# EUROPEAN PATENT APPLICATION

(11) **EP 4 847 524 A2**
(43) Date of publication of application: **02.12.2026**
(21) Application number: 26211666.8
(22) Date of filing: 12.12.2024
(51) Int. Cl.: C07K 14/575

(54) **FATTY ACID CONJUGATED CYCLIC PEPTIDES**

(30) Priority: 15.12.2023 US 202363610462 P; 23.09.2024 US 202463697944 P
(62) Document number(s) of the earlier application(s) in accordance with Art. 76 EPC: 24838120.4 / 4 801 530
(71) Applicant: NEUROCRINE BIOSCIENCES, INC., San Diego, CA 92130 (US)
(72) Inventor: MEZO, Adam, R., San Diego 92130 (US); BEHAN, Dominic, P., San Diego 92131 (US); ERCHEGYI, Judit, San Diego 92130 (US); MILLER, Charleen, L., Escondido 92025 (US); RABINOVICH, Aleksandr, La Jolla 92037 (US); SUZARA, Vincent, La Jolla 92037 (US)
(74) Representative: Carpmaels & Ransford LLP

(57) **Abstract**

This disclosure provides fatty acid conjugated cyclic peptides, pharmaceutical compositions comprising such fatty acid conjugated cyclic peptides and methods of using such fatty acid conjugated cyclic peptides to treat or prevent diseases associated with the abnormal or dysregulated activity of corticotropin releasing factor (CRF) signaling.

## Description

### RELATED APPLICATION

This application claims priority to U.S. Provisional Application No. 63/610,462, filed on December 15, 2023, and U.S. Provisional Application No. 63/697,944, filed on September 23, 2024, the entirety of each of which is incorporated herein by reference.

### SEQUENCE LISTING

This application contains a computer readable Sequence Listing which has been submitted in XML file format with this application, the entire content of which is incorporated by reference herein in its entirety. The Sequence Listing XML file submitted with this application is entitled "14794-018-228_SEQ_LISTING.xml", was created on December 7, 2024, and is 587,519 bytes in size.

### FIELD

This disclosure provides corticotropin releasing factor (CRF) receptor antagonist cyclic peptides, pharmaceutical compositions comprising such cyclic peptides, and methods of using such peptides to treat and/or prevent diseases associated with the abnormal or dysregulated activity of corticotropin releasing factor (CRF) signaling.

### BACKGROUND

In response to stress, either physiological or psychological, corticotropin-releasing factor (CRF) is released into the pituitary portal system via the hypothalamus. CRF results in the release into systemic circulation of adrenocorticotropin hormone (ACTH) which, in turn, stimulates glucocorticoid secretion. Perturbations in the regulatory loops comprising the hypothalamic pituitary axis (HPA) often produce chronically elevated levels of circulating glucocorticoids and HPA dysregulation has a detrimental effect on almost every organ system.

Hyperactivity of the HPA predisposes patients to a variety of illnesses, including, but not limited to, anxiety, depression, Alzheimer's and Parkinson's diseases, obesity, metabolic syndrome, osteoporosis, cardiovascular disease, alcohol and drug abuse, inflammatory bowel disease (IBD) and irritable bowel syndrome (IBS). In addition, prolonged exposure to elevated glucocorticoids can harm the central nervous system, causing hippocampal and prefrontal cortex functional impairments.

Effective HPA modulation and treatment of stress-related conditions mediated by the CRF receptors remains an unmet medical need.

### SUMMARY

The present disclosure provides corticotropin releasing factor receptor antagonist fatty acid conjugated cyclic peptides, or pharmaceutically acceptable salts thereof, pharmaceutical compositions comprising such cyclic peptides or pharmaceutically acceptable salts thereof, and methods of using such peptides or pharmaceutically acceptable salts thereof to treat or prevent diseases associated with the abnormal or dysregulated activity of corticotropin releasing factor (CRF) signaling.

In one aspect, the present disclosure provides a corticotropin releasing factor receptor antagonist fatty acid conjugated cyclic peptide or a pharmaceutically acceptable salt thereof, comprising the following amino acid sequence (SEQ ID NO: 117): 
Y-Asp-R2-R3-R4-R5-R6-R7-Arg-R9-R10-R11-R12-R13-R14-R15-R16-R17-R18-R19-
R20-R21-R22-R23-R24-R25-R26-R27-R28-R29-R30-R31-R32-R33-CONH2
 wherein:
**R2** is Leu or N-Me-Leu;
**R3** is Thr, Ser, or D-Phe;
**R4** is D-Phe, Phe, Leu, Trp, or His;
**R5** is His, Glu, or CML (C^{α}-Me-Leu);
**R6** is Leu, or CML (C^{α}-Me-Leu);
**R7** is Leu, CML (C^{α}-Me-Leu), or N-Me-Leu;
**R9** is Glu, Thr, Nle, CML (C^{α}-Me-Leu), Ala, or **K*;**
**R10** is Val, Met, Leu, Nle, Asp, or Asn;
**R11** is Leu or Ile;
**R12** is Glu, Aib, Ala, or **K*;**
**R13** is Nle, Ile, Leu, Met, Asn, Asp, or **K*;**
**R14** is **K*,** Glu, Ala, Thr, Aib, Na-Me-Ala, or Gln;
**R15** is Asn, Thr, Arg, **K*,** Gln, Glu, Ala, Aad, Gla, pcF, K&, K%, K^, E&, E%, or Lys;
**R16** is Arg, Glu, Gln, Thr, Ala, Aib, or **K*;**
**R17** is Glu, Gln, Nle, Aib, Ala, D-Ala, Asp, or **K*;**
**R18** is Gln, Ser, Ala, or **K*;**
**R19** is CML (C^{α}-Me-Leu), cVal, Glu, Gln, Leu, Aib, N-Me-Leu; D-Ala, CMV(C^{α}-Me-Val), CMP (C^{α}-Me-Phe), cLeu, or **K*;**
**R20** is Ala, Arg, Glu, Aib, or **K*;**
**R21** is Gln, Glu, Aib, Ala, or **K*;**
**R22** is E# or K#;
**R23** is Ala, Leu, Glu, Gln, Aib, D-Ala, Aph(Hor), Nle, or Lys;
**R24** is Glu, Deg, Ala, D-Ala, His, Gln, Lys, Aib, Na-Me-Ala, CML (C^{α}-Me-Leu), N-Me-D-Ala, D-Aph(Cbm), D-CML (C^{α}-Me-D-Leu), or **K*;**
**R25** is K# or E#;
**R26** is Asn, Glu, Lys, K(Ac), Ala, or Aib;
**R27** is Arg, Glu, or **K*;**
**R28** is Lys, Leu, Ile, Glu, K(Ac), Ala, Arg, or K(iPr);
**R29** is Leu, Ile, Ala, Glu, K(Ac), CML (C^{α}-Me-Leu), N-Me-Leu, or **K*;**
**R30** is Nle, Leu, Phe, Met, K(Ac), Asp, Asn, Glu, 1Nal, 2Nal, or Cha;
**R31** is Glu, Lys, CML (C^{α}-Me-Leu), Aib, Ala, or Asp;
**R32** is cVal, CML (C^{α}-Me-Leu), Thr, Ser, Glu, Lys, N6-Me-Lys, Aib, D-Ala,
CMV(C^{α}-Me-Val), CMP(C^{α}-Me-Phe), β-Ala(2-thienyl), β-Ala(3-thienyl), or Ile;
**R33** is Ala, Val, Lys, N-Me-Ala, or Ile; and
Y is Ac or X*;
X* represents ((2-[2-(2-amino-ethoxy)-ethoxy]-acetyl))ₙ-(γGlu)ₘ-diacid, wherein: n is 1, 2 or 3; m is 0, 1, 2, or 3; and wherein the diacid is a C16, C18, C20, or C22 diacid, and wherein the linkage to the 2-[2-(2-amino-ethoxy)-ethoxy]-acetyl ((AEEA)) amine is via the nitrogen of the N-terminus;
**K*** represents Lys-(Z)₀₋₁((2-[2-(2-amino-ethoxy)-ethoxy]-acetyl))ₙ-(γGlu)ₘ-diacid or
   Lys-(PEG12)-(γGlu)₁₋₂-diacid, wherein: Z is Glu, γGlu, Lys, Ala, Gln or His; n is 1, 2 or 3; m is 0, 1, 2, or 3; and wherein the diacid is a C16, C18, C20, or C22 diacid, and wherein the linkage to the 2-[2-(2-amino-ethoxy)-ethoxy]-acetyl ((AEEA)) amine is via the gamma carboxylate of the γGlu residue;
with the proviso that only one amino acid in the Formula I sequence is **K*;** and with the proviso that if K* is present, Y is Ac, and if Y is X*, K* is not present; and with the further proviso that when **R22** is E#, **R25** is K#, and when **R22** is K#, **R25** is
   E#; wherein E# and K# represent a lactam bridge between the side chains of these two amino acids (E=Glu; K=Lys).

In a further aspect, the present disclosure provides a peptide comprising or consisting of Formula (I), or a pharmaceutically acceptable salt thereof, comprising the following amino acid sequence (SEQ ID. NO 107): 
Y-Asp-R2-R3-R4-R5-R6-R7-Arg-R9-R10-R11-R12-R13-R14-R15-R16-R17-R18-
R19-R20-R21-R22-R23-R24-R25-R26-R27-R28-R29-R30-R31-R32-R33-CONH2
(I)
 wherein:
R2 is Leu or N-Me-Leu;
R3 is Thr, Ser, or D-Phe;
R4 is D-Phe, Phe, Leu, Trp, or His;
R5 is His, Glu, or CML (C^{α}-Me-Leu);
R6 is Leu, or CML (C^{α}-Me-Leu);
R7 is Leu, CML (C^{α}-Me-Leu), or N-Me-Leu;
R9 is Glu, Thr, Nle, CML (C^{α}-Me-Leu), Ala, or K*;
R10 is Val, Met, Leu, Nle, Asp, or Asn;
R11 is Leu or Ile;
R12 is Glu, Aib, Ala, or K*;
R13 is Nle, Ile, Leu, Met, Asn, Asp, or K*;
R14 is K*, Glu, Ala, Thr, Aib, N-Me-Ala, or Gln;
R15 is Arg, K*, Gln, Glu, Ala, Aad, Gla, pcF, K&, K%, K^, E&, E%, or Lys;
R16 is Glu, Gln, Thr, Ala, Aib, or K*;
R17 is Glu, Gln, Nle, Aib, Ala, D-Ala, Asp, or K*;
R18 is Gln, Ser, Ala, or K*;
R19 is cVal, cLeu, Glu, Gln, Leu, Aib, CML (C^{α}-Me-Leu), N-Me-Leu, D-Ala, Me-Phe,
CMV(C^{α}-Me-Val), CMP(C^{α}-Me-Phe), or K*;
R20 is Ala, Arg, Glu, Aib, or K*;
R21 is Gln, Glu, Aib, Ala, or K*;
R22 is E# or K#;
R23 is Ala, Leu, Glu, Gln, Aib, D-Ala, Aph(Hor), Nle, or Lys;
R24 is Glu, Deg, Ala, D-Ala, His, Gln, Lys, Aib, N-Me-Ala, CML (C^{α}-Me-Leu), N-Me-D-Ala, D-Aph(Cbm), D-CML (D-C^{α}-Me-Leu), or K*;
R25 is K# or E#;
R26 is Asn, Glu, Lys, K(Ac), Ala, or Aib;
R27 is Arg, Glu, or K*;
R28 is Lys, Leu, Ile, Glu, K(Ac), Ala, Arg, or K(iPr);
R29 is Leu, Ile, Ala, Glu, K(Ac), CML (C^{α}-Me-Leu), N-Me-Leu, or K*;
R30 is Nle, Leu, Phe, Met, K(Ac), Asp, Asn, Glu, 1Nal, 2Nal, or Cha;
R31 is Glu, Lys, CML (C^{α}-Me-Leu), Aib, Ala, or Asp;
R32 is cVal, cLeu, Thr, Ser, Glu, Lys, N6-Me-Lys, Aib, D-Ala, CML (C^{α}-Me-Leu),
CMV(C^{α}-Me-Val), CMP(C^{α}-Me-Phe), β-Ala(2-thienyl), β-Ala(3-thienyl), Phe(Me), or Ile;
R33 is Ala, Val, Lys, N-Me-Ala, or Ile; and
Y is Ac,
K* is Lys-(2-[2-(2-amino-ethoxy)-ethoxy]-acetyl)n-(γGlu)m-diacid, wherein the diacid is a C16, C18, or C20 diacid, and wherein n = 1, 2 or 3 and m is 1, 2, or 3; and wherein the linkage to the 2-[2-(2-amino-ethoxy)-ethoxy]-acetyl amine is via the gamma carboxylate of the γGlu residue; with the proviso that only one amino acid in the sequence is K*; and with the further proviso that when R22 is E#, then R25 is K#, and when R22 is K#, then R25 is E#, wherein E# and K# represent a lactam bridge between the side chains of these two amino acids.

In some embodiments, the present disclosure provides a corticotropin releasing factor receptor antagonist fatty acid conjugated cyclic peptide, or a pharmaceutically acceptable salt thereof, of any one of formulae (Ia), (Ib), (Ic), (Id), (Ie), (If), (Ig), (Ih), or (Ik).

In some embodiments, the present disclosure provides a corticotropin releasing factor receptor antagonist fatty acid conjugated cyclic peptide, or a pharmaceutically acceptable salt thereof, of any one of the peptides listed in Tables B1.1 and B1.2.

In some embodiments, the peptide comprises or is SEQ ID NO: 50: Ac-Asp-Leu-Thr-D-Phe-His-Leu-Leu-Arg-Glu-Val-Leu-Glu-Nle-**K***-Gln-Glu-Glu-Gln-cVal-Ala-Gln-E#-Ala-Glu-K#-Asn-Arg-Lys-Leu-Nle-Glu-cVal-Ile-CONH₂; wherein **K*** represents Lys-(2-[2-(2-amino-ethoxy)-ethoxy]-acetyl)₂-γGlu-diacid, wherein the diacid is a C18 diacid, and wherein E# and K# represent a lactam bridge between the side chains of these two amino acids (E=Glu; K=Lys). In some embodiments, the present disclosure provides pharmaceutically acceptable salts of a peptide comprising or consisting of SEQ ID NO: 50.

In some embodiments, the peptide comprises or is SEQ ID NO: 95: Ac-Asp-Leu-Thr-D-Phe-His-Leu-Leu-Arg-Glu-Val-Leu-Glu-Nle-**K***-Gln-Glu-Glu-Gln-cVal-Ala-Gln-E#-Ala-Glu-K#-Asn-Arg-Lys-Leu-Nle-Glu-cVal-Ala-CONH₂; wherein **K*** represents Lys-(2-[2-(2-amino-ethoxy)-ethoxy]-acetyl)₂-γGlu-diacid, wherein the diacid is a C18 diacid, and wherein E# and K# represent a lactam bridge between the side chains of these two amino acids (E=Glu; K=Lys). In some embodiments, the present disclosure provides pharmaceutically acceptable salts of a peptide comprising or consisting of SEQ ID NO: 95.

In some embodiments, the peptide comprises or is SEQ ID NO: 97: Ac-Asp-Leu-Thr-D-Phe-His-Leu-Leu-Arg-Glu-Val-Leu-Glu-Nle-**K***-Gln-Glu-Glu-Gln-cVal-Ala-Gln-E#-Ala-Glu-K#-Asn-Arg-Lys-Leu-Nle-Glu-cVal-Ala-CONH₂; wherein **K*** represents Lys-(2-[2-(2-amino-ethoxy)-ethoxy]-acetyl)₃-γGlu-diacid, wherein the diacid is a C18 diacid, and wherein E# and K# represent a lactam bridge between the side chains of these two amino acids (E=Glu; K=Lys). In some embodiments, the present disclosure provides pharmaceutically acceptable salts of a peptide comprising or consisting of SEQ ID NO: 97.

In some embodiments, the peptide comprises or is SEQ ID NO: 101: Ac-Asp-Leu-Thr-D-Phe-His-Leu-Leu-Arg-Glu-Val-Leu-Glu-Nle-**K***-Arg-Glu-Glu-Gln-cVal-Ala-Gln-E#-Ala-Glu-K#-Asn-Arg-Lys-Leu-Nle-Glu-cVal-Ala-CONH₂; wherein **K*** represents Lys-(2-[2-(2-amino-ethoxy)-ethoxy]-acetyl)₂-γGlu-diacid, wherein the diacid is a C20 diacid, and wherein E# and K# represent a lactam bridge between the side chains of these two amino acids (E=Glu; K=Lys). In some embodiments, the present disclosure provides pharmaceutically acceptable salts of a peptide comprising or consisting of SEQ ID NO: 101.

In another aspect, the present disclosure provides a pharmaceutical composition which contains a peptide comprising or consisting of SEQ ID NO: 117, or of Formula (I), or pharmaceutically acceptable salts thereof, in admixture with one or more suitable excipients.

In some embodiments, the present disclosure provides a pharmaceutical composition which contains a peptide comprising or consisting of any one of formulae (Ia), (Ib), (Ic), (Id), (Ie), (If), (Ig), (Ih), or (Ik), or a pharmaceutically acceptable salt thereof, in admixture with one or more suitable excipients.

In some embodiments, the present disclosure provides a pharmaceutical composition which comprises or contains a peptide comprising or consisting of any one of the peptides listed in Tables B1.1 and B1.2, or a pharmaceutically acceptable salt thereof, in admixture with one or more suitable excipients.

In some embodiments, the present disclosure provides a pharmaceutical composition which comprises or contains the peptide comprising or consisting of SEQ ID NO: 50: Ac-Asp-Leu-Thr-D-Phe-His-Leu-Leu-Arg-Glu-Val-Leu-Glu-Nle-**K***-Gln-Glu-Glu-Gln-cVal-Ala-Gln-E#-Ala-Glu-K#-Asn-Arg-Lys-Leu-Nle-Glu-cVal-Ile-CONH₂; wherein **K*** represents Lys-(2-[2-(2-amino-ethoxy)-ethoxy]-acetyl)₂-γGlu-diacid, wherein the diacid is a C18 diacid, and wherein E# and K# represent a lactam bridge between the side chains of these two amino acids (E=Glu; K=Lys), or a pharmaceutically acceptable salt thereof, in admixture with one or more suitable excipients.

In some embodiments, the present disclosure provides a pharmaceutical composition which comprises or contains the peptide comprising or consisting of SEQ ID NO: 95: Ac-Asp-Leu-Thr-D-Phe-His-Leu-Leu-Arg-Glu-Val-Leu-Glu-Nle-**K***-Gln-Glu-Glu-Gln-cVal-Ala-Gln-E#-Ala-Glu-K#-Asn-Arg-Lys-Leu-Nle-Glu-cVal-Ala-CONH₂; wherein **K*** represents Lys-(2-[2-(2-amino-ethoxy)-ethoxy]-acetyl)₂-γGlu-diacid, wherein the diacid is a C18 diacid, and wherein E# and K# represent a lactam bridge between the side chains of these two amino acids (E=Glu; K=Lys), or a pharmaceutically acceptable salt thereof, in admixture with one or more suitable excipients.

In some embodiments, the present disclosure provides a pharmaceutical composition which comprises or contains the peptide comprising or consisting of SEQ ID NO: 97: Ac-Asp-Leu-Thr-D-Phe-His-Leu-Leu-Arg-Glu-Val-Leu-Glu-Nle-**K***-Gln-Glu-Glu-Gln-cVal-Ala-Gln-E#-Ala-Glu-K#-Asn-Arg-Lys-Leu-Nle-Glu-cVal-Ala-CONH₂; wherein **K*** represents Lys-(2-[2-(2-amino-ethoxy)-ethoxy]-acetyl)₃-γGlu-diacid, wherein the diacid is a C18 diacid, and wherein E# and K# represent a lactam bridge between the side chains of these two amino acids (E=Glu; K=Lys), or a pharmaceutically acceptable salt thereof, in admixture with one or more suitable excipients.

In some embodiments, the present disclosure provides a pharmaceutical composition which comprises or contains the peptide comprising or consisting of SEQ ID NO: 101: Ac-Asp-Leu-Thr-D-Phe-His-Leu-Leu-Arg-Glu-Val-Leu-Glu-Nle-**K***-Arg-Glu-Glu-Gln-cVal-Ala-Gln-E#-Ala-Glu-K#-Asn-Arg-Lys-Leu-Nle-Glu-cVal-Ala-CONH₂; wherein **K*** represents Lys-(2-[2-(2-amino-ethoxy)-ethoxy]-acetyl)₂-γGlu-diacid, wherein the diacid is a C20 diacid, and wherein E# and K# represent a lactam bridge between the side chains of these two amino acids (E=Glu; K=Lys), or a pharmaceutically acceptable salt thereof, in admixture with one or more suitable excipients.

In another aspect, the present disclosure provides a method of treating a disease in a patient by modulating corticotropin releasing factor (CRF) signaling to prevent, inhibit, or ameliorate the pathology and/or symptoms of a disease, which method comprises administering to a patient a therapeutically effective amount of a peptide comprising or consisting of SEQ ID NO: 117, or of Formula (I), or a pharmaceutically acceptable salt thereof.

In some embodiments, the present disclosure provides a method of treating a disease in a patient by modulating corticotropin releasing factor (CRF) signaling to prevent, inhibit, or ameliorate the pathology and/or symptoms of a disease, which method comprises administering to a patient a therapeutically effective amount of a peptide comprising or consisting of any one of Formulae (Ia), (Ib), (Ic), (Id), (Ie), (If), (Ig), (Ih), or (Ik), or a pharmaceutically acceptable salt thereof.

In some embodiments, the present disclosure provides a method of treating a disease in a patient by modulating corticotropin releasing factor (CRF) signaling to prevent, inhibit, or ameliorate the pathology and/or symptoms of a disease, which method comprises administering to a patient a therapeutically effective amount of a peptide of any one of the peptides listed in Tables B1.1 and B1.2, or a pharmaceutically acceptable salt thereof.

In some embodiments, the present disclosure provides a method of treating a disease in a patient by modulating corticotropin releasing factor (CRF) signaling to prevent, inhibit, or ameliorate the pathology and/or symptoms of a disease, which method comprises administering to a patient a therapeutically effective amount of the peptide comprising or consisting of SEQ ID NO: 50: Ac-Asp-Leu-Thr-D-Phe-His-Leu-Leu-Arg-Glu-Val-Leu-Glu-Nle-**K***-Gln-Glu-Glu-Gln-cVal-Ala-Gln-E#-Ala-Glu-K#-Asn-Arg-Lys-Leu-Nle-Glu-cVal-Ile-CONH₂; wherein **K*** represents Lys-(2-[2-(2-amino-ethoxy)-ethoxy]-acetyl)₂-γGlu-diacid, wherein the diacid is a C18 diacid, and wherein E# and K# represent a lactam bridge between the side chains of these two amino acids (E=Glu; K=Lys). In some embodiments, the method comprises administering a pharmaceutically acceptable salt thereof.

In some embodiments, the present disclosure provides a method of treating a disease in a patient by modulating corticotropin releasing factor (CRF) signaling to prevent, inhibit, or ameliorate the pathology and/or symptoms of a disease, which method comprises administering to a patient a therapeutically effective amount of the peptide comprising or consisting of SEQ ID NO: 95: Ac-Asp-Leu-Thr-D-Phe-His-Leu-Leu-Arg-Glu-Val-Leu-Glu-Nle-**K***-Gln-Glu-Glu-Gln-cVal-Ala-Gln-E#-Ala-Glu-K#-Asn-Arg-Lys-Leu-Nle-Glu-cVal-Ala-CONH₂; wherein **K*** represents Lys-(2-[2-(2-amino-ethoxy)-ethoxy]-acetyl)₂-γGlu-diacid, wherein the diacid is a C18 diacid, and wherein E# and K# represent a lactam bridge between the side chains of these two amino acids (E=Glu; K=Lys). In some embodiments, the method comprises administering a pharmaceutically acceptable salt thereof.

In some embodiments, the present disclosure provides a method of treating a disease in a patient by modulating corticotropin releasing factor (CRF) signaling to prevent, inhibit, or ameliorate the pathology and/or symptoms of a disease, which method comprises administering to a patient a therapeutically effective amount of the peptide comprising or consisting of SEQ ID NO: 97: Ac-Asp-Leu-Thr-D-Phe-His-Leu-Leu-Arg-Glu-Val-Leu-Glu-Nle-**K***-Gln-Glu-Glu-Gln-cVal-Ala-Gln-E#-Ala-Glu-K#-Asn-Arg-Lys-Leu-Nle-Glu-cVal-Ala-CONH₂; wherein **K*** represents Lys-(2-[2-(2-amino-ethoxy)-ethoxy]-acetyl)₃-γGlu-diacid, wherein the diacid is a C18 diacid, and wherein E# and K# represent a lactam bridge between the side chains of these two amino acids (E=Glu; K=Lys). In some embodiments, the method comprises administering a pharmaceutically acceptable salt thereof.

In some embodiments, the present disclosure provides a method of treating a disease in a patient by modulating corticotropin releasing factor (CRF) signaling to prevent, inhibit, or ameliorate the pathology and/or symptoms of a disease, which method comprises administering to a patient a therapeutically effective amount of the peptide comprising or consisting of SEQ ID NO: 101: Ac-Asp-Leu-Thr-D-Phe-His-Leu-Leu-Arg-Glu-Val-Leu-Glu-Nle-**K***-Arg-Glu-Glu-Gln-cVal-Ala-Gln-E#-Ala-Glu-K#-Asn-Arg-Lys-Leu-Nle-Glu-cVal-Ala-CONH₂; wherein **K*** represents Lys-(2-[2-(2-amino-ethoxy)-ethoxy]-acetyl)₂-γGlu-diacid, wherein the diacid is a C20 diacid, and wherein E# and K# represent a lactam bridge between the side chains of these two amino acids (E=Glu; K=Lys). In some embodiments, the method comprises administering a pharmaceutically acceptable salt thereof.

In another aspect, the present disclosure provides the use of a peptide comprising or consisting of Formula (I), or a pharmaceutically acceptable salt thereof, in the manufacture of a medicament for treating a disease in a patient by modulating corticotropin releasing factor (CRF) signaling to prevent, inhibit, or ameliorate pathology and/or symptoms of a disease. In some embodiments, the present disclosure provides a peptide comprising or consisting of SEQ ID NO: 117, or of Formula (I), or pharmaceutically acceptable salts thereof for use in therapy. In some embodiments, the present disclosure provides a peptide comprising or consisting of SEQ ID NO: 117, or of Formula (I), or pharmaceutically acceptable salts thereof for use in the treatment of a disease in a patient by modulating corticotropin releasing factor (CRF) signaling to prevent, inhibit, or ameliorate the pathology and/or symptoms of a disease.

In some embodiments, the present disclosure provides the use of a peptide comprising or consisting of any one of formulae (Ia), (Ib), (Ic), (Id), (Ie), (If), (Ig), (Ih), or (Ik), or a pharmaceutically acceptable salt thereof, in the manufacture of a medicament for treating a disease in a patient by modulating corticotropin releasing factor (CRF) signaling to prevent, inhibit, or ameliorate pathology and/or symptoms of a disease.

In some embodiments, the present disclosure provides the use of a peptide comprising or consisting of any one of the peptides listed in Tables B1.1 and B1.2, or a pharmaceutically acceptable salt thereof, in the manufacture of a medicament for treating a disease in a patient by modulating corticotropin releasing factor (CRF) signaling to prevent, inhibit, or ameliorate pathology and/or symptoms of a disease.

In some embodiments, the present disclosure provides the use of the peptide comprising or consisting of SEQ ID NO: 50: Ac-Asp-Leu-Thr-D-Phe-His-Leu-Leu-Arg-Glu-Val-Leu-Glu-Nle-**K***-Gln-Glu-Glu-Gln-cVal-Ala-Gln-E#-Ala-Glu-K#-Asn-Arg-Lys-Leu-Nle-Glu-cVal-Ile-CONH₂; wherein **K*** represents Lys-(2-[2-(2-amino-ethoxy)-ethoxy]-acetyl)₂-γGlu-diacid, wherein the diacid is a C18 diacid, and wherein E# and K# represent a lactam bridge between the side chains of these two amino acids (E=Glu; K=Lys), in the manufacture of a medicament for treating a disease in a patient by modulating corticotropin releasing factor (CRF) signaling to prevent, inhibit, or ameliorate pathology and/or symptoms of a disease. In some embodiments, the peptide comprises or consists of a pharmaceutically acceptable salt of SEQ ID NO: 50.

In some embodiments, the present disclosure provides the use of the peptide comprising or consisting of SEQ ID NO: 95: Ac-Asp-Leu-Thr-D-Phe-His-Leu-Leu-Arg-Glu-Val-Leu-Glu-Nle-**K***-Gln-Glu-Glu-Gln-cVal-Ala-Gln-E#-Ala-Glu-K#-Asn-Arg-Lys-Leu-Nle-Glu-cVal-Ala-CONH₂; wherein **K*** represents Lys-(2-[2-(2-amino-ethoxy)-ethoxy]-acetyl)₂-γGlu-diacid, wherein the diacid is a C18 diacid, and wherein E# and K# represent a lactam bridge between the side chains of these two amino acids (E=Glu; K=Lys), in the manufacture of a medicament for treating a disease in a patient by modulating corticotropin releasing factor (CRF) signaling to prevent, inhibit, or ameliorate pathology and/or symptoms of a disease. In some embodiments, the peptide comprises or consists of a pharmaceutically acceptable salt of SEQ ID NO: 95.

In some embodiments, the present disclosure provides the use of the peptide comprising or consisting of SEQ ID NO: 97: Ac-Asp-Leu-Thr-D-Phe-His-Leu-Leu-Arg-Glu-Val-Leu-Glu-Nle-**K***-Gln-Glu-Glu-Gln-cVal-Ala-Gln-E#-Ala-Glu-K#-Asn-Arg-Lys-Leu-Nle-Glu-cVal-Ala-CONH₂; wherein **K*** represents Lys-(2-[2-(2-amino-ethoxy)-ethoxy]-acetyl)₃-γGlu-diacid, wherein the diacid is a C18 diacid, and wherein E# and K# represent a lactam bridge between the side chains of these two amino acids (E=Glu; K=Lys), in the manufacture of a medicament for treating a disease in a patient by modulating corticotropin releasing factor (CRF) signaling to prevent, inhibit, or ameliorate pathology and/or symptoms of a disease. In some embodiments, the peptide comprises or consists of a pharmaceutically acceptable salt of SEQ ID NO: 97.

In some embodiments, the present disclosure provides the use of the peptide comprising or consisting of SEQ ID NO: 101: Ac-Asp-Leu-Thr-D-Phe-His-Leu-Leu-Arg-Glu-Val-Leu-Glu-Nle-**K***-Arg-Glu-Glu-Gln-cVal-Ala-Gln-E#-Ala-Glu-K#-Asn-Arg-Lys-Leu-Nle-Glu-cVal-Ala-CONH₂; wherein **K*** represents Lys-(2-[2-(2-amino-ethoxy)-ethoxy]-acetyl)₂-γGlu-diacid, wherein the diacid is a C20 diacid, and wherein E# and K# represent a lactam bridge between the side chains of these two amino acids (E=Glu; K=Lys), in the manufacture of a medicament for treating a disease in a patient by modulating corticotropin releasing factor (CRF) signaling to prevent, inhibit, or ameliorate pathology and/or symptoms of a disease. In some embodiments, the peptide comprises or consists of a pharmaceutically acceptable salt of SEQ ID NO: 101.

In another aspect, the present disclosure provides a peptide comprising or consisting of SEQ ID NO: 117, or of Formula (I), or pharmaceutically acceptable salts thereof, for use in therapy. In another aspect, the present disclosure provides a peptide comprising or consisting of SEQ ID NO: 117, or of Formula (I), or pharmaceutically acceptable salts thereof, for use in the treatment of a disease in a patient in which modulation of corticotropin releasing factor (CRF) signaling contributes to the pathology and/or symptoms of the disease.

In some embodiments, the present disclosure provides a peptide comprising or consisting of any one of formulae (Ia), (Ib), (Ic), (Id), (Ie), (If), (Ig), (Ih), or (Ik), or a pharmaceutically acceptable salt thereof, for use in therapy. In another aspect, the present disclosure provides a peptide comprising or consisting of any one of formulae (Ia), (Ib), (Ic), (Id), (Ie), (If), (Ig), (Ih), or (Ik), or a pharmaceutically acceptable salt thereof, for use in the treatment of a disease in a patient in which modulation of corticotropin releasing factor (CRF) signaling contributes to the pathology and/or symptoms of the disease.

In some embodiments, the present disclosure provides a peptide comprising or consisting of any one of the peptides listed in Tables B1.1 and B1.2, or a pharmaceutically acceptable salt thereof, for use in therapy. In another aspect, the present disclosure provides a peptide comprising or consisting of any one of the peptides listed in Tables B1.1 and B1.2, or a pharmaceutically acceptable salt thereof, for use in the treatment of a disease in a patient in which modulation of corticotropin releasing factor (CRF) signaling contributes to the pathology and/or symptoms of the disease.

In some embodiments, the present disclosure provides the peptide comprising or consisting of SEQ ID NO: 50: Ac-Asp-Leu-Thr-D-Phe-His-Leu-Leu-Arg-Glu-Val-Leu-Glu-Nle-**K***-Gln-Glu-Glu-Gln-cVal-Ala-Gln-E#-Ala-Glu-K#-Asn-Arg-Lys-Leu-Nle-Glu-cVal-Ile-CONH₂; wherein **K*** represents Lys-(2-[2-(2-amino-ethoxy)-ethoxy]-acetyl)₂-γGlu-diacid, wherein the diacid is a C18 diacid, for use in therapy. In some embodiments, the peptide is a pharmaceutically acceptable salt thereof. In another aspect, the present disclosure provides a peptide comprising or consisting of SEQ ID NO: 50 described above, for use in the treatment of a disease in a patient in which modulation of corticotropin releasing factor (CRF) signaling contributes to the pathology and/or symptoms of the disease. In some embodiments, the peptide is a pharmaceutically acceptable salt thereof.

In some embodiments, the present disclosure provides the peptide comprising or consisting of SEQ ID NO: 95: Ac-Asp-Leu-Thr-D-Phe-His-Leu-Leu-Arg-Glu-Val-Leu-Glu-Nle-**K***-Gln-Glu-Glu-Gln-cVal-Ala-Gln-E#-Ala-Glu-K#-Asn-Arg-Lys-Leu-Nle-Glu-cVal-Ala-CONH₂; wherein **K*** represents Lys-(2-[2-(2-amino-ethoxy)-ethoxy]-acetyl)₂-γGlu-diacid, wherein the diacid is a C18 diacid, for use in therapy. In some embodiments, the peptide is a pharmaceutically acceptable salt thereof. In another aspect, the present disclosure provides a peptide comprising or consisting of SEQ ID NO: 95 described above, for use in the treatment of a disease in a patient in which modulation of corticotropin releasing factor (CRF) signaling contributes to the pathology and/or symptoms of the disease. In some embodiments, the peptide is a pharmaceutically acceptable salt thereof.

In some embodiments, the present disclosure provides the peptide comprising or consisting of SEQ ID NO: 97: Ac-Asp-Leu-Thr-D-Phe-His-Leu-Leu-Arg-Glu-Val-Leu-Glu-Nle-**K***-Gln-Glu-Glu-Gln-cVal-Ala-Gln-E#-Ala-Glu-K#-Asn-Arg-Lys-Leu-Nle-Glu-cVal-Ala-CONH₂; wherein **K*** represents Lys-(2-[2-(2-amino-ethoxy)-ethoxy]-acetyl)₃-γGlu-diacid, wherein the diacid is a C18 diacid, for use in therapy. In some embodiments, the peptide is a pharmaceutically acceptable salt thereof. In another aspect, the present disclosure provides a peptide comprising or consisting of SEQ ID NO: 97 described above, for use in the treatment of a disease in a patient in which modulation of corticotropin releasing factor (CRF) signaling contributes to the pathology and/or symptoms of the disease. In some embodiments, the peptide is a pharmaceutically acceptable salt thereof.

In some embodiments, the present disclosure provides the peptide comprising or consisting of SEQ ID NO: 101: Ac-Asp-Leu-Thr-D-Phe-His-Leu-Leu-Arg-Glu-Val-Leu-Glu-Nle-**K***-Arg-Glu-Glu-Gln-cVal-Ala-Gln-E#-Ala-Glu-K#-Asn-Arg-Lys-Leu-Nle-Glu-cVal-Ala-CONH₂; wherein **K*** represents Lys-(2-[2-(2-amino-ethoxy)-ethoxy]-acetyl)₂-γGlu-diacid, wherein the diacid is a C20 diacid, and wherein E# and K# represent a lactam bridge between the side chains of these two amino acids (E=Glu; K=Lys), for use in therapy. In some embodiments, the peptide is a pharmaceutically acceptable salt thereof. In another aspect, the present disclosure provides a peptide comprising or consisting of SEQ ID NO: 101 described above, for use in the treatment of a disease in a patient in which modulation of corticotropin releasing factor (CRF) signaling contributes to the pathology and/or symptoms of the disease. In some embodiments, the peptide is a pharmaceutically acceptable salt thereof.

In another aspect, the present disclosure provides processes for preparing all the peptides or SEQ ID NOs. described herein, or pharmaceutically acceptable salts thereof.

In some embodiments, the present disclosure provides processes for preparing peptides of any one of formulae (Ia), (Ib), (Ic), (Id), (Ie), (If), (Ig), (Ih), or (Ik), or a pharmaceutically acceptable salt thereof.

In some embodiments, the present disclosure provides processes for preparing a peptide comprising or consisting of any one of the peptides listed in Tables B1.1 and B1.2, or a pharmaceutically acceptable salt thereof.

In some embodiments, the present disclosure provides processes for preparing the peptide comprising or consisting of SEQ ID NO: 50: Ac-Asp-Leu-Thr-D-Phe-His-Leu-Leu-Arg-Glu-Val-Leu-Glu-Nle-**K***-Gln-Glu-Glu-Gln-cVal-Ala-Gln-E#-Ala-Glu-K#-Asn-Arg-Lys-Leu-Nle-Glu-cVal-Ile-CONH₂; wherein **K*** represents Lys-(2-[2-(2-amino-ethoxy)-ethoxy]-acetyl)₂-γGlu-diacid, wherein the diacid is a C18 diacid. In some embodiments, the peptide is a pharmaceutically acceptable salt thereof.

In some embodiments, the present disclosure provides processes for preparing the peptide comprising or consisting of SEQ ID NO: 95: Ac-Asp-Leu-Thr-D-Phe-His-Leu-Leu-Arg-Glu-Val-Leu-Glu-Nle-**K***-Gln-Glu-Glu-Gln-cVal-Ala-Gln-E#-Ala-Glu-K#-Asn-Arg-Lys-Leu-Nle-Glu-cVal-Ala-CONH₂; wherein **K*** represents Lys-(2-[2-(2-amino-ethoxy)-ethoxy]-acetyl)₂-γGlu-diacid, wherein the diacid is a C18 diacid. In some embodiments, the peptide is a pharmaceutically acceptable salt thereof.

In some embodiments, the present disclosure provides processes for preparing the peptide comprising or consisting of SEQ ID NO: 97: Ac-Asp-Leu-Thr-D-Phe-His-Leu-Leu-Arg-Glu-Val-Leu-Glu-Nle-**K***-Gln-Glu-Glu-Gln-cVal-Ala-Gln-E#-Ala-Glu-K#-Asn-Arg-Lys-Leu-Nle-Glu-cVal-Ala-CONH₂; wherein **K*** represents Lys-(2-[2-(2-amino-ethoxy)-ethoxy]-acetyl)₃-γGlu-diacid, wherein the diacid is a C18 diacid. In some embodiments, the peptide is a pharmaceutically acceptable salt thereof.

In some embodiments, the present disclosure provides processes for preparing the peptide comprising or consisting of SEQ ID NO: 101: Ac-Asp-Leu-Thr-D-Phe-His-Leu-Leu-Arg-Glu-Val-Leu-Glu-Nle-**K***-Arg-Glu-Glu-Gln-cVal-Ala-Gln-E#-Ala-Glu-K#-Asn-Arg-Lys-Leu-Nle-Glu-cVal-Ala-CONH₂; wherein **K*** represents Lys-(2-[2-(2-amino-ethoxy)-ethoxy]-acetyl)₂-γGlu-diacid, wherein the diacid is a C20 diacid, and wherein E# and K# represent a lactam bridge between the side chains of these two amino acids (E=Glu; K=Lys). In some embodiments, the peptide is a pharmaceutically acceptable salt thereof.

In another aspect, the present disclosure provides a pre-filled subcutaneous injection device comprising a peptide of the disclosure, or a pharmaceutically acceptable salt thereof, or a pharmaceutical composition which contains a peptide of the disclosure, or a pharmaceutically acceptable salt thereof, in admixture with one or more suitable excipients.

In some embodiments, the present disclosure provides a pre-filled subcutaneous injection device comprising a peptide comprising or consisting of SEQ ID NO: 117, or of Formula (I), or pharmaceutically acceptable salts thereof, or a pharmaceutical composition which contains a peptide comprising or consisting of SEQ ID NO: 117, or of Formula (I), or pharmaceutically acceptable salts thereof, in admixture with one or more suitable excipients.

In some embodiments, the present disclosure provides a pre-filled subcutaneous injection device comprising a peptide comprising or consisting of any one of formulae (Ia), (Ib), (Ic), (Id), (Ie), (If), (Ig), (Ih), or (Ik), or a pharmaceutically acceptable salt thereof, or a pharmaceutical composition which contains a peptide comprising or consisting of any one of formulae (Ia), (Ib), (Ic), (Id), (Ie), (If), (Ig), (Ih), or (Ik), or a pharmaceutically acceptable salt thereof, in admixture with one or more suitable excipients.

In some embodiments, the present disclosure provides a pre-filled subcutaneous injection device comprising a peptide comprising or consisting of any one of the peptides listed in Tables B1.1 and B1.2, or a pharmaceutically acceptable salt thereof, or a pharmaceutical composition which comprises or contains a peptide comprising or consisting of any one of the peptides listed in Tables B1.1 and B1.2, or a pharmaceutically acceptable salt thereof, in admixture with one or more suitable excipients.

In some embodiments, the present disclosure provides a pre-filled subcutaneous injection device comprising the peptide comprising or consists of SEQ ID NO: 50: Ac-Asp-Leu-Thr-D-Phe-His-Leu-Leu-Arg-Glu-Val-Leu-Glu-Nle-**K***-Gln-Glu-Glu-Gln-cVal-Ala-Gln-E#-Ala-Glu-K#-Asn-Arg-Lys-Leu-Nle-Glu-cVal-Ile-CONH₂; wherein **K*** represents Lys-(2-[2-(2-amino-ethoxy)-ethoxy]-acetyl)₂-γGlu-diacid, wherein the diacid is a C18 diacid, or a pharmaceutical composition which comprises or contains a peptide comprising or consists of SEQ ID NO: 50, in admixture with one or more suitable excipients. In some embodiments, the peptide is a pharmaceutically acceptable salt thereof.

In some embodiments, the present disclosure provides a pre-filled subcutaneous injection device comprising the peptide comprising or consists of SEQ ID NO: 95: Ac-Asp-Leu-Thr-D-Phe-His-Leu-Leu-Arg-Glu-Val-Leu-Glu-Nle-**K***-Gln-Glu-Glu-Gln-cVal-Ala-Gln-E#-Ala-Glu-K#-Asn-Arg-Lys-Leu-Nle-Glu-cVal-Ala-CONH₂; wherein **K*** represents Lys-(2-[2-(2-amino-ethoxy)-ethoxy]-acetyl)₂-γGlu-diacid, wherein the diacid is a C18 diacid, or a pharmaceutical composition which contains a peptide comprising or consists of SEQ ID NO: 95, in admixture with one or more suitable excipients. In some embodiments, the peptide is a pharmaceutically acceptable salt thereof.

In some embodiments, the present disclosure provides a pre-filled subcutaneous injection device comprising the peptide comprising or consisting of SEQ ID NO: 97: Ac-Asp-Leu-Thr-D-Phe-His-Leu-Leu-Arg-Glu-Val-Leu-Glu-Nle-**K***-Gln-Glu-Glu-Gln-cVal-Ala-Gln-E#-Ala-Glu-K#-Asn-Arg-Lys-Leu-Nle-Glu-cVal-Ala-CONH₂; wherein **K*** represents Lys-(2-[2-(2-amino-ethoxy)-ethoxy]-acetyl)₃-γGlu-diacid, wherein the diacid is a C18 diacid, or a pharmaceutical composition which contains a peptide comprising or consisting of SEQ ID NO: 97, in admixture with one or more suitable excipients. In some embodiments, the peptide is a pharmaceutically acceptable salt thereof.

In some embodiments, the present disclosure provides a pre-filled subcutaneous injection device comprising the peptide comprising or consisting of SEQ ID NO: 101: Ac-Asp-Leu-Thr-D-Phe-His-Leu-Leu-Arg-Glu-Val-Leu-Glu-Nle-K*-Arg-Glu-Glu-Gln-cVal-Ala-Gln-E#-Ala-Glu-K#-Asn-Arg-Lys-Leu-Nle-Glu-cVal-Ala-CONH₂; wherein **K*** represents Lys-(2-[2-(2-amino-ethoxy)-ethoxy]-acetyl)₂-γGlu-diacid, wherein the diacid is a C20 diacid, and wherein E# and K# represent a lactam bridge between the side chains of these two amino acids (E=Glu; K=Lys), or a pharmaceutical composition which contains a peptide comprising or consisting of SEQ ID NO: 101, in admixture with one or more suitable excipients. In some embodiments, the peptide is a pharmaceutically acceptable salt thereof.

In another aspect, the present disclosure provides processes for preparing peptides comprising or of SEQ ID NO: 117, or of Formula (I), or pharmaceutically acceptable salts thereof.

In some embodiments, the present disclosure provides processes for preparing peptides comprising or of any one of formulae (Ia), (Ib), (Ic), (Id), (Ie), (If), (Ig), (Ih), or (Ik), or a pharmaceutically acceptable salt thereof.

In some embodiments, the present disclosure provides processes for preparing a peptide compri sing or consisting of any one of the peptides listed in Tables B1.1 and B1.2, or a pharmaceutically acceptable salt thereof.

In some embodiments, the present disclosure provides processes for preparing the peptide comprising or consisting of SEQ ID NO: 50: Ac-Asp-Leu-Thr-D-Phe-His-Leu-Leu-Arg-Glu-Val-Leu-Glu-Nle-**K***-Gln-Glu-Glu-Gln-cVal-Ala-Gln-E#-Ala-Glu-K#-Asn-Arg-Lys-Leu-Nle-Glu-cVal-Ile-CONH₂; wherein **K*** represents Lys-(2-[2-(2-amino-ethoxy)-ethoxy]-acetyl)₂-γGlu-diacid, wherein the diacid is a C18 diacid, and wherein E# and K# represent a lactam bridge between the side chains of these two amino acids (E=Glu; K=Lys). In some embodiments, the peptide is a pharmaceutically acceptable salt thereof.

In some embodiments, the present disclosure provides processes for preparing the peptide comprising or consisting of SEQ ID NO: 95: Ac-Asp-Leu-Thr-D-Phe-His-Leu-Leu-Arg-Glu-Val-Leu-Glu-Nle-**K***-Gln-Glu-Glu-Gln-cVal-Ala-Gln-E#-Ala-Glu-K#-Asn-Arg-Lys-Leu-Nle-Glu-cVal-Ala-CONH₂; wherein **K*** represents Lys-(2-[2-(2-amino-ethoxy)-ethoxy]-acetyl)₂-γGlu-diacid, wherein the diacid is a C18 diacid, and wherein E# and K# represent a lactam bridge between the side chains of these two amino acids (E=Glu; K=Lys). In some embodiments, the peptide is a pharmaceutically acceptable salt thereof.

In some embodiments, the present disclosure provides processes for preparing the peptide comprising or consisting of SEQ ID NO: 97: Ac-Asp-Leu-Thr-D-Phe-His-Leu-Leu-Arg-Glu-Val-Leu-Glu-Nle-**K***-Gln-Glu-Glu-Gln-cVal-Ala-Gln-E#-Ala-Glu-K#-Asn-Arg-Lys-Leu-Nle-Glu-cVal-Ala-CONH₂; wherein **K*** represents Lys-(2-[2-(2-amino-ethoxy)-ethoxy]-acetyl)₃-γGlu-diacid, wherein the diacid is a C18 diacid, and wherein E# and K# represent a lactam bridge between the side chains of these two amino acids (E=Glu; K=Lys). In some embodiments, the peptide is a pharmaceutically acceptable salt thereof.

In some embodiments, the present disclosure provides processes for preparing the peptide comprising or consisting of SEQ ID NO: 101: Ac-Asp-Leu-Thr-D-Phe-His-Leu-Leu-Arg-Glu-Val-Leu-Glu-Nle-**K***-Arg-Glu-Glu-Gln-cVal-Ala-Gln-E#-Ala-Glu-K#-Asn-Arg-Lys-Leu-Nle-Glu-cVal-Ala-CONH₂; wherein **K*** represents Lys-(2-[2-(2-amino-ethoxy)-ethoxy]-acetyl)₂-γGlu-diacid, wherein the diacid is a C20 diacid, and wherein E# and K# represent a lactam bridge between the side chains of these two amino acids (E=Glu; K=Lys). In some embodiments, the peptide is a pharmaceutically acceptable salt thereof.

For a better understanding of the present disclosure, together with other and further aspects thereof, reference is made to the accompanying drawings, detailed description, and claims.

### BRIEF DESCRIPTION OF FIGURES

FIG. 1 Structure of peptide of SEQ ID NO: 101, Example A1, where **K*** (or Lys¹⁴) is Lys-(2-[2-(2-amino-ethoxy)-ethoxy]-acetyl)₂-γGlu-C20-diacid and a lactam bridge is present between E# (or Glu²²) and K# (or Lys²⁵).
FIG. 2 Structure of peptide of SEQ ID NO: 95, Example A2, where **K*** (or Lys¹⁴) is Lys-(2-[2-(2-amino-ethoxy)-ethoxy]-acetyl)₂-γGlu-C18-diacid and a lactam bridge is present between E# (or Glu²²) and K# (or Lys²⁵).
FIG. 3 Structure of peptide of SEQ ID NO: 97, Example A3, where **K*** (or Lys¹⁴) is Lys-(2-[2-(2-amino-ethoxy)-ethoxy]-acetyl)₃-γGlu-C18-diacid and a lactam bridge is present between E# (or Glu²²) and K# (or Lys²⁵).
FIG. 4 Structure of peptide of SEQ ID NO: 50, Example A4, where **K*** (or Lys¹⁴) is Lys-(2-[2-(2-amino-ethoxy)-ethoxy]-acetyl)₂-γGlu-C18-diacid and a lactam bridge is present between E# (or Glu²²) and K# (or Lys²⁵).
FIG. 5 Structure of peptide of SEQ ID NO: 79, Example A5, where **K*** (or Lys¹⁴) is Lys-(2-[2-(2-amino-ethoxy)-ethoxy]-acetyl)₂-γGlu-C18-diacid and a lactam bridge is present between E# (or Glu²²) and K# (or Lys²⁵).
FIG. 6 Structure of peptide of SEQ ID NO: 9, Example A7, where **K*** (or Lys¹⁴) is Lys-(2-[2-(2-amino-ethoxy)-ethoxy]-acetyl)₂-γGlu-C18-diacid and a lactam bridge is present between E# (or Glu²²) and K# (or Lys²⁵).
FIG. 7 Structure of peptide of SEQ ID NO: 20 Example A8, where **K*** (or Lys²⁹) is Lys-(2-[2-(2-amino-ethoxy)-ethoxy]-acetyl)₂-γGlu-C18-diacid and a lactam bridge is present between E# (or Glu²²) and K# (or Lys²⁵).
FIG. 8 Structure of peptide of SEQ ID NO: 78, Example A9, where **K*** (or Lys¹⁴) is Lys-(2-[2-(2-amino-ethoxy)-ethoxy]-acetyl)₂-γGlu-C18-diacid and a lactam bridge is present between E# (or Glu²²) and K# (or Lys²⁵).
FIG. 9 Structure of peptide of SEQ ID NO: 99, Example A10, where **K*** (or Lys¹⁴) is Lys-(2-[2-(2-amino-ethoxy)-ethoxy]-acetyl)₃-γGlu-C18-diacid and a lactam bridge is present between E# (or Glu²²) and K# (or Lys²⁵).
FIG. 10 Structure of peptide of SEQ ID NO: 100, Example A11, where **K*** (or Lys¹⁴) is Lys-(2-[2-(2-amino-ethoxy)-ethoxy]-acetyl)₂-γGlu-C20-diacid and a lactam bridge is present between E# (or Glu²²) and K# (or Lys²⁵).
FIG. 11 Structure of peptide of SEQ ID NO: 102, Example A12, where **K*** (or Lys¹⁴) is Lys-(2-[2-(2-amino-ethoxy)-ethoxy]-acetyl)₂-γGlu-C20-diacid and a lactam bridge is present between E# (or Glu²²) and K# (or Lys²⁵).
FIG. 12 shows the percentage adrenocorticotropin hormone (ACTH) reduction at 24, 48, 72, 96, 120, 144, 168, and 192 hours after dosing with the peptides of SEQ ID Nos. 50, 95 and 101.

### DETAILED DESCRIPTION

The fatty acid conjugated CRF receptor antagonist cyclic peptides of the present disclosure have various superior results, such as potent CRFR1 antagonist activity, high physical and chemical stability, high solubility, low aggregation, low viscosity, an increased reduction of ACTH more than about 24 hours (for example more than about 25 hours, about 30 hours, about 35 hours, about 40 hours, about 45 hours, about 50 hours) after peptide dosing, and an extended *in vivo* time of action compared to other CRF receptor antagonists. Their improved solubility allows for a larger dose exposure and larger therapeutic margins, making the peptides beneficial for the management of multiple conditions. In certain embodiments, without being bound by the theory, the fatty acid conjugates extend the in *vivo* time of action of the peptides of the disclosure.

In certain embodiments, without being bound by the theory, the amino-acid-sequence features of the cyclic peptides provided herein lead to superior results in comparison to known reference peptides. Such features include but are not limited to the following features: **R14** being K*, **R15** being Gln or Arg, **R16** being Glu, **R19** being cVal, **R24** being Glu, **R32** being cVal, and **R33** being Ala in the formulae provided herein, such as SEQ ID NO: 117. The superior results include but are not limited to higher potency of CRFR1 antagonist activity, physical and chemical stability, high solubility, low aggregation and low viscosity, an increased reduction of ACTH more than about 24 hours (for example more than about 25 hours, about 30 hours, about 35 hours, about 40 hours, about 45 hours, about 50 hours) after peptide dosing, and an extended *in vivo* time of action.

In certain embodiments, a peptide provided herein has CRFR1 antagonist IC₅₀ activity, as measured using a cyclic AMP (cAMP) assay, of less than about 5 nM.

In a further embodiment, a peptide provided herein has a greater than about 30%, about 35%, about 40%, about 45%, about 50%, about 55%, about 60%, about 65%, about 70%, or about 75% reduction in adrenocorticotropin hormone (ACTH) about 48 hours after peptide dosing in adrenalectomized rats.

In a further embodiment, a peptide provided herein has a greater than about 30% to about 60% reduction in adrenocorticotropin hormone (ACTH) about 48 hours after peptide dosing in adrenalectomized rats.

In a further embodiment, a peptide provided herein has a greater than about 50% to about 75% reduction in adrenocorticotropin hormone (ACTH) about 48 hours after peptide dosing in adrenalectomized rats.

In a further embodiment, a peptide provided herein has a greater than about 35% reduction in adrenocorticotropin hormone (ACTH) about 48 hours after peptide dosing in adrenalectomized rats.

In a further embodiment, a peptide provided herein has a greater than about 60% reduction in adrenocorticotropin hormone (ACTH) about 48 hours after peptide dosing in adrenalectomized rats.

In a further embodiment, a peptide provided herein has a pharmacokinetic half-life from about 30 hours to about 140 hours in cynomolgus monkeys.

In a further embodiment, a peptide provided herein has a pharmacokinetic half-life of greater than about 30 hours, about 40 hours, about 50 hours, about 60 hours, about 70 hours, about 80 hours, about 90 hours, or about 100 hours in cynomolgus monkeys.

In a further embodiment, a peptide provided herein has a pharmacokinetic half-life of greater than about 30 hours in cynomolgus monkeys.

In a further embodiment, a peptide provided herein has a pharmacokinetic half-life of greater than about 100 hours in cynomolgus monkeys.

In a further embodiment, a peptide provided herein has a solubility from about 20mg/mL to about 150 mg/mL in a buffer composition at about neutral pH.

In a further embodiment, a peptide provided herein has a solubility more than or equal to about 100 mg/mL in about 10 mM phosphate buffer at pH of about 7.

In a further embodiment, a peptide provided herein has low or no aggregation as measured using size exclusion chromatography (SEC).

In certain embodiments, a peptide provided herein has the combined superior results of: 1) CRFR1 antagonist IC₅₀ activity as measured using a cyclic AMP (cAMP) assay of less than about 5 nM; 2) more than about 35% reduction in ACTH about 48 hours after peptide dosing in adrenalectomized rats; 3) pharmacokinetic half-life of more than about 100 hours in cynomolgus monkeys; 4) solubility more than or equal to about 100 mg/mL in about 10 mM phosphate buffer at pH of about 7; and 5) low or no aggregation as measured using size exclusion chromatography (SEC).

In certain embodiments, a peptide provided herein has the combined superior results of: 1) CRFR1 antagonist IC₅₀ activity as measured using a cyclic AMP (cAMP) assay of less than about 5 nM; 2) more than about 60% reduction in ACTH about 48 hours after peptide dosing in adrenalectomized rats; 3) pharmacokinetic half-life of more than about 100 hours in cynomolgus monkeys; 4) solubility more than or equal to about 100 mg/mL in about 10 mM phosphate buffer at pH of about 7; and 5) low or no aggregation as measured using size exclusion chromatography (SEC).

In certain embodiments, a peptide provided herein has the superior result of a solubility ≥ about 100 mg/mL in about 10 mM phosphate at pH of about 7, wherein the solubility is measured by visual observation. In certain embodiments, a peptide provided herein has the superior result of a solubility ≥ about 100 mg/mL in about 10 mM TRIS at pH of about 8, wherein the solubility is measured by visual observation. In certain embodiments, the solubility is measured at about 4°C. In certain embodiments, the solubility is measured at about room temperature. In certain embodiments, the solubility is measured at about 37°C. In certain embodiments, a peptide provided herein has the superior result of low or no aggregation as measured using size exclusion chromatography (SEC). In certain embodiments, a peptide provided herein has CRFR1 antagonist activity as measured using a cyclic AMP (cAMP) assay. In certain embodiments, a peptide provided herein has the superior result of CRFR1 antagonist IC₅₀ being about 40 nM to about 98 nM in an assay buffer containing about 0.1% BSA as measured using a cyclic AMP (cAMP) assay. In certain embodiments, a peptide provided herein has CRFR2 antagonist activity as measured using a cyclic AMP (cAMP) assay. In certain embodiments, a peptide provided herein has the superior result of CRFR2 antagonist IC₅₀ being about 80 to about 100 nM in an assay buffer containing about 0.1% BSA as measured using a cAMP assay. In certain embodiments, a peptide provided herein has the superior result of being more selective for CRF1 over CRFR2α as measured using a cyclic AMP (cAMP) assay. In certain embodiments, a peptide provided herein has the superior result of being more selective for CRF1 over CRFR2β as measured using a cyclic AMP (cAMP) assay. In certain embodiments, a peptide provided herein has the superior result of being about 10 times more selective for CRF1 over CRFR2α. In certain embodiments, a peptide provided herein has the superior result of being about 8 times more selective for CRF1 over CRFR2β. In certain embodiments, a peptide provided herein has the superior result of being about 500 times more selective for CRF1 over CRFR2α. In certain embodiments, a peptide provided herein has the superior result of being about 48 times more selective for CRF1 over CRFR2β.

### Definitions

The nomenclature used to define the peptides is that specified by Schroder & Lubke, "The Peptides", Academic Press (1965) wherein, in accordance with conventional representation, the amino group appears to the left and the carboxyl group to the right. The standard 3-letter abbreviations are used to identify the alpha-amino acid residues, and where the amino acid residue has isomeric forms, it is the L-form of the amino acid that is represented unless otherwise expressly indicated, e.g., Ser=L-serine, Orn=L-ornithine, Aad=L-2-aminoadipic acid, and Nle=L-norleucine. In addition, Table A describes the amino acids and corresponding abbreviations used in the present disclosure.

**Table A. Amino Acids and corresponding abbreviations.**

| | |
|---|---|
| Cha | 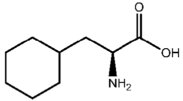 |
| | Cyclohexylalanine |
| cVal | 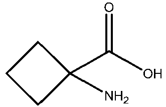 |
| | Cyclovaline or 1-aminocyclobutane-1-carboxylic acid |
| Deg | 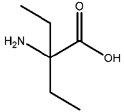 |
| | Diethylglycine, 2-amino-2-ethylbutanoic acid |
| CML (C^{α}-Me-Leu) | 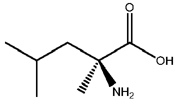 |
| | alpha-methyl-Leucine |
| D-Phe or f | 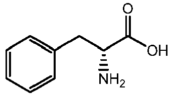 |
| | D-Phenylalanine |
| pcF | 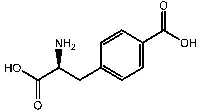 |
| | para-carboxy-Phe |
| K(iPr) | 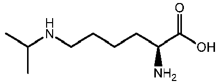 |
| | N-epsilon-isopropyl-lysine |
| K& | 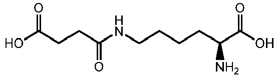 |
| | Lys-(succinic acid) |
| K% | 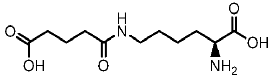 |
| | Lys(1,5-pentane dioic acid) |
| K^ | 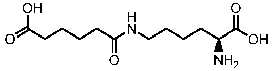 |
| | Lys(adipic acid) |
| E& | 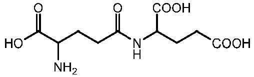 |
| | Glu-(Glu), amide linked |
| E% | 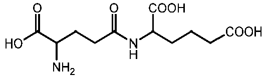 |
| | Glu-(Aad), amide linked (using NH₂-Aad(tBu)-OtBu) |
| E# | Glu with a lactam bridge via side-chain |
| K# | Lys with a lactam bridge via side-chain |

| **K*** | Lysine with a fatty acid conjugate attached at the nitrogen of the side chain of lysine (see examples, below) |
|---|---|
| γGlu | gamma glutamic acid (the linkage, if bound to the (AEEA) amine is via the gamma carboxylate of the Glu residue) |
| N-Me-Leu | 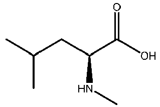 |
| | N-methyl-leucine |
| Nle | 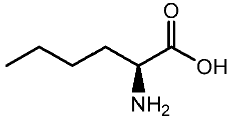 |
| | (S)-2-aminohexanoic acid |
| Aib | 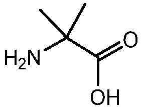 |
| | 2-amino-2-methylpropanoic acid |
| Aad | 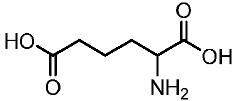 |
| | 2-aminoadipic acid |
| Gla | 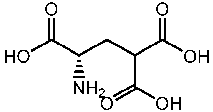 |
| | Gamma-carboxyglutamic acid |
| | (S)-3-aminopropane-1,1,3-tricarboxylic acid |
| D-Ala | 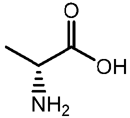 |
| | D-Alanine |
| N-Me-D-Ala | 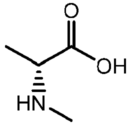 |
| | N-methyl-D-Alanine |
| βAla(2-thienyl) | 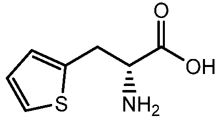 |
| | (R)-2-amino-3-(thiophen-2-yl)propanoic acid |
| βAla(3-thienyl) | 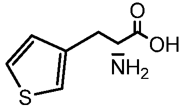 |
| | (R)-2-amino-3-(thiophen-3-yl)propanoic acid |
| CMV | 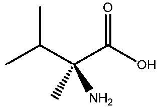 |
| | C^{α}-Methyl-Valine |
| CMP | 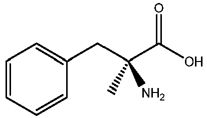 |
| | C^{α}-Methyl-Phenylalanine |
| Aph(Hor) | 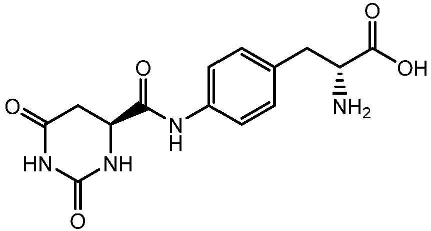 |
| | (R)-2-amino-3-(4-((S)-2,6-dioxohexahydropyrimidine-4-carboxamido)phenyl)propanoic acid |
| N-Me-Ala | 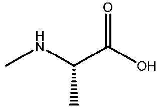 |
| | N-methylalanine |
| D-Aph(Cbm) | 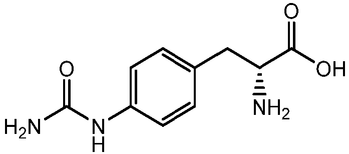 |
| | (R)-2-amino-3-(4-ureidophenyl)propanoic acid |
| Lys(Ac) or K(Ac) | 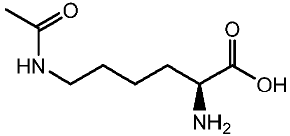 |
| | N⁶-acetyl-L-lysine |
| 1Nal | 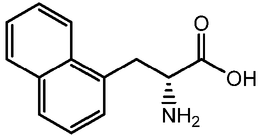 |
| | (R)-2-amino-3-(naphthalen-1-yl)propanoic acid |
| 2Nal | 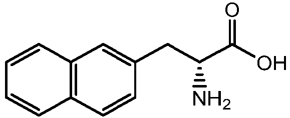 |
| | (R)-2-amino-3-(naphthalen-2-yl)propanoic acid |
| N⁶-Me-Lys | 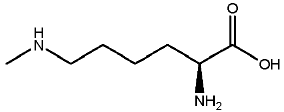 |
| | N⁶-methyl-Lys |
| | (2*S*)-2-amino-6-(methylamino)hexanoic acid |
| PEG12 | 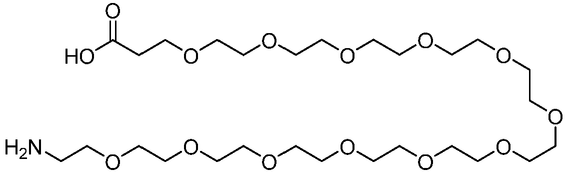 |
| (AEEA) | 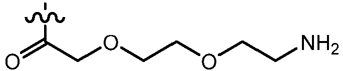 |
| | (2-[2-(2-amino-ethoxy)-ethoxy]-acetyl) |

"CRF Receptor" means corticotropin-releasing hormone receptors or corticotropin-releasing factor receptors (CRFRs) belonging to the superfamily of G-protein-coupled receptors. Two CRFR subtypes have been identified, CRFR1 and CRFR2. CRF1 receptors are widely distributed throughout neocortical, limbic and brain-stem regions of the central nervous system while CRFR2 receptors are limited to discrete brain regions. CRFR2 has three major functional isoforms, alpha, beta, and gamma. In humans, CRFR2-beta is primarily expressed in the brain, while CRFR2-alpha is more broadly expressed and found in the brain and in additional peripheral tissues.

The carbonyl group of "-CONH₂" as recited in the Formulae and amino acid sequences provided herein is the carbonyl group of the respective C-terminal amino acid residue. The structures shown in the figures provide examples showing that the carbonyl group of "-CONH₂" is the carbonyl group of the C-terminal amino acid residue.

### Embodiments

The present disclosure relates to corticotropin releasing factor receptor antagonist fatty acid conjugated cyclic peptides of SEQ ID NO: 117, or of Formula (I), or pharmaceutically acceptable salts thereof. The peptides of SEQ ID NO: 117, or of Formula I, or pharmaceutically acceptable salts thereof, are capable of modulating the activity of corticotropin releasing factor (CRF) signaling to prevent, inhibit, or ameliorate the pathology and/or symptoms of a disease associated with aberrant corticotropin releasing factor (CRF) signaling.

In one aspect, with respect to peptides of Formula (I), are peptides of Formula (Ia), or pharmaceutically acceptable salts thereof (SEQ ID NO: 108): 
Y-Asp-Leu-Thr-D-Phe-His-Leu-Leu-Arg-R9-Val-Leu-R12-R13-R14-R15-R16-R17-R18-
R19-R20-R21-R22-R23-R24-R25-Asn-R27-R28-R29-R30-R31-R32-R33-CONH₂
(Ia)
 wherein: **R9** is Glu or **K*;** R12 is Glu or **K*;** R13 is Nle or **K*;** R14 is Glu, Ala, **K*,** Thr, or Gln; **R15** is Asn, Thr, Arg, **K*,** Gln, Glu, Ala, Aad, Gla, pcF, K&, K%, K^, E&, E%, or Lys; **R16** is Arg, Glu, Ala, Gln, Thr or **K*; R17** is Glu, Asp or **K*; R18** is Gln or **K*; R19** is cVal, CML (C^{α}-Me-Leu), or **K*; R20** is Ala or **K*;** R21 is Gln or **K*; R22** is E# or K#; **R23** is Ala or Lys; **R24** is Deg, Glu, or **K*; R25** is E# or K#; **R27** is Arg or **K*; R28** is Lys or K(iPr); **R29** is Leu or **K*; R30** is Cha or Nle; **R31** is Glu or Asp; **R32** is cVal, CML (C^{α}-Me-Leu), or Ile; **R33** is Ile or Ala; and # represents a lactam bridge between E# and K#; and Y is Ac;
**K*** represents Lys-(2-[2-(2-amino-ethoxy)-ethoxy]-acetyl)ₙ-(γGlu)ₘ-diacid, wherein the diacid is a C16, C18, or C20 diacid, and wherein n = 1, 2 or 3 and m is 1, 2, or 3; with the proviso that only one amino acid in the sequence is **K*;** and with the further proviso that when **R22** is E#, **R25** is K#, and when **R22** is K#, **R25** is E#.

In another aspect, with respect to peptides of Formula (Ia), are peptides of Formula (Ib), or pharmaceutically acceptable salts thereof (SEQ ID NO: 109): 
Ac-Asp-Leu-Thr-D-Phe-His-Leu-Leu-Arg-Glu-Val-Leu-Glu-Nle-R14-R15-R16-R17-Gln-
cVal-Ala-Gln-R22-Ala-Glu-R25-Asn-Arg-Lys-Leu-Nle-Glu-cVal-R33-CONH2
(Ib)
wherein: **R14** is **K*,** Glu, Ala, Thr, or Gln; **R15** is Asn, Thr, Arg, **K*,** Ala, Lys, Glu or Gln; **R16** is Arg, Ala, Glu, Gln, or Thr; **R17** is Glu or Asp; **R22** is E# or K#; **R25** is K# or E#; **R33** is Ala or Ile; and # represents a lactam bridge between E# and K#, and
**K*** represents Lys-(2-[2-(2-amino-ethoxy)-ethoxy]-acetyl)ₙ-(γGlu)ₘ-diacid, wherein the diacid is a C16, C18, or C20 diacid, n is 1, 2 or 3, and m is 1 or 2; with the proviso that one amino acid in the sequence is **K*;** and with the further proviso that when **R22** is E#, then **R25** is K#, and when **R22** is K#, then **R25** is E#.

In another aspect, with respect to peptides of Formula (Ib), are peptides of Formula (Ic), or pharmaceutically acceptable salts thereof (SEQ ID NO: 110): 
Ac-Asp-Leu-Thr-D-Phe-His-Leu-Leu-Arg-Glu-Val-Leu-Glu-Nle-K*-R15-R16-R17-Gln-
cVal-Ala-Gln-R22-Ala-Glu-R25-Asn-Arg-Lys-Leu-Nle-Glu-cVal-R33-CONH2
(Ic)
wherein: **R15** is Asn, Thr, Ala, Glu, Lys, Gln or Arg; **R16** is Arg, Glu, Gln, Ala, or Thr; **R17** is Asp or Glu; **R22** is E# or K#; **R25** is E# or K#; **R33** is Ile or Ala; and # represents a lactam bridge between E# and K#,
**K*** represents Lys-(2-[2-(2-amino-ethoxy)-ethoxy]-acetyl)ₙ-(γGlu)ₘ-diacid, wherein the diacid is a C16, C18, or C20 diacid, n is 1, 2 or 3, and m is 1 or 2; with the further proviso that when **R22** is E#, **R25** is K#, and when **R22** is K#, **R25** is E#.

In some embodiments of Formula (Ic), the diacid is a C18 or C20 diacid.

In some embodiments of Formula (Ic), **R15** is Gln and the diacid of **K*** is a C18 diacid.

In some embodiments of Formula (Ic), **R15** is Gln and the diacid of **K*** is a C20 diacid.

In some embodiments of Formula (Ic), **R15** is Arg and the diacid of **K*** is a C18 diacid.

In some embodiments of Formula (Ic), **R15** is Arg and the diacid of **K*** is a C20 diacid.

In some embodiments of Formula (Ic), **R33** is Ile and the diacid of **K*** is a C18 diacid.

In some embodiments of Formula (Ic), **R33** is Ile and the diacid in **K*** is a C20 diacid.

In some embodiments of Formula (Ic), **R33** is Ala and the diacid in **K*** is a C18 diacid.

In some embodiments of Formula (Ic), **R33** is Ala and the diacid in **K*** is a C20 diacid.

In some embodiments of Formula (Ic), **R15** is Gln, **R33** is Ile, and the diacid in **K*** is a C18 diacid.

In some embodiments of Formula (Ic), **R15** is Gln, **R33** is Ile, and the diacid in **K*** is a C20 diacid.

In some embodiments of Formula (Ic), **R15** is Gln, **R33** is Ala, and the diacid in **K*** is a C18 diacid.

In some embodiments of Formula (Ic), **R15** is Gln, **R33 is** Ala, and the diacid in **K*** is a C20 diacid.

In some embodiments of Formula (Ic), **R15** is Arg, **R33** is Ile, and the diacid in **K*** is a C18 diacid.

In some embodiments of Formula (Ic), **R15** is Arg, **R33** is Ile, and the diacid in **K*** is a C20 diacid.

In some embodiments of Formula (Ic), **R15** is Arg, **R16** is Glu, **R17** is Glu, **R22** is E#, **R25** is K#, **R33** is Ala, n = 2, m = 1, and the diacid in **K*** is a C20 diacid.

In some embodiments of Formula (Ic), **R15** is Gln, **R16** is Glu, **R17** is Glu, **R22** is E#, **R25** is K#, **R33** is Ala, n = 2, m =1, and the diacid of **K*** is a C18 diacid.

In some embodiments of Formula (Ic), **R15** is Gln, **R16** is Glu, **R17** is Glu, **R22** is E#, **R25** is K#, **R33** is Ile, n = 2, m = 1, and the diacid in **K*** is a C18 diacid.

In some embodiments of Formula (Ic), **R15** is Gln, **R16** is Glu, **R17** is Glu, **R22** is E#, **R25** is K#, **R33** is Ala, n = 3, m = 1, and the diacid in **K*** is a C18 diacid.

In another aspect, with respect to peptides of Formula (Ic), are peptides of Formula (Id), or pharmaceutically acceptable salts thereof (SEQ ID NO: 111): 
Ac-Asp-Leu-Thr-D-Phe-His-Leu-Leu-Arg-Glu-Val-Leu-Glu-Nle-K*-R15-R16-Glu-Gln-
cVal-Ala-Gln-R22-Ala-Glu-R25-Asn-Arg-Lys-Leu-Nle-Glu-cVal-R33-CONH2
(Id)
wherein: **R15** is Asn, Thr, Gln or Arg; **R16** is Arg, Ala or Glu; **R22** is E# or K#; **R25** is E# or K#; **R33** is Ile or Ala; and # represents a lactam bridge between E# and K#,
**K*** represents Lys-(2-[2-(2-amino-ethoxy)-ethoxy]-acetyl)ₙ-(γGlu)ₘ-diacid, wherein the diacid is a C18 or C20 diacid, and wherein n = 1, 2 or 3 and m is 1 or 2; with the proviso that when **R22** is E#, then **R25** is K#, and when **R22** is K#, then **R25** is E#.

In some embodiments of Formula (Id), R15 is Gln, **R16** is Glu, **R22** is E#, **R25** is K#, **R33** is Ile, n is 2, m is 1, and the diacid is a C20 diacid.

In some embodiments of Formula (Id), R15 is Gln, **R16** is Glu, **R22** is E#, **R25** is K#, **R33** is Ala, n is 2, m is 1, and the diacid is a C20 diacid.

In some embodiments of Formula (Id), R15 is Gln, **R16** is Glu, **R22** is E#, **R25** is K#, **R33** is Ala, n is 2, m is 1, and the diacid is a C18 diacid.

In some embodiments of Formula (Id), R15 is Gln, **R16** is Glu, **R22** is E#, **R25** is K#, **R33** is Ala, n is 2, m is 2, and the diacid is a C18 diacid.

In some embodiments of Formula (Id), R15 is Gln, **R16** is Glu, **R22** is E#, **R25** is K#, **R33** is Ala, n is 3, m is 1, and the diacid is a C18 diacid.

In some embodiments of Formula (Id), R15 is Gln, **R16** is Ala, **R22** is E#, **R25** is K#, **R33** is Ala, n is 2, m is 1, and the diacid is a C18 diacid.

In some embodiments of Formula (Id), R15 is Gln, **R16** is Glu, **R22** is E#, **R25** is K#, **R33** is Ile, n is 3, m is 1, and the diacid is a C18 diacid.

In some embodiments of Formula (Id), R15 is Arg, **R16** is Glu, **R22** is E#, **R25** is K#, **R33** is Ala, n is 2, m is 1, and the diacid is a C20 diacid.

In some embodiments of Formula (Id), R15 is Arg, **R16** is Glu, **R22** is E#, **R25** is K#, **R33** is Ala, n is 2, m is 1, and the diacid is a C18 diacid.

In some embodiments of Formula (Id), R15 is Gln, **R16** is Glu, **R22** is E#, **R25** is K#, **R33** is Ile, n is 2, m is 2, and the diacid is a C18 diacid.

In another aspect, with respect to peptides of Formula (Ic), are peptides of Formula (Ie), or pharmaceutically acceptable salts thereof (SEQ ID NO: 112): 
Ac-Asp-Leu-Thr-D-Phe-His-Leu-Leu-Arg-Glu-Val-Leu-Glu-Nle-K*-R15-R16-Glu-Gln-
cVal-Ala-Gln-R22-Ala-Glu-R25-Asn-Arg-Lys-Leu-Nle-Glu-cVal-R33-CONH2
(Ie)
wherein: **R15** is Asn, Thr, Gln, Lys, or Arg; **R16** is Arg, Ala or Glu **R22** is E# or K#; **R25** is E# or K#; **R33** is Ile or Ala; and # represents a lactam bridge between E# and K#,
**K*** represents Lys-(2-[2-(2-amino-ethoxy)-ethoxy]-acetyl)ₙ-(γGlu)ₘ-diacid, wherein the diacid is a C16, C18, or C20 diacid, n is 1, 2 or 3, and m is 1, 2 or 3, with the proviso that when **R22** is E#, **R25** is K#, and when **R22** is K#, **R25** is E#.

In some embodiments of Formula (Ie), **R15** is Arg, **R16** is Glu, **R22** is E#, **R25** is K#, **R33** is Ile, n=3, m=1, and the diacid is a C18 diacid.

In some embodiments of Formula (Ie), **R15** is Arg, **R16** is Glu, **R22** is E#, **R25** is K#, **R33** is Ile, n=3, m=2, and the diacid is a C18 diacid.

In some embodiments of Formula (Ie), **R15** is Arg, **R16** is Glu, **R22** is E#, **R25** is K#, **R33** is Ile, n=3, m=3, and the diacid is a C18 diacid.

In some embodiments of Formula (Ie), **R15** is Arg, **R16** is Ala, **R22** is E#, **R25** is K#, **R33** is Ala, n=2, m=1, and the diacid is a C18 diacid.

In some embodiments of Formula (Ie), **R15** is Arg, **R16** is Glu, **R22** is E#, **R25** is K#, **R33** is Ala, n=2, m=1, and the diacid is a C18 diacid.

In some embodiments of Formula (Ie), **R15** is Lys, **R16** is Ala, **R22** is E#, **R25** is K#, **R33** is Ala, n=2, m=1, and the diacid is a C18 diacid.

In some embodiments of Formula (Ie), **R15** is Arg, **R16** is Glu, **R22** is E#, **R25** is K#, **R33** is Ala, n=3, m=1, and the diacid is a C20 diacid.

In another aspect, with respect to peptides of Formula (I), are peptides of Formula (If), or pharmaceutically acceptable salts thereof (SEQ ID NO: 113): 
Ac-Asp-Leu-Thr-D-Phe-His-Leu-Leu-Arg-Glu-Val-Leu-Glu-Nle-R14-K*-R16-Glu-Gln-
cVal-Ala-Gln-R22-Ala-Glu-R25-Asn-Arg-Lys-Leu-Nle-Glu-cVal-R33-CONH2
 (If)
wherein: **R14** is Glu, Thr, Ala, or Gln; **R16** is Arg, Ala or Glu; **R22** is E# or K#; **R25** is E# or K#; **R33** is Ile or Ala; and # represents a lactam bridge between E# and K#,
**K*** represents Lys-X-(2-[2-(2-amino-ethoxy)-ethoxy]-acetyl)ₙ-(γGlu)ₘ-diacid, wherein X is absent or Gln, the diacid is a C16, C18, or C20 diacid, n is 1, 2 or 3, and m is 1, 2 or 3; with the proviso that when **R22** is E#, **R25** is K#, and when **R22** is K#, **R25** is E#.

In some embodiments of Formula (If), **R14** is Thr, **R16** is Glu, **R22** is E#, **R25** is K#, **R33** is Ile, X is absent, n=2, m=1, and the diacid is a C18 diacid.

In some embodiments of Formula (If), **R14** is Gln, **R16** is Glu, **R22** is E#, **R25** is K#, **R33** is Ile, X is absent, n=2, m=1, and the diacid is a C18 diacid.

In some embodiments of Formula (If), **R14** is Ala, **R16** is Glu, **R22** is E#, **R25** is K#, **R33** is Ile, X is absent, n=2, m=1, and the diacid is a C18 diacid.

In some embodiments of Formula (If), **R14** is Glu, **R16** is Glu, **R22** is E#, **R25** is K#, **R33** is Ile, X is absent, n=2, m=2, and the diacid is a C18 diacid.

In some embodiments of Formula (If), **R14** is Gln, **R16** is Glu, **R22** is E#, **R25** is K#, **R33** is Ala, X is absent, n=2, m=1, and the diacid is a C18 diacid.

In some embodiments of Formula (If), **R14** is Gln, **R16** is Ala, **R22** is E#, **R25** is K#, **R33** is Ala, X is absent, n=2, m=1, and the diacid is a C18 diacid.

In some embodiments of Formula (If), **R14** is Thr, **R16** is Glu, **R22** is E#, **R25** is K#, **R33** is Ala, X is absent, n=2, m=1, and the diacid is a C18 diacid.

In some embodiments of Formula (If), **R14** is Thr, **R16** is Ala, **R22** is E#, **R25** is K#, **R33** is Ala, X is absent, n=2, m=1, and the diacid is a C18 diacid.

In some embodiments of Formula (If), **R14** is Thr, **R16** is Ala, **R22** is E#, **R25** is K#, **R33** is Ala, X is absent, n=3, m=1, and the diacid is a C18 diacid.

In some embodiments of Formula (If), **R14** is Gln, **R16** is Ala, **R22** is E#, **R25** is K#, **R33** is Ala, X is absent, n=3, m=1, and the diacid is a C18 diacid.

In another aspect, with respect to peptides of Formula (I), are peptides of Formula (Ig), or pharmaceutically acceptable salts thereof (SEQ ID NO: 114): 
Ac-Asp-Leu-Thr-D-Phe-His-Leu-Leu-Arg-Glu-Val-Leu-Glu-Nle-K*-Gln-R16-Glu-Gln-
cVal-Ala-Gln-R22-Ala-Glu-R25-Asn-Arg-Lys-Leu-Nle-Glu-cVal-R33-CONH2
 (Ig)
wherein: **R16** is Arg, Ala or Gln; **R22** is E# or K#; **R25** is E# or K#; **R33** is Ile or Ala; and # represents a lactam bridge between E# and K#,
**K*** represents Lys-(2-[2-(2-amino-ethoxy)-ethoxy]-acetyl)ₙ-(γGlu)ₘ-diacid, wherein the diacid is a C16, C18, or C20 diacid, n is 1, 2 or 3, and m is 1, 2 or 3, with the proviso that when **R22** is E#, **R25** is K#, and when **R22** is K#, **R25** is E#.

In some embodiments of Formula (Ig), **R16** is Glu, **R22** is E#, **R25** is K#, **R33**
is Ala, n=2, m=1, and the diacid is a C18 diacid.

In some embodiments of Formula (Ig), **R16** is Glu, **R22** is E#, **R25** is K#, **R33** is Ala, n=2, m=2, and the diacid is a C18 diacid.

In some embodiments of Formula (Ig), **R16** is Glu, **R22** is E#, **R25** is K#, **R33** is Ala, n=3, m=1, and the diacid is a C18 diacid.

In some embodiments of Formula (Ig), **R16** is Ala, **R22** is E#, **R25** is K#, **R33** is Ala, n=2, m=1, and the diacid is a C18 diacid.

In some embodiments of Formula (Ig), **R16** is Glu, **R22** is E#, **R25** is K#, **R33** is Ile, n=3, m=1, and the diacid is a C18 diacid.

In some embodiments of Formula (Ig), **R16** is Glu, **R22** is E#, **R25** is K#, **R33** is Ile, n=2, m=1, and the diacid is a C20 diacid.

In some embodiments of Formula (Ig), **R16** is Glu, **R22** is E#, **R25** is K#, **R33** is Ala, n=2, m=1, and the diacid is a C20 diacid.

In another aspect, with respect to peptides of Formula (I), are peptides of Formula (Ih), or pharmaceutically acceptable salts thereof (SEQ ID NO: 115): 
Ac-Asp-Leu-Thr-D-Phe-His-Leu-Leu-Arg-K*-Val-Leu-Glu-Nle-R14-R15-Glu-Glu-Gln-
cVal-Ala-Gln-R22-Ala-Glu-R25-Asn-Arg-Lys-Leu-Nle-Glu-cVal-Ile-CONH2
(Ih)
wherein: **R14** is Glu or Gln; **R15** is Arg, Gln, Glu, or Gla; **R22** is E# or K#; **R25** is E# or K#; and # represents a lactam bridge between E# and K#,
**K*** represents Lys-(2-[2-(2-amino-ethoxy)-ethoxy]-acetyl)ₙ-(γGlu)ₘ-diacid or PEG12-γGlu-diacid, wherein the diacid is a C16, C18, or C20 diacid, n is 1, 2 or 3, and m is 1, 2 or 3; with the proviso that when **R22** is E#, **R25** is K#, and when **R22** is K#, **R25** is E#.

In some embodiments of Formula (Ih), **R14** is Gln, **R15** is Gln, **R22** is E#, **R25** is K#, **K*** is Lys-(2-[2-(2-amino-ethoxy)-ethoxy]-acetyl)ₙ-(γGlu)ₘ-diacid, n=2, m=1, and the diacid is a C18 diacid.

In some embodiments of Formula (Ih), R14 is Glu, **R15 is** Gla, **R22** is E#, **R25** is K#, **K*** is Lys-(2-[2-(2-amino-ethoxy)-ethoxy]-acetyl)n-(γGlu)m-diacid, n=2, m=1, and the diacid is a C18 diacid.

In some embodiments of Formula (Ih), **R14** is Glu, **R15** is Gln, **R22** is E#, **R25** is K#, **K*** is Lys-(2-[2-(2-amino-ethoxy)-ethoxy]-acetyl)ₙ-(γGlu)ₘ-diacid, n=2, m=1, and the diacid is a C16 diacid.

In some embodiments of Formula (Ih), **R14** is Glu, **R15** is Glu, **R22** is E#, **R25** is K#, **K*** is Lys-(2-[2-(2-amino-ethoxy)-ethoxy]-acetyl)ₙ-(γGlu)ₘ-diacid, n=2, m=1, and the diacid is a C18 diacid.

In some embodiments of Formula (Ih), **R14** is Glu, **R15** is Arg, **R22** is E#, **R25** is K#, **K*** is PEG12-γGlu-diacid, and the diacid is a C18 diacid.

In another aspect, with respect to peptides of Formula (I), are peptides of Formula (Ik), or pharmaceutically acceptable salts thereof (SEQ ID NO: 116): 
X-Asp-Leu-Thr-D-Phe-His-Leu-Leu-Arg-R9-Val-Leu-R12-R13-R14-R15-R16-R17-R18-
cVal-R20-R21-R22-Ala-Glu-R25-Asn-R27-Lys-R29-Nle-Glu-cVal-R33-CONH2
(Ik)
wherein: X is Ac or (2-[2-(2-amino-ethoxy)-ethoxy]-acetyl)₂-γGlu-diacid, wherein the diacid is a C16, C18, or C20 diacid;
**R9** is Glu or **K*;** R12 is Glu or **K*; R13** is Nle or **K*; R14** is Thr, Glu, or **K*; R15** is Arg, Ala, Gln, or **K*; R16** is Ala or Glu; **R17** is Glu or **K*; R18** is Gln or **K*; R20** is Ala or **K*; R21** is Gln or **K*; R22** is E# or K#; **R25** is E# or K#; **R27** is Arg or **K*;**
**R29** is Leu or **K*; R33** is Ile or Ala; and
**K*** represents Lys-Z-(2-[2-(2-amino-ethoxy)-ethoxy]-acetyl)ₙ-(γGlu)ₘ-diacid, wherein Z is absent, Lys, Glu, γGlu, Ala, or Gln, the diacid is a C16, C18, or C20 diacid, n is 1, 2 or 3, and m is 1, 2 or 3; with the proviso that only one amino acid in the sequence is **K*;** and with the further proviso that when **R22** is E#, **R25** is K#, and when **R22** is K#, **R25** is E#.

In some embodiments of Formula (Ik), **R9** is **K*, R12 is** Glu, **R13** is Nle, **R14**
is Glu, **R15** is Arg, **R16** is Glu, **R17** is Glu, **R18** is Gln, **R20** is Ala, **R21** is Gln, **R22** is E#, **R25** is K#, **R27** is Arg, **R29** is Leu, **R33** is Ile, Z is absent, n=2, m=1, and the diacid is a C18 diacid.

In some embodiments of Formula (Ik), **R9** is Glu, **R12** is Glu, **R13** is **K*, R14** is Glu, **R15** is Arg, **R16** is Glu, **R17** is Glu, **R18** is Gln, **R20** is Ala, **R21** is Gln, **R22** is E#, **R25** is K#, **R27** is Arg, **R29** is Leu, **R33** is Ile, Z is absent, n=2, m=1, and the diacid is a C18 diacid.

In some embodiments of Formula (Ik), **R9** is Glu, **R12** is Glu, **R13** is Nle, **R14** is **K*, R15** is Arg, **R16** is Glu, **R17** is Glu, **R18** is Gln, **R20** is Ala, **R21** is Gln, **R22** is E#, **R25** is K#, **R27** is Arg, **R29** is Leu, **R33** is Ile, Z is absent, n=2, m=1, and the diacid is a C18 diacid.

In some embodiments of Formula (Ik), **R9** is Glu, **R12** is Glu, **R13** is Nle, **R14** is **K*, R15** is Gln, **R16** is Glu, **R17** is Glu, **R18** is Gln, **R20** is Ala, **R21** is Gln, **R22** is E#, **R25** is K#, **R27** is Arg, **R29** is Leu, **R33** is Ile, Z is absent, n=2, m=1, and the diacid is a C18 diacid.

In some embodiments of Formula (Ik), **R9** is Glu, **R12** is Glu, **R13** is Nle, **R14** is **K*, R15** is Ala, **R16** is Glu, **R17** is Glu, **R18** is Gln, **R20** is Ala, **R21** is Gln, **R22** is E#, **R25** is K#, **R27** is Arg, **R29** is Leu, **R33** is Ile, Z is absent, n=2, m=1, and the diacid is a C18 diacid.

In some embodiments of Formula (Ik), **R9** is Glu, R12 is Glu, R13 is Nle, R14 is Glu, R15 is **K* R16** is Glu, R17 is Glu, R18 is Gln, R20 is Ala, R21 is Gln, **R22** is E#, **R25** is K#, R27 is Arg, R29 is Leu, **R33** is Ile, Z is absent, n=2, m=1, and the diacid is a C18 diacid.

In some embodiments of Formula (Ik), **R9** is Glu, R12 is Glu, R13 is Nle, R14 is Glu, R15 is Arg, **R16** is Glu, R17 is Glu, R18 is Gln, R20 is Ala, R21 is Gln, **R22** is E#, **R25** is K#, R27 is Arg, R29 is Leu, **R33** is Ile, Z is absent, n=2, m=1, and the diacid is a C18 diacid.

In some embodiments of Formula (Ik), **R9** is Glu, R12 is Glu, R13 is Nle, R14 is Glu, R15 is Arg, **R16** is Glu, R17 is Glu, R18 is Gln, R20 is Ala, R21 is Gln, **R22** is E#, **R25** is K#, R27 is Arg, R29 is Leu, **R33** is Ile, Z is absent, n=2, m=1, and the diacid is a C18 diacid.

In some embodiments of Formula (Ik), **R9** is Glu, R12 is Glu, R13 is Nle, R14 is Glu, R15 is Arg, **R16** is Glu, R17 is Glu, R18 is Gln, R20 is Ala, R21 is Gln, **R22** is E#, **R25** is K#, R27 is **K*,** R29 is Leu, **R33** is Ile, Z is absent, n=2, m=1, and the diacid is a C18 diacid.

In some embodiments of Formula (Ik), **R9** is Glu, R12 is Glu, R13 is Nle, R14 is Glu, R15 is Arg, **R16** is Glu, R17 is Glu, R18 is Gln, R20 is Ala, R21 is Gln, **R22** is E#, **R25** is K#, R27 is Arg, R29 is **K*, R33** is Ile, Z is absent, n=2, m=1, and the diacid is a C18 diacid.

In some embodiments of Formula (Ik), R14 is Glu, R9 is **K*,** R12 is Glu, R13 is Nle, R15 is Arg, **R16** is Glu, R17 is Glu, R18 is Gln, R20 is Ala, R21 is Gln, **R22** is E#, **R25** is K#, R27 is Arg, R29 is Leu, **R33** is Ile, Z is absent, n=2, m=1, and the diacid is a C16 diacid.

In some embodiments of Formula (Ik), **R9** is Glu, R12 is K*, R13 is Nle, R14 is Glu, R15 is Arg, R16 is Glu, R17 is Glu, R18 is Gln, R20 is Ala, R21 is Gln, **R22** is E#, **R25** is K#, R27 is Arg, R29 is Leu, **R33** is Ile, Z is absent, n=2, m=1, and the diacid is a C16 diacid.

In some embodiments of Formula (Ik), **R9** is Glu, R12 is Glu, R13 is Nle, R14 is Glu, R15 is Arg, **R16** is Glu, R17 is **K*,** R18 is Gln, R20 is Ala, R21 is Gln, **R22** is E#, **R25** is K#, R27 is Arg, R29 is Leu, **R33** is Ile, Z is absent, n=2, m=1, and the diacid is a C16 diacid.

In some embodiments of Formula (Ik), **R9** is Glu, R12 is Glu, R13 is Nle, R14 is Glu, R15 is Arg, **R16** is Glu, R17 is Glu, R18 is **K*,** R20 is Ala, R21 is Gln, **R22** is E#, **R25** is K#, R27 is Arg, R29 is Leu, **R33** is Ile, Z is absent, n=2, m=1, and the diacid is a C16 diacid.

In some embodiments of Formula (Ik), **R9** is Glu, R12 is Glu, R13 is Nle, R14 is Glu, R15 is Arg, **R16** is Glu, R17 is Glu, R18 is Gln, R20 is **K*,** R21 is Gln, **R22** is E#, **R25** is K#, R27 is Arg, R29 is Leu, **R33** is Ile, Z is absent, n=2, m=1, and the diacid is a C16 diacid.

In some embodiments of Formula (Ik), **R9** is Glu, R12 is Glu, R13 is Nle, R14 is Glu, R15 is Arg, **R16** is Glu, R17 is Glu, R18 is Gln, R20 is Ala, R21 is **K*, R22** is E#, **R25** is K#, R27 is Arg, R29 is Leu, **R33** is Ile, Z is absent, n=2, m=1, and the diacid is a C16 diacid.

In some embodiments of Formula (Ik), **R9** is Glu, R12 is Glu, R13 is Nle, R14 is Thr, R15 is **K*, R16** is Glu, R17 is Glu, R18 is Gln, R20 is Ala, R21 is Gln, **R22** is E#, **R25** is K#, R27 is Arg, R29 is Leu, **R33** is Ile, Z is absent, n=2, m=1, and the diacid is a C16 diacid.

In some embodiments of Formula (Ik), **R9** is Glu, R12 is Glu, R13 is Nle, R14 is Thr, R15 is **K*, R16** is Ala, R17 is Glu, R18 is Gln, R20 is Ala, R21 is Gln, **R22** is E#, **R25** is K#, R27 is Arg, R29 is Leu, **R33** is Ala, Z is absent, n=2, m=1, and the diacid is a C16 diacid.

In some embodiments of Formula (Ik), **R9** is Glu, R12 is Glu, R13 is Nle, R14 is Glu, R15 is **K*, R16** is Glu, R17 is Glu, R18 is Gln, R20 is Ala, R21 is Gln, **R22** is E#, **R25** is K#, R27 is Arg, R29 is Leu, **R33** is Ile, Z is absent, n=2, m=2, and the diacid is a C16 diacid.

In some embodiments of Formula (Ik), **R9** is Glu, R12 is Glu, R13 is Nle, R14 is Glu, R15 is **K*, R16** is Glu, R17 is Glu, R18 is Gln, R20 is Ala, R21 is Gln, **R22** is E#, **R25** is K#, R27 is Arg, R29 is Leu, **R33** is Ile, Z is absent, n=2, m=3, and the diacid is a C16 diacid.

In some embodiments of Formula (Ik), **R9** is Glu, R12 is Glu, R13 is Nle, R14 is Glu, R15 is **K*, R16** is Glu, R17 is Glu, R18 is Gln, R20 is Ala, R21 is Gln, **R22** is E#, **R25** is K#, R27 is Arg, R29 is Leu, **R33** is Ile, Z is Glu, n=2, m=1, and the diacid is a C16 diacid.

In some embodiments of Formula (Ik), **R9** is Glu, R12 is Glu, R13 is Nle, R14 is Glu, R15 is **K*, R16** is Glu, R17 is Glu, R18 is Gln, R20 is Ala, R21 is Gln, **R22** is E#, **R25** is K#, R27 is Arg, R29 is Leu, **R33** is Ile, Z is γGlu, n=2, m=1, and the diacid is a C16 diacid.

In some embodiments of Formula (Ik), **R9** is Glu, R12 is Glu, R13 is Nle, R14 is Glu, R15 is **K*, R16** is Glu, R17 is Glu, R18 is Gln, R20 is Ala, R21 is Gln, **R22** is E#, **R25** is K#, R27 is Arg, R29 is Leu, **R33** is Ile, Z is Lys, n=2, m=1, and the diacid is a C16 diacid.

In some embodiments of Formula (Ik), **R9** is Glu, R12 is Glu, R13 is Nle, R14 is Glu, R15 is **K*, R16** is Glu, R17 is Glu, R18 is Gln, R20 is Ala, R21 is Gln, **R22** is E#, **R25** is K#, R27 is Arg, R29 is Leu, **R33** is Ile, Z is Ala, n=2, m=1, and the diacid is a C16 diacid.

In some embodiments of Formula (Ik), **R9** is Glu, R12 is Glu, R13 is Nle, R14 is Glu, R15 is **K*, R16** is Glu, R17 is Glu, R18 is Gln, R20 is Ala, R21 is Gln, **R22** is E#, **R25** is K#, R27 is Arg, R29 is Leu, **R33** is Ile, Z is Gln, n=2, m=1, and the diacid is a C16 diacid.

In another aspect is a CRF receptor antagonist fatty acid conjugated cyclic peptide comprising or consisting of SEQ ID NO: 117, or Formula (I) with the following sequence: 
Ac-Asp-Leu-Thr-D-Phe-His-Leu-Leu-Arg-Glu-Val-Leu-Glu-Nle-K*-Gln-Glu-Glu-
Gln-cVal-Ala-Gln-E#-Ala-Glu-K#-Asn-Arg-Lys-Leu-Nle-Glu-cVal-Ile-CONH2;
wherein **K*** represents Lys-(2-[2-(2-amino-ethoxy)-ethoxy]-acetyl)₂-γGlu-diacid,
wherein the diacid is a C18 diacid; and wherein E# and K# represent a lactam bridge
   between the side chains of these two amino acids (E=Glu; K=Lys) (SEQ ID NO: 50).
In some embodiments, the peptide is a pharmaceutically acceptable salt thereof.

In another aspect is a CRF receptor antagonist fatty acid conjugated cyclic peptide comprising or consisting of Formula (I) with the following sequence: 
Ac-Asp-Leu-Thr-D-Phe-His-Leu-Leu-Arg-Glu-Val-Leu-Glu-Nle-K*-Gln-Glu-Glu-
Gln-cVal-Ala-Gln-E#-Ala-Glu-K#-Asn-Arg-Lys-Leu-Nle-Glu-cVal-Ala-CONH2;
wherein **K*** represents Lys-(2-[2-(2-amino-ethoxy)-ethoxy]-acetyl)₂-γGlu-diacid,
wherein the diacid is a C18 diacid; and wherein E# and K# represent a lactam bridge
   between the side chains of these two amino acids (E=Glu; K=Lys) (SEQ ID NO: 95).
In some embodiments, the peptide is a pharmaceutically acceptable salt thereof.

In another aspect is a CRF receptor antagonist fatty acid conjugated cyclic peptide comprising or consisting of Formula (I) with the following sequence: 
Ac-Asp-Leu-Thr-D-Phe-His-Leu-Leu-Arg-Glu-Val-Leu-Glu-Nle-K*-Gln-Glu-Glu-
Gln-cVal-Ala-Gln-E#-Ala-Glu-K#-Asn-Arg-Lys-Leu-Nle-Glu-cVal-Ala-CONH2;
wherein K* represents Lys-(2-[2-(2-amino-ethoxy)-ethoxy]-acetyl)₃-γGlu-diacid,
wherein the diacid is a C18 diacid; and wherein E# and K# represent a lactam bridge
   between the side chains of these two amino acids (E=Glu; K=Lys) (SEQ ID NO: 97).
In some embodiments, the peptide is a pharmaceutically acceptable salt thereof.

In another aspect is a CRF receptor antagonist fatty acid conjugated cyclic peptide comprising or consisting of the following sequence: 
Ac-Asp-Leu-Thr-D-Phe-His-Leu-Leu-Arg-Glu-Val-Leu-Glu-Nle-K*-Arg-Glu-Glu-
Gln-cVal-Ala-Gln-E#-Ala-Glu-K#-Asn-Arg-Lys-Leu-Nle-Glu-cVal-Ala-CONH2;
wherein K* represents Lys-(2-[2-(2-amino-ethoxy)-ethoxy]-acetyl)₂-γGlu-diacid,
wherein the diacid is a C20 diacid (SEQ ID NO: 101).
In some embodiments, the peptide is a pharmaceutically acceptable salt thereof.

In one aspect, the CRF receptor antagonist fatty acid conjugated cyclic peptide comprising or consisting of SEQ ID NO: 117, or of Formula (I), or pharmaceutically acceptable salts thereof, as described herein, are biopotent in inhibiting the secretion of ACTH and corticosterone in response to various stimuli and bind to CRFR1 and CRFR2.

In another aspect of the disclosure is a fatty acid conjugated cyclic peptide CRF receptor antagonist, or a pharmaceutically acceptable salt thereof, selected from the peptides in Tables B1.1 and B1.2. In Tables B1.1 and B1.2: **K*** represents the point of attachment of the appropriate fatty acid via the nitrogen of the side chain of lysine; Y represents either an acylated (Ac) N-terminus or X; and X represents the point of attachment of the appropriate fatty acid via the nitrogen of the N-terminus.

**-Table B1.1 Fatty acid conjugated cyclic peptides of the disclosure.**

| **SEQ ID NO:** | **SEQUENCE** | **K* or X*** |
|---|---|---|
| SEQ ID NO:1 | Ac- DLTfHLLREVLE(Nle)ARAEQ(CML)AQE#A(Aib)K#N RKL(Nle)E(CML)I-CONH2 | |
| SEQ ID NO:2 | Ac- DLTfHLLREVLE(Nle)EREEQ(cVal)AQE#AEK#NRKL (Nle)E(cVal)I-CONH2 | |
| SEQ ID NO:3 | X*- DLTfHLLREVLE(Nle)ARAEQ(CML)AQE#ADegK#N RK(iPr)L(Nle)E(CML)I-CONH2 | (AEEA)-(AEEA)-γGlu-C18-diacid |
| SEQ ID NO:4 | X*-DLTfHLLREVLE(Nle) EREEQ(cVal)AQE#AEK#NRKL(Nle)E(cVal)I-CONH2 | (AEEA)-(AEEA)-γGlu-C18-diacid |
| SEQ ID NO:5 | Ac- DLTK*HLLREVLE(Nle)EREEQ(cVal)AQE#AEK#NR KL(Nle)E(cVal)I-CONH2 | (AEEA)-(AEEA)-γGlu-C18-diacid |
| SEQ ID NO:6 | Ac- DLTfHLLRK*VLE(Nle)EREEQ(cVal)AQE#AEK#NRK L(Nle)E(cVal)I-CONH2 | (AEEA)-(AEEA)-γGlu-C18-diacid |
| SEQ ID NO:7 | Ac- DLTfHLLREVLK*(Nle)EREEQ(cVal)AQE#AEK#NRK L(Nle)E(cVal)I-CONH2 | (AEEA)-(AEEA)-γGlu-C18-diacid |
| SEQ ID NO:8 | Ac- DLTfHLLREVLEK*EREEQ(cVal)AQE#AEK#NRKL( Nle)E(cVal)I-CONH2 | (AEEA)-(AEEA)-γGlu-C18-diacid |
| SEQ ID NO:9 | Ac- DLTfHLLREVLE(Nle)K*REEQ(cVal)AQE#AEK#NRK L(Nle)E(cVal)I-CONH2 | (AEEA)-(AEEA)-γGlu-C18-diacid |
| SEQ ID NO:10 | Ac- DLTfHLLREVLE(Nle)EK*EEQ(cVal)AQE#AEK#NRK L(Nle)E(cVal)I-CONH2 | (AEEA)-(AEEA)-γGlu-C18-diacid |
| SEQ ID NO:11 | Ac- DLTfHLLREVLE(Nle)ERK*EQ(cVal)AQE#AEK#NRK L(Nle)E(cVal)I-CONH2 | (AEEA)-(AEEA)-γGlu-C18-diacid |
| SEQ ID NO:12 | Ac- DLTfHLLREVLE(Nle)EREK*Q(cVal)AQE#AEK#NRK L(Nle)E(cVal)I-CONH2 | (AEEA)-(AEEA)-γGlu-C18-diacid |
| SEQ ID NO: 13 | Ac- DLTfHLLREVLE(Nle)EREEK*(cVal)AQE#AEK#NRK L(Nle)E(cVal)I-CONH2 | (AEEA)-(AEEA)-γGlu-C18-diacid |
| SEQ ID NO:14 | Ac- DLTfHLLREVLE(Nle)EREEQK*AQE#AEK#NRKL(Nl e)E(cVal)I-CONH2 | (AEEA)-(AEEA)-γGlu-C18-diacid |
| SEQ ID NO:15 | Ac- DLTfHLLREVLE(Nle)EREEQ(cVal)K*QE#AEK#NRK L(Nle)E(cVal)I-CONH2 | (AEEA)-(AEEA)-γGlu-C18-diacid |
| SEQ ID NO:16 | Ac- DLTfHLLREVLE(Nle)EREEQ(cVal)AK*E#AEK#NRK L(Nle)E(cVal)I-CONH2 | (AEEA)-(AEEA)-γGlu-C18-diacid |
| SEQ ID NO:17 | Ac- DLTfHLLREVLE(Nle)EREEQ(cVal)AQE#AK*K#NRK L(Nle)E(cVal)I-CONH2 | (AEEA)-(AEEA)-γGlu-C18-diacid |
| SEQ ID NO:18 | Ac- DLTfHLLREVLE(Nle)EREEQ(cVal)AQE#AEK#NK*K L(Nle)E(cVal)I-CONH2 | (AEEA)-(AEEA)-γGlu-C18-diacid |
| SEQ ID NO:19 | Ac- DLTfHLLREVLE(Nle)EREEQ(cVal)AQE#AEK#NRK* L(Nle)E(cVal)I-CONH2 | (AEEA)-(AEEA)-γGlu-C18-diacid |
| SEQ ID NO:20 | Ac- DLTfHLLREVLE(Nle)EREEQ(cVal)AQE#AEK#NRK K*(Nle)E(cVal)I-CONH2 | (AEEA)-(AEEA)-γGlu-C18-diacid |
| SEQ ID NO:21 | Ac- DLTfHLLREVLE(Nle)EREEQ(cVal)AQE#AEK#NRKL (Nle)K*(cVal)I-CONH2 | (AEEA)-(AEEA)-γGlu-C18-diacid |
| SEQ ID NO:22 | X*- DLTfHLLREVLE(Nle)EREEQ(cVal)AQE#AEK#NRKL (Nle)E(cVal)I-CONH2 | (AEEA)-(AEEA)-γGlu-C16-diacid |
| SEQ ID NO:23 | Ac- DLTfHLLRK*VLE(Nle)EREEQ(cVal)AQE#AEK#NRK L(Nle)E(cVal)I-CONH2 | (AEEA)-(AEEA)-γGlu-C16-diacid |
| SEQ ID NO:24 | Ac- DLTfHLLREVLK*(Nle)EREEQ(cVal)AQE#AEK#NRK L(Nle)E(cVal)I-CONH2 | (AEEA)-(AEEA)-γGlu-C16-diacid |
| SEQ ID NO:25 | Ac- DLTfHLLREVLE(Nle)EK*EEQ(cVal)AQE#AEK#NRK L(Nle)E(cVal)I-CONH2 | (AEEA)-(AEEA)-γGlu-C16-diacid |
| SEQ ID NO:26 | Ac- DLTfHLLREVLE(Nle)EREK*Q(cVal)AQE#AEK#NRK L(Nle)E(cVal)I-CONH2 | (AEEA)-(AEEA)-γGlu-C16-diacid |
| SEQ ID NO:27 | Ac- DLTfHLLREVLE(Nle)EREEK*(cVal)AQE#AEK#NRK L(Nle)E(cVal)I-CONH2 | (AEEA)-(AEEA)-γGlu-C16-diacid |
| SEQ ID NO:28 | Ac- DLTfHLLREVLE(Nle)EREEQ(cVal)K*QE#AEK#NRK L(Nle)E(cVal)I-CONH2 | (AEEA)-(AEEA)-γGlu-C16-diacid |
| SEQ ID NO:29 | Ac- DLTfHLLREVLE(Nle)EREEQ(cVal)AK*E#AEK#NRK L(Nle)E(cVal)I-CONH2 | (AEEA)-(AEEA)-γGlu-C16-diacid |
| SEQ ID NO:30 | Ac- DLTfHLLREVLE(Nle)EREEQ(cVal)AQE#AEK#NRK K*(Nle)E(cVal)I-CONH2 | (AEEA)-(AEEA)-γGlu-C16-diacid |
| SEQ ID NO:32 | Ac- DLTfHLLRK*VLE(Nle)EEEEQ(cVal)AQE#AEK#NRK L(Nle)E(cVal)I-CONH2 | (AEEA)-(AEEA)-γGlu-C18-diacid |
| SEQ ID NO:33 | Ac- DLTfHLLRK*VLE(Nle)EREEQ(cVal)AQE#AEK#NRK L(Nle)E(cVal)I-CONH2 | PEG12-γGlu-C18-diacid |
| SEQ ID NO:34 | Ac- DLTfHLLRK*VLE(Nle)EREEQ(cVal)AQE#AEK#NRK L(Nle)E(cVal)I-CONH2 | PEG12-(γGlu)₂-C18-diacid |
| SEQ ID NO:36 | Ac- DLTfHLLREVLE(Nle)TK*EEQ(cVal)AQE#AEK#NRK L(Nle)E(cVal)I-CONH2 | (AEEA)-(AEEA)-γGlu-C16-diacid |
| SEQ ID NO:37 | Ac- DLTfHLLREVLE(Nle)EK*EEQ(cVal)AQE#AEK#NRK L(Nle)E(cVal)A-CONH2 | (AEEA)-(AEEA)-γGlu-C16-diacid |
| SEQ ID NO:38 | Ac- DLTfHLLREVLE(Nle)EK*AEQ(cVal)AQE#AEK#NR KL(Nle)E(cVal)I-CONH2 | (AEEA)-(AEEA)-γGlu-C16-diacid |
| SEQ ID NO:39 | Ac- DLTfHLLREVLE(Nle)TK*AEQ(cVal)AQE#AEK#NR KL(Nle)E(cVal)A-CONH2 | (AEEA)-(AEEA)-γGlu-C16-diacid |
| SEQ ID NO:40 | Ac- DLTfHLLREVLE(Nle)EK*EEQ(cVal)AQE#AEK#NRK L(Nle)E(cVal)I-CONH2 | (AEEA)-(AEEA)-γGlu-γGlu-C16-diacid |
| SEQ ID NO:41 | Ac- DLTfHLLREVLE(Nle)EK*EEQ(cVal)AQE#AEK#NRK L(Nle)E(cVal)I-CONH2 | (AEEA)-(AEEA)-γGlu-γGlu-γGlu-C16-diacid |
| SEQ ID NO:42 | Ac- DLTfHLLREVLE(Nle)EK*EEQ(cVal)AQE#AEK#NRK L(Nle)E(cVal)I-CONH2 | (AEEA)-γGlu-γGlu-γGlu-C16-diacid |
| SEQ ID NO:43 | Ac- DLTfHLLREVLE(Nle)EK*EEQ(cVal)AQE#AEK#NRK L(Nle)E(cVal)I-CONH2 | Glu-(AEEA)-(AEEA)-γGlu-C16-diacid |
| SEQ ID NO:44 | Ac- DLTfHLLREVLE(Nle)EK*EEQ(cVal)AQE#AEK#NRK L(Nle)E(cVal)I-CONH2 | γGlu-(AEEA)-(AEEA)-γGlu-C16-diacid |
| SEQ ID NO:45 | Ac- DLTfHLLREVLE(Nle)EK*EEQ(cVal)AQE#AEK#NRK L(Nle)E(cVal)I-CONH2 | Lys-(AEEA)-(AEEA)-γGlu- C16-diacid |
| SEQ ID NO:46 | Ac- DLTfHLLREVLE(Nle)EK*EEQ(cVal)AQE#AEK#NRK L(Nle)E(cVal)I-CONH2 | Ala-(AEEA)- (AEEA)-γGlu-C16-diacid |
| SEQ ID NO:47 | Ac- DLTfHLLREVLE(Nle)EK*EEQ(cVal)AQE#AEK#NRK L(Nle)E(cVal)I-CONH2 | Gln-(AEEA)-(AEEA)-γGlu-C16-diacid |
| SEQ ID NO:48 | Ac- DLTfHLLREVLE(Nle)EK*EEQ(cVal)AQE#AEK#NRK L(Nle)E(cVal)I-CONH2 | Gly-(AEEA)-(AEEA)-γGlu -C16-diacid |
| SEQ ID NO:49 | Ac- DLTfHLLREVLE(Nle)EK*EEQ(cVal)AQE#AEK#NRK L(Nle)E(cVal)I-CONH2 | His-(AEEA)-(AEEA)-γGlu- C16-diacid |
| SEQ ID NO:50 | Ac- DLTfHLLREVLE(Nle)K*QEEQ(cVal)AQE#AEK#NR KL(Nle)E(cVal)I-CONH2 | (AEEA)-(AEEA)-γGlu-C18-diacid |
| SEQ ID NO:51 | Ac- DLTfHLLREVLE(Nle)K*EQEQ(cVal)AQE#AEK#NR KL(Nle)E(cVal)I-CONH2 | (AEEA)-(AEEA)-γGlu-C18-diacid |
| SEQ ID NO:52 | Ac- DLTfHLLREVLE(Nle)K*AEEQ(cVal)AQE#AEK#NR KL(Nle)E(cVal)I-CONH2 | (AEEA)-(AEEA)-γGlu-C18-diacid |
| SEQ ID NO:53 | Ac- DLTfHLLREVLE(Nle)K*EEEQ(cVal)AQE#AEK#NRK L(Nle)E(cVal)I-CONH2 | (AEEA)-(AEEA)-γGlu-C18-diacid |
| SEQ ID NO:54 | Ac- DLTfHLLRK*VLE(Nle)EREEQ(cVal)AQE#AEK#NRK L(Nle)E(cVal)I-CONH2 | (AEEA)-(AEEA)-γGlu-γGlu-C18-diacid |
| SEQ ID NO:55 | Ac- DLTfHLLRK*VLE(Nle)EREEQ(cVal)AQE#AEK#NRK L(Nle)E(cVal)I-CONH2 | (AEEA)-(AEEA)-γGlu-γGlu-γGlu-C18-diacid |
| SEQ ID NO:56 | Ac- DLTfHLLRK*VLE(Nle)EQEEQ(cVal)AQE#AEK#NR KL(Nle)E(cVal)I-CONH2 | (AEEA)-(AEEA)-γGlu-C18-diacid |
| SEQ ID NO:57 | Ac- DLTfHLLRK*VLE(Nle)QQEEQ(cVal)AQE#AEK#NR KL(Nle)E(cVal)I-CONH2 | (AEEA)-(AEEA)-γGlu-C18-diacid |
| SEQ ID NO:58 | Ac- DLTfHLLRK*VLE(Nle)EAadEEQ(cVal)AQE#AEK#N RKL(Nle)E(cVal)I-CONH2 | (AEEA)-(AEEA)-γGlu-C18-diacid |
| SEQ ID NO:59 | Ac- DLTfHLLRK*VLE(Nle)EGlaEEQ(cVal)AQE#AEK#N RKL(Nle)E(cVal)I-CONH2 | (AEEA)-(AEEA)-γGlu-C18-diacid |
| SEQ ID NO:60 | Ac- DLTfHLLRK*VLE(Nle)EpcFEEQ(cVal)AQE#AEK#N RKL(Nle)E(cVal)I-CONH2 | (AEEA)-(AEEA)-γGlu-C18-diacid |
| SEQ ID NO:61 | Ac- DLTfHLLRK*VLE(Nle)EK&EEQ(cVal)AQE#AEK#N RKL(Nle)E(cVal)I-CONH2 | (AEEA)-(AEEA)-γGlu-C18-diacid |
| SEQ ID NO:62 | Ac- DLTfHLLRK*VLE(Nle)EK&EEQ(cVal)AQE#AEK#N RKL(Nle)E(cVal)I-CONH2 | (AEEA)-(AEEA)-γGlu-C18-diacid |
| SEQ ID NO:63 | Ac- DLTfHLLRK*VLE(Nle)EK&EEQ(cVal)AQE#AEK#N RKL(Nle)E(cVal)I-CONH2 | (AEEA)-(AEEA)-γGlu-C18-diacid |
| SEQ ID NO:64 | Ac- DLTfHLLRK*VLE(Nle)EE&EEQ(cVal)AQE#AEK#NR KL(Nle)E(cVal)I-CONH2 | (AEEA)-(AEEA)-γGlu-C18-diacid |
| SEQ ID NO:65 | Ac- DLTfHLLRK*VLE(Nle)EE&EEQ(cVal)AQE#AEK#NR KL(Nle)E(cVal)I-CONH2 | (AEEA)-(AEEA)-γGlu-C18-diacid |
| SEQ ID NO:66 | Ac- DLTfHLLRK*VLE(Nle)EQEEQ(cVal)AQE#AEK#NR KL(Nle)E(cVal)I-CONH2 | (AEEA)-(AEEA)-γGlu-C16-diacid |
| SEQ ID NO:67 | Ac- DLTfHLLRK*VLE(Nle)EREEQ(cVal)AQE#AEK#NRK L(Nle)DSV-CONH2 | (AEEA)-(AEEA)-γGlu-C18-diacid |
| SEQ ID NO:68 | Ac- DLTfHLLRK*VLE(Nle)EREEQ(cVal)AQE#AEK#NRK L(Nle)DIA-CONH2 | (AEEA)-(AEEA)-γGlu-C18-diacid |
| SEQ ID NO:69 | Ac- DLTfHLLRK*VLE(Nle)EREEQ(cVal)AQE#aEK#NRK LChaDIA-CONH2 | (AEEA)-(AEEA)-γGlu-C18-diacid |
| SEQ ID NO:70 | Ac- DLTfHLLREVLE(Nle)EQEEQ(cVal)AQE#AEK#NRK K*(Nle)E(cVal)I-CONH2 | (AEEA)-(AEEA)-γGlu-C18-diacid |
| SEQ ID NO:71 | Ac- DLTfHLLREVLE(Nle)K*REEQ(cVal)AQE#AEK#NRK L(Nle)E(cVal)I-CONH2 | (AEEA)-γGlu-C18-diacid |
| SEQ ID NO:72 | Ac- DLTfHLLREVLE(Nle)K*REEQ(cVal)AQE#AEK#NRK L(Nle)E(cVal)I-CONH2 | (AEEA)-(AEEA)-(AEEA)-γGlu-C18-diacid |
| SEQ ID NO:73 | Ac- DLTfHLLREVLE(Nle)K*REEQ(cVal)AQE#AEK#NRK L(Nle)E(cVal)I-CONH2 | (AEEA)-(AEEA)-γGlu-γGlu-C18-diacid |
| SEQ ID NO:74 | Ac- DLTfHLLREVLE(Nle)K*REEQ(cVal)AQE#AEK#NRK L(Nle)E(cVal)I-CONH2 | (AEEA)-(AEEA)-γGlu-γGlu-γGlu-C18-diacid |
| SEQ ID NO:75 | Ac- DLTfHLLREVLE(Nle)K*KEEQ(cVal)AQE#AEK#NR KL(Nle)E(cVal)I-CONH2 | (AEEA)-(AEEA)-γGlu-C18-diacid |
| SEQ ID NO:76 | Ac- DLTfHLLREVLE(Nle)K*KEEQ(cVal)AQE#AEK#NR KL(Nle)E(cVal)I-CONH2 | (AEEA)-(AEEA)-γGlu-C18-diacid |
| SEQ ID NO:77 | Ac- DLTfHLLREVLE(Nle)K*KADQ(cVal)AQE#AEK#NR KL(Nle)E(cVal)I-CONH2 | (AEEA)-(AEEA)-γGlu-C18-diacid |
| SEQ ID NO:78 | Ac- DLTfHLLREVLE(Nle)K*RAEQ(cVal)AQE#AEK#NR KL(Nle)E(cVal)A-CONH2 | (AEEA)-(AEEA)-γGlu-C18-diacid |
| SEQ ID NO:79 | Ac- DLTfHLLREVLE(Nle)K*REEQ(cVal)AQE#AEK#NRK L(Nle)E(cVal)A-CONH2 | (AEEA)-(AEEA)-γGlu-C18-diacid |
| SEQ ID NO:80 | Ac- DLTfHLLREVLE(Nle)K*KAEQ(cVal)AQE#AEK#NR KL(Nle)E(cVal)A-CONH2 | (AEEA)-(AEEA)-γGlu-C18-diacid |
| SEQ ID NO:81 | Ac- DLTfHLLREVLE(Nle)K*RQEQ(cVal)AQE#AEK#NR KL(Nle)E(cVal)I-CONH2 | (AEEA)-(AEEA)-γGlu-C18-diacid |
| SEQ ID NO:82 | Ac- DLTfHLLREVLE(Nle)K*RTEQ(cVal)AQE#AEK#NRK L(Nle)E(cVal)I-CONH2 | (AEEA)-(AEEA)-γGlu-C18-diacid |
| SEQ ID NO:83 | Ac- DLTfHLLREVLE(Nle)K*REEQ(cVal)AQE#AEK#NRK L(Nle)E(cVal)I-CONH2 | (AEEA)-(AEEA)-γGlu-C20-diacid |
| SEQ ID NO:84 | Ac- DLTfHLLREVLE(Nle)EK*EEQ(cVal)AQE#AEK#NRK L(Nle)E(cVal)I-CONH2 | Gln-(AEEA)-(AEEA)-γGlu-C18-diacid |
| SEQ ID NO:85 | Ac- DLTfHLLREVLE(Nle)TK*EEQ(cVal)AQE#AEK#NRK L(Nle)E(cVal)I-CONH2 | (AEEA)-(AEEA)-γGlu-C18-diacid |
| SEQ ID NO:86 | Ac- DLTfHLLREVLE(Nle)QK*EEQ(cVal)AQE#AEK#NR KL(Nle)E(cVal)I-CONH2 | (AEEA)-(AEEA)-γGlu-C18-diacid |
| SEQ ID NO:87 | Ac- DLTfHLLREVLE(Nle)AK*EEQ(cVal)AQE#AEK#NR KL(Nle)E(cVal)I-CONH2 | (AEEA)-(AEEA)-γGlu-C18-diacid |
| SEQ ID NO:88 | Ac- DLTfHLLREVLE(Nle)EK*EEQ(cVal)AQE#AEK#NRK L(Nle)E(cVal)I-CONH2 | (AEEA)-(AEEA)-γGlu-γGlu-C18-diacid |
| SEQ ID NO:89 | Ac- DLTfHLLREVLE(Nle)QK*EEQ(cVal)AQE#AEK#NR KL(Nle)E(cVal)A-CONH2 | (AEEA)-(AEEA)-γGlu-C18-diacid |
| SEQ ID NO:90 | Ac- DLTfHLLREVLE(Nle)QK*AEQ(cVal)AQE#AEK#NR KL(Nle)E(cVal)A-CONH2 | (AEEA)-(AEEA)-γGlu-C18-diacid |
| SEQ ID NO:91 | Ac- DLTfHLLREVLE(Nle)TK*EEQ(cVal)AQE#AEK#NRK L(Nle)E(cVal)A-CONH2 | (AEEA)-(AEEA)-γGlu-C18-diacid |
| SEQ ID NO:92 | Ac- DLTfHLLREVLE(Nle)TK*AEQ(cVal)AQE#AEK#NR KL(Nle)E(cVal)A-CONH2 | (AEEA)-(AEEA)-γGlu-C18-diacid |
| SEQ ID NO:93 | Ac- DLTfHLLREVLE(Nle)TK*AEQ(cVal)AQE#AEK#NR KL(Nle)E(cVal)A-CONH2 | (AEEA)-(AEEA)-(AEEA)-γGlu-C18-diacid |
| SEQ ID NO:94 | Ac- DLTfHLLREVLE(Nle)QK*AEQ(cVal)AQE#AEK#NR KL(Nle)E(cVal)A-CONH2 | (AEEA)-(AEEA)-(AEEA)-γGlu-C18-diacid |
| SEQ ID NO:95 | Ac- DLTfHLLREVLE(Nle)K*QEEQ(cVal)AQE#AEK#NR KL(Nle)E(cVal)A-CONH2 | (AEEA)-(AEEA)-γGlu-C18-diacid |
| SEQ ID NO:96 | Ac- DLTfHLLREVLE(Nle)K*QEEQ(cVal)AQE#AEK#NR KL(Nle)E(cVal)A-CONH2 | (AEEA)-(AEEA)-γGlu-γGlu-C18-diacid |
| SEQ ID NO:97 | Ac- DLTfHLLREVLE(Nle)K*QEEQ(cVal)AQE#AEK#NR KL(Nle)E(cVal)A-CONH2 | (AEEA)-(AEEA)-(AEEA)-γGlu-C18-diacid |
| SEQ ID NO:98 | Ac- DLTfHLLREVLE(Nle)K*QAEQ(cVal)AQE#AEK#NR KL(Nle)E(cVal)A-CONH2 | (AEEA)-(AEEA)-γGlu-C18-diacid |
| SEQ ID NO:99 | Ac- DLTfHLLREVLE(Nle)K*QEEQ(cVal)AQE#AEK#NR KL(Nle)E(cVal)I-CONH2 | (AEEA)-(AEEA)-(AEEA)-γGlu-C18-diacid |
| SEQ ID NO:100 | Ac- DLTfHLLREVLE(Nle)K*QEEQ(cVal)AQE#AEK#NR KL(Nle)E(cVal)I-CONH2 | (AEEA)-(AEEA)-γGlu-C20-diacid |
| SEQ ID NO:101 | Ac- DLTfHLLREVLE(Nle)K*REEQ(cVal)AQE#AEK#NRK L(Nle)E(cVal)A-CONH2 | (AEEA)-(AEEA)-γGlu-C20-diacid |
| SEQ ID NO:102 | Ac- DLTfHLLREVLE(Nle)K*QEEQ(cVal)AQE#AEK#NR KL(Nle)E(cVal)A-CONH2 | (AEEA)-(AEEA)-γGlu-C20-diacid |
| SEQ ID NO:103 | Ac- DLTfHLLREVLE(Nle)K*QEEQ(cVal)AQE#AEK#NR KL(Nle)E(cVal)A-CONH2 | (AEEA)-(AEEA)-(AEEA)-γGlu-C20-diacid |
| SEQ ID NO:104 | Ac- DLTfHLLREVLE(Nle)K*EREQ(cVal)AQE#AEK#NRK L(Nle)E(cVal)A-CONH2 | (AEEA)-(AEEA)-γGlu-C20-diacid |
| SEQ ID NO:105 | Ac- DLTfHLLREVLE(Nle)K*NEEQ(cVal)AQE#AEK#NR KL(Nle)EcValA-CONH2 | (AEEA)-(AEEA)-γGlu-C20-diacid |
| SEQ ID NO:106 | Ac- DLTfHLLREVLE(Nle)K*TEEQ(cVal)AQE#AEK#NRK L(Nle)E(cVal)A-CONH2 | (AEEA)-(AEEA)-γGlu-C20-diacid |

**Table B1.2 Fatty acid conjugated cyclic peptides of the disclosure.**

| **SEQ ID NO:** | **Description** | **Sequence** |
|---|---|---|
| 107 | Formula (I) | Y-Asp-R2-R3-R4-R5-R6-R7-Arg-R9-R10-R11-R12-R13-R14-R15-R16- R17-R18-R19-R20-R21-R22-R23-R24-R25-R26-R27-R28-R29-R30-R31- R32-R33-CONH2 |
| 108 | Formula (Ia) | Y-Asp-Leu-Thr-D-Phe-His-Leu-Leu-Arg-R9-Val-Leu-R12-R13-R14-R15- R16-R17-R18-R19-R20-R21-R22-R23-R24-R25-Asn-R27-R28-R29-R30- R31-R32-R33-CONH2 |
| 109 | Formula (Ib) | Ac-Asp-Leu-Thr-D-Phe-His-Leu-Leu-Arg-Glu-Val-Leu-Glu-(Nle)-R14- R15-R16-R17-Gln-cVal-Ala-Gln-R22-Ala-Glu-R25-Asn-Arg-Lys-Leu-Nle- Glu-cVal-R33-CONH2 |
| 110 | Formula (Ic) | Ac-Asp-Leu-Thr-D-Phe-His-Leu-Leu-Arg-Glu-Val-Leu-Glu-Nle-K*-R15- R16-R17-Gln-cVal-Ala-Gln-R22-Ala-Glu-R25-Asn-Arg-Lys-Leu-Nle-Glu- cVal-R33-CONH2 |
| 111 | Formula (Id) | Ac-Asp-Leu-Thr-D-Phe-His-Leu-Leu-Arg-Glu-Val-Leu-Glu-Nle-K*-R15- R16-Glu-Gln-cVal-Ala-Gln-R22-Ala-Glu-R25-Asn-Arg-Lys-Leu-Nle-Glu- cVal-R33-CONH2 |
| 112 | Formula (Ie) | Ac-Asp-Leu-Thr-D-Phe-His-Leu-Leu-Arg-Glu-Val-Leu-Glu-Nle-K*-R15- R16-Glu-Gln-cVal-Ala-Gln-R22-Ala-Glu-R25-Asn-Arg-Lys-Leu-Nle-Glu- cVal-R33-CONH2 |
| 113 | Formula (If) | Ac-Asp-Leu-Thr-D-Phe-His-Leu-Leu-Arg-Glu-Val-Leu-Glu-Nle-R14-K*- R16-Glu-Gln-cVal-Ala-Gln-R22-Ala-Glu-R25-Asn-Arg-Lys-Leu-Nle-Glu- cVal-R33-CONH2 |
| 114 | Formula (Ig) | Ac-Asp-Leu-Thr-D-Phe-His-Leu-Leu-Arg-Glu-Val-Leu-Glu-Nle-K*-Gln- R16-Glu-Gln-cVal-Ala-Gln-R22-Ala-Glu-R25-Asn-Arg-Lys-Leu-Nle-Glu- cVal-R33-CONH2 |
| 115 | Formula (Ih) | Ac-Asp-Leu-Thr-D-Phe-His-Leu-Leu-Arg-K*-Val-Leu-Glu-Nle-R14-R15- Glu-Glu-Gln-cVal-Ala-Gln-R22-Ala-Glu-R25-Asn-Arg-Lys-Leu-Nle-Glu- cVal-Ile-CONH2 |
| 116 | Formula (Ik) | X-Asp-Leu-Thr-D-Phe-His-Leu-Leu-Arg-R9-Val-Leu-R12-R13-R14-R15- R16-R17-R18-cVal-R20-R21-R22-Ala-Glu-R25-Asn-R27-Lys-R29-Nle- Glu-cVal-R33-CONH2 |
| 117 | | Y-Asp-R2-R3-R4-R5-R6-R7-Arg-R9-R10-R11-R12-R13-R14-R15-R16- R17-R18-R19-R20-R21-R22-R23-R24-R25-R26-R27-R28-R29-R30-R31- R32-R33-CONH2 |

### Pharmacology and Utility

Stress and the CRF/urocortin system can modulate the behavioral, autonomic, immune, reproductive, and visceral responses to stress. Thus, the peptides of the disclosure have broad applicability to multiple body systems and disease pathways.

Corticotropin releasing factor profoundly stimulates the pituitary-adrenocortical axis, and CRF receptor antagonists are useful to inhibit the functions of this axis in certain types of patients experiencing high ACTH and endogenous glucocorticoid production. For example, CRF receptor antagonists can be useful in regulating pituitary-adrenal function in patients having pituitary Cushing's disease or any CRF-R-bearing tumor.

In some embodiments, the present disclosure provides the use of a peptide comprising or consisting of SEQ ID NO: 117, or of any one of formulae (I), (Ia), (Ib), (Ic), (Id), (Ie), (If), (Ig), (Ih), or (Ik), or a pharmaceutically acceptable salt thereof, in regulating pituitary-adrenal function in patients having pituitary Cushing's disease or any CRF-R-bearing tumor. In another aspect, the present disclosure provides a peptide comprising or consisting of SEQ ID NO: 117, or of any one of formulae (I), (Ia), (Ib), (Ic), (Id), (Ie), (If), (Ig), (Ih), or (Ik), or a pharmaceutically acceptable salt thereof, for use in regulating pituitary-adrenal function in patients having pituitary Cushing's disease or any CRF-R-bearing tumor.

In some embodiments, the present disclosure provides the use of a peptide comprising or consisting of any one of the peptides listed in Tables B1.1 and B1.2, or a pharmaceutically acceptable salt thereof, in regulating pituitary-adrenal function in patients having pituitary Cushing's disease or any CRF-R-bearing tumor. In another aspect, the present disclosure provides a peptide comprising or consisting of any one of the peptides listed in Tables B1.1 and B1.2, or a pharmaceutically acceptable salt thereof, for use in regulating pituitary-adrenal function in patients having pituitary Cushing's disease or any CRF-R-bearing tumor
In some embodiments, the present disclosure provides the use of the peptide comprising or consisting of SEQ ID NO: 50, in regulating pituitary-adrenal function in patients having pituitary Cushing's disease or any CRF-R-bearing tumor. In some embodiments, the peptide is a pharmaceutically acceptable salt thereof. In another aspect, the present disclosure provides the peptide comprising or consisting of SEQ ID NO: 50, for use in regulating pituitary-adrenal function in patients having pituitary Cushing's disease or any CRF-R-bearing tumor. In some embodiments, the peptide is a pharmaceutically acceptable salt thereof.

In some embodiments, the present disclosure provides the use of the peptide comprising or consisting of SEQ ID NO: 95, in regulating pituitary-adrenal function in patients having pituitary Cushing's disease or any CRF-R-bearing tumor. In some embodiments, the peptide is a pharmaceutically acceptable salt thereof. In another aspect, the present disclosure provides the peptide comprising or consisting of SEQ ID NO: 95, for use in regulating pituitary-adrenal function in patients having pituitary Cushing's disease or any CRF-R-bearing tumor. In some embodiments, the peptide is a pharmaceutically acceptable salt thereof.

In some embodiments, the present disclosure provides the use of the peptide comprising or consisting of SEQ ID NO: 97, in regulating pituitary-adrenal function in patients having pituitary Cushing's disease or any CRF-R-bearing tumor. In some embodiments, the peptide is a pharmaceutically acceptable salt thereof. In another aspect, the present disclosure provides the peptide comprising or consisting of SEQ ID NO: 97, for use in regulating pituitary-adrenal function in patients having pituitary Cushing's disease or any CRF-R-bearing tumor. In some embodiments, the peptide is a pharmaceutically acceptable salt thereof.

In some embodiments, the present disclosure provides the use of the peptide comprising or consisting of SEQ ID NO: 101, in regulating pituitary-adrenal function in patients having pituitary Cushing's disease or any CRF-R-bearing tumor. In some embodiments, the peptide is a pharmaceutically acceptable salt thereof. In another aspect, the present disclosure provides the peptide comprising or consisting of SEQ ID NO: 101, for use in regulating pituitary-adrenal function in patients having pituitary Cushing's disease or any CRF-R-bearing tumor. In some embodiments, the peptide is a pharmaceutically acceptable salt thereof.

A peptide of the present disclosure can be used to reduce the effects of ACTH, β-endorphin, β-lipotropin, other pro opiomelanocortin gene products and corticosterone on the brain to thereby influence memory, mood, pain appreciation, etc., and more specifically, alertness, depression and/or anxiety, as well as to modulate the immune system, gastrointestinal tract and adrenocortical growth and function. In some embodiments, the present disclosure provides the use of a peptide comprising or consisting of SEQ ID NO: 117, or of any one of formulae (I), (Ia), (Ib), (Ic), (Id), (Ie), (If), (Ig), (Ih), or (Ik), or a pharmaceutically acceptable salt thereof, to reduce the effects of ACTH, β-endorphin, β-lipotropin, other pro opiomelanocortin gene products and corticosterone on the brain to thereby influence memory, mood, pain appreciation, etc., and more specifically, alertness, depression and/or anxiety, as well as to modulate the immune system, gastrointestinal tract and adrenocortical growth and function. In some embodiments, the present disclosure provides a peptide comprising or consisting of SEQ ID NO: 117, or of any one of formulae (I), (Ia), (Ib), (Ic), (Id), (Ie), (If), (Ig), (Ih), or (Ik), or a pharmaceutically acceptable salt thereof, for use in reducing the effects of ACTH, β-endorphin, β-lipotropin, other pro opiomelanocortin gene products and corticosterone on the brain to thereby influence memory, mood, pain appreciation, etc., and more specifically, alertness, depression and/or anxiety, as well as to modulate the immune system, gastrointestinal tract and adrenocortical growth and function.

In some embodiments, the present disclosure provides the use of a peptide comprising or consisting of any one of the peptides listed in Tables B1.1 and B1.2, or a pharmaceutically acceptable salt thereof, to reduce the effects of ACTH, β-endorphin, β-lipotropin, other pro opiomelanocortin gene products and corticosterone on the brain to thereby influence memory, mood, pain appreciation, etc., and more specifically, alertness, depression and/or anxiety, as well as to modulate the immune system, gastrointestinal tract and adrenocortical growth and function. The present disclosure provides the a peptide comprising or consisting of any one of the peptides listed in Tables B1.1 and B1.2, or a pharmaceutically acceptable salt thereof, for use in the treatment of a disease in which reducing the effects of ACTH, β-endorphin, β-lipotropin, other pro opiomelanocortin gene products and corticosterone on the brain thereby influences memory, mood, pain appreciation, etc., and more specifically, alertness, depression and/or anxiety, as well as to modulate the immune system, gastrointestinal tract and adrenocortical growth and function.

In some embodiments, the present disclosure provides the use of the peptide comprising or consisting of SEQ ID NO: 50, to reduce the effects of ACTH, β-endorphin, β-lipotropin, other pro opiomelanocortin gene products and corticosterone on the brain to thereby influence memory, mood, pain appreciation, etc., and more specifically, alertness, depression and/or anxiety, as well as to modulate the immune system, gastrointestinal tract and adrenocortical growth and function. In some embodiments, the peptide is a pharmaceutically acceptable salt thereof. The present disclosure provides the peptide comprising or consisting of SEQ ID NO: 50, for use in the treatment of a disease in which reducing the effects of ACTH, β-endorphin, β-lipotropin, other pro opiomelanocortin gene products and corticosterone on the brain thereby influences memory, mood, pain appreciation, etc., and more specifically, alertness, depression and/or anxiety, as well as to modulate the immune system, gastrointestinal tract and adrenocortical growth and function. In some embodiments, the peptide is a pharmaceutically acceptable salt thereof.

In some embodiments, the present disclosure provides the use of the peptide comprising or consisting of SEQ ID NO: 95, to reduce the effects of ACTH, β-endorphin, β-lipotropin, other pro opiomelanocortin gene products and corticosterone on the brain to thereby influence memory, mood, pain appreciation, etc., and more specifically, alertness, depression and/or anxiety, as well as to modulate the immune system, gastrointestinal tract and adrenocortical growth and function. In some embodiments, the peptide is a pharmaceutically acceptable salt thereof. The present disclosure provides the peptide the peptide comprising or consisting of SEQ ID NO: 95, for use in the treatment of a disease in which reducing the effects of ACTH, β-endorphin, β-lipotropin, other pro opiomelanocortin gene products and corticosterone on the brain thereby influences memory, mood, pain appreciation, etc., and more specifically, alertness, depression and/or anxiety, as well as to modulate the immune system, gastrointestinal tract and adrenocortical growth and function. In some embodiments, the peptide is a pharmaceutically acceptable salt thereof.

In some embodiments, the present disclosure provides the use of the peptide comprising or consisting of SEQ ID NO: 97, to reduce the effects of ACTH, β-endorphin, β-lipotropin, other pro opiomelanocortin gene products and corticosterone on the brain to thereby influence memory, mood, pain appreciation, etc., and more specifically, alertness, depression and/or anxiety, as well as to modulate the immune system, gastrointestinal tract and adrenocortical growth and function. In some embodiments, the peptide is a pharmaceutically acceptable salt thereof. The present disclosure provides the peptide the peptide comprising or consisting of SEQ ID NO: 97, for use in the treatment of a disease in which reducing the effects of ACTH, β-endorphin, β-lipotropin, other pro opiomelanocortin gene products and corticosterone on the brain thereby influences memory, mood, pain appreciation, etc., and more specifically, alertness, depression and/or anxiety, as well as to modulate the immune system, gastrointestinal tract and adrenocortical growth and function. In some embodiments, the peptide is a pharmaceutically acceptable salt thereof.

In some embodiments, the present disclosure provides the use of the peptide comprising or consisting of SEQ ID NO: 101, to reduce the effects of ACTH, β-endorphin, β-lipotropin, other pro opiomelanocortin gene products and corticosterone on the brain to thereby influence memory, mood, pain appreciation, etc., and more specifically, alertness, depression and/or anxiety, as well as to modulate the immune system, gastrointestinal tract and adrenocortical growth and function. In some embodiments, the peptide is a pharmaceutically acceptable salt thereof. The present disclosure provides the peptide the peptide comprising or consisting of SEQ ID NO: 101, for use in the treatment of a disease in which reducing the effects of ACTH, β-endorphin, β-lipotropin, other pro opiomelanocortin gene products and corticosterone on the brain thereby influences memory, mood, pain appreciation, etc., and more specifically, alertness, depression and/or anxiety, as well as to modulate the immune system, gastrointestinal tract and adrenocortical growth and function. In some embodiments, the peptide is a pharmaceutically acceptable salt thereof.

A peptide of the present disclosure can be of use for decreasing blood flow to the gastrointestinal tract of mammals, particularly humans. A peptide of the present disclosure can be effective to modulate gastrointestinal functions and treat gastrointestinal diseases, including, but not limited to abdominal bowel syndrome and inflammatory diseases such as inflammatory bowel disease (IBD) and irritable bowel syndrome (IBS). A peptide of the present disclosure can be used to treat or ameliorate multiple aspects of gastrointestinal diseases including, but not limited to, gut motility, immune cell activity, barrier function / gut permeability, and pain.

In some embodiments, the present disclosure provides the use of a peptide comprising or consisting of SEQ ID NO: 117, or of any one of formulae (I), (Ia), (Ib), (Ic), (Id), (Ie), (If), (Ig), (Ih), or (Ik), or a pharmaceutically acceptable salt thereof, to treat or ameliorate multiple aspects of gastrointestinal diseases, for example, inflammatory bowel disease (IBD) and irritable bowel syndrome (IBS), including, but not limited to, gut motility, immune cell activity, barrier function / gut permeability, and pain. The present disclosure provides a peptide comprising or consisting of SEQ ID NO: 117, or of any one of formulae (I), (Ia), (Ib), (Ic), (Id), (Ie), (If), (Ig), (Ih), or (Ik), or a pharmaceutically acceptable salt thereof, for use in the treatment of gastrointestinal diseases, for example, inflammatory bowel disease (IBD) and irritable bowel syndrome (IBS), including, but not limited to, gut motility, immune cell activity, barrier function / gut permeability, and pain.

In some embodiments, the present disclosure provides the use of a peptide comprising or consisting of any one of the peptides listed in Tables B1.1 and B1.2, or a pharmaceutically acceptable salt thereof, to treat or ameliorate multiple aspects of gastrointestinal diseases, for example, inflammatory bowel disease (IBD) and irritable bowel syndrome (IBS), including, but not limited to, gut motility, immune cell activity, barrier function / gut permeability, and pain. The present disclosure provides a peptide comprising or consisting of any one of the peptides listed in Tables B1.1 and B1.2, or a pharmaceutically acceptable salt thereof, for use in the treatment of gastrointestinal diseases, for example, inflammatory bowel disease (IBD) and irritable bowel syndrome (IBS), including, but not limited to, gut motility, immune cell activity, barrier function / gut permeability, and pain.

In some embodiments, the present disclosure provides the use of the peptide comprising or consisting of SEQ ID NO: 50, to treat or ameliorate multiple aspects of gastrointestinal diseases, for example, inflammatory bowel disease (IBD) and irritable bowel syndrome (IBS), including, but not limited to, gut motility, immune cell activity, barrier function / gut permeability, and pain. In some embodiments, the peptide is a pharmaceutically acceptable salt thereof. The present disclosure provides a peptide comprising or consisting of SEQ ID NO: 50, for use in the treatment of gastrointestinal diseases, for example, inflammatory bowel disease (IBD) and irritable bowel syndrome (IBS), including, but not limited to, gut motility, immune cell activity, barrier function / gut permeability, and pain. In some embodiments, the peptide is a pharmaceutically acceptable salt thereof.

In some embodiments, the present disclosure provides the use of the peptide comprising or consisting of SEQ ID NO: 95, to treat or ameliorate multiple aspects of gastrointestinal diseases, for example, inflammatory bowel disease (IBD) and irritable bowel syndrome (IBS), including, but not limited to, gut motility, immune cell activity, barrier function / gut permeability, and pain. In some embodiments, the peptide is a pharmaceutically acceptable salt thereof. The present disclosure provides a peptide comprising or consisting of SEQ ID NO: 95, for use in the treatment of gastrointestinal diseases, for example, inflammatory bowel disease (IBD) and irritable bowel syndrome (IBS), including, but not limited to, gut motility, immune cell activity, barrier function / gut permeability, and pain. In some embodiments, the peptide is a pharmaceutically acceptable salt thereof.

In some embodiments, the present disclosure provides the use of the peptide comprising or consisting of SEQ ID NO: 97, to treat or ameliorate multiple aspects of gastrointestinal diseases, for example, inflammatory bowel disease (IBD) and irritable bowel syndrome (IBS), including, but not limited to, gut motility, immune cell activity, barrier function / gut permeability, and pain. In some embodiments, the peptide is a pharmaceutically acceptable salt thereof. The present disclosure provides a peptide comprising or consisting of SEQ ID NO: 97, for use in the treatment of gastrointestinal diseases, for example, inflammatory bowel disease (IBD) and irritable bowel syndrome (IBS), including, but not limited to, gut motility, immune cell activity, barrier function / gut permeability, and pain. In some embodiments, the peptide is a pharmaceutically acceptable salt thereof.

In some embodiments, the present disclosure provides the use of the peptide comprising or consisting of SEQ ID NO: 101, to treat or ameliorate multiple aspects of gastrointestinal diseases, for example, inflammatory bowel disease (IBD) and irritable bowel syndrome (IBS), including, but not limited to, gut motility, immune cell activity, barrier function / gut permeability, and pain. In some embodiments, the peptide is a pharmaceutically acceptable salt thereof. The present disclosure provides a peptide comprising or consisting of SEQ ID NO: 100, for use in the treatment of gastrointestinal diseases, for example, inflammatory bowel disease (IBD) and irritable bowel syndrome (IBS), including, but not limited to, gut motility, immune cell activity, barrier function / gut permeability, and pain. In some embodiments, the peptide is a pharmaceutically acceptable salt thereof.

Pain: CRF-driven inflammation in the gastrointestinal tract and around enteric nerve endings leads to visceral pain. Peptides of the disclosure, therefore, can be useful in reducing visceral pain in, for example, IBS.

In some embodiments, the present disclosure provides the use of a peptide comprising or consisting of SEQ ID NO: 117, or of any one of formulae (I), (Ia), (Ib), (Ic), (Id), (Ie), (If), (Ig), (Ih), or (Ik), or a pharmaceutically acceptable salt thereof, in reducing visceral pain in IBS. The present disclosure provides a peptide comprising or consisting of SEQ ID NO: 117, or of any one of formulae (I), (Ia), (Ib), (Ic), (Id), (Ie), (If), (Ig), (Ih), or (Ik), or a pharmaceutically acceptable salt thereof, for use in the treatment of a patient suffering from IBS, by reducing visceral pain in IBS.

In some embodiments, the present disclosure provides the use of a peptide comprising or consisting of any one of the peptides listed in Tables B1.1 and B1.2, or a pharmaceutically acceptable salt thereof, in reducing visceral pain in IBS. The present disclosure provides a peptide comprising or consisting of any one of the peptides listed in Tables B1.1 and B1.2, or a pharmaceutically acceptable salt thereof, for use in the treatment of a patient suffering from IBS, by reducing visceral pain in IBS.

In some embodiments, the present disclosure provides the use of the peptide comprising or consisting of SEQ ID NO: 50, in reducing visceral pain in IBS. In some embodiments, the peptide is a pharmaceutically acceptable salt thereof. The present disclosure provides the use of the peptide comprising or consisting of SEQ ID NO: 50, for use in the treatment of a patient suffering from IBS, by reducing visceral pain in IBS. In some embodiments, the peptide is a pharmaceutically acceptable salt thereof.

In some embodiments, the present disclosure provides the use of the peptide comprising or consisting of SEQ ID NO: 95, in reducing visceral pain in IBS. In some embodiments, the peptide is a pharmaceutically acceptable salt thereof. The present disclosure provides the use of the peptide comprising or consisting of SEQ ID NO: 95, for use in the treatment of a patient suffering from IBS, by reducing visceral pain in IBS. In some embodiments, the peptide is a pharmaceutically acceptable salt thereof.

In some embodiments, the present disclosure provides the use of the peptide comprising or consisting of SEQ ID NO: 97, in reducing visceral pain in IBS. In some embodiments, the peptide is a pharmaceutically acceptable salt thereof. The present disclosure provides the use of the peptide comprising or consisting of SEQ ID NO: 97, for use in the treatment of a patient suffering from IBS, by reducing visceral pain in IBS. In some embodiments, the peptide is a pharmaceutically acceptable salt thereof.

In some embodiments, the present disclosure provides the use of the peptide comprising or consisting of SEQ ID NO: 101, in reducing visceral pain in IBS. In some embodiments, the peptide is a pharmaceutically acceptable salt thereof. The present disclosure provides the use of the peptide comprising or consisting of SEQ ID NO: 101, for use in the treatment of a patient suffering from IBS, by reducing visceral pain in IBS. In some embodiments, the peptide is a pharmaceutically acceptable salt thereof.

Congenital adrenal hyperplasia (CAH) refers to a group of genetic disorders that affect the adrenal glands and their ability to produce a variety of hormones, for example: cortisol which regulates the response to illness or stress; mineralocorticoids, such as aldosterone, which regulate sodium and potassium levels; and androgens, such as testosterone, which are required for growth and development in both males and females. There are two types of CAH: classic and non-classic. Classic CAH is the rare, more severe form while the non-classic CAH is milder and more common. Classic congenital adrenal hyperplasia (CAH) is a disease that includes a group of autosomal recessive disorders that result in an enzyme deficiency that alters the production of adrenal steroids due to 21-hydroxylase deficiency, a condition that results in little or no cortisol biosynthesis. One clinical manifestation of the absence of cortisol is the lack of feedback inhibition of pituitary adrenocorticotropic hormone (ACTH) secretion. Increased ACTH levels cause adrenal hyperplasia, and the enzyme mutation causes a shunting of cortisol precursor steroids to alternate pathways. Most notably, the shunting of androgens leads to virilization and other developmental complications in females and the over-accumulation of ACTH is associated with the formation of testicular adrenal rest tumors in males. In addition, since the same enzyme (21-hydroxylase) is used in the pathway for the biosynthesis of the mineralocorticoids, a number of these patients suffer from aldosterone deficiency which can result in dehydration and death due to salt-wasting. The prevalence of classic 21-hydroxylase deficiency CAH in the US general population, based on newborn screening, has been documented as 1:10,000 to 1:20,800 (Trakakis, et al., "An update to 21-hydroxylase deficient congenital adrenal hyperplasia," Gynecol. Endocrinol. (2010) 26(1):63-71; Hertzberg et al., "Birth prevalence rates of newborn screening disorders in relation to screening practices in the United States," J. Pediatr. (2011) 159(4):555-560).

Pediatric patients from birth through adolescence, and females in particular, appear to be the most vulnerable population of CAH sufferers and represent the subgroup of patients with the greatest unmet medical need (Cheng and Speiser, "Treatment outcomes in congenital adrenal hyperplasia," Adv. Pediatr. (2012) 59(1):269-281; Merke and Poppas, "Management of adolescents with congenital adrenal hyperplasia," Lancet Diabetes Endocrinol. (2013) 1(4):341-352). Excessive androgen production in these younger patients results in early onset puberty and adrenarche, changes in skeletal maturation patterns, short stature caused by premature growth plate fusion, as well as significant hirsutism and acne problems. While survival is properly ensured through steroid replacement strategies based on physiologic dosing of glucocorticoids (e.g., hydrocortisone) and mineralocorticoids (e.g., fludrocortisone), these doses are often inadequate to suppress the accumulating ACTH and overproduction of progestogens and androgens (e.g., 17-hydroxyprogesterone [17-OHP], androstenedione, and testosterone). The uncontrolled symptoms of androgen excess, indeed, have a substantial impact on the day-to-day functioning and development of these patients.

Currently, exogenous corticosteroids are the standard of care for treating patients with classic CAH. This treatment is used to correct the cortisol deficiency and reduce the excessive ACTH levels and androgen excess. However, the dose and duration of steroid use required to suppress ACTH are typically well above the normal physiological level used for cortisol replacement alone (as in patients with Addison's disease). This increased exposure to glucocorticoids can lead to iatrogenic Cushing's syndrome, increased cardiovascular risk factors, glucose intolerance, reduced growth velocity, and decreased bone mineral density in CAH patients (Elnecave et al., "Bone mineral density in girls with classical congenital adrenal hyperplasia due to CYP21 deficiency," J. Pediatr. Endocrinol. Metab. (2008) 21(12):1155-1162; King et al., "Long-term corticosteroid replacement and bone mineral density in adult women with classical congenital adrenal hyperplasia," J. Clin. Endocrinol. Metab. (2006) 91(3):865-869; Migeon and Wisniewski, "Congenital adrenal hyperplasia owing to 21-hydroxylase deficiency. Growth, development, and therapeutic considerations," Endocrinol. Metab. Clin. North Am. (2001) 30(1):193-206).

Corticotropin releasing factor (CRF) is a hypothalamic hormone released directly into the hypophyseal portal vasculature and acts on specific corticotropin releasing factor 1 (CRF1) receptors on corticotropes in the anterior pituitary to stimulate the release of ACTH. Blockade of these receptors has been shown to decrease the release of ACTH in both animals and humans. High glucocorticoid doses indirectly manage adrenal androgens by lowering CRF release. This leads to reduced CRF₁ receptor activation, reduced ACTH release from the pituitary, and/or lower adrenal androgens. Antagonizing CRF₁ receptors or other HPA axis receptors through a glucocorticoid-independent pathway could more directly reduce ACTH, and/or reduce adrenal androgens. Reducing ACTH and adrenal androgen overproduction more directly through a glucocorticoid-independent pathway could allow for lower glucocorticoid doses and reduce the risk of glucocorticoid-related comorbidities.

Therefore, cyclic peptides of the disclosure that block CRF1 receptors have the potential to directly inhibit the excessive ACTH release that occurs in CAH and thereby allow for normalization of androgen production while using lower, more physiologic doses of hydrocortisone.

One aspect of the present disclosure provides a method of treating congenital adrenal hyperplasia (CAH), in a subject in need thereof, comprising administering to the subject a therapeutically effective amount of a CRF receptor antagonist fatty acid conjugated cyclic peptide comprising or consisting of SEQ ID NO: 117, or of Formula (I), or pharmaceutically acceptable salts thereof.

In a further aspect is a method of treating congenital adrenal hyperplasia (CAH), in a subject in need thereof, comprising administering to the subject a therapeutically effective amount of a CRF receptor antagonist fatty acid conjugated cyclic peptide comprising or consisting of SEQ ID NO: 117, or of Formula (I), or a pharmaceutically acceptable salt thereof, wherein the treating results in reducing the amount of a glucocorticoid or mineralocorticoid (such as hydrocortisone (HC), prednisone, prednisolone, dexamethasone, or fludrocortisone) if administered to the subject by at least 5%, 10%, 15%, 20%, 25%, 30%, 35%, 40%, 45%, 50%, 55%, or 60% compared to the standard of care or recommended daily dose of the glucocorticoid or mineralocorticoid for treating CAH for a subject not treated with CRF receptor antagonist conjugated cyclic peptide of the present disclosure. In certain embodiments, the present disclosure provides administering to the subject the peptide and the reduced amount of the glucocorticoid or mineralocorticoid, e.g., as a combination therapy (e.g., without being limited to the disclosure herein, concurrently as separate therapies or by recommended/prescribed dosage regimens) for treating CAH.

In a further aspect is a method of treating congenital adrenal hyperplasia (CAH), in a subject in need thereof, comprising administering to the subject a therapeutically effective amount of a peptide comprising or consisting of any one of formulae (Ia), (Ib), (Ic), (Id), (Ie), (If), (Ig), (Ih), or (Ik), or a pharmaceutically acceptable salt thereof, wherein the treating results in reducing the amount of a glucocorticoid or mineralocorticoid (such as hydrocortisone (HC), prednisone, prednisolone, dexamethasone, or fludrocortisone) if administered to the subject by at least 5%, 10%, 15%, 20%, 25%, 30%, 35%, 40%, 45%, 50%, 55%, or 60% compared to the standard of care or recommended daily dose of the glucocorticoid or mineralocorticoid for treating CAH for a subject not treated with CRF receptor antagonist conjugated cyclic peptide of the present disclosure. In certain embodiments, the present disclosure provides administering to the subject the peptide and the reduced amount of the glucocorticoid or mineralocorticoid, e.g., as a combination therapy (e.g., without being limited to the disclosure herein, concurrently as separate therapies or by recommended/prescribed dosage regimens) for treating CAH.

In a further aspect is a method of treating congenital adrenal hyperplasia (CAH), in a subject in need thereof, comprising administering to the subject a therapeutically effective amount of any one of the peptides listed in Tables B1.1 and B1.2, or a pharmaceutically acceptable salt thereof, wherein the treating results in reducing the amount of a glucocorticoid or mineralocorticoid (such as hydrocortisone (HC), prednisone, prednisolone, dexamethasone, or fludrocortisone) if administered to the subject by at least 5%, 10%, 15%, 20%, 25%, 30%, 35%, 40%, 45%, 50%, 55%, or 60% compared to the standard of care or recommended daily dose of the glucocorticoid or mineralocorticoid for treating CAH for a subject not treated with CRF receptor antagonist conjugated cyclic peptide of the present disclosure. In certain embodiments, the present disclosure provides administering to the subject the peptide and the reduced amount of the glucocorticoid or mineralocorticoid, e.g., as a combination therapy (e.g., without being limited to the disclosure herein, concurrently as separate therapies or by recommended/prescribed dosage regimens) for treating CAH.

In a further aspect is a method of treating congenital adrenal hyperplasia (CAH), in a subject in need thereof, comprising administering to the subject a therapeutically effective amount of the peptide comprising or consisting of SEQ ID NO: 50, wherein the treating results in reducing the amount of a glucocorticoid or mineralocorticoid (such as hydrocortisone (HC), prednisone, prednisolone, dexamethasone, or fludrocortisone) if administered to the subject by at least 5%, 10%, 15%, 20%, 25%, 30%, 35%, 40%, 45%, 50%, 55%, or 60% compared to the standard of care or recommended daily dose of the glucocorticoid or mineralocorticoid for treating CAH for a subject not treated with CRF receptor antagonist conjugated cyclic peptide of the present disclosure. In some embodiments, the peptide is a pharmaceutically acceptable salt thereof. In certain embodiments, the present disclosure provides administering to the subject the peptide and the reduced amount of the glucocorticoid or mineralocorticoid, e.g., as a combination therapy (e.g., without being limited to the disclosure herein, concurrently as separate therapies or by recommended/prescribed dosage regimens) for treating CAH.

In a further aspect is a method of treating congenital adrenal hyperplasia (CAH), in a subject in need thereof, comprising administering to the subject a therapeutically effective amount of the peptide comprising or consisting of SEQ ID NO: 95, wherein the treating results in reducing the amount of a glucocorticoid or mineralocorticoid (such as hydrocortisone (HC), prednisone, prednisolone, dexamethasone, or fludrocortisone) if administered to the subject by at least 5%, 10%, 15%, 20%, 25%, 30%, 35%, 40%, 45%, 50%, 55%, or 60% compared to the standard of care or recommended daily dose of the glucocorticoid or mineralocorticoid for treating CAH for a subject not treated with CRF receptor antagonist conjugated cyclic peptide of the present disclosure. In some embodiments, the peptide is a pharmaceutically acceptable salt thereof. In certain embodiments, the present disclosure provides administering to the subject the peptide and the reduced amount of the glucocorticoid or mineralocorticoid, e.g., as a combination therapy (e.g., without being limited to the disclosure herein, concurrently as separate therapies or by recommended/prescribed dosage regimens) for treating CAH.

In a further aspect is a method of treating congenital adrenal hyperplasia (CAH), in a subject in need thereof, comprising administering to the subject a therapeutically effective amount of the peptide comprising or consisting of SEQ ID NO: 97, wherein the treating results in reducing the amount of a glucocorticoid or mineralocorticoid (such as hydrocortisone (HC), prednisone, prednisolone, dexamethasone, or fludrocortisone) if administered to the subject by at least 5%, 10%, 15%, 20%, 25%, 30%, 35%, 40%, 45%, 50%, 55%, or 60% compared to the standard of care or recommended daily dose of the glucocorticoid or mineralocorticoid for treating CAH for a subject not treated with CRF receptor antagonist conjugated cyclic peptide of the present disclosure. In some embodiments, the peptide is a pharmaceutically acceptable salt thereof. In certain embodiments, the present disclosure provides administering to the subject the peptide and the reduced amount of the glucocorticoid or mineralocorticoid, e.g., as a combination therapy (e.g., without being limited to the disclosure herein, concurrently as separate therapies or by recommended/prescribed dosage regimens) for treating CAH.

In a further aspect is a method of treating congenital adrenal hyperplasia (CAH), in a subject in need thereof, comprising administering to the subject a therapeutically effective amount of the peptide comprising or consisting of SEQ ID NO: 101, wherein the treating results in reducing the amount of a glucocorticoid or mineralocorticoid (such as hydrocortisone (HC), prednisone, prednisolone, dexamethasone, or fludrocortisone) if administered to the subject by at least 5%, 10%, 15%, 20%, 25%, 30%, 35%, 40%, 45%, 50%, 55%, or 60% compared to the standard of care or recommended daily dose of the glucocorticoid or mineralocorticoid for treating CAH for a subject not treated with CRF receptor antagonist conjugated cyclic peptide of the present disclosure. In some embodiments, the peptide is a pharmaceutically acceptable salt thereof. In certain embodiments, the present disclosure provides administering to the subject the peptide and the reduced amount of the glucocorticoid or mineralocorticoid, e.g., as a combination therapy (e.g., without being limited to the disclosure herein, concurrently as separate therapies or by recommended/prescribed dosage regimens) for treating CAH.

In a further aspect is a method of treating congenital adrenal hyperplasia (CAH), in a subject in need thereof, comprising administering to the subject a therapeutically effective amount of a CRF receptor antagonist fatty acid conjugated cyclic peptide comprising or consisting of SEQ ID NO: 117, or of Formula (I), or pharmaceutically acceptable salts thereof, wherein the treating results in reducing the amount of a glucocorticoid or mineralocorticoid (such as hydrocortisone (HC), prednisone, prednisolone, dexamethasone, or fludrocortisone) if administered to the subject by at least 5%, 10%, 15%, 20%, 25%, 30%, 35%, 40%, 45%, 50%, 55%, or 60% compared to the standard of care or recommended daily dose of the glucocorticoid or mineralocorticoid for treating CAH for a subject not treated with CRF receptor antagonist conjugated cyclic peptide of the present disclosure. In certain embodiments, the present disclosure provides administering to the subject the peptide and the reduced amount of the glucocorticoid or mineralocorticoid, e.g., as a combination therapy (e.g., without being limited to the disclosure herein, concurrently as separate therapies or by recommended/prescribed dosage regimens) for treating CAH.

In a further aspect is a method of treating congenital adrenal hyperplasia (CAH), in a subject in need thereof, comprising administering to the subject a therapeutically effective amount of a CRF receptor antagonist fatty acid conjugated cyclic peptide comprising or consisting of any one of formulae (Ia), (Ib), (Ic), (Id), (Ie), (If), (Ig), (Ih), or (Ik), or a pharmaceutically acceptable salt thereof, wherein the treating results in reducing the amount of a glucocorticoid or mineralocorticoid (such as hydrocortisone (HC), prednisone, prednisolone, dexamethasone, or fludrocortisone) if administered to the subject by at least 5%, 10%, 15%, 20%, 25%, 30%, 35%, 40%, 45%, 50%, 55%, or 60% compared to the standard of care or recommended daily dose of the glucocorticoid or mineralocorticoid for treating CAH for a subject not treated with CRF receptor antagonist conjugated cyclic peptide of the present disclosure. In certain embodiments, the present disclosure provides administering to the subject the peptide and the reduced amount of the glucocorticoid or mineralocorticoid, e.g., as a combination therapy (e.g., without being limited to the disclosure herein, concurrently as separate therapies or by recommended/prescribed dosage regimens) for treating CAH.

In a further aspect is a method of treating congenital adrenal hyperplasia (CAH), in a subject in need thereof, comprising administering to the subject a therapeutically effective amount of a CRF receptor antagonist fatty acid conjugated cyclic peptide of any one of the peptides listed in Tables B1.1 and B1.2, or a pharmaceutically acceptable salt thereof, wherein the treating results in reducing the amount of a glucocorticoid or mineralocorticoid (such as hydrocortisone (HC), prednisone, prednisolone, dexamethasone, or fludrocortisone) if administered to the subject by at least 5%, 10%, 15%, 20%, 25%, 30%, 35%, 40%, 45%, 50%, 55%, or 60% compared to the standard of care or recommended daily dose of the glucocorticoid or mineralocorticoid for treating CAH for a subject not treated with CRF receptor antagonist conjugated cyclic peptide of the present disclosure. In certain embodiments, the present disclosure provides administering to the subject the peptide and the reduced amount of the glucocorticoid or mineralocorticoid, e.g., as a combination therapy (e.g., without being limited to the disclosure herein, concurrently as separate therapies or by recommended/prescribed dosage regimens) for treating CAH.

In a further aspect is a method of treating congenital adrenal hyperplasia (CAH), in a subject in need thereof, comprising administering to the subject a therapeutically effective amount of a CRF receptor antagonist fatty acid conjugated cyclic peptide comprising or consisting of SEQ ID NO: 50, wherein the treating results in reducing the amount of a glucocorticoid or mineralocorticoid (such as hydrocortisone (HC), prednisone, prednisolone, dexamethasone, or fludrocortisone) if administered to the subject by at least 5%, 10%, 15%, 20%, 25%, 30%, 35%, 40%, 45%, 50%, 55%, or 60% compared to the standard of care or recommended daily dose of the glucocorticoid or mineralocorticoid for treating CAH for a subject not treated with CRF receptor antagonist conjugated cyclic peptide of the present disclosure. In some embodiments, the peptide is a pharmaceutically acceptable salt thereof. In certain embodiments, the present disclosure provides administering to the subject the peptide and the reduced amount of the glucocorticoid or mineralocorticoid, e.g., as a combination therapy (e.g., without being limited to the disclosure herein, concurrently as separate therapies or by recommended/prescribed dosage regimens) for treating CAH.

In a further aspect is a method of treating congenital adrenal hyperplasia (CAH), in a subject in need thereof, comprising administering to the subject a therapeutically effective amount of a CRF receptor antagonist fatty acid conjugated cyclic peptide comprising or consisting of SEQ ID NO: 95, wherein the treating results in reducing the amount of a glucocorticoid or mineralocorticoid (such as hydrocortisone (HC), prednisone, prednisolone, dexamethasone, or fludrocortisone) if administered to the subject by at least 5%, 10%, 15%, 20%, 25%, 30%, 35%, 40%, 45%, 50%, 55%, or 60% compared to the standard of care or recommended daily dose of the glucocorticoid or mineralocorticoid for treating CAH for a subject not treated with CRF receptor antagonist conjugated cyclic peptide of the present disclosure. In some embodiments, the peptide is a pharmaceutically acceptable salt thereof. In certain embodiments, the present disclosure provides administering to the subject the peptide and the reduced amount of the glucocorticoid or mineralocorticoid, e.g., as a combination therapy (e.g., without being limited to the disclosure herein, concurrently as separate therapies or by recommended/prescribed dosage regimens) for treating CAH.

In a further aspect is a method of treating congenital adrenal hyperplasia (CAH), in a subject in need thereof, comprising administering to the subject a therapeutically effective amount of a CRF receptor antagonist fatty acid conjugated cyclic peptide comprising or consisting of SEQ ID NO: 97, wherein the treating results in reducing the amount of a glucocorticoid or mineralocorticoid (such as hydrocortisone (HC), prednisone, prednisolone, dexamethasone, or fludrocortisone) if administered to the subject by at least 5%, 10%, 15%, 20%, 25%, 30%, 35%, 40%, 45%, 50%, 55%, or 60% compared to the standard of care or recommended daily dose of the glucocorticoid or mineralocorticoid for treating CAH for a subject not treated with CRF receptor antagonist conjugated cyclic peptide of the present disclosure. In some embodiments, the peptide is a pharmaceutically acceptable salt thereof. In certain embodiments, the present disclosure provides administering to the subject the peptide and the reduced amount of the glucocorticoid or mineralocorticoid, e.g., as a combination therapy (e.g., without being limited to the disclosure herein, concurrently as separate therapies or by recommended/prescribed dosage regimens) for treating CAH.

In a further aspect is a method of treating congenital adrenal hyperplasia (CAH), in a subject in need thereof, comprising administering to the subject a therapeutically effective amount of a CRF receptor antagonist fatty acid conjugated cyclic peptide comprising or consisting of SEQ ID NO: 101, wherein the treating results in reducing the amount of a glucocorticoid or mineralocorticoid (such as hydrocortisone (HC), prednisone, prednisolone, dexamethasone, or fludrocortisone) if administered to the subject by at least 5%, 10%, 15%, 20%, 25%, 30%, 35%, 40%, 45%, 50%, 55%, or 60% compared to the standard of care or recommended daily dose of the glucocorticoid or mineralocorticoid for treating CAH for a subject not treated with CRF receptor antagonist conjugated cyclic peptide of the present disclosure. In some embodiments, the peptide is a pharmaceutically acceptable salt thereof. In certain embodiments, the present disclosure provides administering to the subject the peptide and the reduced amount of the glucocorticoid or mineralocorticoid, e.g., as a combination therapy (e.g., without being limited to the disclosure herein, concurrently as separate therapies or by recommended/prescribed dosage regimens) for treating CAH.

In a further aspect is a method of treating congenital adrenal hyperplasia (CAH), in a subject in need thereof, comprising administering to the subject a therapeutically effective amount of a CRF receptor antagonist fatty acid conjugated cyclic peptide comprising or consisting of SEQ ID NO: 117, or of Formula (I), or pharmaceutically acceptable salts thereof, wherein an adrenocorticotropic hormone (ACTH) level in the human is reduced by at least 10% from baseline.

In a further aspect is a method of treating congenital adrenal hyperplasia (CAH), in a subject in need thereof, comprising administering to the subject a therapeutically effective amount of a CRF receptor antagonist fatty acid conjugated cyclic peptide of any one of formulae (Ia), (Ib), (Ic), (Id), (Ie), (If), (Ig), (Ih), or (Ik), or a pharmaceutically acceptable salt thereof, wherein an adrenocorticotropic hormone (ACTH) level in the human is reduced by at least 10% from baseline.

In a further aspect is a method of treating congenital adrenal hyperplasia (CAH), in a subject in need thereof, comprising administering to the subject a therapeutically effective amount of a CRF receptor antagonist fatty acid conjugated cyclic peptide of any one of the peptides listed in Tables B1.1 and B1.2, or a pharmaceutically acceptable salt thereof, wherein an adrenocorticotropic hormone (ACTH) level in the human is reduced by at least 10% from baseline.

In a further aspect is a method of treating congenital adrenal hyperplasia (CAH), in a subject in need thereof, comprising administering to the subject a therapeutically effective amount of a CRF receptor antagonist fatty acid conjugated cyclic peptide comprising or consisting of SEQ ID NO: 50, wherein an adrenocorticotropic hormone (ACTH) level in the human is reduced by at least 10% from baseline. In some embodiments, the peptide is a pharmaceutically acceptable salt thereof.

In a further aspect is a method of treating congenital adrenal hyperplasia (CAH), in a subject in need thereof, comprising administering to the subject a therapeutically effective amount of a CRF receptor antagonist fatty acid conjugated cyclic peptide comprising or consisting of SEQ ID NO: 95, wherein an adrenocorticotropic hormone (ACTH) level in the human is reduced by at least 10% from baseline. In some embodiments, the peptide is a pharmaceutically acceptable salt thereof.

In a further aspect is a method of treating congenital adrenal hyperplasia (CAH), in a subject in need thereof, comprising administering to the subject a therapeutically effective amount of a CRF receptor antagonist fatty acid conjugated cyclic peptide comprising or consisting of SEQ ID NO: 97, wherein an adrenocorticotropic hormone (ACTH) level in the human is reduced by at least 10% from baseline. In some embodiments, the peptide is a pharmaceutically acceptable salt thereof.

In a further aspect is a method of treating congenital adrenal hyperplasia (CAH), in a subject in need thereof, comprising administering to the subject a therapeutically effective amount of a CRF receptor antagonist fatty acid conjugated cyclic peptide comprising or consisting of SEQ ID NO: 101, wherein an adrenocorticotropic hormone (ACTH) level in the human is reduced by at least 10% from baseline. In some embodiments, the peptide is a pharmaceutically acceptable salt thereof.

In a further aspect is a method of treating congenital adrenal hyperplasia (CAH), in a subject in need thereof, comprising administering to the subject a therapeutically effective amount of a CRF receptor antagonist fatty acid conjugated cyclic peptide comprising or consisting of SEQ ID NO: 117, or of Formula (I), or pharmaceutically acceptable salts thereof, wherein the level of androstenedione (A4) in the subject is reduced from baseline by at least 5%, 10%, 15%, 20%, 25% and is maintained at the reduced level post 24 hours.

In a further aspect is a method of treating congenital adrenal hyperplasia (CAH), in a subject in need thereof, comprising administering to the subject a therapeutically effective amount of a CRF receptor antagonist fatty acid conjugated cyclic peptide of any one of formulae (Ia), (Ib), (Ic), (Id), (Ie), (If), (Ig), (Ih), or (Ik), or a pharmaceutically acceptable salt thereof, wherein the level of androstenedione (A4) in the subject is reduced from baseline by at least 5%, 10%, 15%, 20%, 25% and is maintained at the reduced level post 24 hours.

In a further aspect is a method of treating congenital adrenal hyperplasia (CAH), in a subject in need thereof, comprising administering to the subject a therapeutically effective amount of a CRF receptor antagonist fatty acid conjugated cyclic peptide of any one of the peptides listed in Tables B1.1 and B1.2, or a pharmaceutically acceptable salt thereof, wherein the level of androstenedione (A4) in the subject is reduced from baseline by at least 5%, 10%, 15%, 20%, 25% and is maintained at a reduced level post 24 hours.

In a further aspect is a method of treating congenital adrenal hyperplasia (CAH), in a subject in need thereof, comprising administering to the subject a therapeutically effective amount of a CRF receptor antagonist fatty acid conjugated cyclic peptide comprising or consisting of SEQ ID NO: 50, wherein the level of androstenedione (A4) in the subject is reduced from baseline by at least 5%, 10%, 15%, 20%, 25% and is maintained at the reduced level post 24 hours. In some embodiments, the peptide is a pharmaceutically acceptable salt thereof.

In a further aspect is a method of treating congenital adrenal hyperplasia (CAH), in a subject in need thereof, comprising administering to the subject a therapeutically effective amount of a CRF receptor antagonist fatty acid conjugated cyclic peptide comprising or consisting of SEQ ID NO: 95, wherein the level of androstenedione (A4) in the subject is reduced from baseline by at least 5%, 10%, 15%, 20%, 25% and is maintained at the reduced level post 24 hours. In some embodiments, the peptide is a pharmaceutically acceptable salt thereof.

In a further aspect is a method of treating congenital adrenal hyperplasia (CAH), in a subject in need thereof, comprising administering to the subject a therapeutically effective amount of a CRF receptor antagonist fatty acid conjugated cyclic peptide comprising or consisting of SEQ ID NO: 97, wherein the level of androstenedione (A4) in the subject is reduced from baseline by at least 5%, 10%, 15%, 20%, 25% and is maintained at the reduced level post 24 hours. In some embodiments, the peptide is a pharmaceutically acceptable salt thereof.

In a further aspect is a method of treating congenital adrenal hyperplasia (CAH), in a subject in need thereof, comprising administering to the subject a therapeutically effective amount of a CRF receptor antagonist fatty acid conjugated cyclic peptide comprising or consisting of SEQ ID NO: 101, wherein the level of androstenedione (A4) in the subject is reduced from baseline by at least 5%, 10%, 15%, 20%, 25% and is maintained at the reduced level post 24 hours. In some embodiments, the peptide is a pharmaceutically acceptable salt thereof.

In some embodiments, the present disclosure provides a fatty acid conjugated cyclic peptide comprising or consisting of SEQ ID NO: 117, or of Formula (I), or any other Formulae or SEQ ID NO. disclosed herein, or pharmaceutically acceptable salts thereof for use in the treatment of congenital adrenal hyperplasia (CAH) in a subject in need thereof, wherein the level of androstenedione (A4) in the subject is reduced from baseline by at least 5%, 10%, 15%, 20%, 25% and is maintained at the reduced level post 24 hours.

In some embodiments, the present disclosure provides the use of a fatty acid conjugated cyclic peptide comprising or consisting of SEQ ID NO: 117, or of Formula (I), or any other Formulae or SEQ ID NO. disclosed herein, or pharmaceutically acceptable salts thereof in the manufacture of a medicament for treating congenital adrenal hyperplasia (CAH) in a subject in need thereof, wherein the level of androstenedione (A4) in the subject is reduced from baseline by at least 5%, 10%, 15%, 20%, 25% and is maintained at the reduced level post 24 hours.

In a further aspect is a method of treating congenital adrenal hyperplasia (CAH), in a subject in need thereof, comprising administering to the subject a therapeutically effective amount of a CRF receptor antagonist fatty acid conjugated cyclic peptide comprising or consisting of SEQ ID NO: 117, or of Formula (I), or pharmaceutically acceptable salts thereof, wherein the level of adrenocorticotropic hormone (ACTH) in the subject is reduced by at least 10% from baseline, and wherein the level of 17-hydroxyprogesterone (17-OHP) is reduced in the subject by at least about 10% from baseline.

In a further aspect is a method of treating congenital adrenal hyperplasia (CAH), in a subject in need thereof, comprising administering to the subject a therapeutically effective amount of a CRF receptor antagonist fatty acid conjugated cyclic peptide of any one of formulae (Ia), (Ib), (Ic), (Id), (Ie), (If), (Ig), (Ih), or (Ik), or a pharmaceutically acceptable salt thereof, wherein the level of adrenocorticotropic hormone (ACTH) in the subject is reduced by at least 10% from baseline, and wherein the level of 17-hydroxyprogesterone (17-OHP) is reduced in the subject by at least about 10% from baseline.

In a further aspect is a method of treating congenital adrenal hyperplasia (CAH), in a subject in need thereof, comprising administering to the subject a therapeutically effective amount of a CRF receptor antagonist fatty acid conjugated cyclic peptide of any one of the peptides listed in Tables B1.1 and B1.2, or a pharmaceutically acceptable salt thereof, wherein the level of adrenocorticotropic hormone (ACTH) in the subject is reduced by at least 10% from baseline, and wherein the level of 17-hydroxyprogesterone (17-OHP) is reduced in the subject by at least about 10% from baseline.

In a further aspect is a method of treating congenital adrenal hyperplasia (CAH), in a subject in need thereof, comprising administering to the subject a therapeutically effective amount of a CRF receptor antagonist fatty acid conjugated cyclic peptide comprising or consisting of SEQ ID NO: 50, wherein the level of_adrenocorticotropic hormone (ACTH) in the subject is reduced by at least 10% from baseline, and wherein the level of 17-hydroxyprogesterone (17-OHP) is reduced in the subject by at least about 10% from baseline. In some embodiments, the peptide is a pharmaceutically acceptable salt thereof.

In a further aspect is a method of treating congenital adrenal hyperplasia (CAH), in a subject in need thereof, comprising administering to the subject a therapeutically effective amount of a CRF receptor antagonist fatty acid conjugated cyclic peptide comprising or consisting of SEQ ID NO: 95, wherein the level of adrenocorticotropic hormone (ACTH) in the subject is reduced by at least 10% from baseline, and wherein the level of 17-hydroxyprogesterone (17-OHP) is reduced in the subject by at least about 10% from baseline. In some embodiments, the peptide is a pharmaceutically acceptable salt thereof.

In a further aspect is a method of treating congenital adrenal hyperplasia (CAH), in a subject in need thereof, comprising administering to the subject a therapeutically effective amount of a CRF receptor antagonist fatty acid conjugated cyclic peptide comprising or consisting of SEQ ID NO: 97, wherein the level of adrenocorticotropic hormone (ACTH) in the subject is reduced by at least 10% from baseline, and wherein the level of 17-hydroxyprogesterone (17-OHP) is reduced in the subject by at least about 10% from baseline. In some embodiments, the peptide is a pharmaceutically acceptable salt thereof.

In a further aspect is a method of treating congenital adrenal hyperplasia (CAH), in a subject in need thereof, comprising administering to the subject a therapeutically effective amount of a CRF receptor antagonist fatty acid conjugated cyclic peptide comprising or consisting of SEQ ID NO: 101, wherein the level of adrenocorticotropic hormone (ACTH) in the subject is reduced by at least 10% from baseline, and wherein the level of 17-hydroxyprogesterone (17-OHP) is reduced in the subject by at least about 10% from baseline. In some embodiments, the peptide is a pharmaceutically acceptable salt thereof.

In some embodiments, the present disclosure provides a fatty acid conjugated cyclic peptide comprising or consisting of SEQ ID NO: 117, or of Formula (I), or any other Formulae or SEQ ID NO. disclosed herein, or pharmaceutically acceptable salts thereof for use in the treatment of congenital adrenal hyperplasia (CAH) in a subject in need thereof, wherein the level of adrenocorticotropic hormone (ACTH) in the subject is reduced by at least 10% from baseline, and wherein the level of 17-hydroxyprogesterone (17-OHP) is reduced in the subject by at least about 10% from baseline.

In some embodiments, the present disclosure provides the use of a fatty acid conjugated cyclic peptide comprising or consisting of SEQ ID NO: 117, or of Formula (I), or any other Formulae or SEQ ID NO. disclosed herein, or pharmaceutically acceptable salts thereof in the manufacture of a medicament for treating congenital adrenal hyperplasia (CAH) in a subject in need thereof, wherein the level of adrenocorticotropic hormone (ACTH) in the subject is reduced by at least 10% from baseline, and wherein the level of 17-hydroxyprogesterone (17-OHP) is reduced in the subject by at least about 10% from baseline.

In some embodiments, the present disclosure provides a method of treating classic or non-classic congenital adrenal hyperplasia (CAH), in a subject in need thereof, comprising administering to the subject a therapeutically effective amount of a peptide comprising or consisting of any one of formulae (Ia), (Ib), (Ic), (Id), (Ie), (If), (Ig), (Ih), or (Ik), or a pharmaceutically acceptable salt thereof.

In some embodiments, the present disclosure provides a method of treating classic or non-classic congenital adrenal hyperplasia (CAH), in a subject in need thereof, comprising administering to the subject a therapeutically effective amount of any one of the peptides listed in Tables B1.1 and B1.2, or a pharmaceutically acceptable salt thereof.

In some embodiments, the present disclosure provides a method of treating classic or non-classic congenital adrenal hyperplasia (CAH), in a subject in need thereof, comprising administering to the subject a therapeutically effective amount of the peptide comprising or consisting of SEQ ID NO:50. In some embodiments, the peptide is a pharmaceutically acceptable salt thereof.

In some embodiments, the present disclosure provides a method of treating classic or non-classic congenital adrenal hyperplasia (CAH), in a subject in need thereof, comprising administering to the subject a therapeutically effective amount of the peptide comprising or consisting of SEQ ID NO:95. In some embodiments, the peptide is a pharmaceutically acceptable salt thereof.

In some embodiments, the present disclosure provides a method of treating classic or non-classic congenital adrenal hyperplasia (CAH), in a subject in need thereof, comprising administering to the subject a therapeutically effective amount of the peptide comprising or consisting of SEQ ID NO:97. In some embodiments, the peptide is a pharmaceutically acceptable salt thereof.

In some embodiments, the present disclosure provides a method of treating classic or non-classic congenital adrenal hyperplasia (CAH), in a subject in need thereof, comprising administering to the subject a therapeutically effective amount of the peptide comprising or consisting of SEQ ID NO:101. In some embodiments, the peptide is a pharmaceutically acceptable salt thereof.

In some embodiments, the present disclosure provides a fatty acid conjugated cyclic peptide comprising or consisting of SEQ ID NO: 117, or of Formula (I), or any other Formulae or SEQ ID NO. disclosed herein, or pharmaceutically acceptable salts thereof for use in the treatment of classic or non-classic congenital adrenal hyperplasia (CAH).

Another aspect of the present disclosure provides a method for reducing the severity of one or more symptoms of classic or non-classic CAH selected from hirsutism, precocious puberty, fertility problems, acne, and growth impairment in a subject having classic congenital adrenal hyperplasia, comprising administering a therapeutically effective amount of a CRF receptor antagonist fatty acid conjugated cyclic peptide comprising or consisting of SEQ ID NO: 117, or of Formula (I), or pharmaceutically acceptable salts thereof.

In some embodiments, the present disclosure provides a method for reducing the severity of one or more symptoms of classic or non-classic CAH selected from hirsutism, precocious puberty, fertility problems, acne, and growth impairment in a subject having classic congenital adrenal hyperplasia, comprising administering to the subject a therapeutically effective amount of a peptide comprising or consisting of any one of formulae (Ia), (Ib), (Ic), (Id), (Ie), (If), (Ig), (Ih), or (Ik), or a pharmaceutically acceptable salt thereof.

In some embodiments, the present disclosure provides a method for reducing the severity of one or more symptoms of classic or non-classic CAH selected from hirsutism, precocious puberty, fertility problems, acne, and growth impairment in a subject having classic congenital adrenal hyperplasia, comprising administering to the subject a therapeutically effective amount of any one of the peptides listed in Tables B1.1 and B1.2, or a pharmaceutically acceptable salt thereof.

In some embodiments, the present disclosure provides a method for reducing the severity of one or more symptoms of classic or non-classic CAH selected from hirsutism, precocious puberty, fertility problems, acne, and growth impairment in a subject having classic congenital adrenal hyperplasia, comprising administering to the subject a therapeutically effective amount of the peptide comprising or consisting of SEQ ID NO: 50. In some embodiments, the peptide is a pharmaceutically acceptable salt thereof.

In some embodiments, the present disclosure provides a method for reducing the severity of one or more symptoms of classic or non-classic CAH selected from hirsutism, precocious puberty, fertility problems, acne, and growth impairment in a subject having classic congenital adrenal hyperplasia, comprising administering to the subject a therapeutically effective amount of the peptide comprising or consisting of SEQ ID NO: 95. In some embodiments, the peptide is a pharmaceutically acceptable salt thereof.

In some embodiments, the present disclosure provides a method for reducing the severity of one or more symptoms of classic or non-classic CAH selected from hirsutism, precocious puberty, fertility problems, acne, and growth impairment in a subject having classic congenital adrenal hyperplasia, comprising administering to the subject a therapeutically effective amount of the peptide comprising or consisting of SEQ ID NO: 97. In some embodiments, the peptide is a pharmaceutically acceptable salt thereof.

In some embodiments, the present disclosure provides a method for reducing the severity of one or more symptoms of classic or non-classic CAH selected from hirsutism, precocious puberty, fertility problems, acne, and growth impairment in a subject having classic congenital adrenal hyperplasia, comprising administering to the subject a therapeutically effective amount of the peptide comprising or consisting of SEQ ID NO: 101. In some embodiments, the peptide is a pharmaceutically acceptable salt thereof.

In some embodiments, the present disclosure provides a fatty acid conjugated cyclic peptide comprising or consisting of SEQ ID NO: 117, or of Formula (I), or any other Formulae disclosed herein or SEQ ID NO. or pharmaceutically acceptable salts thereof for use in the treatment of reducing the severity of one or more symptoms of classic or non-classic CAH selected from hirsutism, precocious puberty, fertility problems, acne, and growth impairment in a subject having classic congenital adrenal hyperplasia.

Another aspect of the present disclosure provides a method for reducing the severity of one or more symptoms of classic or non-classic CAH wherein the growth impairment is selected from one or more of accelerated height velocity, accelerated weight velocity, and accelerated bone age.

In some embodiments, the present disclosure provides a method for reducing the severity of one or more symptoms of classic or non-classic CAH wherein the growth impairment is selected from one or more of accelerated height velocity, accelerated weight velocity, and accelerated bone age, comprising administering to the subject a therapeutically effective amount of a peptide comprising or consisting of any one of formulae (I), (Ia), (Ib), (Ic), (Id), (Ie), (If), (Ig), (Ih), or (Ik), or a pharmaceutically acceptable salt thereof.

In some embodiments, the present disclosure provides a method for reducing the severity of one or more symptoms of classic or non-classic CAH wherein the growth impairment is selected from one or more of accelerated height velocity, accelerated weight velocity, and accelerated bone age, comprising administering to the subject a therapeutically effective amount of any one of the peptides listed in Tables B1.1 and B1.2, or a pharmaceutically acceptable salt thereof.

In some embodiments, the present disclosure provides a method for reducing the severity of one or more symptoms of classic or non-classic CAH wherein the growth impairment is selected from one or more of accelerated height velocity, accelerated weight velocity, and accelerated bone age, comprising administering to the subject a therapeutically effective amount of the peptide comprising or consisting of SEQ ID NO: 50. In some embodiments, the peptide is a pharmaceutically acceptable salt thereof.

In some embodiments, the present disclosure provides a method for reducing the severity of one or more symptoms of classic or non-classic CAH wherein the growth impairment is selected from one or more of accelerated height velocity, accelerated weight velocity, and accelerated bone age, comprising administering to the subject a therapeutically effective amount of the peptide comprising or consisting of SEQ ID NO: 95. In some embodiments, the peptide is a pharmaceutically acceptable salt thereof.

In some embodiments, the present disclosure provides a method for reducing the severity of one or more symptoms of classic or non-classic CAH wherein the growth impairment is selected from one or more of accelerated height velocity, accelerated weight velocity, and accelerated bone age, comprising administering to the subject a therapeutically effective amount of the peptide comprising or consisting of SEQ ID NO: 97. In some embodiments, the peptide is a pharmaceutically acceptable salt thereof.

In some embodiments, the present disclosure provides a method for reducing the severity of one or more symptoms of classic or non-classic CAH wherein the growth impairment is selected from one or more of accelerated height velocity, accelerated weight velocity, and accelerated bone age, comprising administering to the subject a therapeutically effective amount of the peptide comprising or consisting of SEQ ID NO: 101. In some embodiments, the peptide is a pharmaceutically acceptable salt thereof.

In some embodiments, the present disclosure provides a fatty acid conjugated cyclic peptide comprising or consisting of SEQ ID NO: 117, or of Formula (I), or any other Formulae or SEQ ID NO. disclosed herein, or pharmaceutically acceptable salts thereof for use in the treatment of reducing the severity of one or more symptoms of classic or non-classic CAH wherein the growth impairment is selected from one or more of accelerated height velocity, accelerated weight velocity, and accelerated bone age.

Another embodiment of the present disclosure provides a method of reducing the dosage of corticosteroid administered to a subject having classic or non-classic CAH for controlling CAH comprising administering to the subject a therapeutically effective amount of a CRF receptor antagonist fatty acid conjugated cyclic peptide comprising or consisting of SEQ ID NO: 117, or of Formula (I), or a pharmaceutically acceptable salt thereof. Wherein the dosage of corticosteroid is reduced compared to the recommended dosages for classic or non-classic CAH patients.

In some embodiments, the present disclosure provides a method of reducing the dosage of corticosteroid administered to a subject having classic or non-classic CAH for controlling CAH, comprising administering to the subject a therapeutically effective amount of a peptide comprising or consisting of any one of formulae (Ia), (Ib), (Ic), (Id), (Ie), (If), (Ig), (Ih), or (Ik), or a pharmaceutically acceptable salt thereof.

In some embodiments, the present disclosure provides a method of reducing the dosage of corticosteroid administered to a subject having classic or non-classic CAH for controlling CAH, comprising administering to the subject a therapeutically effective amount of any one of the peptides listed in Tables B1.1 and B1.2, or a pharmaceutically acceptable salt thereof.

In some embodiments, the present disclosure provides a method of reducing the dosage of corticosteroid administered to a subject having classic or non-classic CAH for controlling CAH, comprising administering to the subject a therapeutically effective amount of the peptide comprising or consisting of SEQ ID NO: 50. In some embodiments, the peptide is a pharmaceutically acceptable salt thereof.

In some embodiments, the present disclosure provides a method of reducing the dosage of corticosteroid administered to a subject having classic or non-classic CAH for controlling CAH, comprising administering to the subject a therapeutically effective amount of the peptide comprising or consisting of SEQ ID NO: 95. In some embodiments, the peptide is a pharmaceutically acceptable salt thereof.

In some embodiments, the present disclosure provides a method of reducing the dosage of corticosteroid administered to a subject having classic or non-classic CAH for controlling CAH, comprising administering to the subject a therapeutically effective amount of the peptide comprising or consisting of SEQ ID NO: 97. In some embodiments, the peptide is a pharmaceutically acceptable salt thereof.

In some embodiments, the present disclosure provides a method of reducing the dosage of corticosteroid administered to a subject having classic or non-classic CAH for controlling CAH, comprising administering to the subject a therapeutically effective amount of the peptide comprising or consisting of SEQ ID NO: 101. In some embodiments, the peptide is a pharmaceutically acceptable salt thereof.

Another aspect of the disclosure provides a method of reducing the severity of one or more side effects of glucocorticoid treatment in a subject having classic or non-classic CAH comprising administering to the subject a therapeutically effective amount of a CRF receptor antagonist fatty acid conjugated cyclic peptide comprising or consisting of SEQ ID NO: 117, or of Formula (I), or pharmaceutically acceptable salts thereof.

In some embodiments, the present disclosure provides a method of reducing the severity of one or more side effects of glucocorticoid treatment in a subject having classic or non-classic CAH, comprising administering to the subject a therapeutically effective amount of a peptide comprising or consisting of any one of formulae (Ia), (Ib), (Ic), (Id), (Ie), (If), (Ig), (Ih), or (Ik), or a pharmaceutically acceptable salt thereof.

In some embodiments, the present disclosure provides a method of reducing the severity of one or more side effects of glucocorticoid treatment in a subject having classic or non-classic CAH, comprising administering to the subject a therapeutically effective amount of any one of the peptides listed in Tables B1.1 and B1.2, or a pharmaceutically acceptable salt thereof.

In some embodiments, the present disclosure provides a method of reducing the severity of one or more side effects of glucocorticoid treatment in a subject having classic or non-classic CAH, comprising administering to the subject a therapeutically effective amount of the peptide comprising or consisting of SEQ ID NO: 50. In some embodiments, the peptide is a pharmaceutically acceptable salt thereof.

In some embodiments, the present disclosure provides a method of reducing the severity of one or more side effects of glucocorticoid treatment in a subject having classic or non-classic CAH, comprising administering to the subject a therapeutically effective amount of the peptide comprising or consisting of SEQ ID NO: 95. In some embodiments, the peptide is a pharmaceutically acceptable salt thereof.

In some embodiments, the present disclosure provides a method of reducing the severity of one or more side effects of glucocorticoid treatment in a subject having classic or non-classic CAH, comprising administering to the subject a therapeutically effective amount of the peptide comprising or consisting of SEQ ID NO: 97. In some embodiments, the peptide is a pharmaceutically acceptable salt thereof.

In some embodiments, the present disclosure provides a method of reducing the severity of one or more side effects of glucocorticoid treatment in a subject having classic or non-classic CAH, comprising administering to the subject a therapeutically effective amount of the peptide comprising or consisting of SEQ ID NO: 101. In some embodiments, the peptide is a pharmaceutically acceptable salt thereof.

In some embodiments, the present disclosure provides a fatty acid conjugated cyclic peptide comprising or consisting of SEQ ID NO: 117, or of Formula (I), or any other Formulae or SEQ ID NO. disclosed herein, or pharmaceutically acceptable salts thereof for use in the treatment of reducing severity of one or more side effects of glucocorticoid treatment in a subject having classic or non-classic CAH.

Testicular adrenal rest tumors (TART) and ovarian adrenal rest tumors (OART) are ACTH-responsive lesions of the testes and ovaries derived from adrenal tissue interchelated within these organs during embryogenesis. In response to high ACTH, hyperplasia of this tissue occurs, resulting in single or multiple lesions that may cause pain and infertility. Currently, there are no non-steroidal, non-surgical solutions to control, shrink, or reduce TART.

One aspect of the disclosure is a method of treating (e.g., controlling, shrinking, and/or reducing) or preventing testicular adrenal rest tumors (TART) or ovarian adrenal rest tumors (OART) in a subject in need thereof, comprising administering to the subject a therapeutically effective amount of a CRF receptor antagonist fatty acid conjugated cyclic peptide comprising or consisting of SEQ ID NO: 117, or of Formula (I), or pharmaceutically acceptable salts thereof. In a further aspect, the subject has congenital adrenal hyperplasia (CAH). In a further aspect, the size and/or numbers of the tumors (TART and/or OART) are decreased or reduced.

In a further aspect of the disclosure is a method of treating (e.g., controlling, shrinking, and/or reducing) or preventing testicular adrenal rest tumors (TART) or ovarian adrenal rest tumors (OART) in a subject in need thereof, comprising administering to the subject a therapeutically effective amount of a peptide comprising or consisting of any one of formulae (Ia), (Ib), (Ic), (Id), (Ie), (If), (Ig), (Ih), or (Ik), or a pharmaceutically acceptable salt thereof. In a further aspect, the subject has congenital adrenal hyperplasia (CAH). In a further aspect, the size and/or numbers of the tumors (TART and/or OART) are decreased or reduced.

In a further aspect of the disclosure is a method of treating (e.g., controlling, shrinking, and/or reducing) or preventing testicular adrenal rest tumors (TART) or ovarian adrenal rest tumors (OART) in a subject in need thereof, comprising administering to the subject a therapeutically effective amount of any one of the peptides listed in Tables B1.1 and B1.2, or a pharmaceutically acceptable salt thereof. In a further aspect, the subject has congenital adrenal hyperplasia (CAH). In a further aspect, the size and/or numbers of the tumors (TART and/or OART) are decreased or reduced.

In a further aspect of the disclosure is a method of treating (e.g., controlling, shrinking, and/or reducing) or preventing testicular adrenal rest tumors (TART) or ovarian adrenal rest tumors (OART) in a subject in need thereof, comprising administering to the subject a therapeutically effective amount of the peptide comprising or consisting of SEQ ID NO: 50. In some embodiments, the peptide is a pharmaceutically acceptable salt thereof. In a further aspect, the subject has congenital adrenal hyperplasia (CAH). In a further aspect, the size and/or numbers of the tumors (TART and/or OART) are decreased or reduced.

In a further aspect of the disclosure is a method of treating (e.g., controlling, shrinking, and/or reducing) or preventing testicular adrenal rest tumors (TART) or ovarian adrenal rest tumors (OART) in a subject in need thereof, comprising administering to the subject a therapeutically effective amount of the peptide comprising or consisting of SEQ ID NO: 95. In some embodiments, the peptide is a pharmaceutically acceptable salt thereof. In a further aspect, the subject has congenital adrenal hyperplasia (CAH). In a further aspect, the size and/or numbers of the tumors (TART and/or OART) are decreased or reduced.

In a further aspect of the disclosure is a method of treating (e.g., controlling, shrinking, and/or reducing) or preventing testicular adrenal rest tumors (TART) or ovarian adrenal rest tumors (OART) in a subject in need thereof, comprising administering to the subject a therapeutically effective amount of the peptide comprising or consisting of SEQ ID NO: 97. In some embodiments, the peptide is a pharmaceutically acceptable salt thereof. In a further aspect, the subject has congenital adrenal hyperplasia (CAH). In a further aspect, the size and/or numbers of the tumors (TART and/or OART) are decreased or reduced.

In a further aspect of the disclosure is a method of treating (e.g., controlling, shrinking, and/or reducing) or preventing testicular adrenal rest tumors (TART) or ovarian adrenal rest tumors (OART) in a subject in need thereof, comprising administering to the subject a therapeutically effective amount of the peptide comprising or consisting of SEQ ID NO: 101. In some embodiments, the peptide is a pharmaceutically acceptable salt thereof. In a further aspect, the subject has congenital adrenal hyperplasia (CAH). In a further aspect, the size and/or numbers of the tumors (TART and/or OART) are decreased or reduced.

In some embodiments, the present disclosure provides a fatty acid conjugated cyclic peptide comprising or consisting of SEQ ID NO: 117, or of Formula (I), or any other Formulae or SEQ ID NO. disclosed herein, or pharmaceutically acceptable salts thereof for use in the treatment of testicular adrenal rest tumors (TART).

In some embodiments, the present disclosure provides a fatty acid conjugated cyclic peptide comprising or consisting of SEQ ID NO: 117, or of Formula (I), or any other Formulae or SEQ ID NO. disclosed herein, or pharmaceutically acceptable salts thereof for use in the treatment of ovarian adrenal rest tumors (OART) .

In some embodiments, the present disclosure provides a fatty acid conjugated cyclic peptide comprising or consisting of SEQ ID NO: 117, or of Formula (I), or any other Formulae or SEQ ID NO. disclosed herein, or pharmaceutically acceptable salts thereof for use in the treatment of congenital adrenal hyperplasia (CAH), wherein the size and/or numbers of the tumors are decreased or reduced.

In some embodiments, the present disclosure provides the use of a fatty acid conjugated cyclic peptide comprising or consisting of SEQ ID NO: 117, or of Formula (I), or any other Formulae or SEQ ID NO. disclosed herein, or pharmaceutically acceptable salts thereof in the manufacture of a medicament for treating testicular adrenal rest tumors (TART).

In some embodiments, the present disclosure provides the use of a fatty acid conjugated cyclic peptide comprising or consisting of SEQ ID NO: 117, or of Formula (I), or any other Formulae or SEQ ID NO. disclosed herein, or pharmaceutically acceptable salts thereof in the manufacture of a medicament for treating ovarian adrenal rest tumors (OART) .

In some embodiments, the present disclosure provides the use of a fatty acid conjugated cyclic peptide comprising or consisting of SEQ ID NO: 117, or of Formula (I), or any other Formulae or SEQ ID NO. disclosed herein, or pharmaceutically acceptable salts thereof in the manufacture of a medicament for treating congenital adrenal hyperplasia (CAH), wherein the size and/or numbers of the tumors are decreased or reduced.

The side effect of glucocorticoid treatment in a subject having CAH can be osteoporosis, avascular necrosis of bone, myopathy, hyperglycemia, diabetes mellitus, dyslipidemia, weight gain, Cushing syndrome, Cushingoid features, growth suppression, adrenal suppression, gastritis, peptic ulcer, gastrointestinal bleeding, visceral perforation, hepatic steatosis, pancreatitis, hypertension, coronary heart disease, ischemic heart disease, heart failure, dermatoprosis, skin atrophy, ecchymosis, purpura, erosions, striae, delayed wound healing, easy bruising, acne, hirsutism, hair loss, mood changes, depression, euphoria, mood lability, irritability, akathisia, anxiety, cognitive impairment, psychosis, dementia, delirium, cataract, glaucoma, ptosis, mydriasis, opportunistic ocular infections, central serous chorioretinopathy, suppression of cell-mediated immunity, predisposition to infections, reactivation of latent infections, or any combination(s) thereof.

Another aspect of the present disclosure provides a method of treating mastocytosis or mast cell activation syndrome, autoimmune disease, CNS disease or neuroinflammation, or irritable bowel syndrome (IBS), comprising administering to the subject a therapeutically effective amount of a CRF receptor antagonist fatty acid conjugated cyclic peptide comprising or consisting of SEQ ID NO: 117, or of Formula (I), or pharmaceutically acceptable salts thereof.

In some embodiments, the present disclosure provides a method of treating mastocytosis or mast cell activation syndrome, autoimmune disease, CNS disease or neuroinflammation, or irritable bowel syndrome (IBS), comprising administering to the subject a therapeutically effective amount of a peptide comprising or consisting of any one of formulae (Ia), (Ib), (Ic), (Id), (Ie), (If), (Ig), (Ih), or (Ik), or a pharmaceutically acceptable salt thereof.

In some embodiments, the present disclosure provides a method of treating mastocytosis or mast cell activation syndrome, autoimmune disease, CNS disease or neuroinflammation, or irritable bowel syndrome (IBS), comprising administering to the subject a therapeutically effective amount of any one of the peptides listed in Tables B1.1 and B1.2, or a pharmaceutically acceptable salt thereof.

In some embodiments, the present disclosure provides a method of treating mastocytosis or mast cell activation syndrome, autoimmune disease, CNS disease or neuroinflammation, or irritable bowel syndrome (IBS), comprising administering to the subject a therapeutically effective amount of the peptide comprising or consisting of SEQ ID NO: 50. In some embodiments, the peptide is a pharmaceutically acceptable salt thereof.

In some embodiments, the present disclosure provides a method of treating mastocytosis or mast cell activation syndrome, autoimmune disease, CNS disease or neurointlammation, or irritable bowel syndrome (IBS), comprising administering to the subject a therapeutically effective amount of the peptide comprising or consisting of SEQ ID NO: 95. In some embodiments, the peptide is a pharmaceutically acceptable salt thereof.

In some embodiments, the present disclosure provides a method of treating mastocytosis or mast cell activation syndrome, autoimmune disease, CNS disease or neuroinflammation, or irritable bowel syndrome (IBS), comprising administering to the subject a therapeutically effective amount of the peptide comprising or consisting of SEQ ID NO: 97. In some embodiments, the peptide is a pharmaceutically acceptable salt thereof.

In some embodiments, the present disclosure provides a method of treating mastocytosis or mast cell activation syndrome, autoimmune disease, CNS disease or neuroinflammation, or irritable bowel syndrome (IBS), comprising administering to the subject a therapeutically effective amount of the peptide comprising or consisting of SEQ ID NO: 101. In some embodiments, the peptide is a pharmaceutically acceptable salt thereof.

In some embodiments, the present disclosure provides a fatty acid conjugated cyclic peptide comprising or consisting of SEQ ID NO: 117, or of Formula (I), or any other Formulae or SEQ ID NO. disclosed herein, or pharmaceutically acceptable salts thereof for use in the treatment of mastocytosis or mast cell activation syndrome, autoimmune disease, CNS disease or neuroinflammation, or irritable bowel syndrome (IBS).

In another aspect of the present disclosure provides a method of treating allergic rhinitis, asthma, cancer, angiogenesis/lymphangiogenesis, atherosclerosis, myocardial infarction, stress/pain/pruritus, rheumatoid arthritis, coeliac disease, multiple sclerosis, bullous dermatoses, inflammatory bowel disease, venom tolerance, helminth infections, viral infections, bacterial and fungal infections, atopic dermatitis, food allergy, angioedema, or urticaria, comprising administering to the subject a therapeutically effective amount of a CRF receptor antagonist fatty acid conjugated cyclic peptide comprising or consisting of SEQ ID NO: 117, or of Formula (I), or a pharmaceutically acceptable salt thereof.

In some embodiments, the present disclosure provides a method of treating allergic rhinitis, asthma, cancer, angiogenesis/lymphangiogenesis, atherosclerosis, myocardial infarction, stress/pain/pruritus, rheumatoid arthritis, coeliac disease, multiple sclerosis, bullous dermatoses, inflammatory bowel disease, venom tolerance, helminth infections, viral infections, bacterial and fungal infections, atopic dermatitis, food allergy, angioedema, or urticaria, comprising administering to the subject a therapeutically effective amount of a peptide comprising or consisting of any one of formulae (Ia), (Ib), (Ic), (Id), (Ie), (If), (Ig), (Ih), or (Ik), or a pharmaceutically acceptable salt thereof.

In some embodiments, the present disclosure provides a method of treating allergic rhinitis, asthma, cancer, angiogenesis/lymphangiogenesis, atherosclerosis, myocardial infarction, stress/pain/pruritus, rheumatoid arthritis, coeliac disease, multiple sclerosis, bullous dermatoses, inflammatory bowel disease, venom tolerance, helminth infections, viral infections, bacterial and fungal infections, atopic dermatitis, food allergy, angioedema, or urticaria, comprising administering to the subject a therapeutically effective amount of any one of the peptides listed in Tables B1.1 and B1.2, or a pharmaceutically acceptable salt thereof.

In some embodiments, the present disclosure provides a method of treating allergic rhinitis, asthma, cancer, angiogenesis/lymphangiogenesis, atherosclerosis, myocardial infarction, stress/pain/pruritus, rheumatoid arthritis, coeliac disease, multiple sclerosis, bullous dermatoses, inflammatory bowel disease, venom tolerance, helminth infections, viral infections, bacterial and fungal infections, atopic dermatitis, food allergy, angioedema, or urticaria, comprising administering to the subject a therapeutically effective amount of the peptide comprising or consisting of SEQ ID NO: 50. In some embodiments, the peptide is a pharmaceutically acceptable salt thereof.

In some embodiments, the present disclosure provides a method of treating allergic rhinitis, asthma, cancer, angiogenesis/lymphangiogenesis, atherosclerosis, myocardial infarction, stress/pain/pruritus, rheumatoid arthritis, coeliac disease, multiple sclerosis, bullous dermatoses, inflammatory bowel disease, venom tolerance, helminth infections, viral infections, bacterial and fungal infections, atopic dermatitis, food allergy, angioedema, or urticaria, comprising administering to the subject a therapeutically effective amount of the peptide comprising or consisting of SEQ ID NO: 95. In some embodiments, the peptide is a pharmaceutically acceptable salt thereof.

In some embodiments, the present disclosure provides a method of treating allergic rhinitis, asthma, cancer, angiogenesis/lymphangiogenesis, atherosclerosis, myocardial infarction, stress/pain/pruritus, rheumatoid arthritis, coeliac disease, multiple sclerosis, bullous dermatoses, inflammatory bowel disease, venom tolerance, helminth infections, viral infections, bacterial and fungal infections, atopic dermatitis, food allergy, angioedema, or urticaria, comprising administering to the subject a therapeutically effective amount of the peptide comprising or consisting of SEQ ID NO: 97. In some embodiments, the peptide is a pharmaceutically acceptable salt thereof.

In some embodiments, the present disclosure provides a method of treating allergic rhinitis, asthma, cancer, angiogenesis/lymphangiogenesis, atherosclerosis, myocardial infarction, stress/pain/pruritus, rheumatoid arthritis, coeliac disease, multiple sclerosis, bullous dermatoses, inflammatory bowel disease, venom tolerance, helminth infections, viral infections, bacterial and fungal infections, atopic dermatitis, food allergy, angioedema, or urticaria, comprising administering to the subject a therapeutically effective amount of the peptide comprising or consisting of SEQ ID NO: 101. In some embodiments, the peptide is a pharmaceutically acceptable salt thereof.

In some embodiments, the present disclosure provides a fatty acid conjugated cyclic peptide comprising or consisting of SEQ ID NO: 117, or of Formula (I), or any other Formulae or SEQ ID NO. disclosed herein, or pharmaceutically acceptable salts thereof for use in the treatment of allergic rhinitis, asthma, cancer, angiogenesis/lymphangiogenesis, atherosclerosis, myocardial infarction, stress/pain/pruritus, rheumatoid arthritis, coeliac disease, multiple sclerosis, bullous dermatoses, inflammatory bowel disease, venom tolerance, helminth infections, viral infections, bacterial and fungal infections, atopic dermatitis, food allergy, angioedema, or urticaria.

Glucocorticoid remediable aldosteronism (GRA) is a common monogenic form of hypertension in humans. It accounts for approx. 1% of cases of Primary Aldosteronism (PA). Although other etiologies of PA are more frequent in women, GRA occurs equally among women and men. In GRA, chimeric gene duplication results from unequal crossing over between the highly homologous 11b-hydroxylase (CYP11B1) and aldosterone synthase (CYP11B2) genes. The chimera is a gene duplication resulting from the fusion of the 5' ACTH responsive promoter region of the 11b-hydroxylase gene to the 3' coding sequences of the aldosterone synthase gene. As a consequence, aldosterone is produced ectopically in the zona fasciculata, and its synthesis is solely under the regulation of ACTH. In patients with GRA, hypertension is often severe and difficult to control with standard antihypertensive therapies. Caution must be taken with the use of potassium-wasting diuretics, as these can induce hypokalemia in affected individuals. Exogenous glucocorticoid administration, which suppresses pituitary ACTH secretion, can improve, or normalize blood pressure, and is considered the first-line treatment for GRA. The use of a CRF receptor antagonist allows for lower doses of glucocorticoid to control the disease and/or eliminate the need for glucocorticoids. Peptides of the disclosure, therefore, are useful for the treatment of glucocorticoid remediable aldosteronism (GRA).

In another aspect of the present disclosure provides a method of treating glucocorticoid remediable aldosteronism (GRA), comprising administering to the subject a therapeutically effective amount of a CRF receptor antagonist fatty acid conjugated cyclic peptide comprising or consisting of Formula (I), or a pharmaceutically acceptable salt thereof.

In some embodiments, the present disclosure provides a method of treating glucocorticoid remediable aldosteronism (GRA), comprising administering to the subject a therapeutically effective amount of a peptide comprising or consisting of any one of formulae (Ia), (Ib), (Ic), (Id), (Ie), (If), (Ig), (Ih), or (Ik), or a pharmaceutically acceptable salt thereof.

In some embodiments, the present disclosure provides a method of treating glucocorticoid remediable aldosteronism (GRA), comprising administering to the subject a therapeutically effective amount of any one of the peptides listed in Tables B1.1 and B1.2, or a pharmaceutically acceptable salt thereof.

In some embodiments, the present disclosure provides a method of treating glucocorticoid remediable aldosteronism (GRA), comprising administering to the subject a therapeutically effective amount of the peptide comprising or consisting of SEQ ID NO: 50. In some embodiments, the peptide is a pharmaceutically acceptable salt thereof.

In some embodiments, the present disclosure provides a method of treating glucocorticoid remediable aldosteronism (GRA), comprising administering to the subject a therapeutically effective amount of the peptide comprising or consisting of SEQ ID NO: 95. In some embodiments, the peptide is a pharmaceutically acceptable salt thereof.

In some embodiments, the present disclosure provides a method of treating glucocorticoid remediable aldosteronism (GRA), comprising administering to the subject a therapeutically effective amount of the peptide comprising or consisting of SEQ ID NO: 97. In some embodiments, the peptide is a pharmaceutically acceptable salt thereof.

In some embodiments, the present disclosure provides a method of treating glucocorticoid remediable aldosteronism (GRA), comprising administering to the subject a therapeutically effective amount of the peptide comprising or consisting of SEQ ID NO: 101. In some embodiments, the peptide is a pharmaceutically acceptable salt thereof.

In some embodiments, the present disclosure provides a fatty acid conjugated cyclic peptide comprising or consisting of SEQ ID NO: 117, or of Formula (I), or any other Formulae or SEQ ID NO. disclosed herein, or pharmaceutically acceptable salts thereof for use in the treatment of glucocorticoid remediable aldosteronism (GRA).

Mast cell activation syndrome (MCAS) encompasses a severe constellation of symptoms within the broader group of disorders of mast cell activation. Mast cell activation can be caused by both IgE-mediated and non-IgE-mediated triggers. Atopic disorders, such as allergic rhinitis and allergic asthma, can affect up to 10% to 30% of the general population. In contrast, mastocytosis and monoclonal mast cell activation syndrome (MMAS) might be as rare as 1 in 10,000 to 20,000 subjects. Current treatments are H1 and/or H2 antagonists, leukotriene modifying agents, cromolyn, glucocorticoids, and/or omalizumab. Stress is a known trigger and/or potential; however, there are no therapies positioned to treat stress-induced mast cell activation or potentiation. CRF₁ and CRF2 receptors reside on human mast cells and modulate mast cell degranulation. Peptides of the disclosure, therefore, offer a long-acting approach to treat this spectrum of disorders.

In another aspect of the present disclosure provides a method of treating mast cell activation syndrome (MCAS), comprising administering to the subject a therapeutically effective amount of a CRF receptor antagonist fatty acid conjugated cyclic peptide comprising or consisting of SEQ ID NO: 117, or of Formula (I), or a pharmaceutically acceptable salt thereof.

In some embodiments, the present disclosure provides a method of treating mast cell activation syndrome (MCAS), comprising administering to the subject a therapeutically effective amount of a peptide comprising or consisting of any one of formulae (Ia), (Ib), (Ic), (Id), (Ie), (If), (Ig), (Ih), or (Ik), or a pharmaceutically acceptable salt thereof.

In some embodiments, the present disclosure provides a method of treating mast cell activation syndrome (MCAS), comprising administering to the subject a therapeutically effective amount of any one of the peptides listed in Tables B1.1 and B1.2, or a pharmaceutically acceptable salt thereof.

In some embodiments, the present disclosure provides a method of treating mast cell activation syndrome (MCAS), comprising administering to the subject a therapeutically effective amount of the peptide comprising or consisting of SEQ ID NO: 50. In some embodiments, the peptide is a pharmaceutically acceptable salt thereof.

In some embodiments, the present disclosure provides a method of treating mast cell activation syndrome (MCAS), comprising administering to the subject a therapeutically effective amount of the peptide comprising or consisting of SEQ ID NO: 95. In some embodiments, the peptide is a pharmaceutically acceptable salt thereof.

In some embodiments, the present disclosure provides a method of treating mast cell activation syndrome (MCAS), comprising administering to the subject a therapeutically effective amount of the peptide comprising or consisting of SEQ ID NO: 97. In some embodiments, the peptide is a pharmaceutically acceptable salt thereof.

In some embodiments, the present disclosure provides a method of treating mast cell activation syndrome (MCAS), comprising administering to the subject a therapeutically effective amount of the peptide comprising or consisting of SEQ ID NO: 101. In some embodiments, the peptide is a pharmaceutically acceptable salt thereof.

In some embodiments, the present disclosure provides a fatty acid conjugated cyclic peptide comprising or consisting of SEQ ID NO: 117, or of Formula (I), or any other Formulae or SEQ ID NO. disclosed herein, or pharmaceutically acceptable salts thereof for use in the treatment of mast cell activation syndrome (MCAS).

Prevalence of autism spectrum disorder (ASD) in patients with mastocytosis, a rare disease occurring in 114,000 children and characterized by an increased number of hypersensitive mast cells in many organs, is about 10 times higher than the general population. Perinatal mast cell activation by infectious, stress-related, environmental or allergic triggers can lead to release of pro-inflammatory and neurotoxic molecules, thus contributing to brain inflammation and ASD pathogenesis, at least in a subgroup of ASD patients.

In another aspect of the present disclosure provides a method of treating autism spectrum disorder (ASD), comprising administering to the subject a therapeutically effective amount of a CRF receptor antagonist fatty acid conjugated cyclic peptide comprising or consisting of Formula (I), or a pharmaceutically acceptable salt thereof.

In some embodiments, the present disclosure provides a method of treating autism spectrum disorder (ASD), comprising administering to the subject a therapeutically effective amount of a peptide comprising or consisting of any one of formulae (Ia), (Ib), (Ic), (Id), (Ie), (If), (Ig), (Ih), or (Ik), or a pharmaceutically acceptable salt thereof.

In some embodiments, the present disclosure provides a method of treating autism spectrum disorder (ASD), comprising administering to the subject a therapeutically effective amount of any one of the peptides listed in Tables B1.1 and B1.2, or a pharmaceutically acceptable salt thereof.

In some embodiments, the present disclosure provides a method of treating autism spectrum disorder (ASD), comprising administering to the subject a therapeutically effective amount of the peptide comprising or consisting of SEQ ID NO: 50. In some embodiments, the peptide is a pharmaceutically acceptable salt thereof.

In some embodiments, the present disclosure provides a method of treating autism spectrum disorder (ASD), comprising administering to the subject a therapeutically effective amount of the peptide comprising or consisting of SEQ ID NO: 95. In some embodiments, the peptide is a pharmaceutically acceptable salt thereof.

In some embodiments, the present disclosure provides a method of treating autism spectrum disorder (ASD), comprising administering to the subject a therapeutically effective amount of the peptide comprising or consisting of SEQ ID NO: 97. In some embodiments, the peptide is a pharmaceutically acceptable salt thereof.

In some embodiments, the present disclosure provides a method of treating autism spectrum disorder (ASD), comprising administering to the subject a therapeutically effective amount of the peptide comprising or consisting of SEQ ID NO: 101. In some embodiments, the peptide is a pharmaceutically acceptable salt thereof.

In some embodiments, the present disclosure provides a fatty acid conjugated cyclic peptide comprising or consisting of SEQ ID NO: 117, or of Formula (I), or any other Formulae or SEQ ID NO. disclosed herein, or pharmaceutically acceptable salts thereof for use in the treatment of autism spectrum disorder (ASD).

The HPA axis is dysregulated in autism with ACTH levels reported to be higher in autistic patients with ACTH levels positively correlated with autistic disease severity. Aspergers patients also have elevated ACTH levels. Diurnal HPA rhythm is disrupted with lower morning and higher evening cortisol levels leading to the hypothesis that autistic patients are less able to adapt to changing situations and daily stressors. Plasma CRF and neurotensin are elevated in autistic patients but were not altered in unaffected control patients. Mast cells have been implicated in the pathophysiology of autism and elevated CRF and neurotensin contribute to mast cell activation. Therefore, correction of HPA axis with a peptide of the disclosure can improve a patient's ability to adapt and respond to changing situations/stressors leading to improved disease scores.

Certain mutational diseases have autistic-like characteristics. These include, but are not limited to, Fragile X syndrome, Rett syndrome, MeCP2 duplication syndrome, tuberous sclerosis, Angelman Syndrome, Downs Syndrome, and mastocytosis. Peptides of the disclosure, therefore, offer an opportunity to treat these mutation diseases.

A migraine is a type of headache characterized by recurrent attacks of moderate to severe throbbing and pulsating pain on one side of the head. Treatments for migraines include, for example: triptans, injectable Botox; VYEPTI^{™} (eptinezumab); and Amovig^{®} (erenumab-aooe). Mast cell degranulation has been implicated in the pathophysiology of migraine. Dura mast cells degranulate after electrical stimulation of the trigeminal or cervical ganglion and after acute psychological stress. Indeed, stress has frequently been cited as the most common migraine trigger. Peripherally acting peptides of the disclosure, therefore, can prevent stress-induced dura mast cell degranulation and trigeminal activation. Further, pituitary adenylate cyclase activating peptide (PACAP) has been implicated in migraine, is elevated during migraine attacks, and PACAP is secreted by activated mast cells. A peptide of the disclosure, therefore, may prevent release of PACAP from mast cells.

In another aspect of the present disclosure provides a method of treating or ameliorating symptoms of migraine, comprising administering to the subject a therapeutically effective amount of a CRF receptor antagonist fatty acid conjugated cyclic peptide comprising or consisting of SEQ ID NO: 117, or of Formula (I), or pharmaceutically acceptable salts thereof.

In some embodiments, the present disclosure provides a method of treating or ameliorating symptoms of migraine, comprising administering to the subject a therapeutically effective amount of a peptide comprising or consisting of any one of formulae (Ia), (Ib), (Ic), (Id), (Ie), (If), (Ig), (Ih), or (Ik), or a pharmaceutically acceptable salt thereof.

In some embodiments, the present disclosure provides a method of treating or ameliorating symptoms of migraine, comprising administering to the subject a therapeutically effective amount of any one of the peptides listed in Tables B1.1 and B1.2, or a pharmaceutically acceptable salt thereof.

In some embodiments, the present disclosure provides a method of treating or ameliorating symptoms of migraine, comprising administering to the subject a therapeutically effective amount of the peptide comprising or consisting of SEQ ID NO: 50. In some embodiments, the peptide is a pharmaceutically acceptable salt thereof.

In some embodiments, the present disclosure provides a method of treating or ameliorating symptoms of migraine, comprising administering to the subject a therapeutically effective amount of the peptide comprising or consisting of SEQ ID NO: 95. In some embodiments, the peptide is a pharmaceutically acceptable salt thereof.

In some embodiments, the present disclosure provides a method of treating or ameliorating symptoms of migraine, comprising administering to the subject a therapeutically effective amount of the peptide comprising or consisting of SEQ ID NO: 97. In some embodiments, the peptide is a pharmaceutically acceptable salt thereof.

In some embodiments, the present disclosure provides a method of treating or ameliorating symptoms of migraine, comprising administering to the subject a therapeutically effective amount of the peptide comprising or consisting of SEQ ID NO: 101. In some embodiments, the peptide is a pharmaceutically acceptable salt thereof.

In some embodiments, the present disclosure provides a fatty acid conjugated cyclic peptide comprising or consisting of SEQ ID NO: 117, or of Formula (I), or any other Formulae or SEQ ID NO. disclosed herein, or pharmaceutically acceptable salts thereof for use in the treatment of, or ameliorating symptoms of, migraine.

Cyclic vomiting syndrome (CVS) is a disorder characterized by recurrent episodes of nausea and severe vomiting with symptom-free intervals between the episodes. In the pediatric population, the prevalence of CVS is 1.9-2.3% and 3-5% of adult patients referred to GI motility centers. Common triggering factors include stress, emotional excitement, and infections. CVS has 3 phases: prodromal phase (when a patient senses an episode is coming), emetic phase, and a well phase between episodes. The diagnosis of CVS is made using the Rome III criteria that considers stereotypical episodes of vomiting lasting less than 1 week, with three or more of these episodes per year, and the absence of nausea or vomiting between episodes. During the prodromal phase, various prophylactic approaches and therapies are employed to alleviate stress and the ensuing emetic phase. These include lying down in a dark, quiet environment or hot bath, high carbohydrate ingestion and/or taking anti-emetic agents (for example, ondansetron and granisetron), anti-anxiety agents (for example, lorazepam), and anti-migraine agents (for example, sumatriptan and zolmitriptan). However, the effectiveness of these therapies has not been proven and oral medications are problematic during the emetic phase. Peptides of the disclosure can be useful for the treatment of CVS.

In another aspect of the present disclosure provides a method of treating or ameliorating symptoms of cyclic vomiting syndrome (CVS), comprising administering to the subject a therapeutically effective amount of a CRF receptor antagonist fatty acid conjugated cyclic peptide comprising or consisting of SEQ ID NO: 117, or of Formula (I), or pharmaceutically acceptable salts thereof.

In some embodiments, the present disclosure provides a method of treating or ameliorating symptoms of cyclic vomiting syndrome (CVS), comprising administering to the subject a therapeutically effective amount of a peptide comprising or consisting of any one of formulae (Ia), (Ib), (Ic), (Id), (Ie), (If), (Ig), (Ih), or (Ik), or a pharmaceutically acceptable salt thereof.

In some embodiments, the present disclosure provides a method of treating or ameliorating symptoms of cyclic vomiting syndrome (CVS), comprising administering to the subject a therapeutically effective amount of any one of the peptides listed in Tables B1.1 and B1.2, or a pharmaceutically acceptable salt thereof.

In some embodiments, the present disclosure provides a method of treating or ameliorating symptoms of cyclic vomiting syndrome (CVS), comprising administering to the subject a therapeutically effective amount of the peptide comprising or consisting of SEQ ID NO: 50. In some embodiments, the peptide is a pharmaceutically acceptable salt thereof.

In some embodiments, the present disclosure provides a method of treating or ameliorating symptoms of cyclic vomiting syndrome (CVS), comprising administering to the subject a therapeutically effective amount of the peptide comprising or consisting of SEQ ID NO: 95. In some embodiments, the peptide is a pharmaceutically acceptable salt thereof.

In some embodiments, the present disclosure provides a method of treating or ameliorating symptoms of cyclic vomiting syndrome (CVS), comprising administering to the subject a therapeutically effective amount of the peptide comprising or consisting of SEQ ID NO: 97. In some embodiments, the peptide is a pharmaceutically acceptable salt thereof.

In some embodiments, the present disclosure provides a method of treating or ameliorating symptoms of cyclic vomiting syndrome (CVS), comprising administering to the subject a therapeutically effective amount of the peptide comprising or consisting of SEQ ID NO: 101. In some embodiments, the peptide is a pharmaceutically acceptable salt thereof.

In some embodiments, the present disclosure provides a fatty acid conjugated cyclic peptide comprising or consisting of SEQ ID NO: 117, or of Formula (I), or any other Formulae or SEQ ID NO. disclosed herein, or pharmaceutically acceptable salts thereof for use in the treatment of, or ameliorating symptoms of, cyclic vomiting syndrome (CVS).

Pancreatitis is an inflammation of the pancreas. A UCN2 experimental acute pancreatitis (AP) model has been studied. UCN2 treatment prevented caerulein-induced degradation of IκB-α in the cytosolic fraction as well as increased levels of p65 subunit of NF-κB in the cytosolic fraction. Pancreatic UCN2 levels decreased in acute pancreatitis compared with saline. UCN2 evoked [Ca2+]i responses in primary acinar cells and abolished caerulein-evoked [Ca2+]i responses at 0.1 nM, and decreased responses by ~50% at 1.0 nM caerulein. UCN2 stimulation resulted in redistribution of a portion of F-actin from the apical to the basolateral pole. UCN2 prevented the massive redistribution of F-actin observed with supraphysiologic doses of caerulein. The protective effects of UCN2, including antiinflammatory and anti-necrotic effects involve activation of the CRF2 receptor, [Ca2+]i signaling, and inhibition of NF-κB activity. In a rat model of acute pancreatitis induced by intraductal infusion of sodium tetracholate, the mast cell stabilizer, sodium cromoglycate (SCG) reduced plasma exudation in the pancreas, colon, and lungs (P < 0.05). Further, there was reduced MPO activity and MCP1 levels in the colon and lungs but not in the pancreas, concluding that mast cells contribute to multi-organ failure and endothelial barrier dysfunction in pancreatitis. Pancreatic neuritis in pancreatic cancer (PCa) and chronic pancreatitis (CP) is composed of cytotoxic T-lymphocytes, macrophages, and mast cells (MC). The specific enrichment of MC around intrapancreatic nerves in neuropathic pain due to PCa and CP suggests the presence of MC-induced visceral hypersensitivity in the pancreas. Therefore, pancreatic and enteric neuropathies seem to share a similar type of neuro-immune interaction in the generation of visceral pain. Peptides of the disclosure can be useful for the treatment of pancreatitis.

In another aspect of the present disclosure provides a method of treating or ameliorating symptoms of pancreatitis, comprising administering to the subject a therapeutically effective amount of a CRF receptor antagonist fatty acid conjugated cyclic peptide comprising or consisting of SEQ ID NO: 117, or of Formula (I), or pharmaceutically acceptable salts thereof.

In some embodiments, the present disclosure provides a method of treating or ameliorating symptoms of pancreatitis, comprising administering to the subject a therapeutically effective amount of a peptide comprising or consisting of any one of formulae (Ia), (Ib), (Ic), (Id), (Ie), (If), (Ig), (Ih), or (Ik), or a pharmaceutically acceptable salt thereof.

In some embodiments, the present disclosure provides a method of treating or ameliorating symptoms of pancreatitis, comprising administering to the subject a therapeutically effective amount of any one of the peptides listed in Tables B1.1 and B1.2, or a pharmaceutically acceptable salt thereof.

In some embodiments, the present disclosure provides a method of treating or ameliorating symptoms of pancreatitis, comprising administering to the subject a therapeutically effective amount of the peptide comprising or consisting of SEQ ID NO: 50. In some embodiments, the peptide is a pharmaceutically acceptable salt thereof.

In some embodiments, the present disclosure provides a method of treating or ameliorating symptoms of pancreatitis, comprising administering to the subject a therapeutically effective amount of the peptide comprising or consisting of SEQ ID NO: 95. In some embodiments, the peptide is a pharmaceutically acceptable salt thereof.

In some embodiments, the present disclosure provides a method of treating or ameliorating symptoms of pancreatitis, comprising administering to the subject a therapeutically effective amount of the peptide comprising or consisting of SEQ ID NO: 97. In some embodiments, the peptide is a pharmaceutically acceptable salt thereof.

In some embodiments, the present disclosure provides a method of treating or ameliorating symptoms of pancreatitis, comprising administering to the subject a therapeutically effective amount of the peptide comprising or consisting of SEQ ID NO: 101. In some embodiments, the peptide is a pharmaceutically acceptable salt thereof.

In some embodiments, the present disclosure provides a fatty acid conjugated cyclic peptide comprising or consisting of SEQ ID NO: 117, or of Formula (I), or any other Formulae or SEQ ID NO. disclosed herein, or pharmaceutically acceptable salts thereof for use in the treatment of, or ameliorating symptoms of, pancreatitis.

Atopic dermatitis (eczema) is a condition that causes dry, itchy, and inflamed skin. Psoriasis (Ps) is a skin disease that causes a rash with itchy, scaly patches, most commonly on the knees, elbows, trunk, and scalp. CRF levels are elevated in patients with Atopic Dermatitis (AD) and Psoriasis (Ps) suggesting a link to stress-induced exacerbation of these diseases. Skin CRF1 expression is lowered in affected skin samples for AD and Psoriasis patients and there is a positive correlation between CRF serum levels and VEGF gene expression in Ps patients with PASI score >10 which is known to be associated with increased skin vascularization. Further, there is a correlation between serum CRH levels and State-Trait Anxiety Inventory scores (STAI-STATE now-construct measuring temporary effects) for Ps patients with PASI > 10 but there was no correlation with the longer term STAI construct (STATE trait-longer term). There was no correlation between STAI scores and CRH serum measurements or VEGF gene expression for the AD patients. Psychological stress can trigger Atopic Dermatitis in NC/Nga mice (a mouse model for atopic dermatitis) and there is an inhibitory effect of CRF. CRH-mediated suppression of IL-10 secretion from regulatory T cells (Tregs) might explain stress-related exacerbations in patients with AD. Mast cells have a role in neurogenic inflammation leading to pain and itch. Therefore, peptides of the disclosure can be useful for the treatment of AD and Ps.

In another aspect of the present disclosure provides a method of treating or ameliorating symptoms of atopic dermatitis (eczema) and Ps, comprising administering to the subject a therapeutically effective amount of a CRF receptor antagonist fatty acid conjugated cyclic peptide comprising or consisting of SEQ ID NO: 117, or of Formula (I), or pharmaceutically acceptable salts thereof.

In some embodiments, the present disclosure provides a method of treating or ameliorating symptoms of atopic dermatitis (eczema) and Ps, comprising administering to the subject a therapeutically effective amount of a peptide comprising or consisting of any one of formulae (Ia), (Ib), (Ic), (Id), (Ie), (If), (Ig), (Ih), or (Ik), or a pharmaceutically acceptable salt thereof.

In some embodiments, the present disclosure provides a method of treating or ameliorating symptoms of atopic dermatitis (eczema), comprising administering to the subject a therapeutically effective amount of any one of the peptides listed in Tables B1.1 and B1.2, or a pharmaceutically acceptable salt thereof.

In some embodiments, the present disclosure provides a method of treating or ameliorating symptoms of atopic dermatitis (eczema), comprising administering to the subject a therapeutically effective amount of the peptide comprising or consisting of SEQ ID NO: 50. In some embodiments, the peptide is a pharmaceutically acceptable salt thereof.

In some embodiments, the present disclosure provides a method of treating or ameliorating symptoms of atopic dermatitis (eczema), comprising administering to the subject a therapeutically effective amount of the peptide comprising or consisting of SEQ ID NO: 95. In some embodiments, the peptide is a pharmaceutically acceptable salt thereof.

In some embodiments, the present disclosure provides a method of treating or ameliorating symptoms of atopic dermatitis (eczema), comprising administering to the subject a therapeutically effective amount of the peptide comprising or consisting of SEQ ID NO: 97. In some embodiments, the peptide is a pharmaceutically acceptable salt thereof.

In some embodiments, the present disclosure provides a method of treating or ameliorating symptoms of atopic dermatitis (eczema), comprising administering to the subject a therapeutically effective amount of the peptide comprising or consisting of SEQ ID NO: 101. In some embodiments, the peptide is a pharmaceutically acceptable salt thereof.

In some embodiments, the present disclosure provides a fatty acid conjugated cyclic peptide comprising or consisting of SEQ ID NO: 117, or of Formula (I), or any other Formulae or SEQ ID NO. disclosed herein, or pharmaceutically acceptable salts thereof for use in the treatment of, or ameliorating symptoms of, atopic dermatitis (eczema).

Rheumatoid Arthritis (RA) is an autoimmune and inflammatory disease where the immune system attacks healthy cells in the body, causing inflammation. Approved drugs to treat RA range from methotrexate and JAK inhibitors (for example, Xeljanz, and Rinvoq) to a variety of biologics that block various cytokines as well as other immune cell proteins such as Embrel (anti-TNF), Kevzara (anti-IL6) and Rituxan (anti-CD20). In addition, although not approved for RA, data suggests that anti-IL17 biologics, for example, Cosentyx (Secukinumab) results in improvement in signs and symptoms and reduced disease activity in patients with active RA who had an inadequate response to TNF inhibitors. Urocortin, CRF1 and CRF2*α* mRNAs, detected by RT-PCR, were expressed in the synovium of 10/10, 10/10 and 2/10 RA patients respectively, but CRF2β was not expressed. Further, urocortin is actively synthesized in the synovium of RA patients. Analysis of synovial tissue from an early arthritis patient cohort (n = 9) established that expression of CRH-R1 significantly (P < 0.03) colocalized with PECAM-1 and E-selectin expression *in vivo*. The expression was enhanced by histamine. Chronic blockade of CRH-R1 with systemic antalarmin (a non-peptide drug that blocks the CRH1 receptor) significantly ameliorated adjuvant induced arthritis (AIA) in LEW/N rats, reducing the severity of inflammation in peripheral joints, evidenced by clinical and histopathology scores, and weight loss associated with disease onset. The effect was seen despite a lowering of corticosterone levels. UCN treatment significantly reduced severity and incidence of collagen-induced arthritis (CIA) and was associated with a reduction of the Th1-driven autoimmune response and generation and/or activation of interleukin-10/ transforming growth factor 1-producing Treg cells. Mast cells have been implicated in RA joint inflammation and numbers correlate with RA disease severity. Therefore, peptides of the disclosure can be useful for the treatment of RA.

In another aspect of the present disclosure provides a method of treating or ameliorating symptoms of rheumatoid arthritis (RA), comprising administering to the subject a therapeutically effective amount of a CRF receptor antagonist fatty acid conjugated cyclic peptide comprising or consisting of SEQ ID NO: 117, or of Formula (I), or pharmaceutically acceptable salts thereof.

In some embodiments, the present disclosure provides a method of treating or ameliorating symptoms of rheumatoid arthritis (RA), comprising administering to the subject a therapeutically effective amount of a peptide comprising or consisting of any one of formulae (Ia), (Ib), (Ic), (Id), (Ie), (If), (Ig), (Ih), or (Ik), or a pharmaceutically acceptable salt thereof.

In some embodiments, the present disclosure provides a method of treating or ameliorating symptoms of rheumatoid arthritis (RA), comprising administering to the subject a therapeutically effective amount of any one of the peptides listed in Tables B1.1 and B1.2, or a pharmaceutically acceptable salt thereof.

In some embodiments, the present disclosure provides a method of treating or ameliorating symptoms of rheumatoid arthritis (RA), comprising administering to the subject a therapeutically effective amount of the peptide comprising or consisting of SEQ ID NO: 50. In some embodiments, the peptide is a pharmaceutically acceptable salt thereof.

In some embodiments, the present disclosure provides a method of treating or ameliorating symptoms of rheumatoid arthritis (RA), comprising administering to the subject a therapeutically effective amount of the peptide comprising or consisting of SEQ ID NO: 95. In some embodiments, the peptide is a pharmaceutically acceptable salt thereof.

In some embodiments, the present disclosure provides a method of treating or ameliorating symptoms of rheumatoid arthritis (RA), comprising administering to the subject a therapeutically effective amount of the peptide comprising or consisting of SEQ ID NO: 97. In some embodiments, the peptide is a pharmaceutically acceptable salt thereof.

In some embodiments, the present disclosure provides a method of treating or ameliorating symptoms of rheumatoid arthritis (RA), comprising administering to the subject a therapeutically effective amount of the peptide comprising or consisting of SEQ ID NO: 101. In some embodiments, the peptide is a pharmaceutically acceptable salt thereof.

In some embodiments, the present disclosure provides a fatty acid conjugated cyclic peptide comprising or consisting of SEQ ID NO: 117, or of Formula (I), or any other Formulae or SEQ ID NO. disclosed herein, or pharmaceutically acceptable salts thereof for use in the treatment of, or ameliorating symptoms of, rheumatoid arthritis (RA).

Obesity, Diabetes and Prader Willi Syndrome (PWS). PWS is a rare genetic condition that affects a child's metabolism and causes changes to their body and behavior. Regarding food intake and body weight reduction, evidence has been reported for a dose-dependent decrease in food intake and body weight in mice treated with urocortin 2 (UCN2) or urocortin 3 (UCN3). UCN2 decreases food intake without stress effects. UCN3 transgenics show decreases in food intake and body weight and increased muscle mass and are resistant to obesity. Urocortin gene transfer by i.v. administration resulted in decreases in body weight. CRF2 knockouts have increased food intake and body weight. CRF2 knockdown in the VMH increases food intake. UCN3 KO mice have increased food intake and body weight. Antisauvagine blocks ICV CRF and urocortin-induced decreases in food intake. Urocortin 2 analogs decrease food intake and body weight. With respect to gastric emptying, urocortin decreases gastric emptying in mice. Regarding pancreatic endocrine effects, CRF1 and CRF2 receptors have been located on beta cells, islets, and delta cells. Min6 cells exhibit glucose-inducible insulin secretion comparable with cultured normal mouse islet cells and glucocorticoids upregulate CRF2 expression in min6 cells. UCN3 is expressed in pancreatic beta-cells and stimulates insulin and glucagon secretion. UCN3 regulates glucose-stimulated insulin secretion and energy homeostasis. UCN3 mediates somatostatin-dependent negative feedback control of insulin secretion. Peptides of the disclosure can be useful for the treatment of obesity, diabetes, and Prader Willi Syndrome (PWS).

In another aspect of the present disclosure provides a method of treating or ameliorating symptoms of Obesity, Diabetes or Prader Willi Syndrome, comprising administering to the subject a therapeutically effective amount of a CRF receptor antagonist fatty acid conjugated cyclic peptide comprising or consisting of SEQ ID NO: 117, or of Formula (I), or pharmaceutically acceptable salts thereof.

In some embodiments, the present disclosure provides a method of treating or ameliorating symptoms of Obesity, Diabetes or Prader Willi Syndrome, comprising administering to the subject a therapeutically effective amount of a peptide comprising or consisting of any one of formulae (Ia), (Ib), (Ic), (Id), (Ie), (If), (Ig), (Ih), or (Ik), or a pharmaceutically acceptable salt thereof.

In some embodiments, the present disclosure provides a method of treating or ameliorating symptoms of Obesity, Diabetes or Prader Willi Syndrome, comprising administering to the subject a therapeutically effective amount of any one of the peptides listed in Tables B1.1 and B1.2, or a pharmaceutically acceptable salt thereof.

In some embodiments, the present disclosure provides a method of treating or ameliorating symptoms of Obesity, Diabetes or Prader Willi Syndrome, comprising administering to the subject a therapeutically effective amount of the peptide comprising or consisting of SEQ ID NO: 50. In some embodiments, the peptide is a pharmaceutically acceptable salt thereof.

In some embodiments, the present disclosure provides a method of treating or ameliorating symptoms of Obesity, Diabetes or Prader Willi Syndrome, comprising administering to the subject a therapeutically effective amount of the peptide comprising or consisting of SEQ ID NO: 95. In some embodiments, the peptide is a pharmaceutically acceptable salt thereof.

In some embodiments, the present disclosure provides a method of treating or ameliorating symptoms of Obesity, Diabetes or Prader Willi Syndrome, comprising administering to the subject a therapeutically effective amount of the peptide comprising or consisting of SEQ ID NO: 97. In some embodiments, the peptide is a pharmaceutically acceptable salt thereof.

In some embodiments, the present disclosure provides a method of treating or ameliorating symptoms of Obesity, Diabetes or Prader Willi Syndrome, comprising administering to the subject a therapeutically effective amount of the peptide comprising or consisting of SEQ ID NO: 101. In some embodiments, the peptide is a pharmaceutically acceptable salt thereof.

In some embodiments, the present disclosure provides a fatty acid conjugated cyclic peptide comprising or consisting of SEQ ID NO: 117, or of Formula (I), or any other Formulae or SEQ ID NO. disclosed herein, or pharmaceutically acceptable salts thereof for use in the treatment of, or ameliorating symptoms of Obesity, Diabetes or Prader Willi syndrome.

Endometriosis is a disease in which tissue similar to the lining of the uterus grows outside the uterus. Plasma urocortin levels were twice as high in women with endometrioma (median 49 pg/mL, interquartile range 41-63 pg/mL) than in the control group ([15-23] pg/mL, P<.001) and significantly higher in the cystic content of endometriomas than in the peritoneal fluid and plasma (P<.05). Urocortin was immunolocalized in endometrioma glands and stromal capillary vessels. Elevated plasma urocortin levels are detected in 88% of the cases of endometrioma with 90% specificity, whereas cancer antigen (CA-125) detected only 65% of the cases with the same specificity. Serum prolactin levels were significantly higher in infertile women with stage III-IV endometriosis (28.9 ± 2.1 ng/mL) than in healthy controls (13.2 ± 2.1 ng/mL). Serum cortisol levels were significantly higher in infertile women with stage III-IV endometriosis (20.1 ± 1.3 ng/mL) than in controls 10.5 ± 1.4 ng/mL). Moreover, since higher levels of cortisol and prolactin are often associated with stress, it is probable that stress might contribute to the development of endometriosis and its progression to advanced stages of the disease. Endometrial expression of urocortin II mRNA was significantly (*P*<.01) higher in the early proliferative phase of the menstrual cycle than in other phases and maternal decidua (MD), whereas urocortin III mRNA was higher (*P*<.01) in MD than in all other endometrial samples. CRH and UCN receptor subtypes CRHR1β and CRHR2α are expressed in endometriotic biopsy sites and they are more strongly expressed (p,<0.01) in eutopic endometrium of women with endometriosis compared to healthy women endometrium at the mRNA and protein level³. CRH, UCN, CRHR1 and CRHR2 mRNA were also more highly expressed in ectopic rather than eutopic endometrium (CRH, UCN, CRHR2α: p,<0.01, CRHR1β: p,<0.05) and protein (CRH and UCN: p,<0.05, CRHR1 and CRHR2: p,<0.01) in women with endometriosis. High numbers of activated mast cells are present in endometriosis sites that were strongly positive for CRH and Urocortin. CRH and Urocortin may activate mast cells and contribute to fibrosis and inflammation in endometriosis. Peptides of the disclosure can be useful for the treatment of endometriosis.

In another aspect of the present disclosure provides a method of treating or ameliorating symptoms of endometriosis, comprising administering to the subject a therapeutically effective amount of a CRF receptor antagonist fatty acid conjugated cyclic peptide comprising or consisting of SEQ ID NO: 117, or of Formula (I), or pharmaceutically acceptable salts thereof.

In some embodiments, the present disclosure provides a method of treating or ameliorating symptoms of endometriosis, comprising administering to the subject a therapeutically effective amount of a peptide comprising or consisting of any one of formulae (Ia), (Ib), (Ic), (Id), (Ie), (If), (Ig), (Ih), or (Ik), or a pharmaceutically acceptable salt thereof.

In some embodiments, the present disclosure provides a method of treating or ameliorating symptoms of endometriosis, comprising administering to the subject a therapeutically effective amount of any one of the peptides listed in Tables B1.1 and B1.2, or a pharmaceutically acceptable salt thereof.

In some embodiments, the present disclosure provides a method of treating or ameliorating symptoms of endometriosis, comprising administering to the subject a therapeutically effective amount of the peptide comprising or consisting of SEQ ID NO: 50. In some embodiments, the peptide is a pharmaceutically acceptable salt thereof.

In some embodiments, the present disclosure provides a method of treating or ameliorating symptoms of endometriosis, comprising administering to the subject a therapeutically effective amount of the peptide comprising or consisting of SEQ ID NO: 95. In some embodiments, the peptide is a pharmaceutically acceptable salt thereof.

In some embodiments, the present disclosure provides a method of treating or ameliorating symptoms of endometriosis, comprising administering to the subject a therapeutically effective amount of the peptide comprising or consisting of SEQ ID NO: 97. In some embodiments, the peptide is a pharmaceutically acceptable salt thereof.

In some embodiments, the present disclosure provides a method of treating or ameliorating symptoms of endometriosis, comprising administering to the subject a therapeutically effective amount of the peptide comprising or consisting of SEQ ID NO: 101. In some embodiments, the peptide is a pharmaceutically acceptable salt thereof.

In some embodiments, the present disclosure provides a fatty acid conjugated cyclic peptide comprising or consisting of SEQ ID NO: 117, or of Formula (I), or any other Formulae or SEQ ID NO. disclosed herein, or pharmaceutically acceptable salts thereof for use in the treatment of, or ameliorating symptoms of, endometriosis.

Polycystic Ovary Syndrome (PCOS) is a hormone problem for women of childbearing age. According to the Centers for Disease Control and Prevention (CDC), women with PCOS are often insulin resistant; their bodies can make insulin but can't use it effectively, increasing their risk for type 2 diabetes. They also have higher levels of androgens (male hormones that females also have), which can stop eggs from being released (ovulation) and cause irregular periods, acne, thinning scalp hair, and excess hair growth on the face and body. Urocortin 2 levels are increased in patients with PCOS. Positive correlations were seen with testosterone levels and insulin resistance in PCOS patients. Urocortin 3 inhibits progesterone from cultured human granulosa cells. Therefore, peptides of the disclosure can be useful for the treatment of PCOS.

In another aspect of the present disclosure provides a method of treating or ameliorating symptoms of PCOS, comprising administering to the subject a therapeutically effective amount of a CRF receptor antagonist fatty acid conjugated cyclic peptide comprising or consisting of SEQ ID NO: 117, or of Formula (I), or pharmaceutically acceptable salts thereof.

In some embodiments, the present disclosure provides a method of treating or ameliorating symptoms of PCOS, comprising administering to the subject a therapeutically effective amount of a peptide comprising or consisting of any one of formulae (Ia), (Ib), (Ic), (Id), (Ie), (If), (Ig), (Ih), or (Ik), or a pharmaceutically acceptable salt thereof.

In some embodiments, the present disclosure provides a method of treating or ameliorating symptoms of PCOS, comprising administering to the subject a therapeutically effective amount of any one of the peptides listed in Tables B1.1 and B1.2, or a pharmaceutically acceptable salt thereof.

In some embodiments, the present disclosure provides a method of treating or ameliorating symptoms of PCOS, comprising administering to the subject a therapeutically effective amount of the peptide comprising or consisting of SEQ ID NO: 50. In some embodiments, the peptide is a pharmaceutically acceptable salt thereof.

In some embodiments, the present disclosure provides a method of treating or ameliorating symptoms of PCOS, comprising administering to the subject a therapeutically effective amount of the peptide comprising or consisting of SEQ ID NO: 95. In some embodiments, the peptide is a pharmaceutically acceptable salt thereof.

In some embodiments, the present disclosure provides a method of treating or ameliorating symptoms of PCOS, comprising administering to the subject a therapeutically effective amount of the peptide comprising or consisting of SEQ ID NO: 97. In some embodiments, the peptide is a pharmaceutically acceptable salt thereof.

In some embodiments, the present disclosure provides a method of treating or ameliorating symptoms of PCOS, comprising administering to the subject a therapeutically effective amount of the peptide comprising or consisting of SEQ ID NO: 101. In some embodiments, the peptide is a pharmaceutically acceptable salt thereof.

In some embodiments, the present disclosure provides a fatty acid conjugated cyclic peptide comprising or consisting of SEQ ID NO: 117, or of Formula (I), or any other Formulae or SEQ ID NO. disclosed herein, or pharmaceutically acceptable salts thereof for use in the treatment of, or ameliorating symptoms of, PCOS.

Hypothalamic amenorrhea is a condition where women do not menstruate due to a problem involving the hypothalamus. The hypothalamus regulates certain aspects of reproduction by releasing gonadotropin-releasing hormone (GnRH), a hormone that works with follicle-stimulating hormone (FSH), luteinizing hormone (LH) and estrogen to control menstruation.

In some embodiments, the present disclosure provides a method of treating or ameliorating symptoms of hypothalamic amenorrhea, comprising administering to the subject a therapeutically effective amount of a peptide comprising or consisting of any one of formulae (Ia), (Ib), (Ic), (Id), (Ie), (If), (Ig), (Ih), or (Ik), or a pharmaceutically acceptable salt thereof.

In some embodiments, the present disclosure provides a method of treating or ameliorating symptoms of hypothalamic amenorrhea, comprising administering to the subject a therapeutically effective amount of any one of the peptides listed in Tables B1.1 and B1.2, or a pharmaceutically acceptable salt thereof.

In some embodiments, the present disclosure provides a method of treating or ameliorating symptoms of hypothalamic amenorrhea, comprising administering to the subject a therapeutically effective amount of the peptide comprising or consisting of SEQ ID NO: 50. In some embodiments, the peptide is a pharmaceutically acceptable salt thereof.

In some embodiments, the present disclosure provides a method of treating or ameliorating symptoms of hypothalamic amenorrhea, comprising administering to the subject a therapeutically effective amount of the peptide comprising or consisting of SEQ ID NO: 95. In some embodiments, the peptide is a pharmaceutically acceptable salt thereof.

In some embodiments, the present disclosure provides a method of treating or ameliorating symptoms of hypothalamic amenorrhea, comprising administering to the subject a therapeutically effective amount of the peptide comprising or consisting of SEQ ID NO: 97. In some embodiments, the peptide is a pharmaceutically acceptable salt thereof.

In some embodiments, the present disclosure provides a method of treating or ameliorating symptoms of hypothalamic amenorrhea, comprising administering to the subject a therapeutically effective amount of the peptide comprising or consisting of SEQ ID NO: 101. In some embodiments, the peptide is a pharmaceutically acceptable salt thereof.

In some embodiments, the present disclosure provides a fatty acid conjugated cyclic peptide comprising or consisting of SEQ ID NO: 117, or of Formula (I), or any other Formulae or SEQ ID NO. disclosed herein, or pharmaceutically acceptable salts thereof for use in the treatment of, or ameliorating symptoms of hypothalamic, amenorrhea.

Heart failure: urocortin is produced in the human heart and stored there mainly in the larger molecular weight forms. Endogenously produced Urocortin may therefore exert its effects mostly through CRF-R2 in an autocrine and/or paracrine manner in the human heart. According to predefined systolic blood pressure (SBP) groups, the percentage of patients in each group was 0.8% (<90 mmHg), 21.7% (90-119 mmHg), 44.9% (120-149 mmHg), 24.5% (150-179 mmHg), and 8.1% (>179 mmHg). In total, 437 (42.4%) patients presented at admission with high SBP defined as >140 mmHg according to the current guidelines. Fifty percent of the patients had SBP higher than 140 mm Hg at admission. Patients with lower SBP at admission had higher in-hospital and post-discharge mortality rates. Higher SBP at admission was associated with lower in-hospital mortality rates: 7.2% (120 mm Hg), 3.6% (120-139 mm Hg), 2.5% (140-161 mm Hg), and 1.7% (161 mm Hg) (*P* < .001 for overall difference). Post-discharge mortality rates in the follow-up cohort by SBP at admission were 14.0%, 8.4%, 6.0%, and 5.4%, respectively (*P* < .001 for overall difference). RT-PCR analysis demonstrated that UCN and CRF-R2 mRNAs were detected in all four chambers. CRF-R1 mRNA was weakly present in some left atria, left ventricles, and in one right ventricle. CRF-R2 mRNA was detected predominantly in the left atrium. CRF mRNA was not detected in any of the four chambers. Immunostaining for both UCN and CRF receptors was detected in cardiac myocytes in all four chambers. UCN-like immunoreactivity was detected in all four chambers by radioimmunoassay (RIA), with the highest concentrations in the left ventricle. On the other hand, CRF-like immunoreactivity was very low or undetectable in the human heart. Immunocytochemistry showed positive staining of UCN3 in the human myocardium and the renal kidney tubules, particularly distal tubules and urocortin II mRNA has been detected in heart and cardiomyocytes. In a mouse model of heart failure, single i.v. bolus administration of UCN2 to a heart failure model (muscle-specific LIM protein-deficient mice) produced significant enhancement of inotropic and lusitropic effects on left ventricular function and improved cardiac output. UCN2 also reduced systemic arterial pressure, associated with a lowering of systemic arterial elastance (end-systolic pressure stroke volume) and systemic vascular resistance. In acute heart failure patients in the UNICORN study (Urocortin-2 in the Treatment of Acute Heart Failure as an Adjunct Over Conventional Therapy), UCN2 infusion in acute decompensated heart failure (ADHF) patients markedly augmented cardiac output without significant reflex tachycardia. Urocortin-2 produced greater falls in systolic blood pressure compared to placebo (16 ± 5.8 mm Hg, *P <* 0.001) with nonsignificant increases in heart rate (5.7 ± 3.8 beats/min, *P* = 0.07) and increased cardiac output (2.1 ± 0.4 l/min vs. - 0.1 ± 0.4 l/min, *P* < 0.001) associated with a 47% reduction in calculated total peripheral resistance (p = 0.015). Plasma UCN2 levels were 7.5-fold higher in patients with heart failure compared to those in healthy controls. Additionally, cardiomyocyte-specific deletion of CRF2 protected mice from pressure overload-induced cardiac dysfunction. Mice treated with a CRF2 peptide antagonist, antisauvagine-30, lost maladaptive cAMP-dependent signaling and did not develop heart failure in response to overload. Patients with non-ischemic dilated cardiomyopathy (NIDCM) had significantly lower SBP and diastolic blood pressure and significantly higher creatinine levels. Of note, patients with NIDCM exhibited significantly higher UCN2 levels (a median 7.5-fold increase) than healthy controls, which remained significant after adjustment for all measured parameters (*P* < 0.01). The increased urocortin levels observed during heart failure are sufficient to lower blood pressure based on other studies. Prevention of urocortin-induced hypotension at admission and discharge may improve mortality. Therefore, peptides of the disclosure can be useful for the treatment of heart failure.

In another aspect of the present disclosure provides a method of treating or ameliorating symptoms of heart failure, comprising administering to the subject a therapeutically effective amount of a CRF receptor antagonist fatty acid conjugated cyclic peptide comprising or consisting of SEQ ID NO: 117, or of Formula (I), or pharmaceutically acceptable salts thereof.

In some embodiments, the present disclosure provides a method of treating or ameliorating symptoms of heart failure, comprising administering to the subject a therapeutically effective amount of a peptide comprising or consisting of any one of formulae (Ia), (Ib), (Ic), (Id), (Ie), (If), (Ig), (Ih), or (Ik), or a pharmaceutically acceptable salt thereof.

In some embodiments, the present disclosure provides a method of treating or ameliorating symptoms of heart failure, comprising administering to the subject a therapeutically effective amount of any one of the peptides listed in Tables B1.1 and B1.2, or a pharmaceutically acceptable salt thereof.

In some embodiments, the present disclosure provides a method of treating or ameliorating symptoms of heart failure, comprising administering to the subject a therapeutically effective amount of the peptide comprising or consisting of SEQ ID NO: 50. In some embodiments, the peptide is a pharmaceutically acceptable salt thereof.

In some embodiments, the present disclosure provides a method of treating or ameliorating symptoms of heart failure, comprising administering to the subject a therapeutically effective amount of the peptide comprising or consisting of SEQ ID NO: 95. In some embodiments, the peptide is a pharmaceutically acceptable salt thereof.

In some embodiments, the present disclosure provides a method of treating or ameliorating symptoms of heart failure, comprising administering to the subject a therapeutically effective amount of the peptide comprising or consisting of SEQ ID NO: 97. In some embodiments, the peptide is a pharmaceutically acceptable salt thereof.

In some embodiments, the present disclosure provides a method of treating or ameliorating symptoms of heart failure, comprising administering to the subject a therapeutically effective amount of the peptide comprising or consisting of SEQ ID NO: 101. In some embodiments, the peptide is a pharmaceutically acceptable salt thereof.

In some embodiments, the present disclosure provides a fatty acid conjugated cyclic peptide comprising or consisting of SEQ ID NO: 117, or of Formula (I), or any other Formulae or SEQ ID NO. disclosed herein or pharmaceutically acceptable salts thereof for use in the treatment of, or ameliorating symptoms of, heart failure.

Orthostatic Hypotension, according to NIH, orthostatic hypotension is defined as a decrease in blood pressure by at least 20 mmHg systolic or 10 mmHg diastolic within 3 minutes of standing and occurs in approximately 6% of the population. According to the Mayo Clinic, orthostatic hypotension can be caused by dehydration, heart problems, endocrine problems (for example, adrenal insufficiency, nervous system disorders (neurogenic) such as Parkinson's Disease, Multi System Atrophy, Pure Autonomic Failure, Lewy Body Dementia, Amyloidosis), and after eating a meal (Postprandial Hypotension). Northera (droxidopa) was approved for treatment of orthostatic dizziness, lightheadedness, or the "feeling that you are about to black out" in adult patients with symptomatic neurogenic orthostatic hypotension caused by primary autonomic failure (Parkinson's disease, multiple system atrophy, and pure autonomic failure), dopamine beta-hydroxylase deficiency, and non-diabetic autonomic neuropathy. It has a black box warning for supine hypertension and although it's MOA is stated as not known Northera is a synthetic amino acid analog that is directly metabolized to norepinephrine by dopa-decarboxylase. Effectiveness beyond two weeks has not been established. Orthostatic hypertension is often characterized by low catecholamine levels. Peptides of the disclosure can be useful for the treatment of orthostatic hypotension.

In another aspect of the present disclosure provides a method of treating or ameliorating symptoms of orthostatic hypotension, comprising administering to the subject a therapeutically effective amount of a CRF receptor antagonist fatty acid conjugated cyclic peptide comprising or consisting of Formula (I), or a pharmaceutically acceptable salt thereof.

In some embodiments, the present disclosure provides a method of treating or ameliorating symptoms of orthostatic hypotension, comprising administering to the subject a therapeutically effective amount of a peptide comprising or consisting of any one of formulae (Ia), (Ib), (Ic), (Id), (Ie), (If), (Ig), (Ih), or (Ik), or a pharmaceutically acceptable salt thereof.

In some embodiments, the present disclosure provides a method of treating or ameliorating symptoms of orthostatic hypotension, comprising administering to the subject a therapeutically effective amount of any one of the peptides listed in Tables B1.1 and B1.2, or a pharmaceutically acceptable salt thereof.

In some embodiments, the present disclosure provides a method of treating or ameliorating symptoms of orthostatic hypotension, comprising administering to the subject a therapeutically effective amount of the peptide comprising or consisting of SEQ ID NO: 50. In some embodiments, the peptide is a pharmaceutically acceptable salt thereof.

In some embodiments, the present disclosure provides a method of treating or ameliorating symptoms of orthostatic hypotension, comprising administering to the subject a therapeutically effective amount of the peptide comprising or consisting of SEQ ID NO: 95. In some embodiments, the peptide is a pharmaceutically acceptable salt thereof.

In some embodiments, the present disclosure provides a method of treating or ameliorating symptoms of orthostatic hypotension, comprising administering to the subject a therapeutically effective amount of the peptide comprising or consisting of SEQ ID NO: 97. In some embodiments, the peptide is a pharmaceutically acceptable salt thereof.

In some embodiments, the present disclosure provides a method of treating or ameliorating symptoms of orthostatic hypotension, comprising administering to the subject a therapeutically effective amount of the peptide comprising or consisting of SEQ ID NO: 101. In some embodiments, the peptide is a pharmaceutically acceptable salt thereof.

In some embodiments, the present disclosure provides a fatty acid conjugated cyclic peptide comprising or consisting of SEQ ID NO: 117, or of Formula (I), or any other Formulae or SEQ ID NO. disclosed herein, or pharmaceutically acceptable salts thereof for use in the treatment of, or ameliorating symptoms of, orthostatic hypotension.

Bladder Cystitis or bladder inflammation happens in certain infections caused by bacteria, for example, a urinary tract infection (UTI). CRF immunoreactivity (IR) was increased significantly (P < 0.01) in the urothelium and sacral parasympathetic nucleus (SPN) after cyclophosphamide (CYP) treatment. CRF IR nerve fibers increased in density in the suburothelial plexus and detrusor smooth muscle whole mounts with CYP-induced cystitis. CRF2 receptor transcript was expressed in the urothelium or detrusor smooth muscle, and CRF2 receptor expression increased in whole bladder with CYP-treatment, whereas no CRF 1 receptor transcript was expressed in either urothelium or detrusor. Immunohistochemical studies demonstrated CRF2 IR in urinary bladder nerve fibers and urothelial cells from control animals, whereas no CRF1 IR was observed. Immunochemical staining revealed CRHR1 expression was mainly in the submucosa, while CRHR2 expression was mainly in uroepithelial cells. Compared to control subjects, the CRHR1 expression was significantly higher, while CRHR2 expression was significantly lower in IC/BPS patients. Further analysis of patients with HIC, NHIC, and control subjects showed that bladder in patients with HIC had significantly higher expression of CRHR1 and significantly lower CRHR2. CRHR2 expression was significantly negatively correlated with O'Leary-Sant score and bladder pain. Most human studies have shown an increase in mast cell numbers and activation in patients with IC. VEGF, another mast cell mediator, causes vasodilation and may therefore be responsible for the hypervascularity and glomerulations, which are characteristic of IC. Studies have also shown an association between increased VEGF and severity of IC-associated pain. In human mast cells, CRH increased cAMP and induced secretion of vascular endothelial growth factor (VEGF) without tryptase, histamine, IL-6, IL-8, or TNF- release. Peptides of the disclosure can be useful for the treatment of bladder cystitis.

In another aspect of the present disclosure provides a method of treating or ameliorating symptoms of bladder cystitis, comprising administering to the subject a therapeutically effective amount of a CRF receptor antagonist fatty acid conjugated cyclic peptide comprising or consisting of SEQ ID NO: 117, or of Formula (I), or pharmaceutically acceptable salts thereof.

In some embodiments, the present disclosure provides a method of treating or ameliorating symptoms of bladder cystitis, comprising administering to the subject a therapeutically effective amount of a peptide comprising or consisting of any one of formulae (Ia), (Ib), (Ic), (Id), (Ie), (If), (Ig), (Ih), or (Ik), or a pharmaceutically acceptable salt thereof.

In some embodiments, the present disclosure provides a method of treating or ameliorating symptoms of bladder cystitis, comprising administering to the subject a therapeutically effective amount of any one of the peptides listed in Tables B1.1 and B1.2, or a pharmaceutically acceptable salt thereof.

In some embodiments, the present disclosure provides a method of treating or ameliorating symptoms of bladder cystitis, comprising administering to the subject a therapeutically effective amount of the peptide comprising or consisting of SEQ ID NO: 50. In some embodiments, the peptide is a pharmaceutically acceptable salt thereof.

In some embodiments, the present disclosure provides a method of treating or ameliorating symptoms of bladder cystitis, comprising administering to the subject a therapeutically effective amount of the peptide comprising or consisting of SEQ ID NO: 95. In some embodiments, the peptide is a pharmaceutically acceptable salt thereof.

In some embodiments, the present disclosure provides a method of treating or ameliorating symptoms of bladder cystitis, comprising administering to the subject a therapeutically effective amount of the peptide comprising or consisting of SEQ ID NO: 97. In some embodiments, the peptide is a pharmaceutically acceptable salt thereof.

In some embodiments, the present disclosure provides a method of treating or ameliorating symptoms of bladder cystitis, comprising administering to the subject a therapeutically effective amount of the peptide comprising or consisting of SEQ ID NO: 101. In some embodiments, the peptide is a pharmaceutically acceptable salt thereof.

Alopecia Areata is an autoimmune disorder that causes the hair of individuals afflicted with the condition to come out, often in clumps. It can be triggered by environmental factors like stress, injury, or illness. But in many cases, there is no clear answer as to why someone develops alopecia areata. CRF over-expressing (OE)-mice display phenotypes of Cushing's syndrome and chronic stress, including alopecia. Peptides of the disclosure can be useful for the treatment of alopecia areata.

In another aspect of the present disclosure provides a method of treating or ameliorating symptoms of alopecia areata, comprising administering to the subject a therapeutically effective amount of a CRF receptor antagonist fatty acid conjugated cyclic peptide comprising or consisting of SEQ ID NO: 117, or of Formula (1), or pharmaceutically acceptable salts thereof.

In some embodiments, the present disclosure provides a method of treating or ameliorating symptoms of alopecia areata, comprising administering to the subject a therapeutically effective amount of a peptide comprising or consisting of any one of formulae (Ia), (Ib), (Ic), (Id), (Ie), (If), (Ig), (Ih), or (Ik), or a pharmaceutically acceptable salt thereof.

In some embodiments, the present disclosure provides a method of treating or ameliorating symptoms of alopecia areata, comprising administering to the subject a therapeutically effective amount of any one of the peptides listed in Tables B1.1 and B1.2, or a pharmaceutically acceptable salt thereof.

In some embodiments, the present disclosure provides a method of treating or ameliorating symptoms of alopecia areata, comprising administering to the subject a therapeutically effective amount of the peptide comprising or consisting of SEQ ID NO: 50. In some embodiments, the peptide is a pharmaceutically acceptable salt thereof.

In some embodiments, the present disclosure provides a method of treating or ameliorating symptoms of alopecia areata, comprising administering to the subject a therapeutically effective amount of the peptide comprising or consisting of SEQ ID NO: 95. In some embodiments, the peptide is a pharmaceutically acceptable salt thereof.

In some embodiments, the present disclosure provides a method of treating or ameliorating symptoms of alopecia areata, comprising administering to the subject a therapeutically effective amount of the peptide comprising or consisting of SEQ ID NO: 97. In some embodiments, the peptide is a pharmaceutically acceptable salt thereof.

In some embodiments, the present disclosure provides a method of treating or ameliorating symptoms of alopecia areata, comprising administering to the subject a therapeutically effective amount of the peptide comprising or consisting of SEQ ID NO: 101. In some embodiments, the peptide is a pharmaceutically acceptable salt thereof.

In some embodiments, the present disclosure provides a fatty acid conjugated cyclic peptide comprising or consisting of SEQ ID NO: 117, or of Formula (I), or any other Formulae or SEQ ID NO. disclosed herein, or pharmaceutically acceptable salts thereof for use in the treatment of, or ameliorating symptoms of, alopecia areata.

### Pharmaceutical Combinations

The disclosure relates to the use of a CRF receptor antagonist fatty acid conjugated cyclic peptide comprising or consisting of SEQ ID NO: 117, or of Formula (I), or a pharmaceutical composition comprising a compound of the Formula (I), in the treatment of one or more of the diseases mentioned herein; wherein the response to treatment is beneficial as demonstrated, for example, by the partial or complete removal of one or more of the symptoms of the disease up to complete cure or remission.

CRF antagonists will block the hypothalamic pituitary axis (HPA) and therefore block ACTH and corticosterone secretion in instances when the desired effects of administration may be on other functions (e.g., immune, neuronal, etc.). Therefore, hormonal replacement therapy (for example, administration of ACTH and/or corticosterone) can be used as an adjunct to CRF antagonist therapy, as necessary to maintain homeostasis.

### Pharmaceutical Compositions

In another aspect, the present disclosure provides pharmaceutically acceptable compositions which comprise a therapeutically effective amount of one or more of the fatty acid conjugated cyclic peptides described above, or a pharmaceutically acceptable salt thereof, formulated together with one or more pharmaceutically acceptable carriers (additives) and/or diluents. As described below, the pharmaceutical compositions of the present disclosure can be formulated for administration in solid or liquid form, including those adapted for the following: (1) oral administration, for example, drenches (aqueous or non-aqueous solutions or suspensions); or (2) parenteral administration, for example, by subcutaneous, intramuscular, or intravenous, injection as, for example, a sterile solution or suspension.

In some embodiments, the present disclosure provides pharmaceutically acceptable compositions of a therapeutically effective amount of one or more of the fatty acid conjugated cyclic peptides comprising or consisting of SEQ ID NO: 117, or of Formula (1), or pharmaceutically acceptable salts thereof.

In some embodiments, the present disclosure provides pharmaceutically acceptable compositions of a therapeutically effective amount of a peptide comprising or consisting of any one of formulae (Ia), (Ib), (Ic), (Id), (Ie), (If), (Ig), (Ih), or (Ik), or a pharmaceutically acceptable salt thereof.

In some embodiments, the present disclosure provides pharmaceutically acceptable compositions of a therapeutically effective amount of any one of peptides listed in Tables B1.1 and B1.2, or a pharmaceutically acceptable salt thereof.

In some embodiments, the present disclosure provides pharmaceutically acceptable compositions of a therapeutically effective amount of the peptide comprising or consisting of SEQ ID NO: 50. In some embodiments, the peptide is a pharmaceutically acceptable salt thereof.

In some embodiments, the present disclosure provides pharmaceutically acceptable compositions of a therapeutically effective amount of the peptide comprising or consisting of SEQ ID NO: 95. In some embodiments, the peptide is a pharmaceutically acceptable salt thereof.

In some embodiments, the present disclosure provides pharmaceutically acceptable compositions of a therapeutically effective amount of the peptide comprising or consisting of SEQ ID NO: 97. In some embodiments, the peptide is a pharmaceutically acceptable salt thereof.

In some embodiments, the present disclosure provides pharmaceutically acceptable compositions of a therapeutically effective amount of the peptide comprising or consisting of SEQ ID NO: 101. In some embodiments, the peptide is a pharmaceutically acceptable salt thereof.

Peptides of the disclosure can be self-administered, for example, using a prefilled subcutaneous injectable device, or administered under the guidance of a physician in single or multiple doses for the treatment of diseases or disorders outlined above. The effective dosage generally depends on the intended route of administration and other factors such as age and weight of the patient, and upon the illness being treated. Usually, the dosage will be from about 0.5 to about 1 milligram of the peptide per kilogram of the body weight of the host animal. Fatty acid conjugates extend the in vivo time of action of the peptides of the disclosure. The fatty acid conjugate binds albumin, taking advantage of the 20-day human albumin half-life, to achieve a minimum of weekly dosing. Fatty acid conjugated cyclic peptides of the disclosure, or any other Formulae or SEQ ID NO. disclosed herein, , or pharmaceutically acceptable salts thereof, have a long half-life in the body and are, therefore, suitable for once weekly, twice monthly, or monthly dosing. Weekly dosing in humans is about 50 mg to about 250 mg, about 75 mg to about 230 mg and, based on solubility, can be delivered in less than 1 mL volumes from a subcutaneous injection. Weekly, twice monthly, or monthly dosing is particularly compelling for patients with compliance concerns and/or a practical need around multiple dosing throughout the day.

### Process for Making Peptides of the Disclosure

The peptides described herein are synthesized by a suitable method, such as by exclusively solid-phase techniques, by partial solid-phase techniques, by fragment condensation or by classical solution addition.

Common to chemical syntheses of peptides is the protection of the labile side chain groups of the various amino acid moieties with suitable protecting groups which will prevent a chemical reaction from occurring at that site until the group is ultimately removed. Usually also common is the protection of an alpha-amino group on an amino acid or a fragment while that entity reacts at the carboxyl group, followed by the selective removal of the alpha-amino protecting group to allow subsequent reaction to take place at that location. Accordingly, it is common that, as a step in the synthesis, an intermediate compound is produced which includes each of the amino acid residues located in its desired sequence in the peptide chain with various of these residues having side chain protecting groups.

In one aspect of the disclosure, the side chain protecting groups comprise a hydrogen or an alpha-amino protecting group. The alpha-amino protecting groups contemplated include, but are not limited to, those known to be useful in the art in the stepwise synthesis of polypeptides. Classes of alpha-amino protecting groups include, but are not limited to: (1)butyloxycarbonyl (ng groups, such as formyl (For), acrylyl (Acr), benzoyl (Bz) and acetyl (Ac) which can be used at the N-terminal; (2) aromatic urethan-type protecting groups, such as benzyloxycarbonyl (Z) and substituted Z, such as p-chlorobenzyloxycarbonyl, p-nitrobenzyloxycarbonyl, p-bromobenzyloxycarbonyl, p-methoxybenzyloxycarbonyl; (3) aliphatic urethan protecting groups, such as t-butyloxycarbonyl (BOC), diisopropylmethoxycarbonyl, isopropyloxycarbonyl, ethoxycarbonyl, allyloxycarbonyl (Alloc); (4) cycloalkyl urethan-type protecting groups, such as fluorenyl methyloxycarbonyl (Fmoc), cyclopentyloxy-carbonyl, adamantyloxycarbonyl, and cyclohexyloxy-carbonyl; and (5) thiourethan-type protecting groups, such as phenylthiocarbonyl.

Protecting groups for the hydroxyl group of Thr or Ser include, but are not limited to, protecting groups selected from the class consisting of acetyl (Ac), tert-butyl (tBu), triphenylmethyl (trityl), tetrahydropyranyl, benzyl ether (Bzl), and 2,6-dichlorobenzyl (DCB). In some embodiments, there is no protecting group on the hydroxyl group.

Protecting groups for the guanidino group of Arg include, but are not limited to, protecting groups selected from the class consisting of nitro, p-toluenesulfonyl (Tos), Z, 2,2,4,6,7-Pentamethyl-2,3-dihydrobenzofuran-5-sulfonyl (Pbf), adamantyloxycarbonyl and BOC, or hydrogen.

Protecting groups for the amido group of Asn or Gln include, but are not limited to, trityl (Trt), xanthyl (Xan) or hydrogen. Asn or Gln is often coupled without side chain protection in the presence of hydroxybenzotriazole (HOBt).

Protecting groups for the β- or γ-carboxyl group of Asp or Glu include, but are not limited to, ester-forming protecting groups (e.g., of allyl (OAll), cyclohexyl (OChx), benzyl (OBzl), 2,6-dichlorobenzyl, methyl, ethyl, trityl (Trt), 2-phenylisopropyl (2-PhiPr), and t-butyl (Ot-Bu)) and hydrogen.

Protecting groups for the side chain amino substituent of Lys or Orn include, but are not limited to, Z, 2-chlorobenzyloxycarbonyl(2Cl-Z), 1-(4,4-dimethyl-2,6-dioxocyclohex-1-ylidene)ethyl (Dde), Tos, t-amyloxycarbonyl (Aoc), 4-methoxytrityl (MMT), 4-methyltrityl (MTT), BOC, and aromatic or aliphatic urethan-type protecting groups as specified hereinbefore, or hydrogen.

Protecting groups for the imidazole nitrogen in His include, but are not limited to, Tos, 2,4-dinitrophenyl (DNP), BOC, and hydrogen. Protecting groups for the hydroxyl group in Tyr include, but are not limited to, DCB and hydrogen. When Met is present, the sulfur can be protected, if desired, with oxygen.

The selection of a side chain amino protecting group is not critical except that it cannot be one that is removed during deprotection of the alpha-amino groups during the synthesis. Hence, the alpha-amino protecting group and the side chain amino protecting group cannot be the same.

The particular amino acid chosen for each R-group determines whether there will also be a protecting group attached as specified hereinbefore and as generally known in the art. In selecting a particular side chain protecting group to be used in the synthesis of the peptides, the following rules are followed: (a) the protecting group is stable to the reagent and under the reaction conditions selected for removing the alpha-amino protecting group at each step of the synthesis, (b) the protecting group retains its protecting properties and is not split off under coupling conditions and (c) the side chain protecting group must be removable, upon the completion of the synthesis containing the desired amino acid sequence, under reaction conditions that will not alter the peptide chain.

For the acylated N-terminus, an acyl group having 15 carbon atoms or less is present, in some embodiments 12 or less. Acylated groups are selected from, but not limited to, acetyl (Ac), formyl (For), acrylyl (Acr), benzoyl (Bz), propionyl, butyroyl, valeroyl, hexanoyl, octanoyl, decanoyl, and tetradecanoyl. In some embodiments, to facilitate labeling, an acylating agent containing a hydroxy aryl moiety, such as 4-hydroxy-phenylpropionic acid (des-NH₂-Tyr) or 4-hydroxy-phenylacetic acid, can be used. Also, the N-terminus may alternatively comprise a suitable sugar or lipid, which are equivalents that can be used to adjust hydrophilicity.

In one aspect of the disclosure, the fatty acid conjugated cyclic peptides are synthesized by classical peptide solution synthesis, and such synthesis can be useful for synthesizing large quantities. To obtain limited quantities, for example, less than 1 kg, solid phase synthesis can be used, such as that described by Merrifield, J. Am. Chem. Soc., 85, p 2149 (1964), the disclosure of which is herein incorporated by reference in its entirety, which facilitates the CRF antagonist peptides being prepared efficiently and then quickly tested to determine biological activity. This facilitates the ready preparation and evaluation of various CRF antagonist peptides. Solid-phase synthesis is commenced from the C-terminus of the peptide by coupling a protected alpha-amino acid to a suitable resin as generally set forth in U.S. Pat. No. 4,244,946 issued Jan. 21, 1981, to Rivier et al., the disclosure of which is herein incorporated by reference in its entirety. Such a starting material for an antagonist based upon human CRF can be prepared by attaching alpha-amino-protected amino acid (for example, R33) to a resin (for example, MBHA resin).

The amino acid (for example, R33) protected by Fmoc or another appropriate protecting group is coupled to the MBHA resin using a coupling reagent (for example, HBTU, or the like) in an appropriate solution (for example, methylene chloride, and/or dimethylformamide (DMF), and/or N-methyl pyrrolidone (NMP), or the like). Following the coupling of protected amino acid to the resin support, the alpha-amino protecting group is removed (for example, by using trifluoroacetic acid (TFA) in methylene chloride, TFA alone, piperidine in DMF, with HCl in dioxane, or the like). The deprotection is carried out at a temperature between about 0°C and about 70°C. Other standard cleaving reagents and conditions for removal of specific alpha-amino protecting groups can be used, as described in Schroder & Lubke, "The Peptides", Vol. 1, 72-75 (Academic Press 1965).

After removal of the alpha-amino protecting group (for example, R33), the remaining alpha-amino-and side chain-protected amino acids are coupled stepwise in the desired order to obtain an intermediate compound such as defined herein. As an alternative to adding each amino acid separately in the synthesis, some of them can firstly be coupled to one another in solution phase prior to addition to the solid phase reactor.

Activating or coupling reagents for use in the solid phase synthesis of the peptides are well known in the peptide art. Examples of such reagents include, but are not limited to, suitable carbodiimides, such as N,N'-diisopropyl carbodiimide (DIC), N,N'-dicyclohexyl carbodiimide (DCC), N-ethyl-N'-(3-dimethylaminopropyl) carbodiimide (EDC), Hexafluorophosphate Benzotriazole Tetramethyl Uronium (HBTU), 2-(1H-Benzotriazole-1-yl)-1,1,3,3-tetramethylaminium tetrafluoroborate (TBTU), Hexafluorophosphate Azabenzotriazole Tetramethyl Uronium (HATU), Benzotriazol-1-yloxy)tris(dimethylamino)phosphonium hexafluorophosphate (BOP) and benzotriazol-1-yloxytripyrrolidinophosphonium hexafluorophosphate (PyBop), or the like. Other activating reagents and their use in peptide coupling are described by: Schroder & Lubke, "The Peptides", Academic Press (1965), in Chapter III; and by Kapoor, J. Phar. Scl. 59, pp 1- 27 (1970). P-nitrophenyl ester (ONp) can also be used to activate the carboxyl end of Asn or Gln for coupling.

Each protected amino acid or amino acid sequence is introduced into the solid phase reactor in about a threefold excess, and the coupling is carried out in a medium of dimethylformamide (DMF):CH₂Cl₂ (1:1) or in CH₂Cl₂ alone at room temperature. Alternatively, coupling may be carried out at an elevated temperature up to about 70°C in NMP or in a mixture of toluene:DMSO (70:30) or the peptides can be synthesized with Fmoc (9-fluorenmethyloxycarbonyl) chemistry on the CEM Multi-channeled Liberty Blue HT-12 automated microwave assisted peptide synthesizer. N-α-Fmoc-protected amino acids are coupled with DIC and Ethyl cyano(hydroxyimino)acetate (OxymaPure) in DMF and microwave irradiation at 90°C for 2 min is applied.

In instances where the coupling is carried out manually, the success of the coupling reaction at each stage of the synthesis is monitored by the ninhydrin reaction, as described by E. Kaiser et al., Anal. Biochem., 34, 595 (1970), the contents of which are herein incorporated by reference in its entirety. In cases where incomplete coupling occurs, the coupling procedure is repeated before removal of the alpha-amino protecting group prior to the coupling of the next amino acid. The coupling reactions can be performed automatically, as on a CSBio Model 356 automatic synthesizer, using a program such as that reported in Rivier et al., Biopolymers, 17, pp.1927-1938, (1978), the contents of which are herein incorporated by reference in its entirety.

After the desired amino acid sequence has been completed, the intermediate peptide is removed from the resin support unless it is desired to form the cyclizing bond while attached to the resin, as described hereinafter. Removal is affected by treatment with a reagent (for example, liquid hydrogen fluoride (HF), a TFA / TIS / H₂O / DTT solution, or the like), which not only cleaves the peptide from the resin but also cleaves all remaining side chain protecting groups and the alpha-amino protecting group, if still present (unless it is an acyl group which is intended to be present in the final peptide), to obtain the peptide.

To effect an amide cyclizing linkage (lactam bridge), cyclization may be carried out while the partially protected peptide remains attached to the resin as disclosed in U.S. Pat. Nos. 5,064,939 and 5,043,322, the contents of each of which are herein incorporated by reference in their entireties. Such a procedure effectively creates an amide cyclizing bond between the two desired side chains while other residues, such as Asp, Glu and/or Lys, in the peptide intermediate retain their side-chain protection.

When cyclizing via an amide bond between a side-chain carboxyl group of the 22-position residue of the peptides disclosed herein and a side-chain amino group of the 25-position residue. The protected peptide can be synthesized on a MBHA or BHA resin. The benzyl ester of the particular carboxyl acid side chain can be derivatized to the hydrazide while the peptide is still attached to the resin and then reacted with a selectively deprotected amino-side chain as set forth in U.S. Pat. No. 5,043,322, the contents of which are herein incorporated by reference in its entirety. In some embodiments, cyclization is accomplished by using a base-labile protecting group, for example, OFm, for the carboxyl side chain of the residue to be involved in the amide-bond bridge and using Fmoc as a protecting group for the amino side chain on the other residue that is to be involved. The alpha-amino protecting group on the residue at the N-terminus of the intermediate and all the other side chain protecting groups remain in place while the two base-labile groups are removed using piperidine, or the like. Following such selective removal, a reaction to accomplish cyclization is carried out by treating with PyBOP reagent and DIPEA base which effects substantially complete generation of the amide bond. Following cyclization, the peptide is completely deprotected and cleaved from the resin using a reagent, such as HF, or the like. Optionally, a BOC-protecting group is first removed from the N-terminus using TFA, or the like, particularly if the N-terminus is to be acylated.

Alternatively, cyclization of peptides by creating such amide linkages can also be effected using teachings of U.S. Pat. No. 4,115,554 (Sep. 19, 1978); U.S. Pat. No. 4,133,805 (Jan. 9, 1979); U.S. Pat. No. 4,140,767 (Feb. 20, 1979); U.S. Pat. No. 4,161,521 (Jul. 17, 1979); U.S. Pat. No. 4,191,754 (Mar. 4, 1980); U.S. Pat. No. 4,238,481 (Dec. 9, 1980); U.S. Pat. No. 4,244,947 (Jan. 13, 1981); and U.S. Pat. No. 4,261,885 (Apr. 14, 1981), the contents of each of which are herein incorporated by reference in their entireties.

As described herein, the present disclosure also contemplates a synthesis of the peptides disclosed herein through a fragment approach, where peptide fragments are first synthesized as described herein, and the fragments are coupled together. For example, a peptide having the following sequence can be synthesized as three fragments: Ac-Asp-Leu-Thr-D-Phe-His-Leu-Leu-Arg-Glu-Val-Leu-Glu-Nle-**K***-Arg-Glu-Glu-Gln-cVal-Ala-Gln-Glu#-Ala-Glu-Lys#-Asn-Arg-Lys-Leu-Nle-Glu-cVal-Ala-CONH₂
Fragment 1: Ac-Asp-Leu-Thr-D-Phe-His-Leu-Leu-Arg-Glu-Val-Leu-Glu-OH.
Fragment 2: Fmoc-Nle-**K***-Arg-Glu-Glu-Gln-cVal-OH.
Fragment 3: H-Ala-Gln-Glu#-Ala-Glu-Lys#-Asn-Arg-Lys-Leu-Nle-Glu-cVal-Ala-CONH₂.
Fragment 2 would first be coupled to Fragment 3 using methods described herein, followed by coupling of Fragment 1 to previously coupled Fragment 2-3.

In some aspects of the disclosure, the N-terminus is acetylated. In another aspect of the disclosure, the C-terminus is amidated. In another aspect of the disclosure, the N-terminus is acetylated, and the C-terminus is amidated. In another aspect of the disclosure, a lactam bridge between Glu#²² and Lys#²⁵ is performed on resin prior to coupling of Fragment 2 to Fragment 3. This fragment synthesis approach can be achieved through a solution assembly of fragments in solution, or a hybrid approach of assembly on resin.

A candidate CRF antagonist peptide can be evaluated in a binding assay using a known CRF receptor, such as that described in the examples below.

### Examples

The following Examples are illustrative of the disclosure without limiting the scope thereof.

### Experimental Method A: Peptide Synthesis.

### Example A1

### Synthesis of Peptide of SEQ ID NO: 101

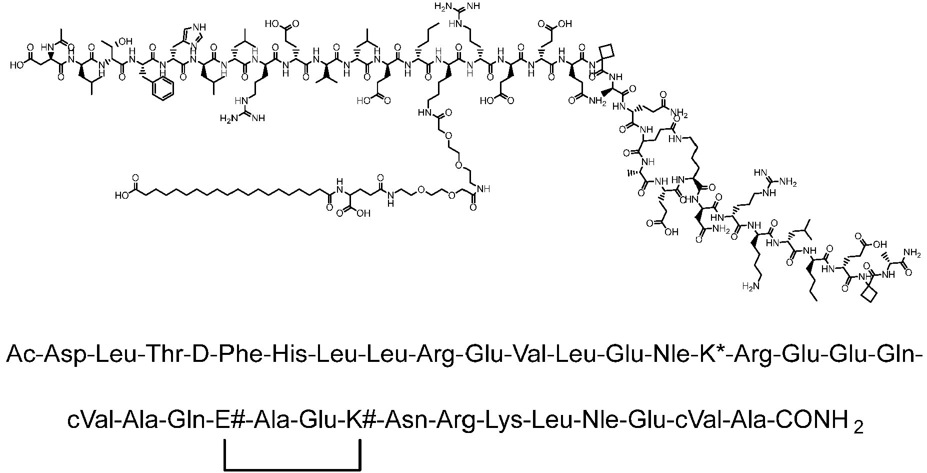

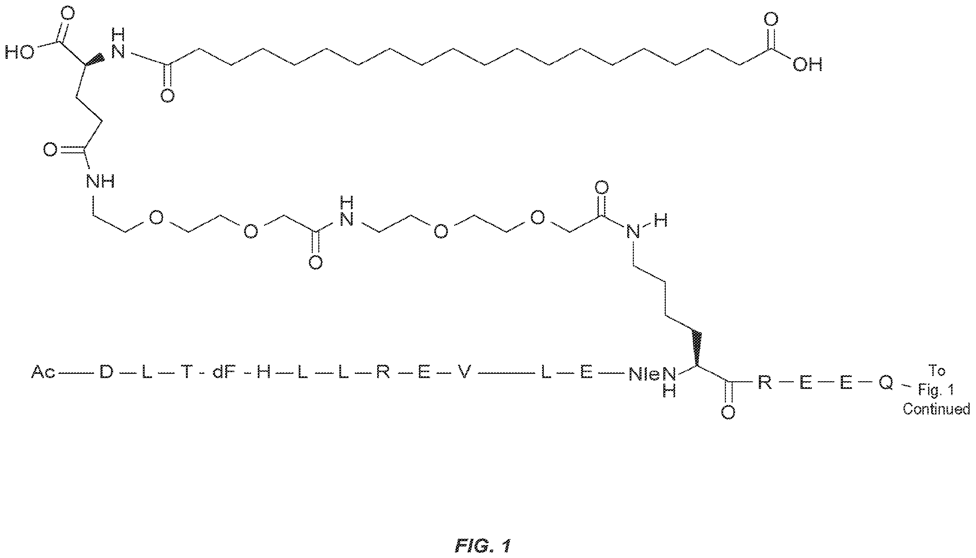

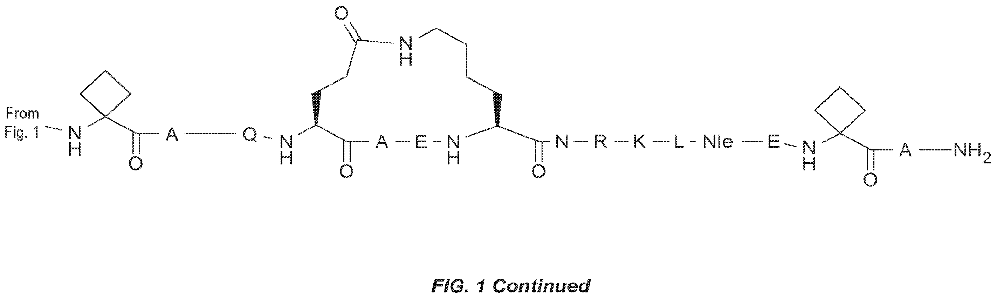

Peptide of SEQ ID NO: 101, wherein K* is Lys-(2-[2-(2-amino-ethoxy)-ethoxy]-acetyl)₂-γGlu-C20-diacid (Table B1.1, Formula I, FIG. 1), was produced by solid phase synthesis following chromatography, concentration with a rotovap to remove solvent, and freeze-drying (Synthesis 1). The solid phase peptide synthesis (SPSS) batch was done on a CSBio536 automated synthesizer on 10 mmol scale in a 1000 mL reactor. The resin bed was agitated by overhead stirring. Potential critical coupling steps were monitored by the qualitative Kaiser test as an in-process control (IPC). Additional modification steps as the on-resin cyclization or site chain modification with the C18 moiety were monitored with test cleavages on small scale. The peptide was synthesized from C-terminus to N-terminus linearly using standard Fmoc chemistry on Fmoc protected Rink amide resin. The synthesis involved the use of DIC/Oxymapure^{®} chemistry. The loading factor of the initial Fmoc-Rink amide resin was 0.4 mmol g⁻¹. After the complete synthesis, the resin was washed 5 times with IPA followed by 5 times with DIPE. The resin was finally dried in a vacuum oven. The general conditions for the SPPS are depicted in Table 1A whereas the corresponding coupling and reaction times for each cycle are listed in Table 1B.

**Table 1A. SPSS Steps for Peptide of SEQ ID NO: 101 Synthesis (Synthesis 1).**

| | Fmoc-removal |
|---|---|
| Deprotection | 2 x 20% piperidine in DMF, 5 + 20 minutes, 10 mL/g resin |
| Wash | 6 x DMF, 5 minutes, 10 mL/g resin |

| | Coupling Fmoc-AA-OH |
|---|---|
| Coupling | 2.5 eq. Fmoc-AA-OH, 3.75 eq. DIC, 3.75 eq. Oxyma in DMF, 0.12 M coupling conc |
| Acetylation | 4.5 eq. Ac₂O, 4.5 eq. collidine in DMF, 10 minutes |
| Wash | 4 x DMF, 5 minutes, 10 mL/g resin |

| | Alloc-, Allyl-removal |
|---|---|
| Deprotection | 0.15 eq. Pd(PPh₃)₄, 25 eq. Phenylsilane in DCM |
| Wash | 6 x DCM, 5 minutes, 10 mL/g resin 5 x DMF, 5 minutes, 10 mL/gᵣₑₛᵢₙ |

| | Cyclization |
|---|---|
| Cyclization | 1.5 eq. PyBOP, 3 eq. DIPEA in DMF |
| Wash | 4 x DMF, 5 minutes, 10 mL/g resin |
| Acetylation | 4.5 eq. Ac₂O, 4.5 eq. collidine in DMF, 10 minutes |
| Wash | 4 x DMF, 5 minutes, 10 mL/g resin |
| Fmoc-removal | 2 x 20% piperidine in DMF, 5 + 20 minutes, 10 mL/g resin |
| Wash | 6 x DMF, 5 minutes, 10 mL/g resin |

| | MTT-Removal |
|---|---|
| Deprotection | HFIP/DCM/TIPS 30/70/1, 5 x 30 minutes, 10 mL/g resin |
| Wash | HFIP/DCM/TIPS 30/70/1, 5 x 30 minutes, 10 mL/g resin |

| | Final Wash |
|---|---|
| | 5 × IPA (10 mL/gᵣₑₛᵢₙ), 5 minutes each 5 x DIPE (10 mL/g resin), 5 minutes each |

**Table 1B. Coupling Time for Peptide of SEQ ID NO: 101 Synthesis (Synthesis 1).**

| Cycle | AA used | Coupling time (h) |
|---|---|---|
| 1 | Fmoc-Ala-OH | 3 |
| 2 | Fmoc-cVal-OH | 3 |
| 3 | Fmoc-Glu(OtBu)-OH | 3 |
| 4 | Fmoc-Nle-OH | 3 |
| 5 | Fmoc-Leu-OH | 3 |
| 6 | Fmoc-Lys(Boc)-OH | 3 |
| 7 | Fmoc-Arg(pbf)-OH | 6 |
| 8 | Fmoc-Asn(Trt)-OH | 3 |
| 9 | Fmoc-Lys(Alloc)-OH | 3 |
| 10 | Fmoc-Glu(OtBu)-OH | 3 |
| 11 | Fmoc-Ala-OH | 3 |
| 12 | Fmoc-Glu(OAll)-OH | 3 |
| 13 | Fmoc-Gln(Trt)-OH | 3 |
| 13.1 | Alloc-, Allyl-removal | 3 |
| 13.2 | Cyclization | 3 |
| 14 | Fmoc-Ala-OH | 12 |
| 15 | Fmoc-cVal-OH | 3 |
| 16 | Fmoc-Gln(Trt)-OH | 3 |
| 17 | Fmoc-Glu(OtBu)-OH | 3 |
| 18 | Fmoc-Glu(OtBu)-OH | 3 |
| 19 | Fmoc-Arg(pbf)-OH | 6 |
| 20 | Fmoc-Lys(MTT)-OH | 3 |
| 21 | Fmoc-Nle-OH | 3 |
| 22 | Fmoc-Glu(OtBu)-OH | 3 |
| 23 | Fmoc-Leu-OH | 3 |
| 24 | Fmoc-Val-OH | 3 |
| 25 | Fmoc-Glu(OtBu)-OH | 3 |
| 26 | Fmoc-Arg(pbf)-OH | 6 |
| 27 | Fmoc-Leu-OH | 3 |
| 28 | Fmoc-Leu-OH | 3 |
| 29 | Fmoc-His(Trt)-OH | 3 |
| 30 | Fmoc-D-Phe-OH | 3 |
| 31 | Fmoc-Thr(Otbu)-OH | 3 |
| 32 | Fmoc-Leu-OH | 3 |
| 33 | Fmoc-Asp(OtBu)-OH | 3 |
| 33.1 | Acetylation | 20 minutes |
| 33.2 | MTT removal | 5 x 30 minutes |
| 34 | Fmoc-(AEEA)-OH | 3 |
| 35 | Fmoc-(AEEA)-OH | 3 |
| 36 | Fmoc-Glu-OtBu | 6 |
| 37 | C20-Diacid-OtBu | 6 |
| 38 | Final wash | 5 x 5 minutes IPA |
| | | 5 x 5 minutes DIPE |

Cleavage of peptide from the resin and global deprotection was performed with a cocktail (10 mL/g dry peptidyl resin) consisting of a mixture of TFA/TIS/H₂O (95/2.5/2.5, v/v/v). The cleavage cocktail was added to the resin bound peptide and the mixture was stirred at room temperature for 3 hours. Afterwards, the cleavage mixture was filtered using a P3 frit, and the residual resin was rinsed twice with TFA (2 x 0.25 mL/g peptidyl resin). DIPE (8-10 vol-eq. of cleavage cocktail volume) was precooled to 0°C. The cleavage solution was added dropwise to the anti-solvent at a rate in which the solution temperature did not exceed 8°C (stir speed: 150 rpm). The addition rate was increased as needed during the addition time. The mixture containing the precipitated peptide was then warmed to room temperature and aged for 1 hour. The crude peptide was obtained via centrifugation, additional DIPE washes, and drying under vacuum.

Crude peptide was purified by RP-HPLC in a two-step approach. Chromatography was carried out on a BioRAD NGC Quest 10 Plus system using a C18 column. For the first RP-HPLC step, ammonium acetate/acetonitrile buffer was used. The product was eluted with an acetonitrile gradient. For 10 mmol scale, 10 runs were performed with 1.5 g crude loading each. Fractions with purity ≥ 75% were pooled together into one final pool which was further purified in a second chromatography step. Prior to loading, the pool was diluted 1: 1 with acetic acid (buffer A). An acetonitrile (buffer B) gradient was applied. Fractions with purity ≥ 85% were pooled together. Acetonitrile was evaporated using a rotary evaporator and the concentrate was lyophilized. The resulting 1.85 g of lyophilized product was filled into a 100 ml glass bottle.

Alternatively, the peptide of SEQ ID NO: 101 was synthesized using standard Fmoc chemistry (Synthesis 2):
Resin preparation: The Rink Amide MBHA resin (1.50 mmol, 1.00 eq., Sub 0.33 mmol/g) in DMF (25.0 mL) was agitated with N₂ for 2 hours at 20°C. The resin was washed with DMF (50.0 mL, five times).

Deprotection: 20% Piperidine in DMF (50.0 mL) was added and the mixture was agitated with N₂ for 10 minutes at 20°C. The resin was washed with DMF (50.0 mL, five times) and filtered to prepare for amino acid coupling.

Coupling: A solution of HBTU (2.85 eq.) and Fmoc-Ala-OH (3.00 eq.) in DMF (25.00 mL) was added to the resin. DIEA (6.00 eq.) was added, and the mixture was agitated with N₂ at 20°C for 30 minutes. The resin was washed with DMF (50.0 mL, five times).

The Deprotection and Coupling steps were then repeated for the coupling of the following amino acids (1-29) outlined in Table 1C.

**Table 1C. Peptide of SEQ ID NO: 101 Part 1 (Synthesis 2).**

| # | Materials | Coupling Reagents |
|---|---|---|
| 1 | Fmoc-cVal-OH (2.00 eq.) | HATU (1.90 eq.) and DIEA (4.00 eq.) |
| 2 | Fmoc-Glu(OtBu)-OH (3.00 eq.) | HBTU (2.85 eq.) and DIEA (6.00 eq.) |
| 3 | Fmoc-Nle-OH (3.00 eq.) | HBTU (2.85 eq.) and DIEA (6.00 eq.) |
| 4 | Fmoc-Leu-OH (3.00 eq.) | HBTU (2.85 eq.) and DIEA (6.00 eq.) |
| 5 | Fmoc-Lys(Boc)-OH (3.00 eq.) | HBTU (2.85 eq.) and DIEA (6.00 eq.) |
| 6 | Fmoc-Arg(Pbf)-OH (3.00 eq.) | HOAT (3.00 eq.) and DIC (3.00 eq.) |
| 7 | Fmoc-Asn(Trt)-OH (3.00 eq.) | HBTU (2.85 eq.) and DIEA (6.00 eq.) |
| 8 | Fmoc-cyclo[Glu-Ala-Glu(OtBu)-Lys]-OH (2.00 eq.) | HATU (2.85 eq.) and DIEA (6.00 eq.) |
| 9 | Fmoc-Gln(Trt)-OH (3.00 eq.) | HBTU (2.85 eq.) and DIEA (6.00 eq.) |
| 10 | Fmoc-Ala-OH (3.00 eq.) | HBTU (2.85 eq.) and DIEA (6.00 eq.) |
| 11 | Fmoc-cVal-OH (2.00 eq.) | HATU (1.90 eq.) and DIEA (4.00 eq.) |
| 12 | Fmoc-Gln(Trt)-OH (3.00 eq.) | HBTU (2.85 eq.) and DIEA (6.00 eq.) |
| 13 | Fmoc-Glu(OtBu)-OH (3.00 eq.) | HBTU (2.85 eq.) and DIEA (6.00 eq.) |
| 14 | Fmoc-Glu(OtBu)-OH (3.00 eq.) | HBTU (2.85 eq.) and DIEA (6.00 eq.) |
| 15 | Fmoc-Arg(Pbf)-OH (3.00 eq.) | HBTU (2.85 eq.) and DIEA (6.00 eq.) |
| 16 | Fmoc-Lys(Dde)-OH (3.00 eq.) | HATU (2.85 eq.) and DIEA (6.00 eq.) |
| 17 | Fmoc-Nle-OH (2.00 eq.) | HATU (1.90 eq.) and DIEA (4.00 eq.) |
| 18 | Fmoc-Glu(OtBu)-OH (3.00 eq.) | HATU (2.85 eq.) and DIEA (6.00 eq.) |
| 19 | Fmoc-Leu-OH (3.00 eq.) | HOAT (3.00 eq.) and DIC (3.00 eq.) |
| 20 | Fmoc-Val-OH (3.00 eq.) | HATU (2.85 eq.) and DIEA (6.00 eq.) |
| 21 | Fmoc-Glu(OtBu)-OH (3.00 eq.) | HATU (2.85 eq.) and DIEA (6.00 eq.) |
| 22 | Fmoc-Arg(Pbf)-OH (3.00 eq.) | HATU (2.85 eq.) and DIEA (6.00 eq.) |
| 23 | Fmoc-Leu-OH (3.00 eq.) | HATU (2.85 eq.) and DIEA (6.00 eq.) |
| 24 | Fmoc-Leu-OH (3.00 eq.) | HATU (2.85 eq.) and DIEA (6.00 eq.) |
| 25 | Fmoc-His(Trt)-OH (3.00 eq.) | HATU (2.85 eq.) and DIEA (6.00 eq.) |
| 26 | Fmoc-D-Phe-OH (3.00 eq.) | HATU (2.85 eq.) and DIEA (6.00 eq.) |
| 27 | Fmoc-Thr(tBu)-OH (3.00 eq.) | HATU (2.85 eq.) and DIEA (6.00 eq.) |
| 28 | Fmoc-Leu-OH (3.00 eq.) | HATU (2.85 eq.) and DIEA (6.00 eq.) |
| 29 | Fmoc-Asp(OtBu)-OH (3.00 eq.) | HATU (2.85 eq.) and DIEA (6.00 eq.) |

10% Ac₂O/5% DIEA/85% DMF was added into the resin and the mixture was agitated with N₂ at 20°C for 30 minutes. The resin was washed with DMF (50.0 mL, five times).

3% hydrazine hydrate/DMF was added into the resin and the mixture was agitated with N₂ at 20°C for 10 minutes. The resin was washed with DMF (50.0 mL, five times).

The Deprotection and Coupling steps were then repeated for the coupling of the following amino acids (30-33) as outlined in Table 1D.

**Table 1D. Peptide of SEQ ID NO: 101 Part 2 (Synthesis 2).**

| # | **Materials** | **Coupling Reagents** |
|---|---|---|
| 30 | Fmoc-(AEEA)-OH (3.00 eq.) | HATU (2.85 eq.) and DIEA (6.00 eq.) |
| 31 | Fmoc-(AEEA)-OH (3.00 eq.) | HATU (2.85 eq.) and DIEA (6.00 eq.) |
| 32 | Fmoc-Glu-OtBu (3.00 eq) | HATU (2.85 eq.) and DIEA (6.00 eq.) |
| 33 | 20-(tert-butoxy)-20-oxoicosanoic acid (2.00 eq.) | HBTU (1.90 eq.) and DIEA (4.00 eq.) |

Peptide Cleavage and Purification: Cleavage solution (90.0 mL, 92.5% TFA/2.50% TIS/2.50% H₂O/2.50% 3-Mercaptopropionic acid) was added to the flask containing resin at room temperature and stirred for 2 hours.

The peptide was precipitated with cold isopropyl ether (900 mL). The peptide was filtered, and the filter cake was collected. The filter cake was washed with isopropyl ether (900 mL, twice). The filter cake was dried under vacuum for 2 hours to obtain crude peptide (5.00 g). The crude peptide was confirmed via LCMS (MS cal.: 4711.49, MS observed: [M+4H]4+ = 1178.8, Rt=1.433 minutes).

The crude peptide was purified by prep-HPLC (TFA condition: A: 0.075 % TFA in H₂O, B: ACN) and then was purified by prep-HPLC (HCl condition, A: 0.005%HCl, B: H₂O) to give the final product (333.5 mg, 68.24 µmol, 6.67% yield, 97.15% purity, HCl) as a white solid. The peptide was confirmed via LCMS (MS cal.: 4711.49, MS observed: [M+4H]4+ = 1178.6, Rt=1.330 minutes) and HPLC (Rt = 10.732 minutes, purity: 97.15%, Rt = 10.732 minutes). Purification conditions were as described in Table 1E.

**Table 1E. Peptide of SEQ ID NO: 101 Purification Conditions (Synthesis 2).**

| First Separation Condition | |
|---|---|
| Dissolution Condition | Crude peptide was dissolved in 20% ACN-H₂0 |
| Instrument | Gilson GX-281 |
| Mobile Phase | A: H₂O (0.075% TFA in H₂O) |
| | B: CH₃CN |
| Gradient | 10-10%-8 minutes, 25-55%-50 minutes, Retention time: 46 minutes |
| Column | Luna, 10 µm, C18, 100A, (650 mm*100 mm) |
| Flow Rate | 20 mL/minute |
| Wavelength | 220/254 nm |
| Oven Temperature | 30°C |

| Second Separation Condition | |
|---|---|
| Dissolution Condition | Lyophilized material from first purification was dissolved in 20% ACN-H₂O |
| Instrument | Gilson GX-281 |
| Mobile Phase | A: H₂O (0.075% TFA in H₂O) |
| | B: CH₃CN |
| Gradient | 31-51%-50 minutes, Retention time: 31 minutes |
| Column | Column 1: Luna, 10 µm, C18, 100A, (25 mm*120 mm) |
| | Column 2: Gemini, 5 µm, C18, 110A (40 mm*150 mm) |
| | Dual column connections |
| Flow Rate | 20 mL/minute |
| Wavelength | 220/254 nm |
| Over Temperature | 30°C |

| HCl Salt Exchange Condition | |
|---|---|
| Dissolution Condition | Lyophilized material from second purification was dissolved in 35% ACN-H₂O |
| Instrument | Gilson GX-281 |
| Mobile Phase | A: H₂O (0.005% HCl in H₂O) |
| | B: CH₃CN |
| Gradient | 0-0%-25 minutes 2M NH₄Cl, 0-0%-10 minutes 0.005% HCl, 30-48%-38 minutes, Retention time: 54 minutes |
| Column | Column 1: Luna, 10 µm, C18, 100A, (25 mm*120 mm) |
| | Column 2: Gemini, 5 µm, C18, 110A (40 mm*150 mm) |
| | Dual column connections |
| Flow Rate | 20 mL/minute |
| Wavelength | 220/254 nm |
| Oven Temperature | 30°C |

### Example A2

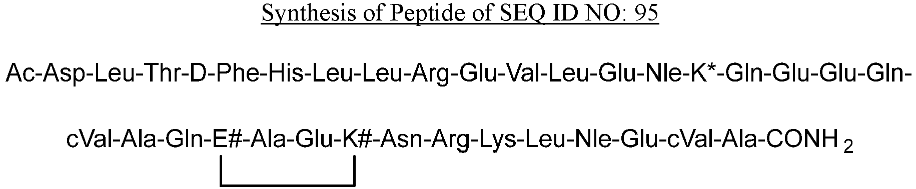
Synthesis of Peptide of SEQ ID NO: 95

Ac-Asp-Leu-Thr-D-Phe-His-Leu-Leu-Arg-Glu-Val-Leu-Glu-Nle-K*-Gln-Glu-Glu-Gln-

cVal-Ala-Gln-E#-Ala-Glu-K#-Asn-Arg-Lys-Leu-Nle-Glu-cVal-Ala-CONH 2


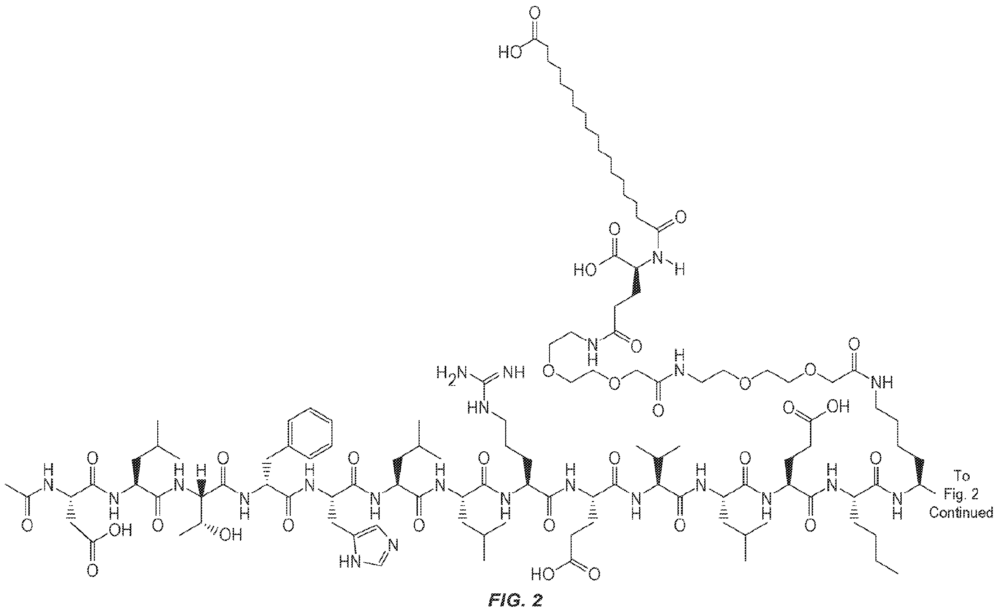

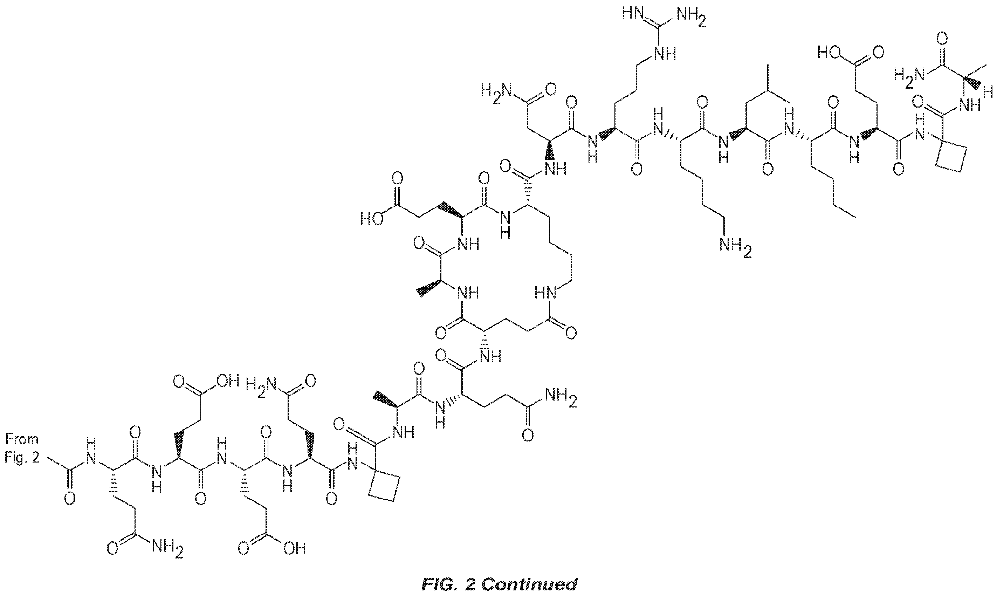

Peptide of SEQ ID NO: 95, wherein K* is Lys-(2-[2-(2-amino-ethoxy)-ethoxy]-acetyl)₂-γGlu-C18-diacid (Table B1.1, Formula I, FIG. 2), was synthesized using standard Fmoc chemistry based on the following procedure (Synthesis 1):

Resin preparation: A solution of Rink Amine MBHA resin (0.2 mmol, 1.00 eq, Sub 0.33 mmol/g) in DMF (15.0 mL) was agitated with N₂ at 25°C for 1.0 hour.

Deprotection: 20% piperidine in DMF (15.0 mL) was added and the resin was agitated with N₂ at 25°C for 30 minutes. The resin was washed with DMF (15.0 mL, five times) and filtered.

Coupling: A solution of HBTU (2.85 eq) and Fmoc-Ala-OH (3.00 eq) in DMF (4.0 mL) was added to the resin. DIEA (6.00 eq) was added, and the mixture was agitated with N₂ at 25°C for 30-60 minutes. The coupling reaction was monitored by Ninhydrin and Chloranil tests. The resin was washed with DMF (15.0 mL, five times).

The steps for Deprotection and Coupling were repeated for the coupling of the following amino acids (1-34) as outlined in Table 2A.

**Table 2A. Peptide of SEQ ID NO: 95 Synthesis Procedure (Synthesis 1).**

| # | Materials | Coupling reagents |
|---|---|---|
| 1 | Fmoc-cVal-OH (2.00 eq) | HATU (1.90 eq) and DIEA (4.00 eq) |
| 2 | Fmoc-Glu(OtBu)-OH (3.00 eq) | HBTU (2.85 eq) and DIEA (6.00 eq) |
| 3 | Fmoc-Nle-OH (2.00 eq) | HATU (1.90 eq) and DIEA (4.00 eq) |
| 4 | Fmoc-Leu-OH (3.00 eq) | HBTU (2.85 eq) and DIEA (6.00 eq) |
| 5 | Fmoc-Lys(Boc)-OH (3.00 eq) | HBTU (2.85 eq) and DIEA (6.00 eq) |
| 6 | Fmoc-Arg(Pbf)-OH (3.00 eq) | HBTU (2.85 eq) and DIEA (6.00 eq) |
| 7 | Fmoc-Asn(Trt)-OH (3.00 eq) | HBTU (2.85 eq) and DIEA (6.00 eq) |
| 8 | Fmoc-cyclo[Glu-Ala-Glu(OtBu)-Lys]-OH (2.00 eq) | HATU (1.90 eq) and DIEA (4.00 eq) |
| | | Coupling time: 60 minutes |
| 9 | Fmoc-Gln(Trt)-OH (3.00 eq) | HATU (2.85 eq) and DIEA (6.00 eq) |
| 10 | Fmoc-Ala-OH (3.00 eq) | HATU (2.85 eq) and DIEA (6.00 eq) |
| 11 | Fmoc-cVal-OH (2.00 eq) | HATU (1.90 eq) and DIEA (4.00 eq) |
| 12 | Fmoc-Gln(Trt)-OH (3.00 eq) | HATU (2.85 eq) and DIEA (6.00 eq) |
| | | Coupling time: 60 minutes |
| 13 | Fmoc-Glu(OtBu)-OH (3.00 eq) | HATU (2.85 eq) and DIEA (6.00 eq) |
| 14 | Fmoc- Glu(OtBu)-OH (3.00 eq) | HATU (2.85 eq) and DIEA (6.00 eq) |
| 15 | Fmoc-Gln(Trt)-OH (3.00 eq) | HATU (2.85 eq) and DIEA (6.00 eq) |
| 16 | Fmoc-Lys(Alloc)-OH (2.00 eq) | HATU (1.90 eq) and DIEA (4.00 eq) |
| 17 | Fmoc-Nle-OH (3.00 eq) | HATU (2.85 eq) and DIEA (6.00 eq) |
| 18 | Fmoc-Glu(OtBu)-OH (3.00 eq) | HATU (2.85 eq) and DIEA (6.00 eq) |
| 19 | Fmoc-Leu-OH (3.00 eq) | HATU (2.85 eq) and DIEA (6.00 eq) |
| 20 | Fmoc-Val-OH (3.00 eq) | HATU (2.85 eq) and DIEA (6.00 eq) |
| 21 | Fmoc-Glu(OtBu)-OH (3.00 eq) | HATU (2.85 eq) and DIEA (6.00 eq) |
| 22 | Fmoc-Arg(Pbf)-OH(3.00 eq) | HATU (2.85 eq) and DIEA (6.00 eq) |
| 23 | Fmoc-Leu-OH (3.00 eq) | HATU (2.85 eq) and DIEA (6.00 eq) |
| 24 | Fmoc-Leu-OH (3.00 eq) | HATU (2.85 eq) and DIEA (6.00 eq) |
| 25 | Fmoc-His(Trt)-OH (3.00 eq) | HATU (2.85 eq) and DIEA (6.00 eq) |
| 26 | Fmoc-D-Phe-OH (3.00 eq) | HATU (2.85 eq) and DIEA (6.00 eq) |
| 27 | Fmoc-Thr(tBu)-OH (3.00 eq) | HATU (2.85 eq) and DIEA (6.00 eq) |
| 28 | Fmoc-Leu-OH (3.00 eq) | HATU (2.85 eq) and DIEA (6.00 eq) |
| 29 | Fmoc-Asp(OtBu)-OH (3.00 eq) | HATU (2.85 eq) and DIEA (6.00 eq) |
| 30 | Ac | 10% Ac₂O/5% NMM/85% DMF |
| - | De-Alloc & OAll | PhSiH₃ (10.0 eq) and Pd(PPh₃)₄(0.1 eq) in DCM(15 minutes, three times) |
| 31 | Fmoc-(AEEA)-OH (2.00 eq) | HATU (1.90 eq) and DIEA (4.00 eq) |
| 32 | Fmoc-(AEEA)-OH (2.00 eq) | HATU (1.90 eq) and DIEA (4.00 eq) |
| 33 | Fmoc-Glu-OtBu (2.00 eq) | HATU (1.90 eq) and DIEA (4.00 eq) |
| 34 | 18-OtBu-18-Oxooctadecanoic acid (2.00 eq) | HATU (1.90 eq) and DIEA (4.00 eq) |

Peptide Cleavage: Cleavage solution (20.0 mL, 92.0% TFA/ 2.50% TIS /2.50% H₂O /3.0% DTT) was added to the flask containing resin at room temperature followed by stirring for 1.5 hour. The peptide was then precipitated with cold isopropyl ether (100 mL) and filtered. The filter cake was washed with isopropyl ether (50 mL, two times). The crude peptide was dried under vacuum for 1 hour to get the crude peptide (0.9 g), and the crude peptide was confirmed via LCMS (MS cal.: 4655.38, MS observed: [M+4H]⁴⁺ = 1164.90; Rt= 1.043-1.114 minutes.

The crude peptide was purified by prep-HPLC (TFA condition: A: 0.075 % TFA in H₂O, B: ACN) to give the final product (92.1 mg, 18.79 µmol, 9.44% yield, 95.00% purity, TFA) as a white solid. The peptide was confirmed via LCMS (MS cal.: 4655.38, MS observed: [M+4H]⁴⁺ = 1164.90, Rt= 1.55 minutes) and HPLC (ES26095-1-P7B, Rt= 7.76 minutes). Purification conditions were as outlined in Table 2B.

**Table 2B. Peptide of SEQ ID NO: 95 Purification Conditions (Synthesis 1)**

| Separation condition | |
|---|---|
| Dissolution condition | Dissolve in 20%ACN-H₂O |
| Instrument | Gilson GX-281 |
| Mobile Phase | A: H₂O (0.075%TFA in H₂O) |
| | B: CH₃CN |
| Gradient | 32-52-40 minutes, Retention time: 29 minutes |
| Column | YMC-Actus Triart C18, 250*30 mm, 5 um, 120Å |
| Flow Rate | 20 mL/minute |
| Wavelength | 220/254 nm |
| Oven Temperature | 25°C |

Alternatively, the peptide was synthesized (Synthesis 2) using standard Fmoc chemistry as described below:
Resin preparation: The Rink Amide MBHA resin (1.00 mmol, 1.00 eq., Sub 0.33 mmol/g) in DMF (20.0 mL) was agitated with N₂ for 2 hours at 20°C. The resin was then washed with DMF (40.0 mL, five times).

Deprotection: 20% Piperidine in DMF (40.0 mL) was added and the mixture was agitated with N₂ for 10 minutes at 20°C. The resin was then washed with DMF (40.0 mL, five times) and filtered to prepare for amino acid coupling.

Coupling: A solution of HBTU (2.85 eq.) and Fmoc-Ala-OH (3.00 eq.) in DMF (20.0 mL) was added to the resin, the DIEA (6.00 eq.) was added, and the mixture was agitated with N₂ at 20°C for 30 minutes. The resin was washed with DMF (40.0 mL, five times).

The Deprotection and Coupling steps were then repeated for the coupling of following amino acids (1-29) outlined in Table 2C.

**Table 2C. Peptide of SEQ ID NO: 95 Synthesis 2 Part 1 (Synthesis 2).**

| # | Materials | Coupling Reagents |
|---|---|---|
| 1 | Fmoc-cVal-Oh (2.00 eq.) | HATU (1.90 eq.) and DIEA (4.00 eq.) |
| 2 | Fmoc-Glu(OtBu)-OH (3.00 eq.) | HATU (2.85 eq.) and DIEA (6.00 eq.) |
| 3 | Fmoc-Nle-OH (3.00 eq.) | HATU (2.85 eq.) and DIEA (6.00 eq.) |
| 4 | Fmoc-Leu-OH (3.00 eq.) | HATU (2.85 eq.) and DIEA (6.00 eq.) |
| 5 | Fmoc-Lys(Boc)-OH (3.00 eq.) | HATU (2.85 eq.) and DIEA (6.00 eq.) |
| 6 | Fmoc-Arg(Pbf)-OH (3.00 eq.) | HATU (2.85 eq.) and DIEA (6.00 eq.) |
| 7 | Fmoc-Asn(Trt)-OH (3.00 eq.) | HATU (2.85 eq.) and DIEA (6.00 eq.) |
| 8 | Fmoc-cyclo[Glu-Ala-Glu(OtBu)-Lys]-OH (2.00 eq.) | HATU (1.90 eq.) and DIEA (4.00 eq.) |
| 9 | Fmoc-Gln(Trt)-OH (3.00 eq.) | HATU (2.85 eq.) and DIEA (6.00 eq.) |
| 10 | Fmoc-Ala-OH (3.00 eq.) | HATU (2.85 eq.) and DIEA (6.00 eq.) |
| 11 | Fmoc-cVal-OH (2.00 eq.) | HATU (1.90 eq.) and DIEA (4.00 eq.) |
| 12 | Fmoc-Gln(Trt)-OH (3.00 eq.) | HATU (2.85 eq.) and DIEA (6.00 eq.) |
| 13 | Fmoc-Glu(OtBu)-OH (3.00 eq.) | HATU (2.85 eq.) and DIEA (6.00 eq.) |
| 14 | Fmoc-Glu(OtBu)-OH (3.00 eq.) | HATU (2.85 eq.) and DIEA (6.00 eq.) |
| 15 | Fmoc-Gln(Trt)-OH (3.00 eq.) | HATU (2.85 eq.) and DIEA (6.00 eq.) |
| 16 | Fmoc-Lys(Dde)-OH (3.00 eq.) | HATU (2.85 eq.) and DIEA (6.00 eq.) |
| 17 | Fmoc-Nle-OH (2.00 eq.) | HATU (1.90 eq.) and DIEA (4.00 eq.) |
| 18 | Fmoc-Glu(OtBu)-OH (3.00 eq.) | HATU (2.85 eq.) and DIEA (6.00 eq.) |
| 19 | Fmoc-Leu-OH (3.00 eq.) | HOAT (3.00 eq.) and DIC (3.00 eq.) |
| 20 | Fmoc-Val-OH (3.00 eq.) | HATU (2.85 eq.) and DIEA (6.00 eq.) |
| 21 | Fmoc-Glu(OtBu)-OH (3.00 eq.) | HATU (2.85 eq.) and DIEA (6.00 eq.) |
| 22 | Fmoc-Arg(Pbf)-OH (3.00 eq.) | HATU (2.85 eq.) and DIEA (6.00 eq.) |
| 23 | Fmoc-Leu-OH (3.00 eq.) | HATU (2.85 eq.) and DIEA (6.00 eq.) |
| 24 | Fmoc-Leu-OH (3.00 eq.) | HATU (2.85 eq.) and DIEA (6.00 eq.) |
| 25 | Fmoc-His(Trt)-OH (3.00 eq.) | HATU (2.85 eq.) and DIEA (6.00 eq.) |
| 26 | Fmoc-D-Phe-OH (3.00 eq.) | HATU (2.85 eq.) and DIEA (6.00 eq.) |
| 27 | Fmoc-Thr(tBu)-OH (3.00 eq.) | HATU (2.85 eq.) and DIEA (6.00 eq.) |
| 28 | Fmoc-Leu-OH (3.00 eq.) | HATU (2.85 eq.) and DIEA (6.00 eq.) |
| 29 | Fmoc-Asp(OtBu)-OH (3.00 eq.) | HATU (2.85 eq.) and DIEA (6.00 eq.) |

10% Ac2O/5% DIEA/85% DMF was added into the resin, the mixture was agitated with N₂ at 20°C for 30 minutes, and the resin was washed with DMF (40.0 mL, five times).

3% hydrazine hydrate/DMF was added into the resin, and the mixture was agitated with N₂ at 20°C for 10 minutes. The resin was then washed with DMF (40.0 mL, five times).

The Deprotection and Coupling steps were then repeated for the coupling of the following amino acids (30-33) as outlined in Table 2D.

**Table 2D. Peptide of SEQ ID NO: 95 Synthesis 2 Part 2 (Synthesis 2).**

| # | Materials | Coupling Reagents |
|---|---|---|
| 30 | Fmoc-(AEEA)-OH (3.00 eq.) | HATU (2.85 eq.) and DIEA (6.00 eq.) |
| 31 | Fmoc-(AEEA)-OH (3.00 eq.) | HATU (2.85 eq.) and DIEA (6.00 eq.) |
| 32 | Fmoc-Glu-OtBu (3.00 eq.) | HATU (2.85 eq.) and DIEA (6.00 eq.) |
| 33 | 18-(tert-Butoxy)-18-oxooctadecanoic acid (3.00 eq.) | HATU (2.85 eq.) and DIEA (6.00 eq.) |

Peptide Cleavage and Purification: Cleavage solution (100.0 mL, 92.5% TFA/2.50% TIS/2.50% H₂O/2.50% 3-Mercaptopropionic acid) was added to the flask containing resin at room temperature and stirred for 2 hours.

The peptide was precipitated with cold isopropyl ether (900 mL). The filter cake was collected, and washed with isopropyl ether (900 mL, twice). The filter cake was dried under vacuum for 2 hours to obtain crude peptide (4.60 g). The crude peptide was confirmed via LCMS: MS cal.: 4655.38, MS observed: [M+4H]4+ = 1164.8; Rt = 1.579 minutes).

The crude peptide was purified by prep-HPLC (TFA condition: A: 0.075 % TFA in H₂O, B: ACN) and then was purified by prep-HPLC (HCl condition, A: 0.025%HCl, B: H₂O) to give the final product (460.9 mg, 89.57 µmol, 8.95% yield, 97.60% purity, HCl) as a white solid. The peptide was confirmed via LCMS: MS cal.: 4655.38, MS observed: [M+4H]4+ = 1164.6; Rt=1.249 min) and HPLC: Rt = 12.532 min).

### Example A3

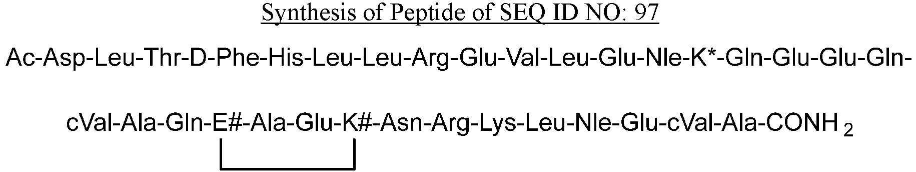
Synthesis of Peptide of SEQ ID NO: 97
Ac-Asp-Leu-Thr-D-Phe-His-Leu-Leu-Arg-Glu-Val-Leu-Glu-Nle-K*-Gln-Glu-Glu-Gln-
cVal-Ala-Gln-E#-Ala-Glu-K#-Asn-Arg-Lys-Leu-Nle-Glu-cVal-Ala-CONH 2

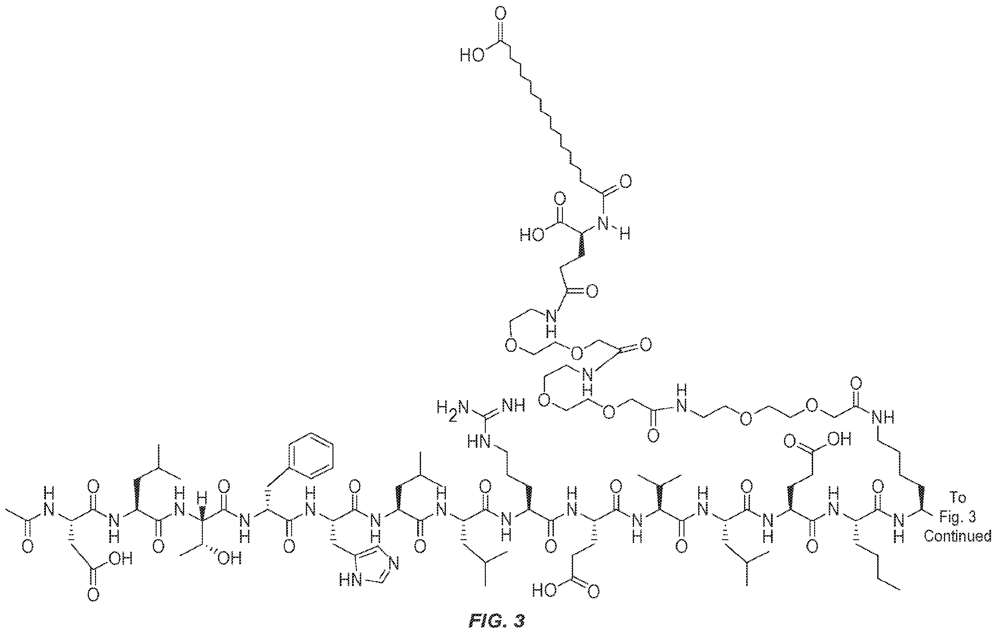

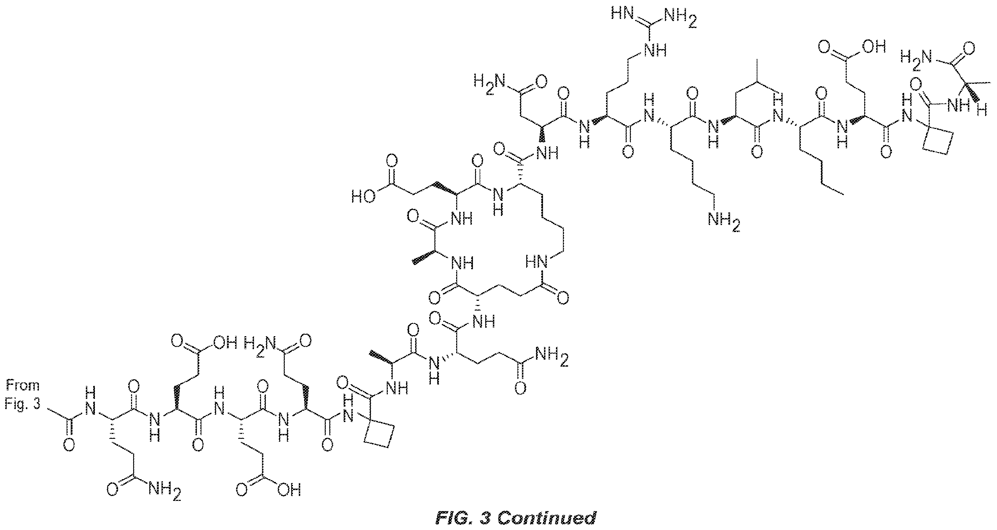

Peptide of SEQ ID NO: 97, wherein K* is Lys-(2-[2-(2-amino-ethoxy)-ethoxy]-acetyl)₃-γGlu-C18-diacid (Table B1.1, Formula I, FIG. 3), was synthesized using standard Fmoc chemistry based on the following procedure:
Resin preparation: A solution of Rink Amine MBHA resin (0.2 mmol, 1.00 eq, Sub 0.33 mmol/g) in DMF (15.0 mL) was agitated with N₂ at 25°C for 1.0 hour.

Deprotection: 20% piperidine in DMF (15.0 mL) was added and the resin was agitated with N₂ at 25°C for 30 minutes. The resin was washed with DMF (15.0 mL, five times) and filtered.

Coupling: A solution of HBTU (2.85 eq) and Fmoc-Ala-OH (3.00 eq) in DMF (4.0 mL) was added to the resin, DIEA (6.00 eq) was added, and the mixture was agitated with N₂ at 25°C for 30-60 minutes. The coupling reaction was monitored by Ninhydrin and Chloranil tests. The resin was washed with DMF (15.0 mL, five times).

The steps for Deprotection and Coupling were repeated for the coupling of following amino acids (1-35) as outlined in Table 3A.

**Table 3A. Peptide of SEQ ID NO: 97 Synthesis Procedure.**

| # | Materials | Coupling Reagents |
|---|---|---|
| 1 | Fmoc-cVal-OH (2.00 eq) | HATU (1.90 eq) and DIEA (4.00 eq) |
| 2 | Fmoc-Glu(OtBu)-OH (3.00 eq) | HBTU (2.85 eq) and DIEA (6.00 eq) |
| 3 | Fmoc-Nle-OH (2.00 eq) | HATU (1.90 eq) and DIEA (4.00 eq) |
| 4 | Fmoc-Leu-OH (3.00 eq) | HBTU (2.85 eq) and DIEA (6.00 eq) |
| 5 | Fmoc-Lys(Boc)-OH (3.00 eq) | HBTU (2.85 eq) and DIEA (6.00 eq) |
| 6 | Fmoc-Arg(Pbf)-OH (3.00 eq) | HBTU (2.85 eq) and DIEA (6.00 eq) |
| 7 | Fmoc-Asn(Trt)-OH (3.00 eq) | HBTU (2.85 eq) and DIEA (6.00 eq) |
| 8 | Fmoc-cyclo[Glu-Ala-Glu(OtBu)-Lys]-OH (2.00 eq) | HATU (1.90 eq) and DIEA (4.00 eq) |
| | | Coupling time: 60 minutes |
| 9 | Fmoc-Gln(Trt)-OH (3.00 eq) | HATU (2.85 eq) and DIEA (6.00 eq) |
| 10 | Fmoc-Ala-OH (3.00 eq) | HATU (2.85 eq) and DIEA (6.00 eq) |
| 11 | Fmoc-cVal-OH (2.00 eq) | HATU (1.90 eq) and DIEA (4.00 eq) |
| 12 | Fmoc-Gln(Trt)-OH (3.00 eq) | HATU (2.85 eq) and DIEA (6.00 eq) |
| | | Coupling time: 60 minutes |
| 13 | Fmoc-Glu(OtBu)-OH (3.00 eq) | HATU (2.85 eq) and DIEA (6.00 eq) |
| 14 | Fmoc- Glu(OtBu)-OH (3.00 eq) | HATU (2.85 eq) and DIEA (6.00 eq) |
| 15 | Fmoc-Gln(Trt)-OH (3.00 eq) | HATU (2.85 eq) and DIEA (6.00 eq) |
| 16 | Fmoc-Lys(Alloc)-OH (2.00 eq) | HATU (1.90 eq) and DIEA (4.00 eq) |
| 17 | Fmoc-Nle-OH (3.00 eq) | HATU (2.85 eq) and DIEA (6.00 eq) |
| 18 | Fmoc-Glu(OtBu)-OH (3.00 eq) | HATU (2.85 eq) and DIEA (6.00 eq) |
| 19 | Fmoc-Leu-OH (3.00 eq) | HATU (2.85 eq) and DIEA (6.00 eq) |
| 20 | Fmoc-Val-OH (3.00 eq) | HATU (2.85 eq) and DIEA (6.00 eq) |
| 21 | Fmoc-Glu(OtBu)-OH (3.00 eq) | HATU (2.85 eq) and DIEA (6.00 eq) |
| 22 | Fmoc-Arg(Pbf)-OH(3.00 eq) | HATU (2.85 eq) and DIEA (6.00 eq) |
| 23 | Fmoc-Leu-OH(3.00 eq) | HATU (2.85 eq) and DIEA (6.00 eq) |
| 24 | Fmoc-Leu-OH (3.00 eq) | HATU (2.85 eq) and DIEA (6.00 eq) |
| 25 | Fmoc-His(Trt)-OH (3.00 eq) | HATU (2.85 eq) and DIEA (6.00 eq) |
| 26 | Fmoc-D-Phe-OH (3.00 eq) | HATU (2.85 eq) and DIEA (6.00 eq) |
| 27 | Fmoc-Thr(tBu)-OH (3.00 eq) | HATU (2.85 eq) and DIEA (6.00 eq) |
| 28 | Fmoc-Leu-OH (3.00 eq) | HATU (2.85 eq) and DIEA (6.00 eq) |
| 29 | Fmoc-Asp(OtBu)-OH (3.00 eq) | HATU (2.85 eq) and DIEA (6.00 eq) |
| 30 | Ac | 10% Ac₂O/5% NMM/85% DMF |
| - | De-Alloc & OAll | PhSiH₃ (10.0 eq) and Pd(PPh₃)₄ |
| 31 | Fmoc-(AEEA)-OH (2.00 eq) | PhSiH₃ (10.0 eq) and Pd(PPh₃)₄(0.1 eq) in DCM(15 minutes, three times) |
| 32 | Fmoc-(AEEA)-OH (2.00 eq) | HATU (1.90 eq) and DIEA (4.00 eq) |
| 33 | Fmoc-(AEEA)-OH (2.00 eq) | HATU (1.90 eq) and DIEA (4.00 eq) |
| 34 | Fmoc-Glu-OtBu (2.00 eq) | HATU (1.90 eq) and DIEA (4.00 eq) |
| 35 | 18-OtBu-18-Oxooctadecanoic acid (2.00 eq) | HATU (1.90 eq) and DIEA (4.00 eq) |

Peptide Cleavage: Cleavage solution (20.0 mL, 92.0% TFA/ 2.50% TIS /2.50% H₂O /3.0% DTT) was added to the flask containing resin at room temperature and stirred for 1.5 hours. The peptide was precipitated with cold isopropyl ether (100 mL), and filtered. The filter cake was washed with isopropyl ether (50 mL, twice). The crude peptide was dried under vacuum for 1-hour (1.0 g) LCMS (MS cal.: 4800.53, MS observed: [M+4H]⁴⁺ = 1201.20, Rt = 1.043-1.105 minutes).

The crude peptide was purified by prep-HPLC (TFA condition: A: 0.075 % TFA in H₂O, B: ACN) to give the final product (101.1 mg, 20.07 µmol, 10.03% yield, 95.30% purity, TFA) as a white solid. LCMS (MS cal.: 4800.53, MS observed: [M+4H]⁴⁺ =1201.20, Rₜ= 1.052-1.159 minutes) and HPLC (Rt= 7.54 minutes). Purification conditions were as outlined in Table 3B.

**Table 3B. Peptide of SEQ ID NO: 97 Purification Conditions.**

| Separation condition | |
|---|---|
| Dissolution condition | Dissolve in 20%ACN-H₂O |
| Instrument | Gilson GX-281 |
| Mobile Phase | A: H₂O (0.075%TFA in H₂O) |
| | B: CH₃CN |
| Gradient | 30-60-40 minutes, Retention time: 27 minutes |
| Column | YMC-Actus Triart C18, 250*30 mm, 5 um, 120Å |
| Flow Rate | 20 mL/minute |
| Wavelength | 220/254 nm |
| Oven Temperature | 25°C |

### Example A4

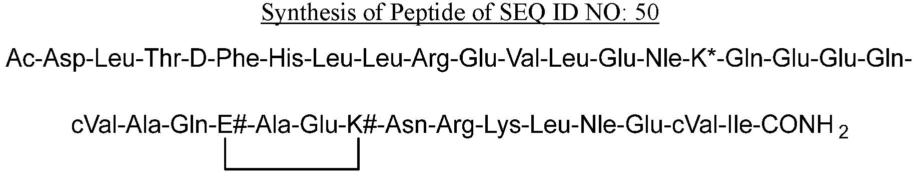
Synthesis of Peptide of SEQ ID NO: 50

Ac-Asp-Leu-Thr-D-Phe-His-Leu-Leu-Arg-Glu-Val-Leu-Glu-Nle-K*-Gln-Glu-Glu-Gln-

cVal-Ala-Gln-E#-Ala-Glu-K#-Asn-Arg-Lys-Leu-Nle-Glu-cVal-Ile-CONH 2


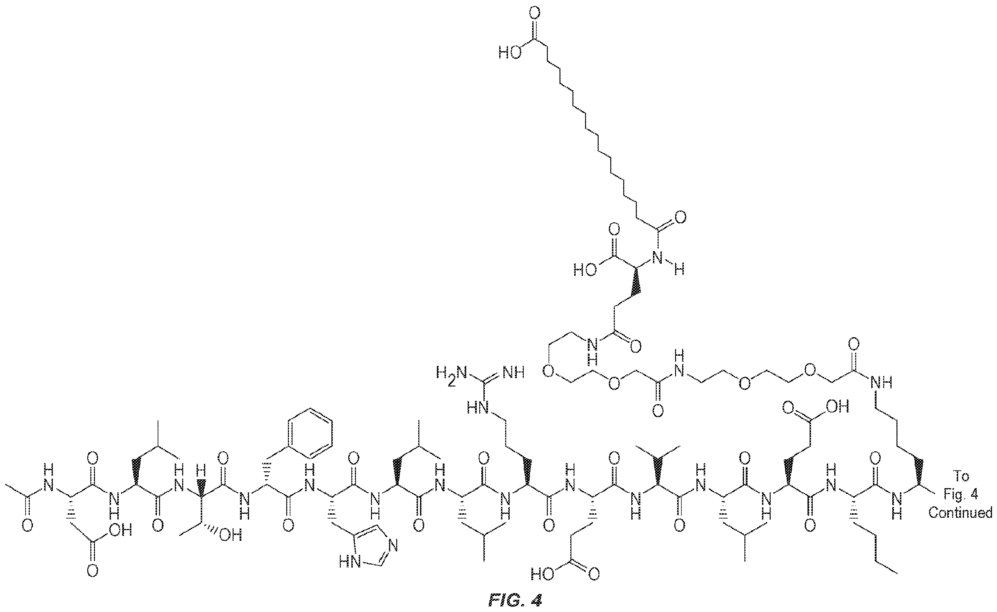

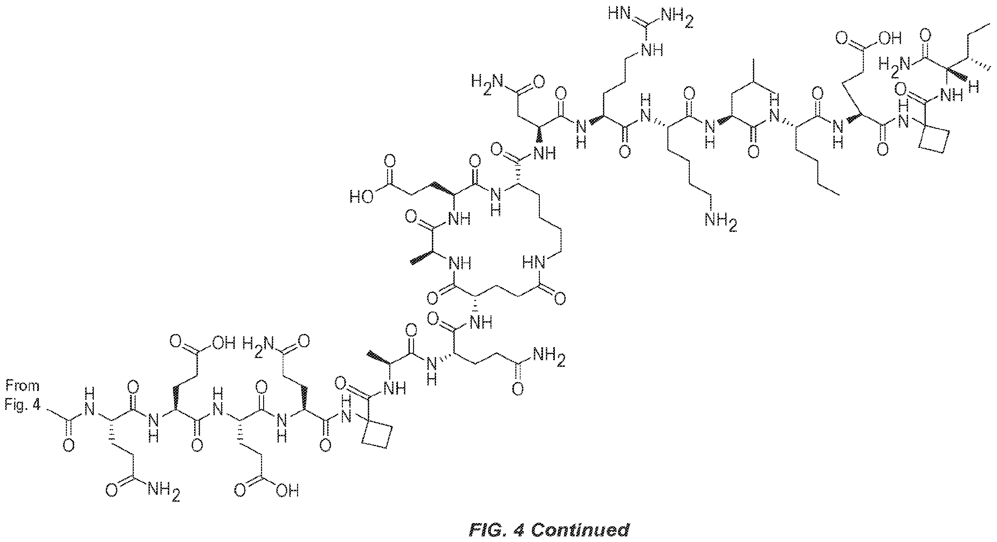

Peptide of SEQ ID NO: 50, wherein K* is Lys-(2-[2-(2-amino-ethoxy)-ethoxy]-acetyl)₂-γGlu-C18-diacid (Table B1.1, Formula I, FIG. 4), was synthesized using standard Fmoc chemistry based on the following procedure:
Resin preparation: A solution of Rink Amine MBHA resin (0.7 mmol, 1.00 eq, Sub 0.33 mmol/g) in DMF (30.0 mL) was agitated with N₂ at 25°C for 1.0 hour.

Deprotection: 20% piperidine in DMF (30.0 mL) was added and the resin was agitated with N₂ at 25°C for 30 minutes. The resin was washed with DMF (30.0 mL, five times) and filtered.

Coupling: A solution of HBTU (2.85 eq) and Fmoc-Ile-OH (3.00 eq) in DMF (15.0 mL) was added to the resin. DIEA (6.00 eq) was added, and the mixture was agitated with N₂ at 25°C for 30-60 minutes. The coupling reaction was monitored by Ninhydrin and Chloranil tests. The resin was washed with DMF (30.0 mL, five times).

The steps for Deprotection and Coupling were repeated for the coupling of following amino acids (1-34) as outlined in Table 4A.

**Table 4A. Peptide of SEQ ID NO: 50 Synthesis Procedure.**

| # | Materials | Coupling reagents |
|---|---|---|
| 1 | Fmoc-cVal-OH (2.00 eq) | HATU (1.90 eq) and DIEA (4.00 eq) |
| 2 | Fmoc-Glu(OtBu)-OH (3.00 eq) | HBTU (2.85 eq) and DIEA (6.00 eq) |
| 3 | Fmoc-Nle-OH (2.00 eq) | HATU (1.90 eq) and DIEA (4.00 eq) |
| 4 | Fmoc-Leu-OH (3.00 eq) | HBTU (2.85 eq) and DIEA (6.00 eq) |
| 5 | Fmoc-Lys(Boc)-OH (3.00 eq) | HBTU (2.85 eq) and DIEA (6.00 eq) |
| 6 | Fmoc-Arg(Pbf)-OH (3.00 eq) | HBTU (2.85 eq) and DIEA (6.00 eq) |
| 7 | Fmoc-Asn(Trt)-OH (3.00 eq) | HBTU (2.85 eq) and DIEA (6.00 eq) |
| 8 | Fmoc-cyclo[Glu-Ala-Glu(OtBu)-Lys]-OH (2.00 eq) | HATU (1.90 eq) and DIEA (4.00 eq) |
| | | Coupling time: 60 minutes |
| 9 | Fmoc-Gln(Trt)-OH (3.00 eq) | HATU (2.85 eq) and DIEA (6.00 eq) |
| 10 | Fmoc-Ala-OH (3.00 eq) | HATU (2.85 eq) and DIEA (6.00 eq) |
| 11 | Fmoc-cVal-OH (2.00 eq) | HATU (1.90 eq) and DIEA (4.00 eq) |
| 12 | Fmoc-Gln(Trt)-OH (3.00 eq) | HATU (2.85 eq) and DIEA (6.00 eq) |
| | | Coupling time: 60 minutes |
| 13 | Fmoc-Glu(OtBu)-OH (3.00 eq) | HATU (2.85 eq) and DIEA (6.00 eq) |
| 14 | Fmoc- Glu(OtBu)-OH (3.00 eq) | HATU (2.85 eq) and DIEA (6.00 eq) |
| 15 | Fmoc-Gln(Trt)-OH (3.00 eq) | HATU (2.85 eq) and DIEA (6.00 eq) |
| 16 | Fmoc-Lys(Alloc)-OH (2.00 eq) | HATU (1.90 eq) and DIEA (4.00 eq) |
| 17 | Fmoc-Nle-OH (3.00 eq) | HATU (2.85 eq) and DIEA (6.00 eq) |
| 18 | Fmoc-Glu(OtBu)-OH (3.00 eq) | HATU (2.85 eq) and DIEA (6.00 eq) |
| 19 | Fmoc-Leu-OH (3.00 eq) | HATU (2.85 eq) and DIEA (6.00 eq) |
| 20 | Fmoc-Val-OH (3.00 eq) | HATU (2.85 eq) and DIEA (6.00 eq) |
| 21 | Fmoc-Glu(OtBu)-OH (3.00 eq) | HATU (2.85 eq) and DIEA (6.00 eq) |
| 22 | Fmoc-Arg(Pbf)-OH(3.00 eq) | HATU (2.85 eq) and DIEA (6.00 eq) |
| 23 | Fmoc-Leu-OH(3.00 eq) | HATU (2.85 eq) and DIEA (6.00 eq) |
| 24 | Fmoc-Leu-OH (3.00 eq) | HATU (2.85 eq) and DIEA (6.00 eq) |
| 25 | Fmoc-His(Trt)-OH (3.00 eq) | HATU (2.85 eq) and DIEA (6.00 eq) |
| 26 | Fmoc-D-Phe-OH (3.00 eq) | HATU (2.85 eq) and DIEA (6.00 eq) |
| 27 | Fmoc-Thr(tBu)-OH (3.00 eq) | HATU (2.85 eq) and DIEA (6.00 eq) |
| 28 | Fmoc-Leu-OH (3.00 eq) | HATU (2.85 eq) and DIEA (6.00 eq) |
| 29 | Fmoc-Asp(OtBu)-OH (3.00 eq) | HATU (2.85 eq) and DIEA (6.00 eq) |
| 30 | Ac | 10% Ac₂O/5% NMM/85% DMF |
| - | De-Alloc & OAll | PhSiH₃ (10.0 eq) and Pd(PPh₃)₄(0.1 eq) in DCM(15 minutes, three times) |
| 31 | Fmoc-(AEEA)-OH (2.00 eq) | HATU (1.90 eq) and DIEA (4.00 eq) |
| 32 | Fmoc-(AEEA)-OH (2.00 eq) | HATU (1.90 eq) and DIEA (4.00 eq) |
| 33 | Fmoc-Glu-OtBu (2.00 eq) | HATU (1.90 eq) and DIEA (4.00 eq) |
| 34 | 18-OtBu-18-Oxooctadecanoic acid (2.00 eq) | HATU (1.90 eq) and DIEA (4.00 eq) |

Peptide Cleavage: Cleavage solution (80.0 mL, 92.0% TFA/ 2.50% TIS /2.50% H₂O /3.0% DTT) was added to the flask containing resin at room temperature and stirred for 1.5 hours. The peptide was precipitated with cold isopropyl ether (500 mL) and filtered. The filter cake was washed with isopropyl ether (200 mL, two times). The crude peptide was dried under vacuum for 1 hour to get the crude peptide (3.0 g). LCMS: MS cal.: 4697.46, MS observed: [M+4H]⁴⁺ = 1175.49; Rₜ = 1.066-1.145 minutes.

The crude peptide was purified by prep-HPLC (TFA condition: A: 0.075 % TFA in H₂O, B: ACN) and transferred to HCl salt by Prep-HPLC (HCl condition: A: 0.05% HCl in H₂O, B: ACN) to give the final product (283.0 mg, 58.62 µmol, 8.37% yield, 97.30% purity, HCl) as a white solid. LCMS: MS cal.: 4697.46, MS observed: [M+4H]⁴⁺ =1175.30; Rₜ= 1.65 minutes; and HPLC: Rt= 6.32 minutes. Purification conditions were as outlined in Table 4B.

**Table 4B. Peptide of SEQ ID NO: 50 Purification Conditions.**

| Separation condition | |
|---|---|
| Dissolution condition | Dissolve in 20%ACN-H₂O |
| Instrument | Welch Sail-1000 |
| Mobile Phase | A: H₂O (0.075%TFA in H₂O) |
| | B: CH₃CN |
| Gradient | 35-55-40 minutes, Retention time: 29 minutes |
| Column | Welch Ultimate^{®} XB-C18, 250*50 mm, 7 um, 120Å |
| Flow Rate | 150 mL/minute |
| Wavelength | 220/254 nm |
| Oven Temperature | 25°C |

| Re-separation condition | |
|---|---|
| Dissolution condition | Dissolve in ACN-H₂O |
| Instrument | Gilson GX-281 |
| Mobile Phase | A: H₂O (0.05% HCl in H₂O) |
| | B: CH₃CN |
| Gradient | 10-70-30 minutes, Retention time: 19 minutes |
| Column | YMC-Actus Triart C18, 250*30 mm, 5 um, 120Å |
| Flow Rate | 20 mL/minute |
| Wavelength | 220/254 nm |
| Oven Temperature | 25°C |

### Example A5

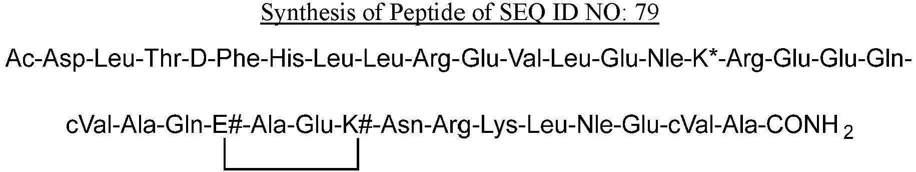
Synthesis of Peptide of SEQ ID NO: 79

Ac-Asp-Leu-Thr-D-Phe-His-Leu-Leu-Arg-Glu-Val-Leu-Glu-Nle-K*-Arg-Glu-Glu-Gln-

cVal-Ala-Gln-E#-Ala-Glu-K#-Asn-Arg-Lys-Leu-Nle-Glu-cVal-Ala-CONH 2


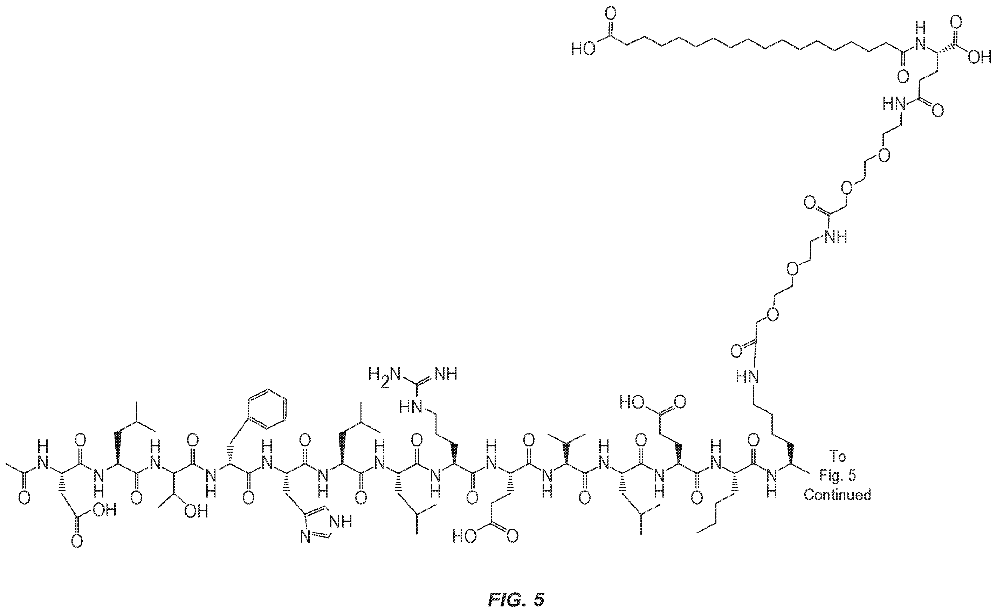

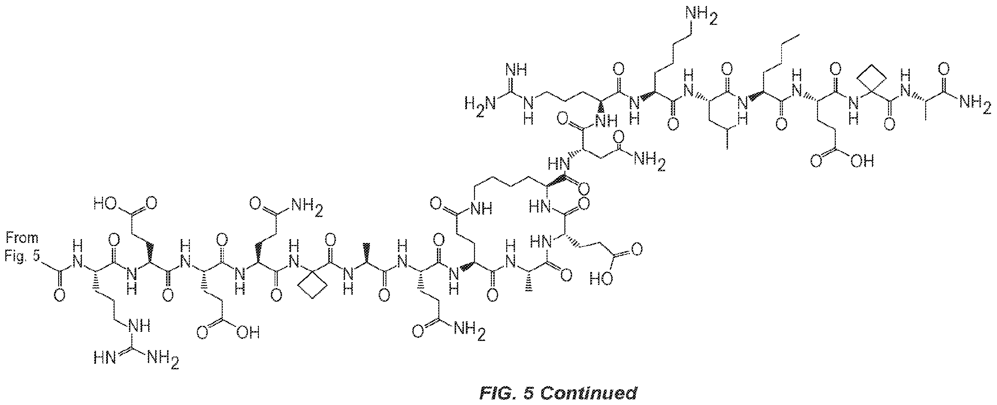

Peptide of SEQ ID NO: 79, wherein K* is Lys-(2-[2-(2-amino-ethoxy)-ethoxy]-acetyl)₂-γGlu-C18-diacid (Table B1.1, Formula I, FIG. 5), was synthesized using standard Fmoc chemistry based on the following procedure:
Resin preparation: A solution of Rink Amine MBHA resin (0.7 mmol, 1.00 eq, Sub 0.33 mmol/g) in DMF (30.0 mL) was agitated with N₂ at 25°C for 1.0 hour.

Deprotection: 20% piperidine in DMF (30.0 mL) was added and the resin was agitated with N₂ at 25°C for 30 minutes. The resin was washed with DMF (30.0 mL, 5 times) and filtered.

Coupling: A solution of HBTU (2.85 eq) and Fmoc-Ala-OH (3.00 eq) in DMF (15.0 mL) was added to the resin. DIEA (6.00 eq) was added, and the mixture was agitated with N₂ at 25°C for 30-60 minutes. The coupling reaction was monitored by Ninhydrin and Chloranil tests. The resin was washed with DMF (30.0 mL, 5 times).

The steps for Deprotection and Coupling were repeated for the coupling of following amino acids (1-34) outlined in Table 5A.

**Table 5A. Synthesis of Peptide of SEQ ID NO: 79.**

| # | Materials | Coupling reagents |
|---|---|---|
| 1 | Fmoc-cVal-OH (2.00 eq) | HATU (1.90 eq) and DIEA (4.00 eq) |
| 2 | Fmoc-Glu(OtBu)-OH (3.00 eq) | HBTU (2.85 eq) and DIEA (6.00 eq) |
| 3 | Fmoc-Nle-OH (2.00 eq) | HATU (1.90 eq) and DIEA (4.00 eq) |
| 4 | Fmoc-Leu-OH (3.00 eq) | HBTU (2.85 eq) and DIEA (6.00 eq) |
| 5 | Fmoc-Lys(Boc)-OH (3.00 eq) | HBTU (2.85 eq) and DIEA (6.00 eq) |
| 6 | Fmoc-Arg(Pbf)-OH (3.00 eq) | HBTU (2.85 eq) and DIEA (6.00 eq) |
| 7 | Fmoc-Asn(Trt)-OH (3.00 eq) | HBTU (2.85 eq) and DIEA (6.00 eq) |
| 8 | Fmoc-cyclo[Glu-Ala-Glu(OtBu)-Lys]-OH (2.00 eq) | HATU (1.90 eq) and DIEA (4.00 eq) |
| | | Coupling time: 60 minutes |
| 9 | Fmoc-Gln(Trt)-OH (3.00 eq) | HATU (2.85 eq) and DIEA (6.00 eq) |
| 10 | Fmoc-Ala-OH (3.00 eq) | HATU (2.85 eq) and DIEA (6.00 eq) |
| 11 | Fmoc-cVal-OH (2.00 eq) | HATU (1.90 eq) and DIEA (4.00 eq) |
| 12 | Fmoc-Gln(Trt)-OH (3.00 eq) | HATU (2.85 eq) and DIEA (6.00 eq) |
| | | Coupling time: 60 minutes |
| 13 | Fmoc-Glu(OtBu)-OH (3.00 eq) | HATU (2.85 eq) and DIEA (6.00 eq) |
| 14 | Fmoc- Glu(OtBu)-OH (3.00 eq) | HATU (2.85 eq) and DIEA (6.00 eq) |
| 15 | Fmoc-Arg(Pbf)-OH(3.00 eq) | HATU (2.85 eq) and DIEA (6.00 eq) |
| 16 | Fmoc-Lys(Alloc)-OH (2.00 eq) | HATU (1.90 eq) and DIEA (4.00 eq) |
| 17 | Fmoc-Nle-OH (3.00 eq) | HATU (2.85 eq) and DIEA (6.00 eq) |
| 18 | Fmoc-Glu(OtBu)-OH (3.00 eq) | HATU (2.85 eq) and DIEA (6.00 eq) |
| 19 | Fmoc-Leu-OH (3.00 eq) | HATU (2.85 eq) and DIEA (6.00 eq) |
| 20 | Fmoc-Val-OH (3.00 eq) | HATU (2.85 eq) and DIEA (6.00 eq) |
| 21 | Fmoc-Glu(OtBu)-OH (3.00 eq) | HATU (2.85 eq) and DIEA (6.00 eq) |
| 22 | Fmoc-Arg(Pbf)-OH(3.00 eq) | HATU (2.85 eq) and DIEA (6.00 eq) |
| 23 | Fmoc-Leu-OH(3.00 eq) | HATU (2.85 eq) and DIEA (6.00 eq) |
| 24 | Fmoc-Leu-OH (3.00 eq) | HATU (2.85 eq) and DIEA (6.00 eq) |
| 25 | Fmoc-His(Trt)-OH (3.00 eq) | HATU (2.85 eq) and DIEA (6.00 eq) |
| 26 | Fmoc-D-Phe-OH (3.00 eq) | HATU (2.85 eq) and DIEA (6.00 eq) |
| 27 | Fmoc-Thr(tBu)-OH (3.00 eq) | HATU (2.85 eq) and DIEA (6.00 eq) |
| 28 | Fmoc-Leu-OH (3.00 eq) | HATU (2.85 eq) and DIEA (6.00 eq) |
| 29 | Fmoc-Asp(OtBu)-OH (3.00 eq) | HATU (2.85 eq) and DIEA (6.00 eq) |
| 30 | Ac | 10% Ac₂O/5% NMM/85% DMF |
| - | De-Alloc & OAll | PhSiH₃ (10.0 eq) and Pd(PPh₃)₄(0.1 eq) in DCM(15 minutes, three times) |
| 31 | Fmoc-(AEEA)-OH (2.00 eq) | HATU (1.90 eq) and DIEA (4.00 eq) |
| 32 | Fmoc-(AEEA)-OH (2.00 eq) | HATU (1.90 eq) and DIEA (4.00 eq) |
| 33 | Fmoc-Glu-OtBu(2.00 eq) | HATU (1.90 eq) and DIEA (4.00 eq) |
| 34 | 18-OtBu-18-Oxooctadecanoic acid (2.00 eq) | HATU (1.90 eq) and DIEA (4.00 eq) |

Peptide Cleavage: Cleavage solution (80.0 mL, 92.0% TFA/ 2.50% TIS /2.50% H₂O /3.0% DTT) was added to the flask containing resin at room temperature and stirred for 1.5 hour. The peptide was then precipitated with cold isopropyl ether (500 mL), and filtered. The filter cake was washed with isopropyl ether (200 mL, twice). The crude peptide was dried under vacuum for 1 hour (3.4 g). LCMS: MS cal.: 4683.43, MS observed: [M+4H]⁴⁺ = 1171.90; Rt = 1.016-1.096 minutes.

The crude peptide was then purified by prep-HPLC (TFA condition: A: 0.075 % TFA in H₂O, B: ACN) and transferred to HCl salt by Prep-HPLC (HCl condition: A: 0.05% HCl in H₂O, B: ACN) to give the final product (318.2 mg, 66.51 µmol, 9.50% yield, 97.90% purity, HCl) as a white solid. LCMS: MS cal.: 4683.43, MS observed: [M+4H]⁴⁺ =1172.00; Rₜ= 1.043-1.141 minutes) and HPLC: Rₜ= 7.46 minutes). Purification conditions were as outlined below in Table 5B.

**Table 5B. Peptide of SEQ ID NO: 79 Purification Conditions.**

| Separation condition | |
|---|---|
| Dissolution condition | Dissolve in 20%ACN-H₂O |
| Instrument | Welch Sail-1000 |
| Mobile Phase | A: H₂O (0.075%TFA in H₂O) |
| | B: CH₃CN |
| Gradient | 32-52-40 minutes, Retention time: 30 minutes |
| Column | Welch Ultimate^{®} XB-C18, 250*50 mm, 7 um, 120Å |
| Flow Rate | 150 mL/minute |
| Wavelength | 220/254 nm |
| Oven Temperature | 25°C |

| Re-separation condition | |
|---|---|
| Dissolution condition | Dissolve in ACN-H₂O |
| Instrument | Gilson GX-281 |
| Mobile Phase | A: H₂O (0.05% HCl in H₂O) |
| | B: CH₃CN |
| Gradient | 10-70-30 minutes, Retention time: 17.5 minutes |
| Column | YMC-Actus Triart C18, 250*30 mm, 5 um, 120Å |
| Flow Rate | 20 mL/minute |
| Wavelength | 220/254 nm |
| Oven Temperature | 25^{°}C |

### Example A6

### Synthesis of Lactam Intermediate

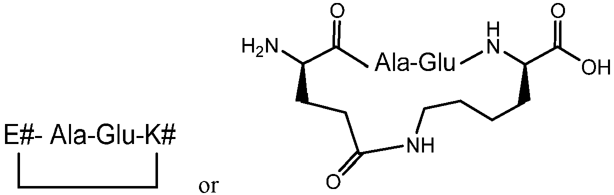

The peptide was synthesized using standard Fmoc chemistry as described below:
Resin preparation: To a solution of 2-CTC Resin (200.0 mmol, 1.00 eq, Sub 0.64 mmol/g) and Fmoc-Lys(Alloc)-OH (1.00 eq) in DCM (2.0 L) was added DIEA (6.00 eq), and the mixture was agitated with N₂ at 20°C for 4.0 hours. The MeOH (320.0 mL) was added and agitation was continued with N₂ at 20°C for 0.5 hours. The mixture was filtered, and washed with DMF (2.5 L, three times) and MeOH (2.0 L, three times).

Deprotection: 20% piperidine in DMF (2.5 L) was added and the resin was agitated with N₂ at 20°C for 30 minutes. The resin was washed with DMF (2.5 L, five times) and filtered.

Coupling: A solution of HBTU (1.90 eq) and Fmoc-Glu(OtBu)-OH (2.00 eq) in DMF (1.5 L) was added to the resin. DIEA (4.00 eq) was added, and the mixture was agitated with N₂ at 20°C for 30 minutes. The coupling reaction was monitored by Ninhydrin and Chloranil tests. The resin was washed with DMF (2.5 L, five times).

The Deprotection and Coupling steps were repeated for the coupling of the following amino acids (1-3) outlined in Table 6A.

**Table 6A. Synthesis of Lactam Intermediate.**

| # | Materials | Coupling reagents |
|---|---|---|
| 1 | Fmoc-Ala-OH (2.00 eq) | HBTU (1.90 eq) and DIEA (4.00 eq) |
| 2 | Fmoc-Glu(Oall)-OH (2.00 eq) | HBTU (1.90 eq) and DIEA (4.00 eq) |
| - | De Alloc & OAll | PhSiH₃ (10.0 eq) and Pd(PPh₃)₄ (0.1 eq) in DCM 15 minutes, three times |
| 3 | Cyclization | HOAT (2.00 eq) and DIC (2.00 eq) |
| | | Coupling time: 48h, two times |

Peptide Cleavage and Purification: Cleavage solution (20.0% HFIP/DCM) was added to the flask containing resin at room temperature and stirred for 0.5 hours, three times. The DCM was concentrated under reduced pressure LCMS: MS cal.: 735.8, MS observed: [M+H]⁺ = 736.7; Rt =1.114 minutes.

The crude peptide was purified by prep-HPLC (TFA condition: A: 0.075 % TFA in H₂O, B: ACN) to give the final product (41.8 g, 55.6 mmol, 27.7% yield, 98.04% purity, TFA) as a white solid. LCMS: MS cal.: 735.8, MS observed: [M+H]⁺ = 736.5; Rt= 1.552 minutes) and HPLC , Rt= 7.315 minutes. Purification conditions were as outlined below in Table 6B.

**Table 6B. Lactam Intermediate Purification Conditions.**

| Separation condition | |
|---|---|
| Dissolution condition | Dissolve in DMF |
| Instrument | Hanbon DAC-100 |
| Mobile Phase | A: H₂O (0.075% TFA in H₂O) |
| | B: CH₃CN |
| Gradient | 35-65-30 minutes, Retention time: 23 minutes |
| Column | Welch Ultimate^{®} XB-C18 250*100 mm, 10 um, 120Å |
| Flow Rate | 250 mL/minute |
| Wavelength | 220/254 nm |
| Oven Temperature | 25°C |

### Example A7

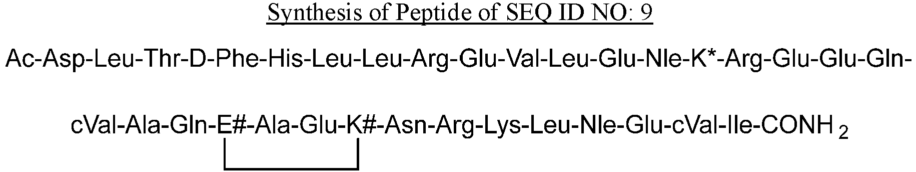
Synthesis of Peptide of SEQ ID NO: 9

Ac-Asp-Leu-Thr-D-Phe-His-Leu-Leu-Arg-Glu-Val-Leu-Glu-Nle-K*-Arg-Glu-Glu-Gln-

cVal-Ala-Gln-E#-Ala-Glu-K#-Asn-Arg-Lys-Leu-Nle-Glu-cVal-Ile-CONH 2


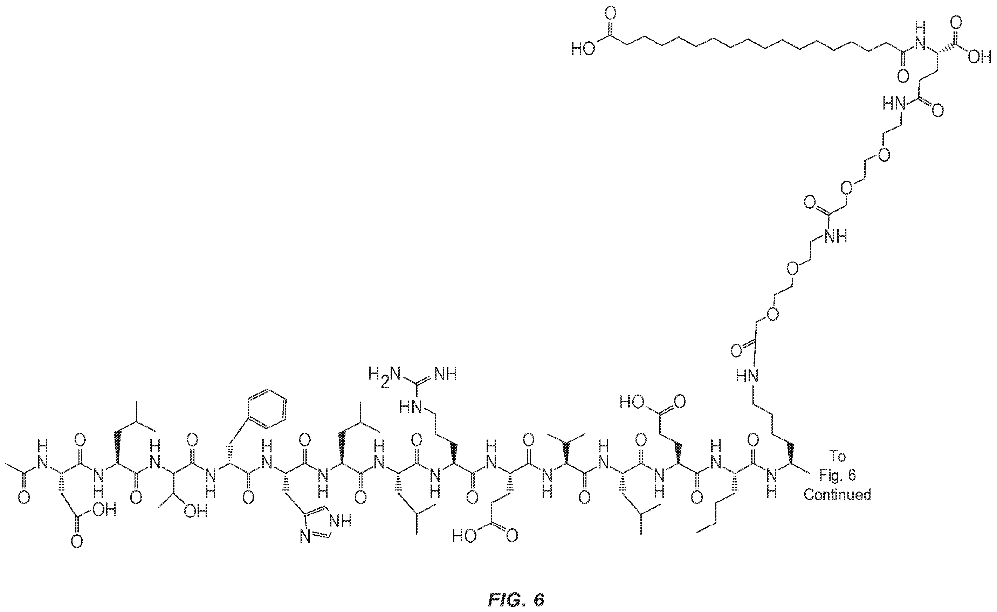

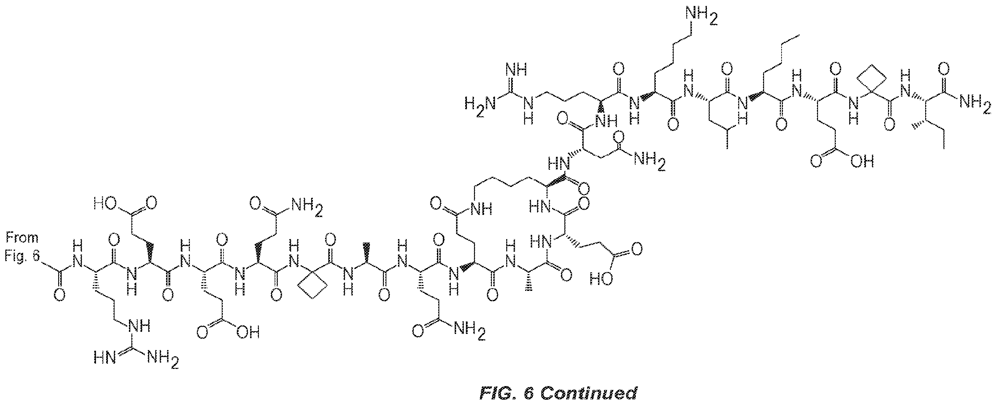

Peptide of SEQ ID NO: 9, wherein K* is Lys-(2-[2-(2-amino-ethoxy)-ethoxy]-acetyl)₂-γGlu-C18-diacid (Table B1.1, Formula I, FIG. 6), was synthesized using standard Fmoc chemistry based on the following procedure:
Resin preparation: A solution of Rink Amine MBHA resin (0.7 mmol, 1.00 eq, Sub 0.33 mmol/g) in DMF (30.0 mL) was agitated with N₂ at 25°C for 1.0 hour.

Deprotection: 20% piperidine in DMF (10.0 mL) was added and the resin was agitated with N₂ at 25°C for 30 minutes. The resin was washed with DMF (10.0 mL, 5 times) and filtered.

Coupling: A solution of HBTU (2.85 eq) and Fmoc-Ile-OH (3.00 eq) in DMF (3.0 mL) was added to the resin. DIEA (6.00 eq) was added, and the mixture was agitated with N₂ at 25°C for 30-60 minutes. The coupling reaction was monitored by Ninhydrin and Chloranil tests. The resin was washed with DMF (10.0 mL, 5 times).

The steps for Deprotection and Coupling were repeated for the coupling of following amino acids (1-34) outlined in Table 7A.

**Table 7A. Synthesis of Peptide of SEQ ID NO: 9.**

| # | Materials | Coupling reagents |
|---|---|---|
| 1 | Fmoc-cVal-OH (2.00 eq) | HATU (1.90 eq) and DIEA (4.00 eq) |
| 2 | Fmoc-Glu(OtBu)-OH (3.00 eq) | HBTU (2.85 eq) and DIEA (6.00 eq) |
| 3 | Fmoc-Nle-OH (2.00 eq) | HATU (1.90 eq) and DIEA (4.00 eq) |
| 4 | Fmoc-Leu-OH (3.00 eq) | HBTU (2.85 eq) and DIEA (6.00 eq) |
| 5 | Fmoc-Lys(Boc)-OH (3.00 eq) | HBTU (2.85 eq) and DIEA (6.00 eq) |
| 6 | Fmoc-Arg(Pbf)-OH (3.00 eq) | HBTU (2.85 eq) and DIEA (6.00 eq) |
| 7 | Fmoc-Asn(Trt)-OH (3.00 eq) | HBTU (2.85 eq) and DIEA (6.00 eq) |
| 8 | Fmoc-cyclo[Glu-Ala-Glu(OtBu)-Lys]-OH (2.00 eq) | HATU (1.90 eq) and DIEA (4.00 eq) |
| | | Coupling time: 60 minutes |
| 9 | Fmoc-Gln(Trt)-OH (3.00 eq) | HATU (2.85 eq) and DIEA (6.00 eq) |
| 10 | Fmoc-Ala-OH (3.00 eq) | HATU (2.85 eq) and DIEA (6.00 eq) |
| 11 | Fmoc-cVal-OH (2.00 eq) | HATU (1.90 eq) and DIEA (4.00 eq) |
| 12 | Fmoc-Gln(Trt)-OH (3.00 eq) | HATU (2.85 eq) and DIEA (6.00 eq) |
| | | Coupling time: 60 minutes |
| 13 | Fmoc-Glu(OtBu)-OH (3.00 eq) | HATU (2.85 eq) and DIEA (6.00 eq) |
| 14 | Fmoc- Glu(OtBu)-OH (3.00 eq) | HATU (2.85 eq) and DIEA (6.00 eq) |
| 15 | Fmoc-Arg(Pbf)-OH (3.00 eq) | HATU (2.85 eq) and DIEA (6.00 eq) |
| 16 | Fmoc-Lys(Alloc)-OH (2.00 eq) | HATU (1.90 eq) and DIEA (4.00 eq) |
| 17 | Fmoc-Nle-OH (3.00 eq) | HATU (2.85 eq) and DIEA (6.00 eq) |
| 18 | Fmoc-Glu(OtBu)-OH (3.00 eq) | HATU (2.85 eq) and DIEA (6.00 eq) |
| 19 | Fmoc-Leu-OH (3.00 eq) | HATU (2.85 eq) and DIEA (6.00 eq) |
| 20 | Fmoc-Val-OH (3.00 eq) | HATU (2.85 eq) and DIEA (6.00 eq) |
| 21 | Fmoc-Glu(OtBu)-OH (3.00 eq) | HATU (2.85 eq) and DIEA (6.00 eq) |
| 22 | Fmoc-Arg(Pbf)-OH(3.00 eq) | HATU (2.85 eq) and DIEA (6.00 eq) |
| 23 | Fmoc-Leu-OH(3.00 eq) | HATU (2.85 eq) and DIEA (6.00 eq) |
| 24 | Fmoc-Leu-OH (3.00 eq) | HATU (2.85 eq) and DIEA (6.00 eq) |
| 25 | Fmoc-His(Trt)-OH (3.00 eq) | HATU (2.85 eq) and DIEA (6.00 eq) |
| 26 | Fmoc-D-Phe-OH (3.00 eq) | HATU (2.85 eq) and DIEA (6.00 eq) |
| 27 | Fmoc-Thr(tBu)-OH (3.00 eq) | HATU (2.85 eq) and DIEA (6.00 eq) |
| 28 | Fmoc-Leu-OH (3.00 eq) | HATU (2.85 eq) and DIEA (6.00 eq) |
| 29 | Fmoc-Asp(OtBu)-OH (3.00 eq) | HATU (2.85 eq) and DIEA (6.00 eq) |
| 30 | Ac | 10% Ac₂O/5% NMM/85% DMF |
| - | De-Alloc | PhSiH₃ (10.0 eq) and Pd(PPh₃)₄(0.1 eq) in DCM(15 min*3 times) |
| 31 | Fmoc-(AEEA)-OH (2.00 eq) | HATU (1.90 eq) and DIEA (4.00 eq) |
| 32 | Fmoc-(AEEA)-OH (2.00 eq) | HATU (1.90 eq) and DIEA (4.00 eq) |
| 33 | Fmoc-Glu-OtBu (2.00 eq) | HATU (1.90 eq) and DIEA (4.00 eq) |
| 34 | 18-OtBu-18-Oxooctadecanoic acid (2.00 eq) | HATU (1.90 eq) and DIEA (4.00 eq) |

Peptide Cleavage: Cleavage solution (40.0 mL, 92.0% TFA/ 2.50% TIS /2.50% H2O /3.0% DTT) was added to the flask containing resin at room temperature and stirred for 1.5 hour. The peptide was precipitated with cold isopropyl ether (200 mL) and filtered. The filter cake was washed with isopropyl ether (50 mL, two times). The crude peptide was dried under vacuum for 1 hour (1.0 g). LCMS: MS cal.: 4725.44, MS observed: [M+4H]⁴⁺ = 1182.36; Rt = 0.66 minutes.

The crude peptide was then purified by prep-HPLC (TFA condition: A: 0.075 % TFA in H₂O, B: ACN) and transferred to AcOH salt by Prep-HPLC (AcOH condition: A: 0.5% AcOH in H₂O, B: ACN) to give the final product (24.4 mg, 5.16 µmol, 2.55% yield, 98.90% purity, AcOH) as a white solid. LCMS: MS cal.: 4725.44, MS observed: [M+4H]⁴⁺ =1182.36; Rt=1.80 minutes; and HPLC Rt= 5.88 minutes. Purification conditions were as outlined below in Table 7B.

**Table 7B. Peptide of SEQ ID NO: 9. Purification Conditions.**

| Separation condition | |
|---|---|
| Dissolution condition | Dissolve in 20% ACN-H₂O |
| Instrument | Gilson GX-281 |
| Mobile Phase | A: H₂O (0.075%TFA in H₂O) |
| | B: CH₃CN |
| Gradient | 30-55-40 minutes, Retention time: 32 minutes |
| Column | YMC-Actus Triart C18, 250*30 mm, 5 um, 120Å |
| Flow Rate | 20 mL/minute |
| Wavelength | 220/254 nm |
| Oven Temperature | 25°C |

| Re-separation condition | |
|---|---|
| Dissolution condition | Dissolve in ACN-H₂O, washed with 20% NH4Ac (A: 0.1 M NH4Ac in H2O, B: ACN) for 30 minutes |
| Instrument | Gilson GX-281 |
| Mobile Phase | A: H₂O (0.5% AcOH in H₂O) |
| | B: CH₃CN |
| Gradient | 27-47-40 minutes, Retention time: 22.5 minutes |
| Column | YMC-Actus Triart C18, 250*30 mm, 5 um, 120Å |
| Flow Rate | 20 mL/minute |
| Wavelength | 220/254 nm |
| Oven Temperature | 25°C |

### Example A8

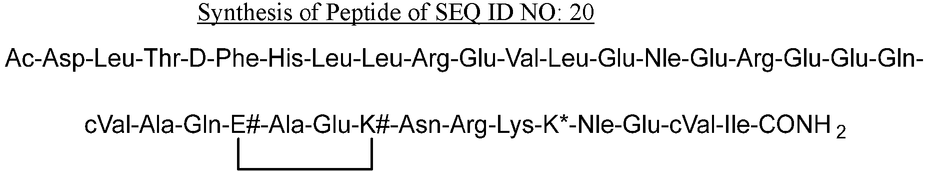
Synthesis of Peptide of SEQ ID NO: 20

Ac-Asp-Leu-Thr-D-Phe-His-Leu-Leu-Arg-Glu-Val-Leu-Glu-Nle-Glu-Arg-Glu-Glu-Gln-

cVal-Ala-Gln-E#-Ala-Glu-K#-Asn-Arg-Lys-K*-Nle-Glu-cVal-Ile-CONH 2


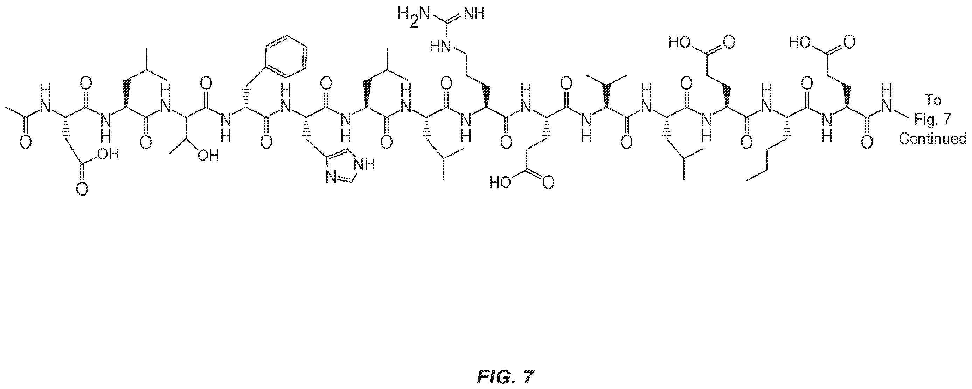

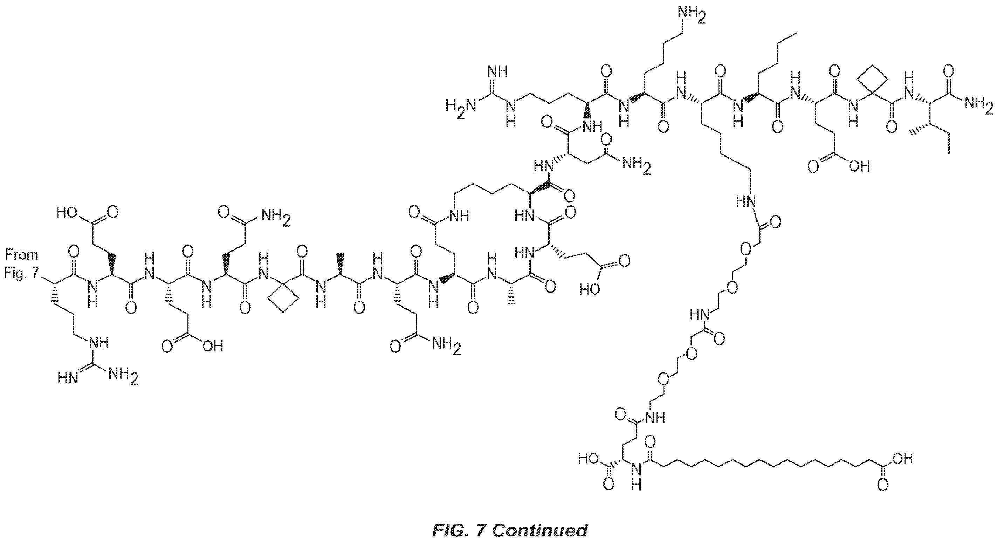

Peptide of SEQ ID NO: 20, wherein K* is Lys-(2-[2-(2-amino-ethoxy)-ethoxy]-acetyl)₂-γGlu-C18-diacid (Table B1.1, Formula I, FIG. 7), was synthesized using standard Fmoc chemistry based on the following procedure:
Resin preparation: A solution of Rink Amine MBHA resin (0.2 mmol, 1.00 eq, Sub 0.33 mmol/g) in DMF (10.0 mL) was agitated with N₂ at 25°C for 1.0 hour.

Deprotection: 20% piperidine in DMF (10.0 mL) was added and the resin was agitated with N₂ at 25°C for 30 minutes. The resin was washed with DMF (10.0 mL, 5 times) and filtered.

Coupling: A solution of HBTU (2.85 eq) and Fmoc-Ile-OH (3.00 eq) in DMF (15.0 mL) was added to the resin. DIEA (6.00 eq) was added, and the mixture was agitated with N₂ at 25°C for 30-60 minutes. The coupling reaction was monitored by Ninhydrin and Chloranil tests. The resin was washed with DMF (10.0 mL, 5 times).

The steps for Deprotection and Coupling were repeated for the coupling of following amino acids (1-34) outlined in Table 8A.

**Table 8A. Synthesis of Peptide of SEQ ID NO: 20.**

| # | Materials | Coupling reagents |
|---|---|---|
| 1 | Fmoc-cVal-OH (2.00 eq) | HATU (1.90 eq) and DIEA (4.00 eq) |
| 2 | Fmoc-Glu(OtBu)-OH (3.00 eq) | HBTU (2.85 eq) and DIEA (6.00 eq) |
| 3 | Fmoc-Nle-OH (2.00 eq) | HATU (1.90 eq) and DIEA (4.00 eq) |
| 4 | Fmoc-Lys(Alloc)-OH (2.00 eq) | HATU (1.90 eq) and DIEA (4.00 eq) |
| 5 | Fmoc-Lys(Boc)-OH (3.00 eq) | HBTU (2.85 eq) and DIEA (6.00 eq) |
| 6 | Fmoc-Arg(Pbf)-OH (3.00 eq) | HBTU (2.85 eq) and DIEA (6.00 eq) |
| 7 | Fmoc-Asn(Trt)-OH (3.00 eq) | HBTU (2.85 eq) and DIEA (6.00 eq) |
| 8 | Fmoc-cyclo[Glu-Ala-Glu(OtBu)-Lys]-OH (2.00 eq) | HATU (1.90 eq) and DIEA (4.00 eq) |
| | | Coupling time: 60 minutes |
| 9 | Fmoc-Gln(Trt)-OH (3.00 eq) | HATU (2.85 eq) and DIEA (6.00 eq) |
| 10 | Fmoc-Ala-OH (3.00 eq) | HATU (2.85 eq) and DIEA (6.00 eq) |
| 11 | Fmoc-cVal-OH (2.00 eq) | HATU (1.90 eq) and DIEA (4.00 eq) |
| 12 | Fmoc-Gln(Trt)-OH (3.00 eq) | HATU (2.85 eq) and DIEA (6.00 eq) |
| | | Coupling time: 60 minutes |
| 13 | Fmoc-Glu(OtBu)-OH (3.00 eq) | HATU (2.85 eq) and DIEA (6.00 eq) |
| 14 | Fmoc- Glu(OtBu)-OH (3.00 eq) | HATU (2.85 eq) and DIEA (6.00 eq) |
| 15 | Fmoc-Arg(Pbf)-OH (3.00 eq) | HATU (2.85 eq) and DIEA (6.00 eq) |
| 16 | Fmoc- Glu(OtBu)-OH (3.00 eq) | HATU (2.85 eq) and DIEA (6.00 eq) |
| 17 | Fmoc-Nle-OH (3.00 eq) | HATU (2.85 eq) and DIEA (6.00 eq) |
| 18 | Fmoc-Glu(OtBu)-OH (3.00 eq) | HATU (2.85 eq) and DIEA (6.00 eq) |
| 19 | Fmoc-Leu-OH (3.00 eq) | HATU (2.85 eq) and DIEA (6.00 eq) |
| 20 | Fmoc-Val-OH (3.00 eq) | HATU (2.85 eq) and DIEA (6.00 eq) |
| 21 | Fmoc-Glu(OtBu)-OH (3.00 eq) | HATU (2.85 eq) and DIEA (6.00 eq) |
| 22 | Fmoc-Arg(Pbf)-OH(3.00 eq) | HATU (2.85 eq) and DIEA (6.00 eq) |
| 23 | Fmoc-Leu-OH(3.00 eq) | HATU (2.85 eq) and DIEA (6.00 eq) |
| 24 | Fmoc-Leu-OH (3.00 eq) | HATU (2.85 eq) and DIEA (6.00 eq) |
| 25 | Fmoc-His(Trt)-OH (3.00 eq) | HATU (2.85 eq) and DIEA (6.00 eq) |
| 26 | Fmoc-D-Phe-OH (3.00 eq) | HATU (2.85 eq) and DIEA (6.00 eq) |
| 27 | Fmoc-Thr(tBu)-OH (3.00 eq) | HATU (2.85 eq) and DIEA (6.00 eq) |
| 28 | Fmoc-Leu-OH (3.00 eq) | HATU (2.85 eq) and DIEA (6.00 eq) |
| 29 | Fmoc-Asp(OtBu)-OH (3.00 eq) | HATU (2.85 eq) and DIEA (6.00 eq) |
| 30 | Ac | 10% Ac₂O/5% NMM/85% DMF |
| - | De-Alloc | PhSiH₃ (10.0 eq) and Pd(PPh₃)₄(0.1 eq) in DCM(15 min*3 times) |
| 31 | Fmoc-(AEEA)-OH (2.00 eq) | HATU (1.90 eq) and DIEA (4.00 eq) |
| 32 | Fmoc-(AEEA)-OH (2.00 eq) | HATU (1.90 eq) and DIEA (4.00 eq) |
| 33 | Fmoc-Glu-OtBu (2.00 eq) | HATU (1.90 eq) and DIEA (4.00 eq) |
| 34 | 18-OtBu-18-Oxooctadecanoic acid (2.00 eq) | HATU (1.90 eq) and DIEA (4.00 eq) |

Peptide Cleavage: Cleavage solution (40.0 mL, 92.0% TFA/ 2.50% TIS /2.50% H₂O /3.0% DTT) was added to the flask containing resin at room temperature and stirred for 1.5 hour. The peptide was then precipitated with cold isopropyl ether (200 mL),and filtered. The filter cake was washed with isopropyl ether (50 mL, twice). The crude peptide was dried under vacuum for 1 hour (1.0 g). LCMS: MS cal.: 4741.4, MS observed: [M+4H]⁴⁺ = 1186.35; Rt = 0.65 minutes.

The crude peptide was purified by prep-HPLC (TFA condition: A: 0.075 % TFA in H₂O, B: ACN) and transferred to AcOH salt by Prep-HPLC (AcOH condition: A: 0.5% AcOH in H₂O, B: ACN) to give the final product (27 mg, 5.58 µmol, 2.79% yield, 98.10% purity, AcOH) as a white solid. LCMS 4741.4, MS observed: [M+4H]⁴⁺ =1186.35; Rt= 1.83 minutes; and HPLC Rt= 5.48 minutes. Purification conditions were as outlined below in Table 8B.

**Table 8B. Peptide of SEQ ID NO: 20 Purification Conditions.**

| Separation condition | |
|---|---|
| Dissolution condition | Dissolve in 20%ACN-H₂O |
| Instrument | Gilson GX-281 |
| Mobile Phase | A: H₂O (0.075%TFA in H₂O) |
| | B: CH₃CN |
| Gradient | 42-62-40 minutes, Retention time: 17 minutes |
| Column | YMC-Actus Triart C18, 250*30 mm, 5 um, 120Å |
| Flow Rate | 20 mL/minute |
| Wavelength | 220/254 nm |
| Oven Temperature | 25^{°}C |

| Re-separation condition | |
|---|---|
| Dissolution condition | Dissolve in ACN-H₂O, washed with 20% NH4Ac (A: 0.1 M NH4Ac in H2O, B: ACN) for 30 minutes |
| Instrument | Gilson GX-281 |
| Mobile Phase | A: H₂O (0.5% AcOH in H₂O) |
| | B: CH₃CN |
| Gradient | 27-47-40 minutes, Retention time: 22 minutes |
| Column | YMC-Actus Triart C18, 250*30 mm, 5 mm, 120Å |
| Flow Rate | 20 mL/minute |
| Wavelength | 220/254 nm |
| Oven Temperature | 25^{°}C |

### Example A9

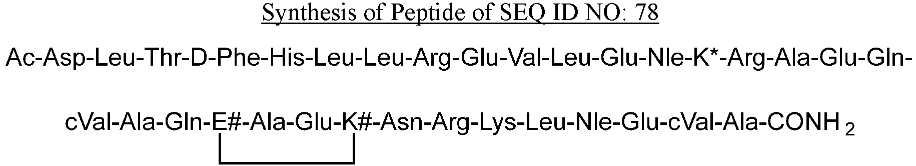
Synthesis of Peptide of SEQ ID NO: 78

Ac-Asp-Leu-Thr-D-Phe-His-Leu-Leu-Arg-Glu-Val-Leu-Glu-Nle-K*-Arg-Ala-Glu-Gln-

cVal-Ala-Gln-E#-Ala-Glu-K#-Asn-Arg-Lys-Leu-Nle-Glu-cVal-Ala-CONH 2


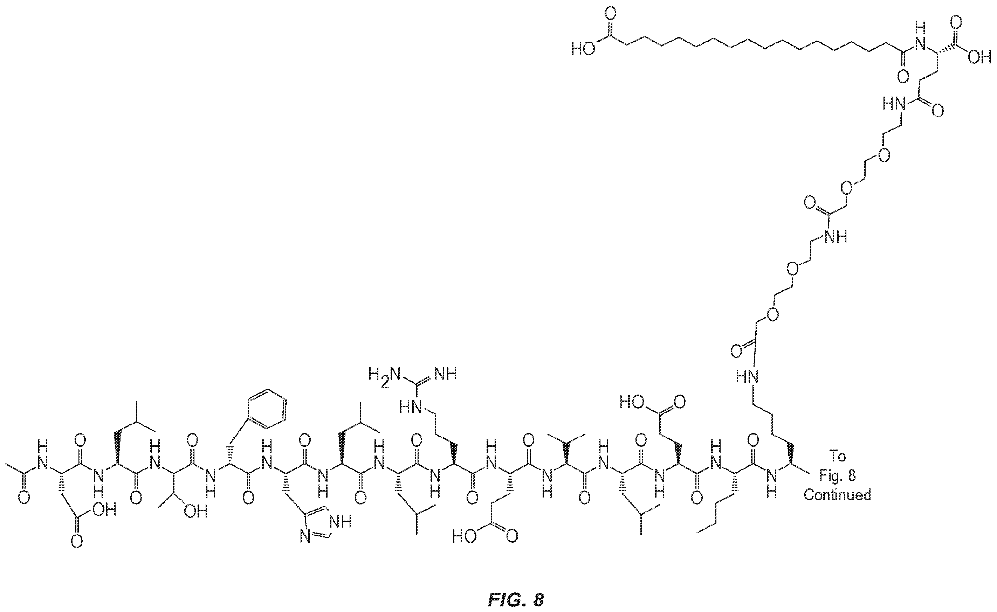

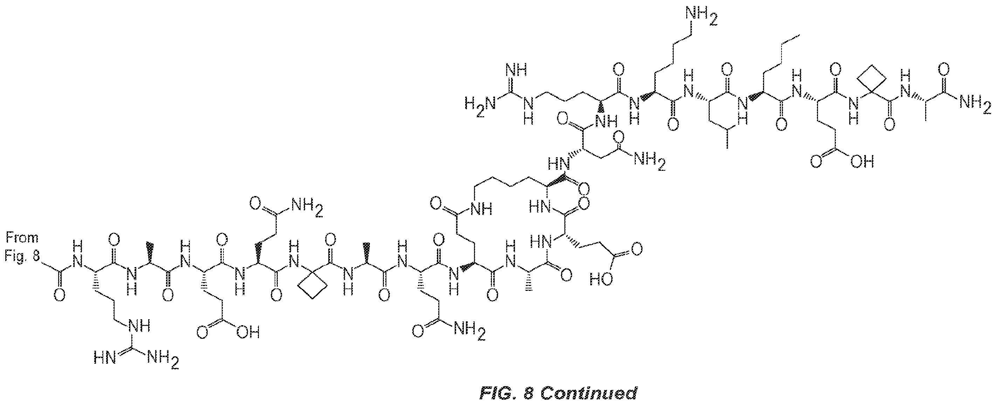

Peptide of SEQ ID NO: 78, wherein K* is Lys-(2-[2-(2-amino-ethoxy)-ethoxy]-acetyl)₂-γGlu-C18-diacid (Table B1.1, Formula I, FIG. 8), was synthesized using standard Fmoc chemistry based on the following procedure:
Resin preparation: A solution of Rink Amine MBHA resin (0.2 mmol, 1.00 eq, Sub 0.33 mmol/g) in DMF (10.0 mL) was agitated with N₂ at 25°C for 1.0 hour.

Deprotection: 20% piperidine in DMF (10.0 mL) was added and the resin was agitated with N₂ at 25°C for 30 minutes. The resin was washed with DMF (10.0 mL, 5 times) and filtered.

Coupling: A solution of HBTU (2.85 eq) and Fmoc-Ala-OH (3.00 eq) in DMF (3.0 mL) was added to the resin. DIEA (6.00 eq) was added, and the mixture was agitated with N₂ at 25°C for 30-60 minutes. The coupling reaction was monitored by Ninhydrin and Chloranil tests. The resin was washed with DMF (10.0 mL, 5 times).

The steps for Deprotection and Coupling were repeated for the coupling of following amino acids (1-34) outlined in Table 9A.

**Table 9A. Peptide of SEQ ID NO: 78 Synthesis.**

| # | Materials | Coupling reagents |
|---|---|---|
| 1 | Fmoc-cVal-OH (2.00 eq) | HATU (1.90 eq) and DIEA (4.00 eq) |
| 2 | Fmoc-Glu(OtBu)-OH (3.00 eq) | HBTU (2.85 eq) and DIEA (6.00 eq) |
| 3 | Fmoc-Nle-OH (2.00 eq) | HATU (1.90 eq) and DIEA (4.00 eq) |
| 4 | Fmoc-Leu-OH (2.00 eq) | HATU (1.90 eq) and DIEA (4.00 eq) |
| 5 | Fmoc-Lys(Boc)-OH (3.00 eq) | HBTU (2.85 eq) and DIEA (6.00 eq) |
| 6 | Fmoc-Arg(Pbf)-OH (3.00 eq) | HBTU (2.85 eq) and DIEA (6.00 eq) |
| 7 | Fmoc-Asn(Trt)-OH (3.00 eq) | HBTU (2.85 eq) and DIEA (6.00 eq) |
| 8 | Fmoc-cyclo[Glu-Ala-Glu(OtBu)-Lys]-OH (2.00 eq) | HATU (1.90 eq) and DIEA (4.00 eq) |
| | | Coupling time: 60 minutes |
| 9 | Fmoc-Gln(Trt)-OH (3.00 eq) | HATU (2.85 eq) and DIEA (6.00 eq) |
| 10 | Fmoc-Ala-OH (3.00 eq) | HATU (2.85 eq) and DIEA (6.00 eq) |
| 11 | Fmoc-cVal-OH (2.00 eq) | HATU (1.90 eq) and DIEA (4.00 eq) |
| 12 | Fmoc-Gln(Trt)-OH (3.00 eq) | HATU (2.85 eq) and DIEA (6.00 eq) |
| | | Coupling time: 60 minutes |
| 13 | Fmoc-Glu(OtBu)-OH (3.00 eq) | HATU (2.85 eq) and DIEA (6.00 eq) |
| 14 | Fmoc- Ala-OH (3.00 eq) | HATU (2.85 eq) and DIEA (6.00 eq) |
| 15 | Fmoc-Arg(Pbf)-OH (3.00 eq) | HATU (2.85 eq) and DIEA (6.00 eq) |
| 16 | Fmoc- Lys(Alloc)-OH (3.00 eq) | HATU (2.85 eq) and DIEA (6.00 eq) |
| 17 | Fmoc-Nle-OH (3.00 eq) | HATU (2.85 eq) and DIEA (6.00 eq) |
| 18 | Fmoc-Glu(OtBu)-OH (3.00 eq) | HATU (2.85 eq) and DIEA (6.00 eq) |
| 19 | Fmoc-Leu-OH (3.00 eq) | HATU (2.85 eq) and DIEA (6.00 eq) |
| 20 | Fmoc-Val-OH (3.00 eq) | HATU (2.85 eq) and DIEA (6.00 eq) |
| 21 | Fmoc-Glu(OtBu)-OH (3.00 eq) | HATU (2.85 eq) and DIEA (6.00 eq) |
| 22 | Fmoc-Arg(Pbf)-OH(3.00 eq) | HATU (2.85 eq) and DIEA (6.00 eq) |
| 23 | Fmoc-Leu-OH(3.00 eq) | HATU (2.85 eq) and DIEA (6.00 eq) |
| 24 | Fmoc-Leu-OH (3.00 eq) | HATU (2.85 eq) and DIEA (6.00 eq) |
| 25 | Fmoc-His(Trt)-OH (3.00 eq) | HATU (2.85 eq) and DIEA (6.00 eq) |
| 26 | Fmoc-D-Phe-OH (3.00 eq) | HATU (2.85 eq) and DIEA (6.00 eq) |
| 27 | Fmoc-Thr(tBu)-OH (3.00 eq) | HATU (2.85 eq) and DIEA (6.00 eq) |
| 28 | Fmoc-Leu-OH (3.00 eq) | HATU (2.85 eq) and DIEA (6.00 eq) |
| 29 | Fmoc-Asp(OtBu)-OH (3.00 eq) | HATU (2.85 eq) and DIEA (6.00 eq) |
| 30 | Ac | 10% Ac₂O/5% NMM/85% DMF |
| - | De-Alloc | PhSiH₃ (10.0 eq) and Pd(PPh₃)₄(0.1 eq) in DCM(15 min*3 times) |
| 31 | Fmoc-(AEEA)-OH (2.00 eq) | HATU (1.90 eq) and DIEA (4.00 eq) |
| 32 | Fmoc-(AEEA)-OH (2.00 eq) | HATU (1.90 eq) and DIEA (4.00 eq) |
| 33 | Fmoc-Glu-OtBu (2.00 eq) | HATU (1.90 eq) and DIEA (4.00 eq) |
| 34 | 18-OtBu-18-Oxooctadecanoic acid (2.00 eq) | HATU (1.90 eq) and DIEA (4.00 eq) |

Peptide Cleavage: Cleavage solution (40.0 mL, 92.0% TFA/ 2.50% TIS /2.50% H₂O /3.0% DTT) was added to the flask containing resin at room temperature and stirred for 1.5 hour. The peptide was then precipitated with cold isopropyl ether (200 mL) and filtered. The filter cake was washed with isopropyl ether (50 mL, two times). The crude peptide was dried under vacuum for 1 hour (1.1 g). LCMS: MS cal.: 4625.32, MS observed: [M+4H]⁴⁺ = 1157.33; Rt = 1.05 minutes).

The crude peptide was purified by prep-HPLC (TFA condition: A: 0.075 % TFA in H₂O, B: ACN) to give the final product (100 mg, 20.64 µmol, 10.80% yield, 95.50% purity, TFA) as a white solid. LCMS: MS cal.: 4625.32, MS observed: [M+4H]⁴⁺ =1157.33; Rₜ= 1.61 minutes; and HPLC Rt= 5.64 minutes. Purification conditions were as outlined below in Table 9B.

**Table 9B. Peptide of SEQ ID NO: 78 Purification Conditions.**

| Separation condition | |
|---|---|
| Dissolution condition | Dissolve in 20%ACN-H₂O |
| Instrument | Gilson GX-281 |
| Mobile Phase | A: H₂O (0.075%TFA in H₂O) |
| | B: CH₃CN |
| Gradient | 32-52-40 minutes, Retention time: 27 minutes |
| Column | YMC-Actus Triart C18, 250*30 mm, 5 um, 120Å |
| Flow Rate | 20 mL/minute |
| Wavelength | 220/254 nm |
| Oven Temperature | 25^{°}C |

### Example A10

Synthesis of Peptide of SEQ ID NO: 99

Ac-Asp-Leu-Thr-D-Phe-His-Leu-Leu-Arg-Glu-Val-Leu-Glu-Nle-K*-Gln-Glu-Glu-Gln-

cVal-Ala-Gln-E#-Ala-Glu-K#-Asn-Arg-Lys-Leu-Nle-Glu-cVal-Ile-CONH 2

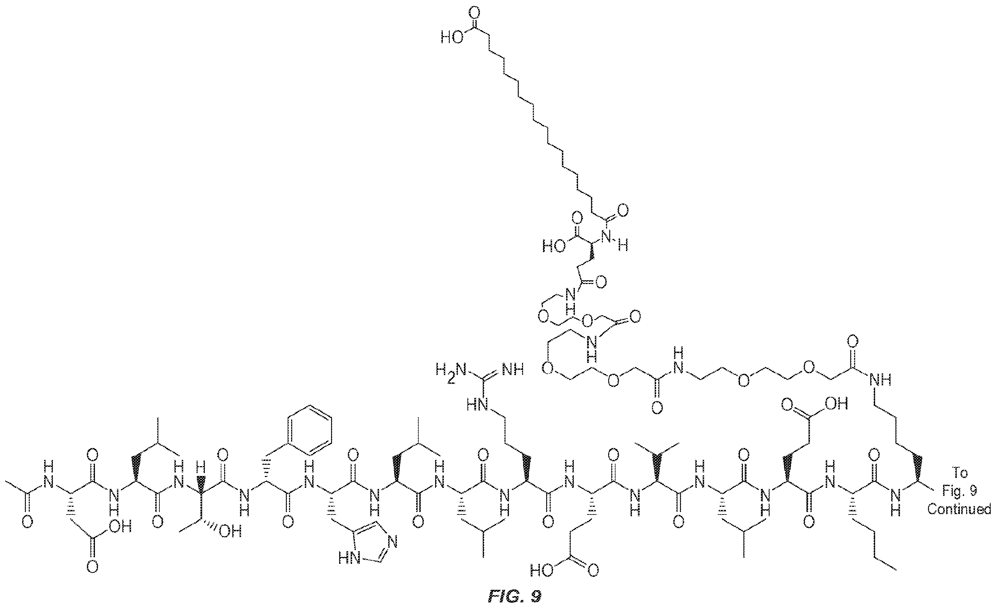

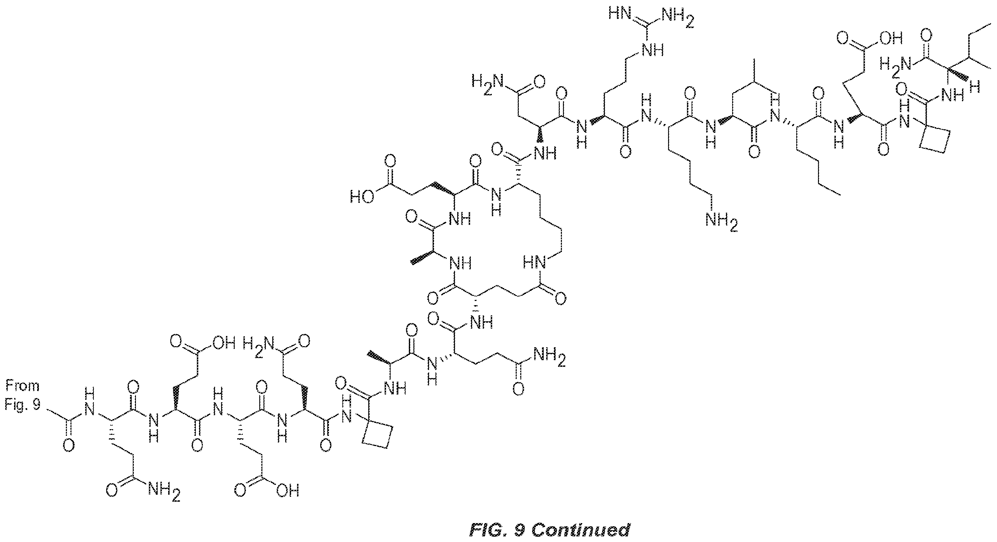

Peptide of SEQ ID NO: 99, wherein K* is Lys-(2-[2-(2-amino-ethoxy)-ethoxy]-acetyl)₃-γGlu-C18-diacid (Table B1.1, Formula I, FIG. 9), was synthesized using standard Fmoc chemistry based on the following procedure:
Resin preparation: A solution of Rink Amine MBHA resin (0.2 mmol, 1.00 eq, Sub 0.33 mmol/g) in DMF (15.0 mL) was agitated with N₂ at 25°C for 1.0 hour.

Deprotection: 20% piperidine in DMF (15.0 mL) was added and the resin was agitated with N₂ at 25°C for 30 minutes. The resin was washed with DMF (15.0 mL, 5 times) and filtered.

Coupling: A solution of HBTU (2.85 eq) and Fmoc-Ile-OH (3.00 eq) in DMF (3.0 mL) was added to the resin. DIEA (6.00 eq) was added, and the mixture was agitated with N₂ at 25°C for 30-60 minutes. The coupling reaction was monitored by Ninhydrin and Chloranil tests. The resin was washed with DMF (15.0 mL, 5 times).

The steps for Deprotection and Coupling were repeated for the coupling of following amino acids (1-35) outlined in Table 11A.

**Table 11A. Peptide of SEQ ID NO: 99 Synthesis.**

| # | Materials | Coupling reagents |
|---|---|---|
| 1 | Fmoc-cVal-OH (2.00 eq) | HATU (1.90 eq) and DIEA (4.00 eq) |
| 2 | Fmoc-Glu(OtBu)-OH (3.00 eq) | HBTU (2.85 eq) and DIEA (6.00 eq) |
| 3 | Fmoc-Nle-OH (2.00 eq) | HATU (1.90 eq) and DIEA (4.00 eq) |
| 4 | Fmoc-Leu-OH (3.00 eq) | HBTU (2.85 eq) and DIEA (6.00 eq) |
| 5 | Fmoc-Lys(Boc)-OH (3.00 eq) | HBTU (2.85 eq) and DIEA (6.00 eq) |
| 6 | Fmoc-Arg(Pbf)-OH (3.00 eq) | HBTU (2.85 eq) and DIEA (6.00 eq) |
| 7 | Fmoc-Asn(Trt)-OH (3.00 eq) | HBTU (2.85 eq) and DIEA (6.00 eq) |
| 8 | Fmoc-cyclo[Glu-Ala-Glu(OtBu)-Lys]-OH (2.00 eq) | HATU (1.90 eq) and DIEA (4.00 eq) |
| | | Coupling time: 60 minutes |
| 9 | Fmoc-Gln(Trt)-OH (3.00 eq) | HATU (2.85 eq) and DIEA (6.00 eq) |
| 10 | Fmoc-Ala-OH (3.00 eq) | HATU (2.85 eq) and DIEA (6.00 eq) |
| 11 | Fmoc-cVal-OH (2.00 eq) | HATU (1.90 eq) and DIEA (4.00 eq) |
| 12 | Fmoc-Gln(Trt)-OH (3.00 eq) | HATU (2.85 eq) and DIEA (6.00 eq) |
| | | Coupling time: 60 minutes |
| 13 | Fmoc-Glu(OtBu)-OH (3.00 eq) | HATU (2.85 eq) and DIEA (6.00 eq) |
| 14 | Fmoc- Glu(OtBu)-OH (3.00 eq) | HATU (2.85 eq) and DIEA (6.00 eq) |
| 15 | Fmoc-Gln(Trt)-OH (3.00 eq) | HATU (2.85 eq) and DIEA (6.00 eq) |
| 16 | Fmoc-Lys(Alloc)-OH (2.00 eq) | HATU (1.90 eq) and DIEA (4.00 eq) |
| 17 | Fmoc-Nle-OH (3.00 eq) | HATU (2.85 eq) and DIEA (6.00 eq) |
| 18 | Fmoc-Glu(OtBu)-OH (3.00 eq) | HATU (2.85 eq) and DIEA (6.00 eq) |
| 19 | Fmoc-Leu-OH (3.00 eq) | HATU (2.85 eq) and DIEA (6.00 eq) |
| 20 | Fmoc-Val-OH (3.00 eq) | HATU (2.85 eq) and DIEA (6.00 eq) |
| 21 | Fmoc-Glu(OtBu)-OH (3.00 eq) | HATU (2.85 eq) and DIEA (6.00 eq) |
| 22 | Fmoc-Arg(Pbf)-OH(3.00 eq) | HATU (2.85 eq) and DIEA (6.00 eq) |
| 23 | Fmoc-Leu-OH(3.00 eq) | HATU (2.85 eq) and DIEA (6.00 eq) |
| 24 | Fmoc-Leu-OH (3.00 eq) | HATU (2.85 eq) and DIEA (6.00 eq) |
| 25 | Fmoc-His(Trt)-OH (3.00 eq) | HATU (2.85 eq) and DIEA (6.00 eq) |
| 26 | Fmoc-D-Phe-OH (3.00 eq) | HATU (2.85 eq) and DIEA (6.00 eq) |
| 27 | Fmoc-Thr(tBu)-OH (3.00 eq) | HATU (2.85 eq) and DIEA (6.00 eq) |
| 28 | Fmoc-Leu-OH (3.00 eq) | HATU (2.85 eq) and DIEA (6.00 eq) |
| 29 | Fmoc-Asp(OtBu)-OH (3.00 eq) | HATU (2.85 eq) and DIEA (6.00 eq) |
| 30 | Ac | 10% Ac₂O/5% NMM/85% DMF |
| - | De-Alloc | PhSiH₃ (10.0 eq) and Pd(PPh₃)₄ |
| 31 | Fmoc-(AEEA)-OH (2.00 eq) | HATU (1.90 eq) and DIEA (4.00 eq) |
| 32 | Fmoc-(AEEA)-OH (2.00 eq) | HATU (1.90 eq) and DIEA (4.00 eq) |
| 33 | Fmoc-(AEEA)-OH (2.00 eq) | HATU (1.90 eq) and DIEA (4.00 eq) |
| 34 | Fmoc-Glu-OtBu (2.00 eq) | HATU (1.90 eq) and DIEA (4.00 eq) |
| 35 | 18-OtBu-18-Oxooctadecanoic acid (2.00 eq) | HATU (1.90 eq) and DIEA (4.00 eq) |

Peptide Cleavage: Cleavage solution (40.0 mL, 92.0% TFA/ 2.50% TIS /2.50% H₂O /3.0% DTT) was added to the flask containing resin at room temperature and stirred for 1.5 hour. The peptide was precipitated with cold isopropyl ether (200 mL) and filtered. The filter cake was washed with isopropyl ether (50 mL, two times). The crude peptide was dried under vacuum for 1 hour (1.1 g). LCMS: MS cal.: 4842.54, MS observed: [M+4H]⁴⁺ = 1211.64; Rt = 1.08 minutes.

The crude peptide was then purified by prep-HPLC (TFA condition: A: 0.075 % TFA in H₂O, B: ACN) to give the final product (92.0 mg, 18.50 µmol, 9.25% yield, 97.40% purity, TFA) as a white solid. LCMS: MS cal.: 4842.54, MS observed: [M+4H]⁴⁺ =1211.64; Rₜ= 1.55 minutes; and HPLC Rt= 7.97 minutes. Purification conditions were as outlined below in Table 11B.

**Table 11B. Peptide of SEQ ID NO: 99 Purification Conditions.**

| Separation condition | |
|---|---|
| Dissolution condition | Dissolve in 20%ACN-H₂O |
| Instrument | Gilson GX-281 |
| Mobile Phase | A: H₂O (0.075%TFA in H₂O) |
| | B: CH₃CN |
| Gradient | 32-62-40 minutes, Retention time: 27 minutes |
| Column | YMC-Actus Triart C18, 250*30 mm, 5 um, 120Å |
| Flow Rate | 20 mL/minute |
| Wavelength | 220/254 nm |
| Oven Temperature | 25^{°}C |

### Example A11

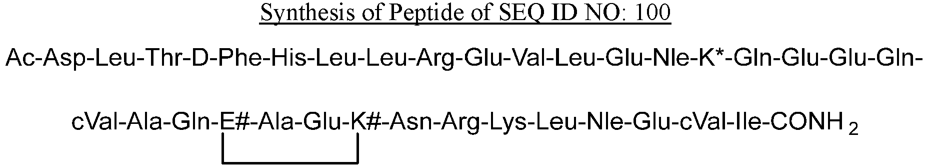
Synthesis of Peptide of SEQ ID NO: 100

Ac-Asp-Leu-Thr-D-Phe-His-Leu-Leu-Arg-Glu-Val-Leu-Glu-Nle-K*-Gln-Glu-Glu-Gln-

cVal-Ala-Gln-E#-Ala-Glu-K#-Asn-Arg-Lys-Leu-Nle-Glu-cVal-Ile-CONH 2


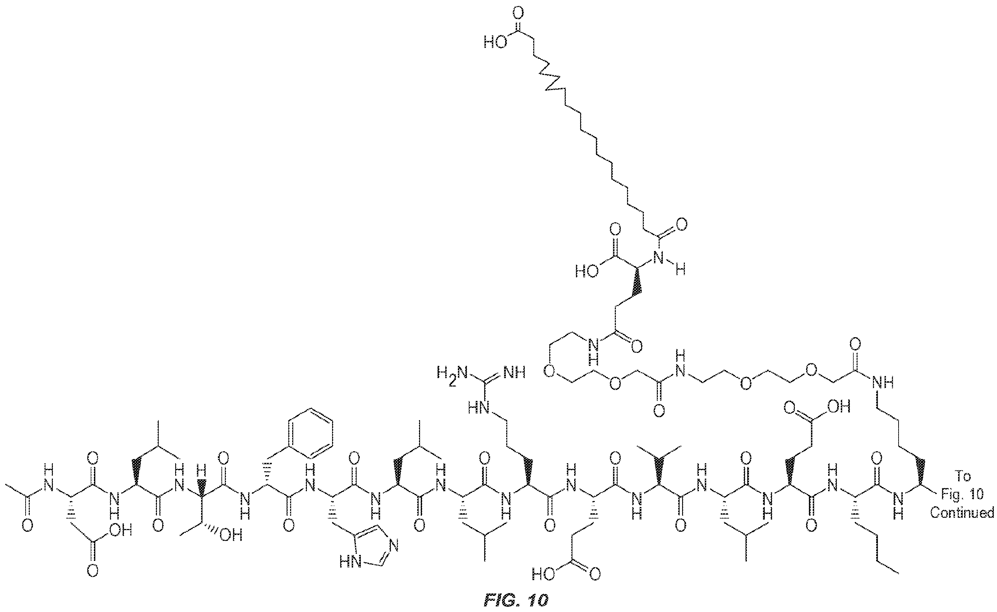

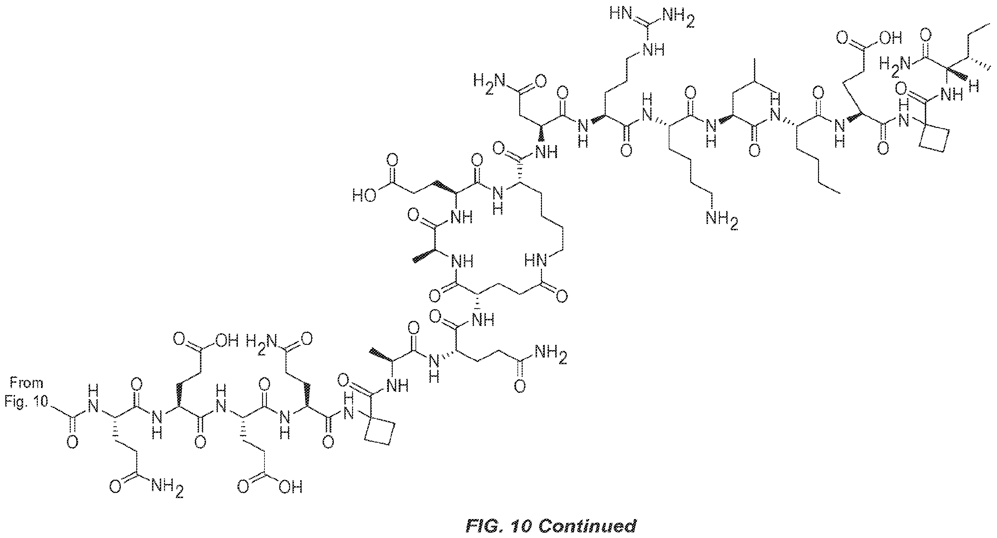

Peptide of SEQ ID NO: 100, wherein K* is Lys-(2-[2-(2-amino-ethoxy)-ethoxy]-acetyl)₂-γGlu-C20-diacid (Table B1.1, Formula I, FIG. 10), was synthesized using standard Fmoc chemistry based on the following procedure:
Resin preparation: A solution of Rink Amine MBHA resin (0.2 mmol, 1.00 eq, Sub 0.33 mmol/g) in DMF (15.0 mL) was agitated with N₂ at 25°C for 1.0 hour.

Deprotection: 20% piperidine in DMF (15.0 mL) was added and the resin was agitated with N₂ at 25°C for 30 minutes. The resin was washed with DMF (15.0 mL, 5 times) and filtered.

Coupling: A solution of HBTU (2.85 eq) and Fmoc-Ile-OH (3.00 eq) in DMF (3.0 mL) was added to the resin. DIEA (6.00 eq) was added, and the mixture was agitated with N₂ at 25°C for 30-60 minutes. The coupling reaction was monitored by Ninhydrin and Chloranil tests. The resin was washed with DMF (15.0 mL, 5 times).

The steps for Deprotection and Coupling were repeated for the coupling of following amino acids (1-34) outlined in Table 12A.

**Table 12A. Peptide of SEQ ID NO: 100 Synthesis.**

| # | Materials | Coupling reagents |
|---|---|---|
| 1 | Fmoc-cVal-OH (2.00 eq) | HATU (1.90 eq) and DIEA (4.00 eq) |
| 2 | Fmoc-Glu(OtBu)-OH (3.00 eq) | HBTU (2.85 eq) and DIEA (6.00 eq) |
| 3 | Fmoc-Nle-OH (2.00 eq) | HATU (1.90 eq) and DIEA (4.00 eq) |
| 4 | Fmoc-Leu-OH (3.00 eq) | HBTU (2.85 eq) and DIEA (6.00 eq) |
| 5 | Fmoc-Lys(Boc)-OH (3.00 eq) | HBTU (2.85 eq) and DIEA (6.00 eq) |
| 6 | Fmoc-Arg(Pbf)-OH (3.00 eq) | HBTU (2.85 eq) and DIEA (6.00 eq) |
| 7 | Fmoc-Asn(Trt)-OH (3.00 eq) | HBTU (2.85 eq) and DIEA (6.00 eq) |
| 8 | Fmoc-cyclo[Glu-Ala-Glu(OtBu)-Lys]-OH (2.00 eq) | HATU (1.90 eq) and DIEA (4.00 eq) Coupling time: 60 minutes |
| 9 | Fmoc-Gln(Trt)-OH (3.00 eq) | HATU (2.85 eq) and DIEA (6.00 eq) |
| 10 | Fmoc-Ala-OH (3.00 eq) | HATU (2.85 eq) and DIEA (6.00 eq) |
| 11 | Fmoc-cVal-OH (2.00 eq) | HATU (1.90 eq) and DIEA (4.00 eq) |
| 12 | Fmoc-Gln(Trt)-OH (3.00 eq) | HATU (2.85 eq) and DIEA (6.00 eq) Coupling time: 60 minutes |
| 13 | Fmoc-Glu(OtBu)-OH (3.00 eq) | HATU (2.85 eq) and DIEA (6.00 eq) |
| 14 | Fmoc- Glu(OtBu)-OH (3.00 eq) | HATU (2.85 eq) and DIEA (6.00 eq) |
| 15 | Fmoc-Gln(Trt)-OH (3.00 eq) | HATU (2.85 eq) and DIEA (6.00 eq) |
| 16 | Fmoc-Lys(Alloc)-OH (2.00 eq) | HATU (1.90 eq) and DIEA (4.00 eq) |
| 17 | Fmoc-Nle-OH (3.00 eq) | HATU (2.85 eq) and DIEA (6.00 eq) |
| 18 | Fmoc-Glu(OtBu)-OH (3.00 eq) | HATU (2.85 eq) and DIEA (6.00 eq) |
| 19 | Fmoc-Leu-OH (3.00 eq) | HATU (2.85 eq) and DIEA (6.00 eq) |
| 20 | Fmoc-Val-OH (3.00 eq) | HATU (2.85 eq) and DIEA (6.00 eq) |
| 21 | Fmoc-Glu(OtBu)-OH (3.00 eq) | HATU (2.85 eq) and DIEA (6.00 eq) |
| 22 | Fmoc-Arg(Pbf)-OH(3.00 eq) | HATU (2.85 eq) and DIEA (6.00 eq) |
| 23 | Fmoc-Leu-OH(3.00 eq) | HATU (2.85 eq) and DIEA (6.00 eq) |
| 24 | Fmoc-Leu-OH (3.00 eq) | HATU (2.85 eq) and DIEA (6.00 eq) |
| 25 | Fmoc-His(Trt)-OH (3.00 eq) | HATU (2.85 eq) and DIEA (6.00 eq) |
| 26 | Fmoc-D-Phe-OH (3.00 eq) | HATU (2.85 eq) and DIEA (6.00 eq) |
| 27 | Fmoc-Thr(tBu)-OH (3.00 eq) | HATU (2.85 eq) and DIEA (6.00 eq) |
| 28 | Fmoc-Leu-OH (3.00 eq) | HATU (2.85 eq) and DIEA (6.00 eq) |
| 29 | Fmoc-Asp(OtBu)-OH (3.00 eq) | HATU (2.85 eq) and DIEA (6.00 eq) |
| 30 | Ac | 10% Ac₂O/5% NMM/85% DMF |
| - | De-Alloc | PhSiH₃ (10.0 eq) and Pd(PPh₃)₄(0.1 eq) in DCM(15 minutes*3 times) |
| 31 | Fmoc-(AEEA)-OH (2.00 eq) | HATU (1.90 eq) and DIEA (4.00 eq) |
| 32 | Fmoc-(AEEA)-OH (2.00 eq) | HATU (1.90 eq) and DIEA (4.00 eq) |
| 33 | Fmoc-Glu-OtBu (2.00 eq) | HATU (1.90 eq) and DIEA (4.00 eq) |
| 34 | 20-(Tert-Butoxy)-20-OxoicosanoicAcid (2.00 eq) | HATU (1.90 eq) and DIEA (4.00 eq) |

Peptide Cleavage: Cleavage solution (40.0 mL, 92.0% TFA/ 2.50% TIS /2.50% H₂O /3.0% DTT) was added to the flask containing resin at room temperature and stirred for 1.5 hour. The peptide was then precipitated with cold isopropyl ether (200 mL) and filtered. The filter cake was washed with isopropyl ether (50 mL, two times). The crude peptide was dried under vacuum for 1 hour (1.0 g). LCMS: MS cal.: 4725.44, MS observed: [M+4H]⁴⁺ = 1182.36; Rt = 1.16 minutes.

The crude peptide was then purified by prep-HPLC (TFA condition: A: 0.075 % TFA in H₂O, B: ACN) to give the final product (51.0 mg, 10.49 µmol, 5.30% yield, 97.20% purity, TFA) as a white solid. LCMS: MS cal.: 4725.44, MS observed: [M+4H]⁴⁺ =1182.36; Rₜ= 1.6 minutes; and HPLC Rₜ= 5.84 minutes). Purification conditions were as outlined below in Table 12B.

**Table 12B. Peptide of SEQ ID NO: 100 Purification Conditions.**

| Separation condition | |
|---|---|
| Dissolution condition | Dissolve in 20%ACN-H₂O |
| Instrument | Gilson GX-281 |
| Mobile Phase | A: H₂O (0.075%TFA in H₂O) |
| | B: CH₃CN |
| Gradient | 37-57-40 minutes, Retention time: 23 minutes |
| Column | YMC-Actus Triart C18, 250*30 mm, 5 µm, 120Å |
| Flow Rate | 20 mL/minute |
| Wavelength | 220/254 nm |
| Oven Temp. | 25^{°}C |

### Example A12

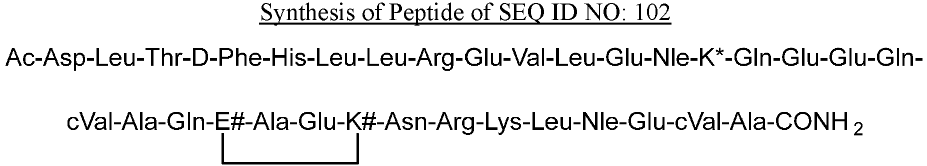
Synthesis of Peptide of SEQ ID NO: 102

Ac-Asp-Leu-Thr-D-Phe-His-Leu-Leu-Arg-Glu-Val-Leu-Glu-Nle-K*-Gln-Glu-Glu-Gln-

cVal-Ala-Gln-E#-Ala-Glu-K#-Asn-Arg-Lys-Leu-Nle-Glu-cVal-Ala-CONH 2


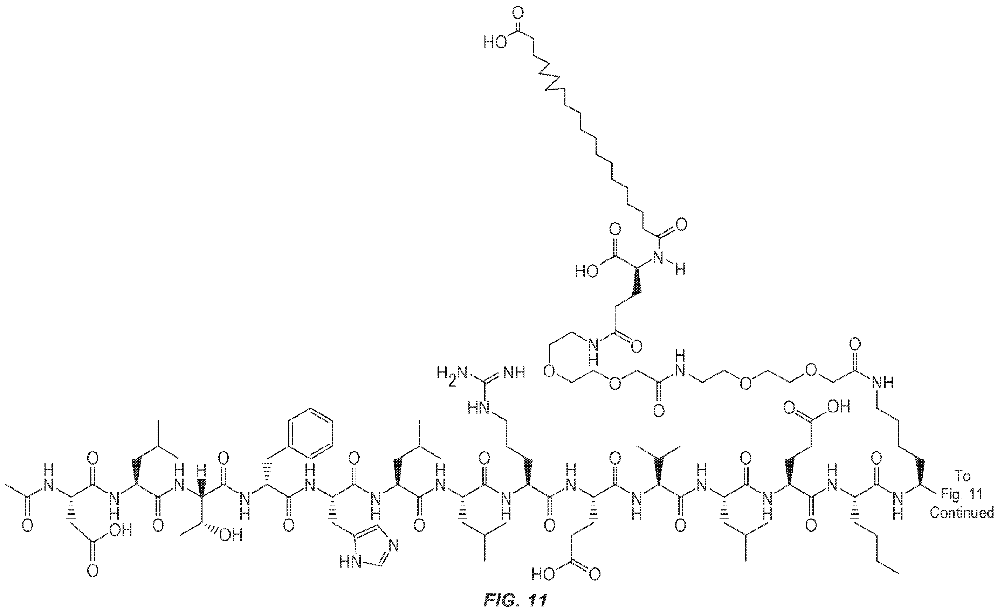

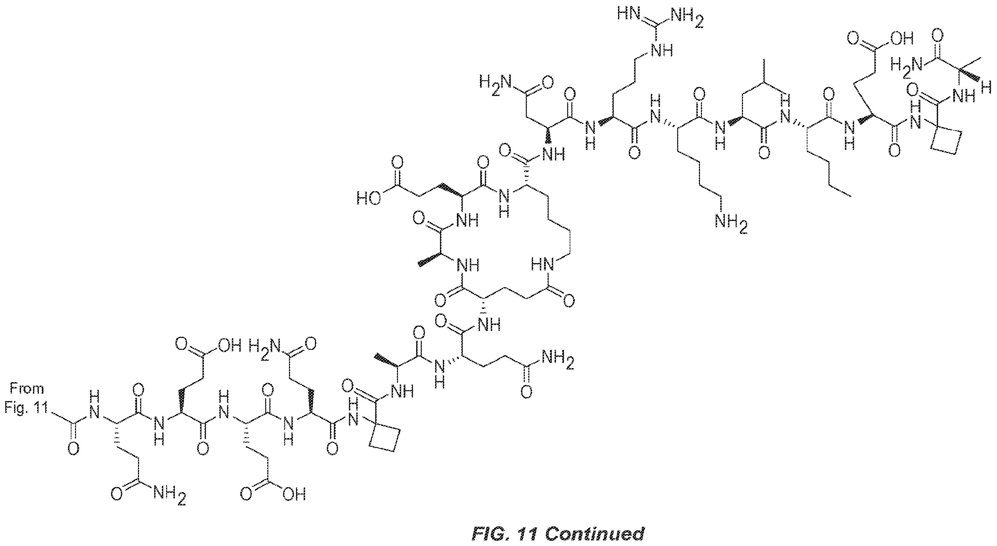

Peptide of SEQ ID NO: 102, wherein K* is Lys-(2-[2-(2-amino-ethoxy)-ethoxy]-acetyl)₂-γGlu-C20-diacid (Table B1.1, Formula I, FIG. 11), was synthesized using standard Fmoc chemistry based on the following procedure:
Resin preparation: A solution of Rink Amine MBHA resin (0.2 mmol, 1.00 eq, Sub 0.33 mmol/g) in DMF (15.0 mL) was agitated with N₂ at 25°C for 1.0 hour.

Deprotection: 20% piperidine in DMF (15.0 mL) was added and the resin was agitated with N₂ at 25°C for 30 minutes. The resin was washed with DMF (15.0 mL, 5 times) and filtered.

Coupling: A solution of HBTU (2.85 eq) and Fmoc-Ala-OH (3.00 eq) in DMF (3.0 mL) was added to the resin. DIEA (6.00 eq) was added, and the mixture was agitated with N₂ at 25°C for 30-60 minutes. The coupling reaction was monitored by Ninhydrin and Chloranil tests. The resin was washed with DMF (15.0 mL, 5 times).

The steps for Deprotection and Coupling were repeated for the coupling of following amino acids (1-34) outlined in Table 13A.

**Table 13A. Peptide of SEQ ID NO: 102 Synthesis.**

| # | Materials | Coupling reagents |
|---|---|---|
| 1 | Fmoc-cVal-OH (2.00 eq) | HATU (1.90 eq) and DIEA (4.00 eq) |
| 2 | Fmoc-Glu(OtBu)-OH (3.00 eq) | HBTU (2.85 eq) and DIEA (6.00 eq) |
| 3 | Fmoc-Nle-OH (2.00 eq) | HATU (1.90 eq) and DIEA (4.00 eq) |
| 4 | Fmoc-Leu-OH (3.00 eq) | HBTU (2.85 eq) and DIEA (6.00 eq) |
| 5 | Fmoc-Lys(Boc)-OH (3.00 eq) | HBTU (2.85 eq) and DIEA (6.00 eq) |
| 6 | Fmoc-Arg(Pbf)-OH (3.00 eq) | HBTU (2.85 eq) and DIEA (6.00 eq) |
| 7 | Fmoc-Asn(Trt)-OH (3.00 eq) | HBTU (2.85 eq) and DIEA (6.00 eq) |
| 8 | Fmoc-cyclo[Glu-Ala-Glu(OtBu)-Lys]-OH (2.00 eq) | HATU (1.90 eq) and DIEA (4.00 eq) |
| 9 | Fmoc-Gln(Trt)-OH (3.00 eq) | HATU (2.85 eq) and DIEA (6.00 eq) |
| 10 | Fmoc-Ala-OH (3.00 eq) | HATU (2.85 eq) and DIEA (6.00 eq) |
| 11 | Fmoc-cVal-OH (2.00 eq) | HATU (1.90 eq) and DIEA (4.00 eq) |
| 12 | Fmoc-Gln(Trt)-OH (3.00 eq) | HATU (2.85 eq) and DIEA (6.00 eq) |
| 13 | Fmoc-Glu(OtBu)-OH (3.00 eq) | HATU (2.85 eq) and DIEA (6.00 eq) |
| 14 | Fmoc- Glu(OtBu)-OH (3.00 eq) | HATU (2.85 eq) and DIEA (6.00 eq) |
| 15 | Fmoc-Gln(Trt)-OH (3.00 eq) | HATU (2.85 eq) and DIEA (6.00 eq) |
| 16 | Fmoc-Lys(Alloc)-OH (2.00 eq) | HATU (1.90 eq) and DIEA (4.00 eq) |
| 17 | Fmoc-Nle-OH (3.00 eq) | HATU (2.85 eq) and DIEA (6.00 eq) |
| 18 | Fmoc-Glu(OtBu)-OH (3.00 eq) | HATU (2.85 eq) and DIEA (6.00 eq) |
| 19 | Fmoc-Leu-OH (3.00 eq) | HATU (2.85 eq) and DIEA (6.00 eq) |
| 20 | Fmoc-Val-OH (3.00 eq) | HATU (2.85 eq) and DIEA (6.00 eq) |
| 21 | Fmoc-Glu(OtBu)-OH (3.00 eq) | HATU (2.85 eq) and DIEA (6.00 eq) |
| 22 | Fmoc-Arg(Pbf)-OH(3.00 eq) | HATU (2.85 eq) and DIEA (6.00 eq) |
| 23 | Fmoc-Leu-OH(3.00 eq) | HATU (2.85 eq) and DIEA (6.00 eq) |
| 24 | Fmoc-Leu-OH (3.00 eq) | HATU (2.85 eq) and DIEA (6.00 eq) |
| 25 | Fmoc-His(Trt)-OH (3.00 eq) | HATU (2.85 eq) and DIEA (6.00 eq) |
| 26 | Fmoc-D-Phe-OH (3.00 eq) | HATU (2.85 eq) and DIEA (6.00 eq) |
| 27 | Fmoc-Thr(tBu)-OH (3.00 eq) | HATU (2.85 eq) and DIEA (6.00 eq) |
| 28 | Fmoc-Leu-OH (3.00 eq) | HATU (2.85 eq) and DIEA (6.00 eq) |
| 29 | Fmoc-Asp(OtBu)-OH (3.00 eq) | HATU (2.85 eq) and DIEA (6.00 eq) |
| 30 | Ac | 10% Ac₂O/5% NMM/85% DMF |
| - | De-Alloc | PhSiH₃ (10.0 eq) and Pd(PPh₃)₄ |
| 31 | Fmoc-(AEEA)-OH (2.00 eq) | HATU (1.90 eq) and DIEA (4.00 eq) |
| 32 | Fmoc-(AEEA)-OH (2.00 eq) | HATU (1.90 eq) and DIEA (4.00 eq) |
| 33 | Fmoc-Glu-OtBu (2.00 eq) | HATU (1.90 eq) and DIEA (4.00 eq) |
| 34 | 20-(Tert-Butoxy)-20-OxoicosanoicAcid (2.00 eq) | HATU (1.90 eq) and DIEA (4.00 eq) |

Peptide Cleavage: Cleavage solution (40.0 mL, 92.0% TFA/ 2.50% TIS /2.50% H₂O /3.0% DTT) was added to the flask containing resin at room temperature and stirred for 1.5 hour. The peptide was then precipitated with cold isopropyl ether (200 mL) and filtered. The filter cake was washed with isopropyl ether (50 mL, twice). The crude peptide was dried under vacuum for 1 hour (1.0 g). LCMS: MS cal.: 4683.36, MS observed: [M+4H]⁴⁺ = 1171.84; Rt = 1.15 minutes.

The crude peptide was then purified by prep-HPLC (TFA condition: A: 0.075 % TFA in H₂O, B: ACN) to give the final product (95.5 mg, 19.37 µmol, 9.68% yield, 95% purity, TFA) as a white solid. LCMS: MS cal.: 4683.36, MS observed: [M+4H]⁴⁺ =1171.84; Rₜ= 1.59 minutes; and HPLC Rt= 5.53 minutes. Purification conditions were as outlined below in Table 13B.

**Table 13B. Peptide of SEQ ID NO: 102 Purification Conditions.**

| Separation condition | |
|---|---|
| Dissolution condition | Dissolve in 20%ACN-H₂O |
| Instrument | Gilson GX-281 |
| Mobile Phase | A: H₂O (0.075%TFA in H₂O) |
| | B: CH₃CN |
| Gradient | 36-56-40 minutes, Retention time: 30 minutes |
| Column | YMC-Actus Triart C18, 250*30 mm, 5 mm, 120Å |
| Flow Rate | 20 mL/minute |
| Wavelength | 220/254 nm |
| Oven Temperature | 25^{°}C |

Alternatively, SEQ ID NO:2, SEQ ID NO:3, SEQ ID NO:4, SEQ ID NO:5, SEQ ID NO:6, SEQ ID NO:7, SEQ ID NO:12, SEQ ID NO:19, SEQ ID NO:21, SEQ ID NO:32, SEQ ID NO:33, and SEQ ID NO:34 were synthesized using Fmoc chemistry where the appropriate N-α-Fmoc-protected amino acid (5 equivalents, for example, R33) was coupled to a ProTide Rink Amide resin using 10 equivalents N,N'-diisopropyl carbodiimide (DIC) and 10 equivalents ethyl cyano(hydroxyimino)acetate (OxymaPure) coupling reagents in DMF. The reactants were microwave irradiated at 90°C for 2 minutes in a CEM Liberty Blue HT-12 automated microwave assisted peptide synthesizer. Following the coupling of the protected amino acid to the resin support, the alpha-amino protecting group was removed using 10% piperazine in ethanol: N-methyl-2-pyrrolidone (NMP) (1:10) containing 0.1 M 1-hydroxybenzotriazole (HOBt) at 90°C for 1 minute. After removal of the alpha-amino protecting group (for example, R33), the remaining alpha-amino-and side chain-protected amino acids were coupled stepwise in the desired order using the same protocol described above until the sequence was completed to position R22.

Introducing Fmoc-Glu(*O*-*2*-*PhiPr*)-OH (at R22) and Fmoc-Lys(Mtt)-OH (at **R25)** allowed the selective removal of the side chain protective groups on the resin without the deprotection of the other side chain protective groups and cleavage of the peptide from the resin. After coupling Fmoc-Glu(*O-2-PhiPr*)-OH, the resin was removed from the synthesizer to form the lactam bridge manually. After swelling the resin in CH₂Cl₂ (DCM) for 10 minutes, freshly made reagent 1% trifluoroacetic acid (TFA) in DCM containing 1% MeOH was added to the resin and stirred at room temperature for 1 hour, then the reagent was removed by filtration and fresh reagent was added to the resin followed by stirring, overnight (16 h). The reagent was removed by filtration and the resin was washed 4 times with DCM, 4 times with 5% N,N-diisopropylethylamine (DIPEA) in DCM, 5 times with DCM, 4 times with 0.1 M OxymaPure in DCM and 5 times with DCM. The lactam bridge formation was carried out on the resin with DIC and OxymaPure activation in the synthesizer at 90°C for 10 minutes. This reaction was repeated 2 more times with fresh reagents. After several washes, the synthesis was continued in the synthesizer till completion of the whole sequence. The Fmoc-protecting group was removed from the N-terminus using 10% piperazine in ethanol : N-Methyl-2-pyrrolidone (NMP) (1:10) containing 0.1 M 1-hydroxybenzotriazole (HOBt) at 90°C for 1 minute and the N-terminus was acylated with selected reagents like acetic anhydride.

Cleavage of the peptide from the resin and simultaneous deprotection of the side chains of the amino acids was carried out in 95 %TFA / 2.5% H₂O/ 2.5% triisopropylsilane (TIS) cleavage cocktail. The resin was stirred at room temperature for 3 hours, then pre-cooled diethyl ether was added to precipitate the crude peptide. The mixture was centrifuged, and the supernatant was discarded. The precipitated peptide and resin mixture was treated with diethyl ether, centrifuged, the supernatant discarded, and the crude precipitate was separated from the resin by washing and filtering with the mixture of 50 % acetonitrile / 50 % water. The solution obtained was lyophilized resulting in the crude peptide in dry powder form.

The crude peptide was purified on a Luna 5µm C18(2) 100 Å HPLC column (250 mm x 10 mm, Phenomenex) with an Agilent LC 1260 Enhanced LC HPLC system. Eluents: TFA, water and acetonitrile were HPLC grade. Eluent A is: 0.1% TFA/water, eluent B is: 0.1% TFA/90% acetonitrile/water. A linear gradient ranging from 35% to 90 % solvent B in 30 minutes, at a flow rate of 3 mL/min and UV detection at 220 nm was used. Fractions with the purity of >90% were pooled together and lyophilized.

The following peptides of Table 14 were made using the above procedures or modifications to the above procedure using the appropriate starting materials and intermediates. In Table 14: **K*** represents the point of attachment of the appropriate fatty acid via the nitrogen of the side chain of lysine; Y represents either an acylated (Ac) N-terminus or X; and X represents the point of attachment of the appropriate fatty acid via the nitrogen of the N-terminus.

**Table 14. Fatty acid conjugated cyclic peptides of the disclosure.**

| **SEQ ID NO:** | **SEQUENCE** | **K* or X*** |
|---|---|---|
| SEQ ID NO:1 | Ac- DLTfHLLREVLE(Nle)ARAEQ(CML)AQE#AAibK#NRK L(Nle)E(CML)I-CONH2 | |
| SEQ ID NO:2 | Ac- DLTfHLLREVLE(Nle)EREEQ(cVal)AQE#AEK#NRKL(N le)E(cVal)I-CONH2 | |
| SEQ ID NO:3 | X*-DLTfHLLREVLE(Nle)ARAEQ(CML)AQE#A(Deg) K#NRK(iPr)L(Nle)E(CML)I-CONH2 | (AEEA)-(AEEA)-γGlu-C18-diacid |
| SEQ ID NO:4 | X*- DLTfHLLREVLE(Nle)EREEQ(cVal)AQE#AEK#NRKL(N le)E(cVal)I-CONH2 | (AEEA)-(AEEA)-γGlu-C18-diacid |
| SEQ ID NO:5 | Ac- DLTK*HLLREVLE(Nle)EREEQ(cVal)AQE#AEK#NRKL (Nle)E(cVal)I-CONH2 | (AEEA)-(AEEA)-γGlu-C18-diacid |
| SEQ ID NO:6 | Ac- DLTfHLLRK*VLE(Nle)EREEQ(cVal)AQE#AEK#NRKL( Nle)E(cVal)I-CONH2 | (AEEA)-(AEEA)-γGlu-C18-diacid |
| SEQ ID NO:7 | Ac- DLTfHLLREVLK*(Nle)EREEQ(cVal)AQE#AEK#NRKL( Nle)E(cVal)I-CONH2 | (AEEA)-(AEEA)-γGlu-C18-diacid |
| SEQ ID NO:8 | Ac- DLTfHLLREVLEK*EREEQ(cVal)AQE#AEK#NRKL(Nle )E(cVal)I-CONH2 | (AEEA)-(AEEA)-γGlu-C18-diacid |
| SEQ ID NO:9 | Ac- DLTfHLLREVLE(Nle)K*REEQ(cVal)AQE#AEK#NRKL( Nle)E(cVal)I-CONH2 | (AEEA)-(AEEA)-γGlu-C18-diacid |
| SEQ ID NO:10 | Ac- DLTfHLLREVLE(Nle)EK*EEQ(cVal)AQE#AEK#NRKL( Nle)E(cVal)I-CONH2 | (AEEA)-(AEEA)-γGlu-C18-diacid |
| SEQ ID NO:11 | Ac- DLTfHLLREVLE(Nle)ERK*EQ(cVal)AQE#AEK#NRKL( Nle)E(cVal)I-CONH2 | (AEEA)-(AEEA)-γGlu-C18-diacid |
| SEQ ID NO:12 | Ac- DLTfHLLREVLE(Nle)EREK*Q(cVal)AQE#AEK#NRKL( Nle)E(cVal)I-CONH2 | (AEEA)-(AEEA)-γGlu-C18-diacid |
| SEQ ID NO: 13 | Ac- DLTfHLLREVLE(Nle)EREEK*(cVal)AQE#AEK#NRKL( Nle)E(cVal)I-CONH2 | (AEEA)-(AEEA)-γGlu-C18-diacid |
| SEQ ID NO:14 | Ac- DLTfHLLREVLE(Nle)EREEQK*AQE#AEK#NRKL(Nle) E(cVal)I-CONH2 | (AEEA)-(AEEA)-γGlu-C18-diacid |
| SEQ ID NO:15 | Ac- DLTfHLLREVLE(Nle)EREEQ(cVal)K*QE#AEK#NRKL( Nle)E(cVal)I-CONH2 | (AEEA)-(AEEA)-γGlu-C18-diacid |
| SEQ ID NO:16 | Ac- DLTfHLLREVLE(Nle)EREEQ(cVal)AK*E#AEK#NRKL( Nle)E(cVal)I-CONH2 | (AEEA)-(AEEA)-γGlu-C18-diacid |
| SEQ ID NO:17 | Ac- DLTfHLLREVLE(Nle)EREEQ(cVal)AQE#AK*K#NRKL( Nle)E(cVal)I-CONH2 | (AEEA)-(AEEA)-γGlu-C18-diacid |
| SEQ ID NO:18 | Ac- DLTfHLLREVLE(Nle)EREEQ(cVal)AQE#AEK#NK*KL( Nle)E(cVal)I-CONH2 | (AEEA)-(AEEA)-γGlu-C18-diacid |
| SEQ ID NO:19 | Ac- DLTfHLLREVLE(Nle)EREEQ(cVal)AQE#AEK#NRK*L( Nle)E(cVal)I-CONH2 | (AEEA)-(AEEA)-γGlu-C18-diacid |
| SEQ ID NO:20 | Ac- DLTfHLLREVLE(Nle)EREEQ(cVal)AQE#AEK#NRKK*( Nle)E(cVal)I-CONH2 | (AEEA)-(AEEA)-γGlu-C18-diacid |
| SEQ ID NO:21 | Ac- DLTfHLLREVLE(Nle)EREEQ(cVal)AQE#AEK#NRKL(N le)K*(cVal)I-CONH2 | (AEEA)-(AEEA)-γGlu-C18-diacid |
| SEQ ID NO:22 | X*- DLTfHLLREVLE(Nle)EREEQ(cVal)AQE#AEK#NRKL(N le)E(cVal)I-CONH2 | (AEEA)-(AEEA)-γGlu-C16-diacid |
| SEQ ID NO:23 | Ac- DLTfHLLRK*VLE(Nle)EREEQ(cVal)AQE#AEK#NRKL( Nle)E(cVal)I-CONH2 | (AEEA)-(AEEA)-γGlu-C16-diacid |
| SEQ ID NO:24 | Ac- DLTfHLLREVLK*(Nle)EREEQ(cVal)AQE#AEK#NRKL( Nle)E(cVal)I-CONH2 | (AEEA)-(AEEA)-γGlu-C16-diacid |
| SEQ ID NO:25 | Ac- DLTfHLLREVLE(Nle)EK*EEQ(cVal)AQE#AEK#NRKL( Nle)E(cVal)I-CONH2 | (AEEA)-(AEEA)-γGlu-C16-diacid |
| SEQ ID NO:26 | Ac- DLTfHLLREVLE(Nle)EREK*Q(cVal)AQE#AEK#NRKL( Nle)E(cVal)I-CONH2 | (AEEA)-(AEEA)-γGlu-C16-diacid |
| SEQ ID NO:27 | Ac- DLTfHLLREVLE(Nle)EREEK*(cVal)AQE#AEK#NRKL( Nle)E(cVal)I-CONH2 | (AEEA)-(AEEA)-γGlu-C16-diacid |
| SEQ ID NO:28 | Ac DLTfHLLREVLE(Nle)EREEQ(cVal)K*QE#AEK#NRKL( Nle)E(cVal)I-CONH2 | (AEEA)-(AEEA)-γGlu-C16-diacid |
| SEQ ID NO:29 | Ac- DLTfHLLREVLE(Nle)EREEQ(cVal)AK*E#AEK#NRKL( Nle)E(cVal)I-CONH2 | (AEEA)-(AEEA)-γGlu-C16-diacid |
| SEQ ID NO:30 | Ac- DLTfHLLREVLE(Nle)EREEQ(cVal)AQE#AEK#NRKK*( Nle)E(cVal)I-CONH2 | (AEEA)-(AEEA)-γGlu-C16-diacid |
| SEQ ID NO:32 | Ac- DLTfHLLRK*VLE(Nle)EEEEQ(cVal)AQE#AEK#NRKL( Nle)E(cVal)I-CONH2 | (AEEA)-(AEEA)-γGlu-C18-diacid |
| SEQ ID NO:33 | Ac- DLTfHLLRK*VLE(Nle)EREEQ(cVal)AQE#AEK#NRKL( Nle)E(cVal)I-CONH2 | PEG12-γGlu-C18-diacid |
| SEQ ID NO:34 | Ac- DLTfHLLRK*VLE(Nle)EREEQ(cVal)AQE#AEK#NRKL( Nle)E(cVal)I-CONH2 | PEG12-(γGlu)₂-C18-diacid |
| SEQ ID NO:36 | Ac- DLTfHLLREVLE(Nle)TK*EEQ(cVal)AQE#AEK#NRKL( Nle)E(cVal)I-CONH2 | (AEEA)-(AEEA)-γGlu-C16-diacid |
| SEQ ID NO:37 | Ac- DLTfHLLREVLE(Nle)EK*EEQ(cVal)AQE#AEK#NRKL( Nle)E(cVal)A-CONH2 | (AEEA)-(AEEA)-γGlu-C16-diacid |
| SEQ ID NO:38 | Ac- DLTfHLLREVLE(Nle)EK*AEQ(cVal)AQE#AEK#NRKL( Nle)E(cVal)I-CONH2 | (AEEA)-(AEEA)-γGlu-C16-diacid |
| SEQ ID NO:39 | Ac- DLTfHLLREVLE(Nle)TK*AEQ(cVal)AQE#AEK#NRKL( Nle)E(cVal)A-CONH2 | (AEEA)-(AEEA)-γGlu-C16-diacid |
| SEQ ID NO:40 | Ac- DLTfHLLREVLE(Nle)EK*EEQ(cVal)AQE#AEK#NRKL( Nle)E(cVal)I-CONH2 | (AEEA)-(AEEA)-γGlu-γGlu-C16-diacid |
| SEQ ID NO:41 | Ac- DLTfHLLREVLE(Nle)EK*EEQ(cVal)AQE#AEK#NRKL( Nle)E(cVal)I-CONH2 | (AEEA)-(AEEA)-γGlu-γGlu-γGlu-C16-diacid |
| SEQ ID NO:42 | Ac- DLTfHLLREVLE(Nle)EK*EEQ(cVal)AQE#AEK#NRKL( Nle)E(cVal)I-CONH2 | (AEEA)-γGlu-γGlu-γGlu-C16-diacid |
| SEQ ID NO:43 | Ac- DLTfHLLREVLE(Nle)EK*EEQ(cVal)AQE#AEK#NRKL( Nle)E(cVal)I-CONH2 | Glu-(AEEA)-(AEEA)-γGlu-C16-diacid |
| SEQ ID NO:44 | Ac- DLTfHLLREVLE(Nle)EK*EEQ(cVal)AQE#AEK#NRKL( Nle)E(cVal)I-CONH2 | γGlu-(AEEA)-(AEEA)-γGlu-C16-diacid |
| SEQ ID NO:45 | Ac- DLTfHLLREVLE(Nle)EK*EEQ(cVal)AQE#AEK#NRKL( Nle)E(cVal)I-CONH2 | Lys-(AEEA)-(AEEA)-γGlu-C16-diacid |
| SEQ ID NO:46 | Ac- DLTfHLLREVLE(Nle)EK*EEQ(cVal)AQE#AEK#NRKL( Nle)E(cVal)I-CONH2 | Ala-(AEEA)-(AEEA)-γGlu-C16-diacid |
| SEQ ID NO:47 | Ac- DLTfHLLREVLE(Nle)EK*EEQ(cVal)AQE#AEK#NRKL( Nle)E(cVal)I-CONH2 | Gln-(AEEA)-(AEEA)-γGlu-C16-diacid |
| SEQ ID NO:48 | Ac- DLTfHLLREVLE(Nle)EK*EEQ(cVal)AQE#AEK#NRKL( Nle)E(cVal)I-CONH2 | Gly-(AEEA)-(AEEA)-γGlu - C16-diacid |
| SEQ ID NO:49 | Ac- DLTfHLLREVLE(Nle)EK*EEQ(cVal)AQE#AEK#NRKL( Nle)E(cVal)I-CONH2 | His-(AEEA)-(AEEA)-γGlu-C16-diacid |
| SEQ ID NO:50 | Ac- DLTfHLLREVLE(Nle)K*QEEQ(cVal)AQE#AEK#NRKL( Nle)E(cVal)I-CONH2 | (AEEA)-(AEEA)-γGlu-C18-diacid |
| SEQ ID NO:51 | Ac- DLTfHLLREVLE(Nle)K*EQEQ(cVal)AQE#AEK#NRKL( Nle)E(cVal)I-CONH2 | (AEEA)-(AEEA)-γGlu-C18-diacid |
| SEQ ID NO:52 | Ac- DLTfHLLREVLE(Nle)K*AEEQ(cVal)AQE#AEK#NRKL( Nle)E(cVal)I-CONH2 | (AEEA)-(AEEA)-γGlu-C18-diacid |
| SEQ ID NO:53 | Ac- DLTfHLLREVLE(Nle)K*EEEQ(cVal)AQE#AEK#NRKL( Nle)E(cVal)I-CONH2 | (AEEA)-(AEEA)-γGlu-C18-diacid |
| SEQ ID NO:54 | Ac- DLTfHLLRK*VLE(Nle)EREEQ(cVal)AQE#AEK#NRKL( Nle)E(cVal)I-CONH2 | (AEEA)-(AEEA)-γGlu-γGlu-C18-diacid |
| SEQ ID NO:55 | Ac- DLTfHLLRK*VLE(Nle)EREEQ(cVal)AQE#AEK#NRKL( Nle)E(cVal)I-CONH2 | (AEEA)-(AEEA)-γGlu-γGlu-γGlu-C18-diacid |
| SEQ ID NO:56 | Ac- DLTfHLLRK*VLE(Nle)EQEEQ(cVal)AQE#AEK#NRKL( Nle)E(cVal)I-CONH2 | (AEEA)-(AEEA)-γGlu-C18-diacid |
| SEQ ID NO:57 | Ac- DLTfHLLRK*VLE(Nle)QQEEQ(cVal)AQE#AEK#NRKL (Nle)E(cVal)I-CONH2 | (AEEA)-(AEEA)-γGlu-C18-diacid |
| SEQ ID NO:58 | Ac- DLTfHLLRK*VLE(Nle)EAadEEQ(cVal)AQE#AEK#NRK L(Nle)E(cVal)I-CONH2 | (AEEA)-(AEEA)-γGlu-C18-diacid |
| SEQ ID NO:59 | Ac- DLTfHLLRK*VLE(Nle)EGlaEEQ(cVal)AQE#AEK#NRK L(Nle)E(cVal)I-CONH2 | (AEEA)-(AEEA)-γGlu-C18-diacid |
| SEQ ID NO:60 | Ac- DLTfHLLRK*VLE(Nle)EpcFEEQ(cVal)AQE#AEK#NRK L(Nle)E(cVal)I-CONH2 | (AEEA)-(AEEA)-γGlu-C18-diacid |
| SEQ ID NO:61 | Ac- DLTfHLLRK*VLE(Nle)EK&EEQ(cVal)AQE#AEK#NRK L(Nle)E(cVal)I-CONH2 | (AEEA)-(AEEA)-γGlu-C18-diacid |
| SEQ ID NO:62 | Ac- DLTfHLLRK*VLE(Nle)EK&EEQ(cVal)AQE#AEK#NRK L(Nle)E(cVal)I-CONH2 | (AEEA)-(AEEA)-γGlu-C18-diacid |
| SEQ ID NO:63 | Ac- DLTfHLLRK*VLE(Nle)EK&EEQ(cVal)AQE#AEK#NRK L(Nle)E(cVal)I-CONH2 | (AEEA)-(AEEA)-γGlu-C18-diacid |
| SEQ ID NO:64 | Ac- DLTfHLLRK*VLE(Nle)EE&EEQ(cVal)AQE#AEK#NRK L(Nle)E(cVal)I-CONH2 | (AEEA)-(AEEA)-γGlu-C18-diacid |
| SEQ ID NO:65 | Ac- DLTfHLLRK*VLE(Nle)EE&EEQ(cVal)AQE#AEK#NRK L(Nle)E(cVal)I-CONH2 | (AEEA)-(AEEA)-γGlu-C18-diacid |
| SEQ ID NO:66 | Ac- DLTfHLLRK*VLE(Nle)EQEEQ(cVal)AQE#AEK#NRKL( Nle)E(cVal)I-CONH2 | (AEEA)-(AEEA)-γGlu-C16-diacid |
| SEQ ID NO:67 | Ac- DLTfHLLRK*VLE(Nle)EREEQ(cVal)AQE#AEK#NRKL( Nle)DSV-CONH2 | (AEEA)-(AEEA)-γGlu-C18-diacid |
| SEQ ID NO:68 | Ac- DLTfHLLRK*VLE(Nle)EREEQ(cVal)AQE#AEK#NRKL( Nle)DIA-CONH2 | (AEEA)-(AEEA)-γGlu-C18-diacid |
| SEQ ID NO:69 | Ac- DLTfHLLRK*VLE(Nle)EREEQ(cVal)AQE#aEK#NRKL( Cha)DIA-CONH2 | (AEEA)-(AEEA)-γGlu-C18-diacid |
| SEQ ID NO:70 | Ac- DLTfHLLREVLE(Nle)EQEEQ(cVal)AQE#AEK#NRKK*( Nle)E(cVal)I-CONH2 | (AEEA)-(AEEA)-γGlu-C18-diacid |
| SEQ ID NO:71 | Ac- DLTfHLLREVLE(Nle)K*REEQ(cVal)AQE#AEK#NRKL( Nle)E(cVal)I-CONH2 | (AEEA)-γGlu-C18-diacid |
| SEQ ID NO:72 | Ac- DLTfHLLREVLE(Nle)K*REEQ(cVal)AQE#AEK#NRKL( Nle)E(cVal)I-CONH2 | (AEEA)-(AEEA)-(AEEA)-γGlu-C18-diacid |
| SEQ ID NO:73 | Ac- DLTfHLLREVLE(Nle)K*REEQ(cVal)AQE#AEK#NRKL( Nle)E(cVal)I-CONH2 | (AEEA)-(AEEA)-γGlu-γGlu-C18-diacid |
| SEQ ID NO:74 | Ac- DLTfHLLREVLE(Nle)K*REEQ(cVal)AQE#AEK#NRKL( Nle)E(cVal)I-CONH2 | (AEEA)-(AEEA)-γGlu-γGlu-γGlu-C18-diacid |
| SEQ ID NO:75 | Ac- DLTfHLLREVLE(Nle)K*KEEQ(cVal)AQE#AEK#NRKL( Nle)E(cVal)I-CONH2 | (AEEA)-(AEEA)-γGlu-C18-diacid |
| SEQ ID NO:76 | Ac- DLTfHLLREVLE(Nle)K*KEEQ(cVal)AQE#AEK#NRKL( Nle)E(cVal)I-CONH2 | (AEEA)-(AEEA)-γGlu-C18-diacid |
| SEQ ID NO:77 | Ac- DLTfHLLREVLE(Nle)K*KADQ(cVal)AQE#AEK#NRKL (Nle)E(cVal)I-CONH2 | (AEEA)-(AEEA)-γGlu-C18-diacid |
| SEQ ID NO:78 | Ac- DLTfHLLREVLE(Nle)K*RAEQ(cVal)AQE#AEK#NRKL (Nle)E(cVal)A-CONH2 | (AEEA)-(AEEA)-γGlu-C18-diacid |
| SEQ ID NO:79 | Ac- DLTfHLLREVLE(Nle)K*REEQ(cVal)AQE#AEK#NRKL( Nle)E(cVal)A-CONH2 | (AEEA)-(AEEA)-γGlu-C18-diacid |
| SEQ ID NO:80 | Ac- DLTfHLLREVLE(Nle)K*KAEQ(cVal)AQE#AEK#NRKL (Nle)E(cVal)A-CONH2 | (AEEA)-(AEEA)-γGlu-C18-diacid |
| SEQ ID NO:81 | Ac- DLTfHLLREVLE(Nle)K*RQEQ(cVal)AQE#AEK#NRKL (Nle)E(cVal)I-CONH2 | (AEEA)-(AEEA)-γGlu-C18-diacid |
| SEQ ID NO:82 | Ac- DLTfHLLREVLE(Nle)K*RTEQ(cVal)AQE#AEK#NRKL( Nle)E(cVal)I-CONH2 | (AEEA)-(AEEA)-γGlu-C18-diacid |
| SEQ ID NO:83 | Ac- DLTfHLLREVLE(Nle)K*REEQ(cVal)AQE#AEK#NRKL( Nle)E(cVal)I-CONH2 | (AEEA)-(AEEA)-γGlu-C20-diacid |
| SEQ ID NO:84 | Ac- DLTfHLLREVLE(Nle)EK*EEQ(cVal)AQE#AEK#NRKL( Nle)E(cVal)I-CONH2 | Gln-(AEEA)-(AEEA)-γGlu-C18-diacid |
| SEQ ID NO:85 | Ac- DLTfHLLREVLE(Nle)TK*EEQ(cVal)AQE#AEK#NRKL( Nle)E(cVal)I-CONH2 | (AEEA)-(AEEA)-γGlu-C18-diacid |
| SEQ ID NO:86 | Ac- DLTfHLLREVLE(Nle)QK*EEQ(cVal)AQE#AEK#NRKL( Nle)E(cVal)I-CONH2 | (AEEA)-(AEEA)-γGlu-C18-diacid |
| SEQ ID NO:87 | Ac- DLTfHLLREVLE(Nle)AK*EEQ(cVal)AQE#AEK#NRKL( Nle)E(cVal)I-CONH2 | (AEEA)-(AEEA)-γGlu-C18-diacid |
| SEQ ID NO:88 | Ac- DLTfHLLREVLE(Nle)EK*EEQ(cVal)AQE#AEK#NRKL( Nle)E(cVal)I-CONH2 | (AEEA)-(AEEA)-γGlu-γGlu-C18-diacid |
| SEQ ID NO:89 | Ac- DLTfHLLREVLE(Nle)QK*EEQ(cVal)AQE#AEK#NRKL( Nle)E(cVal)A-CONH2 | (AEEA)-(AEEA)-γGlu-C18-diacid |
| SEQ ID NO:90 | Ac- DLTfHLLREVLE(Nle)QK*AEQ(cVal)AQE#AEK#NRKL (Nle)E(cVal)A-CONH2 | (AEEA)-(AEEA)-γGlu-C18-diacid |
| SEQ ID NO:91 | Ac- DLTfHLLREVLE(Nle)TK*EEQ(cVal)AQE#AEK#NRKL( Nle)E(cVal)A-CONH2 | (AEEA)-(AEEA)-γGlu-C18-diacid |
| SEQ ID NO:92 | Ac- DLTfHLLREVLE(Nle)TK*AEQ(cVal)AQE#AEK#NRKL( Nle)E(cVal)A-CONH2 | (AEEA)-(AEEA)-γGlu-C18-diacid |
| SEQ ID NO:93 | Ac- DLTfHLLREVLE(Nle)TK*AEQ(cVal)AQE#AEK#NRKL( Nle)E(cVal)A-CONH2 | (AEEA)-(AEEA)-(AEEA)-γGlu-C18-diacid |
| SEQ ID NO:94 | Ac- DLTfHLLREVLE(Nle)QK*AEQ(cVal)AQE#AEK#NRKL (Nle)E(cVal)A-CONH2 | (AEEA)-(AEEA)-(AEEA)-γGlu-C18-diacid |
| SEQ ID NO:95 | Ac- DLTfHLLREVLE(Nle)K*QEEQ(cVal)AQE#AEK#NRKL( Nle)E(cVal)A-CONH2 | (AEEA)-(AEEA)-γGlu-C18-diacid |
| SEQ ID NO:96 | Ac- DLTfHLLREVLE(Nle)K*QEEQ(cVal)AQE#AEK#NRKL( Nle)E(cVal)A-CONH2 | (AEEA)-(AEEA)-γGlu-γGlu-C18-diacid |
| SEQ ID NO:97 | Ac- DLTfHLLREVLE(Nle)K*QEEQ(cVal)AQE#AEK#NRKL( Nle)E(cVal)A-CONH2 | (AEEA)-(AEEA)-(AEEA)-γGlu-C18-diacid |
| SEQ ID NO:98 | Ac- DLTfHLLREVLE(Nle)K*QAEQ(cVal)AQE#AEK#NRKL (Nle)E(cVal)A-CONH2 | (AEEA)-(AEEA)-γGlu-C18-diacid |
| SEQ ID NO:99 | Ac- DLTfHLLREVLE(Nle)K*QEEQ(cVal)AQE#AEK#NRKL( Nle)E(cVal)I-CONH2 | (AEEA)-(AEEA)-(AEEA)-γGlu-C18-diacid |
| SEQ ID NO:100 | Ac- DLTfHLLREVLE(Nle)K*QEEQ(cVal)AQE#AEK#NRKL( Nle)E(cVal)I-CONH2 | (AEEA)-(AEEA)-γGlu-C20-diacid |
| SEQ ID NO:101 | Ac- DLTfHLLREVLE(Nle)K*REEQ(cVal)AQE#AEK#NRKL( Nle)E(cVal)A-CONH2 | (AEEA)-(AEEA)-γGlu-C20-diacid |
| SEQ ID NO:102 | Ac- DLTfHLLREVLE(Nle)K*QEEQ(cVal)AQE#AEK#NRKL( Nle)E(cVal)A-CONH2 | (AEEA)-(AEEA)-γGlu-C20-diacid |
| SEQ ID NO:103 | Ac- DLTfHLLREVLE(Nle)K*QEEQ(cVal)AQE#AEK#NRKL( Nle)E(cVal)A-CONH2 | (AEEA)-(AEEA)-(AEEA)-γGlu-C20-diacid |
| SEQ ID NO:104 | Ac- DLTfHLLREVLE(Nle)K*EREQ(cVal)AQE#AEK#NRKL( Nle)E(cVal)A-CONH2 | (AEEA)-(AEEA)-γGlu-C20-diacid |
| SEQ ID NO:105 | Ac- DLTfHLLREVLE(Nle)K*NEEQ(cVal)AQE#AEK#NRKL( Nle)E(cVal)A-CONH2 | (AEEA)-(AEEA)-γGlu-C20-diacid |
| SEQ ID NO:106 | Ac- DLTfHLLREVLE(Nle)K*TEEQ(cVal)AQE#AEK#NRKL( Nle)E(cVal)A-CONH2 | (AEEA)-(AEEA)-γGlu-C20-diacid |

### Experimental Method B: Peptide Solubility and Stability

Peptides of the disclosure were analyzed for solubility, stability, and aggregation, according to the examples, below.

### Example B1

### Sample Preparation and Solubility

Preliminary screening methods for the evaluation of multiple fatty acid peptide analogs included a two-stage approach. First, formulations were prepared at target concentrations of approximately 10, 20, 40 or 50 mg/mL in 150 mM phosphate, pH 7.5. These formulations were titrated using NaOH, as needed, to the target pH 7.5 and were evaluated (visual assessment only). For visibly insoluble formulations, an additional buffer was added to dissolve the peptide. Once dissolved, all formulations were filtered through a 0.2 µm PVDF filter membrane and analyzed by RP-HPLC for soluble content. Formulations were incubated at 4°C, room temperature, and 37°C. At day 0 and 7 the formulations were analyzed by visual observation for solubility, size exclusion chromatography (SEC) for aggregation, & RP-HPLC for chemical stability studies.

### Example B2

### Size Exclusion Chromatography

Size exclusion chromatography (SEC) was conducted using an Agilent 1260 Infinity II-UV equipped with a Sepax Zenix-C SEC-300 (3 µm, 300 Å, 4.6 x 300 mm). UV absorption at 220 nm was monitored during the analysis. Formulated samples from day 0 and 7 were used directly for injection with injection volume of 1 µL. Separation was achieved using an isocratic buffer system containing 150 mM sodium phosphate (pH 7.5) at room temperature, with a constant flow rate at 0.35 mL/minute, and a run time of 15 minutes. A mixture of bovine γ-globulins (MW approximately 150,000 Da, at 1mg/ml), egg white albumin (MW approximately 44,300 Da, at 1mg/ml), ribonuclease A type I-A (MW approximately 13,700 Da, 1mg/ml), and uracil (MW 112.09 Da, at 0. 1mg/ml) was used as external standards. Peaks with estimated molecular weight greater than 150,000 were considered as aggregations. Area under curve of peaks were used to analyze the percentage of aggregations.

### Example B3

### Chemical Stability Analysis

Chemical stability analysis was conducted on an Agilent 1260 Infinity II LC-UV instrument with a Kinetex XB-C18 reversed phase column (2.6 µM, 100 Å, 150mm× 4.6 mm). Formulated samples from day 0, 7, and 30 were diluted into 5 mg/ml solutions with 150 mM phosphate, pH 7.5 buffer with injection volume of 5 µL. A linear gradient from 30% B to 70% B over 40 minutes was used for the run (buffer A: 100% water, 0.1% TFA; buffer B: 100% aqueous acetonitrile, 0.1% TFA, flow rate 0.5ml/minute). UV absorption at 220nm was monitored throughout the run, the area under the curve of peaks on the UV trace were analyzed to give purity information for each sample. The purity of samples at day 7 were compared to that at day 0 to provide % purity loss.

For example, the peptide of SEQ ID NO: 101 in either 10mM phosphate at pH 7 or 10mM TRIS at pH8, has high solubility (>100 mg/mL), high physical and chemical stability, no aggregation, and low viscosity.

### Experimental Method C: in vitro Testing of Peptides

Peptides of the disclosure were analyzed for in vitro and in vivo activity, according to the examples, below.

### Example C1

### Cells and Cell Culturing

Human CHO-KI CRF-R1 (hCRF-R1; Catalog # 95-0047C2) and human CHO-K1 CRF2 (hCRF-R2; Catalog # 95-0048C2) cell lines were purchased from Eurofins.

CHO-K1 Complete Media: DMEM Version (complete medium):Dulbecco's Minimal Essential Media with High Glucose, 4.5% (DMEM-HG), 10% [vol] U.S. Sourced, Certified Fetal Bovine Serum (FBS), and 1X [standard mammalian cell culture concentration] of the following: L-Glutamine, Non-Essential Amino Acids, Hepes Buffer (10 mM final concentration) and a standard antibiotic/antimycotic mix (penicillin, streptomycin, amphotericin B (Gibbco Cat # 15240062).

CHO-K1 Complete Media - HF12 Version is the same as the CHO-K1 Complete Media, DMEM Version (complete medium), except that Ham's F-12 was used as the base media. Cell Plating 2 Reagent (CPR2) - Eurofins DiscoverX Assay Complete Cell Plating 2 Reagent (Cat# 93-0563R2A). Lipofecamine LTX (Invitrogen^{™} Cat# 15338100). FuGENE 6 (Promega Cat# E2691). Bovine Albumin Fraction V, Fatty Acid Free: MP Biomedicals, Cat. 152401, Lot. S8210.

On Day 0, cryovial(s) containing the frozen cells (approx. 1.0E+6 cells/vial) stored in liquid nitrogen were removed and thawed in a 37°C water bath for 60 seconds and then diluted into fresh CHO-K1 Complete Media that was pre-warmed to 37°C. The cells were further diluted into a total of 11 ml of media (9.1E+5 cells/ml) and then distributed into 96-well Corning (3903) cell culture-treated assay plates using a multi-channel pipettor. Typical seeding densities were 1:4 (approx. 7.5E+3 cells/well) for CRF-R1 established line cells (Eurofins Cat# 95-0047C2) and 1:1 (approx. 3.0E+4 cells/well) for CRF-R2 established line cells (Eurofins Cat# 95-0048C2). Cells were then incubated at 37°C, 5% CO₂(g), 100% humidity for 3-4 days, checked daily, until approximately 90% (CRFR1 cells) and 60% (hCRF-R2 cells) confluency, at which time cells were used in cAMP Assays. Occasionally, the HF12 version of complete media was used to culture both hCRF-R1 and hCRFR-2 cell lines for cAMP Assays.

Eurofins Assay Ready (division arrested) versions of the above hCRF-R1 and hCRF-R2 (hCRF-R1 and hCRF-R2 eXpress cell/assay kits) cells were removed from liquid nitrogen storage and thawed in a 37°C water bath for 30 seconds and then diluted into fresh Cell Plating 2 Reagent that was pre-warmed to 37°C. The cells were further diluted into a total of 11 ml of CPR2 media (3.41E+5 cells/ml) and then distributed using a multi-channel pipettor into 96-well Eurofins assay plates according to manufacturing instructions.

### Example C2

### cAMP Assays

cAMP was determined utilizing a DiscoverX HitHunter assay Enzyme Fragment Complementation (EF) technology purchased from Eurofins as described in the manufacturing instructions with some adjustments. IC50's were calculated from antagonist dose response curves utilizing interpolated cAMP levels and/or relative light unit (RLU) data. In addition, eXpress assay ready cAMP kits were also utilized with hCRF-R1 and hCRF-R2 division arrested cells according to the manufacturing instructions. Briefly, antagonist dilutions were prepared in assay buffer: Gibco Hank's Buffered Salt Solution (Gibco 30-410, HBSS) supplemented with + 10 mM HEPES Buffer (Gibco 15630-080), containing 6X Tween-20 (Gibco BP337-100; final concentration in wells 0.01%). After removal of the culture media in cells, assay buffer (30 µl) was added to each of the wells on the plate utilizing a multichannel pipettor (INTEGRA VIAFLO 300 µl). The various antagonist dilutions (7.5 µl) at 6X concentration were added to the wells according to the assay protocol plate map and the plate was incubated for 30 min at 37°C. To compare the effects of albumin on the antagonist IC₅₀, assays were also performed by diluting the compounds as described above in assay buffer containing 0.1% [w:v] bovine serum albumin (BSA) or no BSA (Bovine Albumin Fraction V, Fatty Acid Free: MP Biomedicals, Cat. 152401, Lot. S8210). Immediately thereafter, 7.5 µl (6X) of agonist (hCRF for hCRF-R1, hUCN1 for hCRF-R2) was added at a final concentration of 1 nM/well; approximately EC₈₀) followed by a further incubation for 30 min at 37°C. Following the incubation of the antagonists and agonists, 15 µl of Eurofins HitHunter cAMP antibody solution and 60 µl of the Working Detection Solution were added followed by a 1 h incubation at room temperature in the dark. Finally, 60 µl of cAMP Solution A was added followed by a further incubation overnight (approx. 18 h). The total final reaction volume was 180 µl/well. The resulting chemiluminescent signal was measured in a Tecan Spark luminometer.

Table 15 below shows the tradeoff between solubility and activity (CRFR1 IC₅₀ (± BSA), and CRFR2 IC₅₀ (± BSA)) data for peptides of the disclosure. For example, SEQ ID NO: 101 has a CRFR1 IC₅₀ of 98 nM in BSA, a CRFR2 IC₅₀ of 89nM in BSA, and is soluble at ≥45mg/mL, whereas SEQ ID NO: 25 has a CRFR1 IC₅₀ of 98 nM in BSA, a CRFR2 IC₅₀ of 124nM in BSA, but is insoluble.

**Table 15**

| **SEQ ID NO:** | **CRFR1 (IC₅₀ nM)** | | **CRFR2 (IC₅₀ nM)** | | **Solubility 150 mM phosphate pH 7.4 (mg/mL)** |
|---|---|---|---|---|---|
| | **No Albumin** | **BSA (0.1%)** | **No Albumin** | **BSA (0.1%)** | |
| 1 | 1.7 | 3.2 | 0.25 | 0.29 | 0.012 |
| 2 | 3.7 | 8.0 | 0.78 | 1.0 | 2.55 |
| 3 | 15 | 123 | 6.1 | 53 | |
| 4 | 2.4 | 111 | 2.0 | 57 | 20 |
| 5 | 24 | 252 | 37 | 280 | |
| 6 | 2.3 | 40 | 0.66 | 23 | 25 |
| 7 | 2.8 | 52 | 1.3 | 18 | Insoluble at 10 mg/ml |
| 8 | 17 | 399 | 1.7 | 33 | < 15 |
| 9 | 1.1 | 15 | 0.67 | 16 | 10 |
| 10 | 2.3 | 118 | 7.2 | 266 | 10 |
| 11 | 9.4 | 546 | 0.84 | 34 | < 10 |
| 12 | 3.2 | 143 | 0.68 | 26 | Insoluble at 10 mg/ml |
| 13 | 2.5 | 81 | 1.1 | 13 | 10 |
| 14 | 8.7 | 224 | 3.5 | 110 | < 10 |
| 15 | 9.9 | 653 | 0.88 | 42 | 15 |
| 16 | 1.6 | 812 | 0.56 | 52 | < 10 |
| 17 | 1.5 | 160 | 0.38 | 19 | < 10 |
| 18 | 449 | 354 | 4.1 | 201 | 30 |
| 19 | 3.8 | 132 | 5.7 | 54 | |
| 20 | 1.3 | 22 | 0.53 | 20 | ≥ 20 |
| 21 | 2.9 | 135 | 13 | 67 | |
| 22 | 0.21 | 19 | 0.49 | 31 | 20 |
| 23 | 4.3 | 66 | 0.70 | 4.4 | 20 |
| 24 | 1.6 | 15 | 2.2 | 7.9 | 10 |
| 25 | 4.2 | 98 | 14 | 124 | Insoluble |
| 26 | 2.0 | 12 | 0.43 | 4.4 | 10 |
| 27 | 3.0 | 108 | 2.1 | 18 | 10 |
| 28 | 2.5 | 20 | 0.88 | 14 | 10 |
| 29 | 3.6 | 139 | 2.5 | 23 | 20 |
| 30 | 2.6 | 11 | 1.5 | 11 | |
| 32 | 0.82 | 53 | 1.3 | 46 | 20 |
| 33 | 0.63 | 350 | 0.26 | 50 | 20 |
| 34 | 1.6 | 138 | 1.3 | 31 | |
| 36 | 3.4 | 81 | 66 | 182 | ≥20 |
| 37 | 21 | 142 | >1000 | 356 | <2 |
| 38 | 2.4 | 73 | 2.3 | 106 | >2 |
| 39 | 6.3 | 135 | 576 | 142 | ≥20 |
| 40 | 6.6 | 125 | 12 | 229 | ≥20 |
| 41 | 16 | >1000 | 23 | >1000 | ≥10 |
| 42 | 16 | 156 | 7.6 | 580 | >2 |
| 43 | 14 | 116 | 53 | >1000 | ≥20 |
| 44 | 12 | 115 | 37 | 246 | ≥20 |
| 45 | 4.3 | 63 | 9.7 | 48 | >2 |
| 46 | 2.5 | 53 | 4.2 | 76 | ≥10 |
| 47 | 3.6 | 95 | 16 | >1000 | ≥10 |
| 48 | 6.1 | 100 | 33 | 189 | >2 |
| 49 | 6.9 | 113 | 12 | 90 | >2 |
| 50 | 2.1 | 72 | 6.4 | 58.4 | ≥20 |
| 51 | 2.0 | 90 | 1.8 | 123 | <2 |
| 52 | 1.9 | 105 | 1.4 | 104 | ≥20 |
| 53 | 3.0 | 149 | 15 | 204 | ≥5 |
| 54 | 6.7 | 100 | 0.90 | 33 | <2 |
| 55 | 12 | 238 | 40 | 25 | ≥5 |
| 56 | 2.0 | 156 | 0.30 | 23 | ≥5 |
| 57 | 2.0 | 79 | 0.32 | 17 | ≥20 |
| 58 | 1.5 | 290 | 1.1 | 45 | <2 |
| 59 | 5.7 | 549 | 1.1 | 200 | ≥20 |
| 60 | 5.5 | 603 | 0.24 | 63 | ≥5 |
| 61 | 3.0 | 140 | 1.0 | 78 | <2 |
| 62 | 1.7 | 109 | 0.72 | 63 | <2 |
| 63 | 2.7 | 96 | 2.6 | 30 | <2 |
| 64 | 3.0 | 158 | 5.9 | 96 | ≥5 |
| 65 | 5.1 | >1000 | 5.0 | 237 | <2 |
| 66 | 14 | 116 | 5.6 | 43 | ≥20 |
| 67 | 1.6 | 74 | 0.15 | 8.5 | <2 |
| 68 | 2.3 | 72 | 0.51 | 37 | <2 |
| 69 | 2.4 | 41 | 0.36 | 16 | >2 |
| 70 | 3.4 | 346 | 0.5 | 101 | <2 |
| 71 | 0.64 | 7.6 | 0.28 | 6.0 | <2 |
| 72 | 0.32 | 5.8 | 1.3 | 8.0 | ≥10 |
| 73 | 1.1 | 29 | 2.3 | 22 | ≥10 |
| 74 | 12 | 54 | 3.3 | 30 | ≥20 |
| 75 | 0.64 | 3.7 | 0.61 | 9.8 | >2 |
| 76 | 0.28 | 2.9 | 0.16 | 1.5 | <2 |
| 77 | 0.15 | 2.5 | 0.13 | 1.7 | <2 |
| 78 | 0.58 | 4.3 | 0.59 | 2.8 | ≥20 |
| 79 | 1.7 | 17 | 2.5 | 17 | ≥45 |
| 80 | 0.18 | 2.8 | 0.26 | 5.1 | ≥10 |
| 81 | 0.27 | 5.9 | 0.19 | 4.0 | <2 |
| 82 | 1.1 | 6.4 | 0.060 | 3.1 | <2 |
| 83 | 0.98 | 56 | 0.19 | 12 | <2 |
| 84 | 1.5 | 25 | 4.9 | 145 | ≥ 20 |
| 85 | 0.90 | 25 | 11 | 403 | ≥ 20 |
| 86 | 1.1 | 9.8 | 4.2 | 154 | ≥ 20 |
| 87 | 0.75 | 15 | 5.3 | 242 | ≥ 10 |
| 88 | 1.7 | 39 | 8.2 | 192 | ≥ 40 |
| 89 | 2.3 | 64 | 56 | 875 | ≥ 40 |
| 90 | 2.2 | 68 | 3.6 | 126 | ≥ 20 |
| 91 | 7.9 | 212 | 0.60 | >1000 | ≥ 40 |
| 92 | 4.7 | 151 | 22 | 166 | ≥ 40 |
| 93 | 3.0 | 77 | 5.9 | 128 | ≥ 40 |
| 94 | 3.1 | 61 | 1.2 | 75 | ≥ 40 |
| 95 | 3.3 | 40 | 17 | 90 | ≥45 |
| 96 | 2.7 | 41 | 94 | 166 | ≥45 |
| 97 | 2.7 | 38 | 49 | 164 | ≥45 |
| 98 | 3.1 | 30 | 6.3 | 28 | ≥45 |
| 99 | 4.4 | 140 | 13 | 127 | ≥45 |
| 100 | 1.6 | 594 | 0.32 | 81 | ≥45 |
| 101 | 3.5 | 98 | 3.1 | 89 | ≥45 |
| 102 | 2.2 | >1000 | 3.1 | 421 | ≥45 |
| 103 | 3.2 | 70 | 4.4 | 81 | ≥45 |
| 104 | 2.5 | 35 | 1.6 | 26 | ≥45 |
| 105 | 2.1 | 44 | 4.3 | 94 | ≥45 |
| 106 | 3.0 | 65 | 3.7 | 28 | ≥45 |

### Example C3

### Cell Transfections

For transient transfections, plasmid constructs were supplied by GenScript for rat, cynomolgus monkey, and canine CRF-R1 alpha, and CRF-R2 beta subtypes, as well as human CRF-R1 alpha and CRF-R2 alpha, beta and gamma subtypes all cloned into a pCDNA3.1 (*Neo*^{R}) vector. Briefly, Chinese hamster ovary strain K1 (CHO-K1) parental cells were expanded from a liquid nitrogen-stored working bank vial (1.0E+6 cells) by thawing at 37°C for 60 seconds, followed by expansion in 30 ml CHO-K1 Complete Media in a T-225 flask. Cells were passaged at least once by aspirating-out used culture media and washing adherent cells once in phosphate buffered saline without divalent cations (Intermountain BSS-PBS-1X6), followed by dislodging the adherent cells using Enzyme-free cell dissociation buffer (EFDB, Millipore Sigma C5789) and subsequent continued culture in complete media.

On Day 0, CHO-K1 parental cells were harvested when ~ 90% confluent, seeded into 6-well plates (Costar 3516) at approximately 400,000 cells/well, and cultured overnight in complete media. On Day +1 the cells were monitored for optimal confluence (60-75%), health, cell distribution and morphology, and the media was changed by aspiration with the addition of 2mL fresh complete media. Lipid:DNA complexes were prepared by either: (i) mixing 2.5 µg of plasmid DNA into 500 µl OptiMEM reduced serum media (Gibco Cat# 31985062) together with 9 µl of Lipofecamine LTX (Invitrogen^{™} Cat# 15338100) followed by incubation for 25 minutes at room temperature; or (ii) mixing 2.0 µg of plasmid DNA into 82 µl OptiMEM with 16 µl of FuGENE 6 (Promega Cat# E2691), at a 4:1 lipid:pDNA volume ratio, followed by incubation for 45 minutes at room temperature. Lipid:DNA complexes were subsequently added dropwise directly onto cells on the 6-well plate. On Day +3, transfected cells were harvested with 1 ml EFDB and resuspended into 10 ml complete media (total volume 11 ml) and transferred (1.45E+5 cells/well) into Corning 96-well plates for assay.

Some stable polyclonal pools were prepared after culturing transiently transfected parental cells for 2-3 weeks under chemical selection. Initially, cells were incubated for approximately 2-3 days under a high dose of Geneticin (G418 Sulfate, Gibco 10131035, 1.0E-3 mg/ml) that was subsequently lowered to 2.5-5.0E-4 mg/ml. The stable pools were assayed as described under the cAMP sections and aliquots were frozen and stored under liquid nitrogen for future use.

All data was analyzed utilizing GraphPad, Prism software by Dotmatics. All antagonist (IC₅₀) and agonist (EC₅₀) data were expressed as a percent of Emax (Relative Light Unit) Response.

Table 16, below, details the IC50 data for CRF1 and the isoforms of CRF2 (alpha, beta, and gamma). Each number in the table is a mean of 3 data points (n=3). For example, the peptide of SEQ ID NO: 101 has an IC₅₀ of 1.6nM for CRFR1, an IC₅₀ of 16nM for CRFR2α, and an IC₅₀ of 12.0nM for CRFR2β. In other words, the peptide of SEQ ID NO: 101 was approximately 10 times more selective for CRF1 over CRFR2α and approximately 8 times more selective for CRF1 over CRFR2β.

**Table 16**

| **Peptide** | **CRFR1 (IC₅₀ nM)** | **CRFR2α (IC₅₀ nM)** | **CRFR2β (IC₅₀ nM)** | **CRFR2γ (IC₅₀ nM)** |
|---|---|---|---|---|
| **Rat** | | | | |
| *Astressin C | 0.32 | n/a | 6.9 | n/a |
| SEQ ID NO: 101 | 1.4 | n/a | 40 | n/a |
| SEQ ID NO: 95 | 0.85 | n/a | 48 | n/a |

| **Dog** | | | | |
|---|---|---|---|---|
| *Astressin C | 0.82 | n/a | 2.1 | n/a |
| SEQ ID NO: 101 | 2.4 | n/a | 24 | n/a |
| SEQ ID NO: 95 | 1.3 | n/a | 32 | n/a |

| **Cyno** | | | | |
|---|---|---|---|---|
| *Astressin C | 0.21 | n/a | 1.3 | n/a |
| SEQ ID NO: 101 | 1.2 | n/a | 24 | n/a |
| SEQ ID NO: 95 | 1.0 | n/a | 28 | n/a |

| **Human** | | | | |
|---|---|---|---|---|
| *Astressin C | 0.63 | 1.1 | 0.90 | 0.32 |
| SEQ ID NO: 101 | 1.6 | 16 | 12.0 | 3.8 |
| SEQ ID NO: 95 | 0.74 | 406 | 36 | 1.6 |

| | | | | |
|---|---|---|---|---|
| *Astressin C" has the peptide sequence Ac-DLTfHLLREVLE(Nle)ARAEQ(CML)AQE#A(Aib)K#NRKL-(Nle)-E-(CML)-I-CONH₂; wherein E# and K# form a lactam bridge. | | | | |

### Experimental Method D: in vivo Testing of Peptides

The following procedures were used to evaluate the efficacy of the peptides disclosed herein *in vivo* and were approved by IACUC.

### Example D1

### In vivo testing of Peptides

Male, Sprague Dawley rats (226-250g/ 55-58 days old) underwent adrenalectomy surgery at Charles River Laboratories prior to shipment. Once delivered, postoperative care was provided by facility personnel. Rats (n=9-10 per group) were maintained single-housed with a 12:12 light/dark cycle. To compensate for the removal of adrenal mineralocorticoids and promote weight maintenance, they were supplemented with 0.9% NaCl and 1% sucrose in their drinking water. Additional pellets, medium high fat/sweet (Research Diets) were provided daily on the cage floor in addition to regular rat chow. Rats were allowed to recover and habituate for a week. Cage change occurred at least two days prior to the start of the experiment and again after one week if required for longer experiments. Water changes occurred every 3-4 days after experiments to avoid additional stress.

All experiments were conducted in the morning, to avoid circadian differences in adrenocorticotropic hormone (ACTH). Prior to collection of a whole blood sample via the tail vein, experimental animals were placed in a rat warmer 15-20 minutes prior to the designated time points. The animals were then placed in a rat restrainer and a 24-gauge catheter was placed in the tail vein. The blood (0.15-0.2mL/ sample) was collected into a 600µL Sarstedt tube that contained 1.2-2.0 mg EDTA-K₃/mL. After baseline blood collection, rats were subcutaneously injected with freshly made test article solution. Blood was collected once a day for up to 1 week for screening experiments or up to 3 weeks for the dose response-repeat dose experiments. No more than 10% of blood volume for survival bleeds were taken during any two-week period. Screening experiment timepoints were: baseline-dose, 24 hours, 48 hours and 72 hours; while dose response-repeat dose experiment timepoints were: baseline-dose 1 hour, 24 hours, 48 hours-dose 2, 72 hours, 96 hours-dose 3, 120 hours, 144 hours, and 168 hours.

The whole blood was centrifuged for 3 minutes at 10,000rpm. Approximately 75-150µL of plasma was extracted, placed into an appropriately labeled micro test tube and frozen on dry ice. The plasma was stored at -80°C until time of analysis, performed with a Milliplex MAP kit for Rat Stress Hormone - ACTH (Cat. #RSHMAG-69K). Plates were run with technical duplicates for samples from screening experiments or technical triplicates for samples from the dose response-repeat dose experiments.

Table 17 below shows the percentage adrenocorticotropin hormone (ACTH) reduction versus vehicle (normalized to t=0) at 24 hours and 48 hours after dosing at 700nmol/kg or 350nmol/kg with peptides of the disclosure using the single dose screening method above. The mean value is given for each peptide/dose together with the standard error of mean (SEM). N represents the number of animals per peptide. For example, peptide 101, after dosing at 350nmol/kg, has a 68.4%) (±3.8) reduction in ACTH at 24 hours and a 62.2% (±5.1) reduction of ACTH at 48 hours.

**Table 17**

| | | **24hr % ACTH reduction** | | **48hr % ACTH reduction** | | |
|---|---|---|---|---|---|---|
| **SEQ ID NO:** | **Dose (nmol/kg)** | **Mean** | **SEM** | **Mean** | **SEM** | **N** |
| **4** | 700 | 0.4 | 5.6 | -15.4 | 11.3 | 11 |
| **6** | 700 | -34.9 | 6.0 | -23.0 | 5.5 | 10 |
| **9** | 700 | -70.9 | 7.7 | -22.8 | 12.0 | 8 |
| **10** | 700 | -52.7 | 5.8 | -19.6 | 6.7 | 9 |
| **13** | 700 | -35.4 | 15.1 | 13.0 | 15.5 | 8 |
| **20** | 700 | -19.3 | 20.1 | 12.5 | 15.2 | 9 |
| **23** | 700 | -26.9 | 3.8 | 3.9 | 8.0 | 10 |
| **26** | 700 | -35.9 | 9.9 | -0.1 | 11.6 | 10 |
| **30** | 700 | -22.6 | 14.0 | 21.1 | 16.7 | 10 |
| **50** | 700 | -69.7 | 3.6 | -51.7 | 8.2 | 10 |
| **52** | 700 | -57.6 | 8.5 | -42.2 | 15.0 | 10 |
| **53** | 700 | -53.9 | 8.1 | -38.8 | 11.3 | 10 |
| **74** | 700 | -30.0 | 8.7 | -5.3 | 9.2 | 10 |
| **78** | 700 | -72.1 | 2.3 | -48.9 | 5.0 | 10 |
| **79** | 700 | -63.6 | 7.8 | -39.2 | 7.0 | 10 |
| | | | | | | |
| **50** | 350 | -50.8 | 3.6 | -48.2 | 9.5 | 6 |
| **95** | 350 | -56.6 | 7.1 | -38.3 | 7.9 | 10 |
| **96** | 350 | -20.9 | 5.4 | -31.4 | 6.4 | 10 |
| **97** | 350 | -39.0 | 7.1 | -52.9 | 4.4 | 9 |
| **99** | 350 | -42.0 | 7.1 | -35.6 | 7.9 | 8 |
| **101** | 350 | -68.4 | 3.8 | -62.2 | 5.1 | 10 |

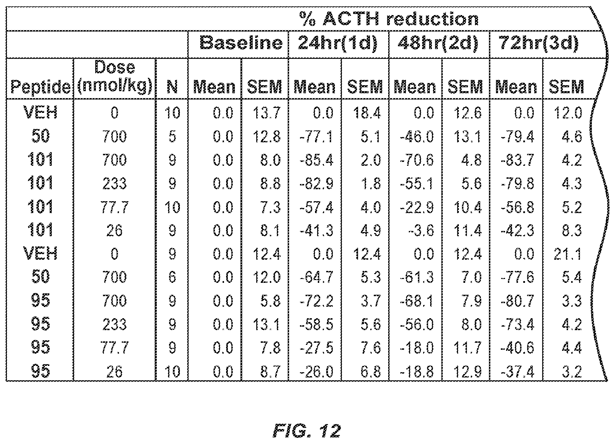

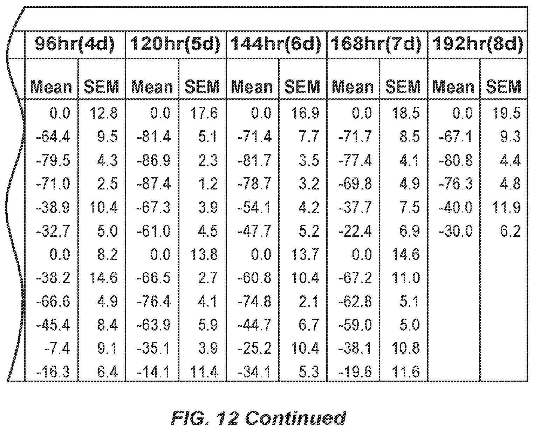

The table in FIG. 12 shows the percentage adrenocorticotropin hormone (ACTH) reduction versus vehicle (normalized to t=0) at 24, 48, 72, 96, 120, 144, 168, and 192 hours after repeat dosing of the peptides of SEQ ID Nos. 50, 95 and 101. Days dosed were baseline, 48, and 96 hours. The mean value is given for each peptide/dose together with the standard error of mean (SEM). N represents the number of animals per peptide. For example, the peptide of SEQ ID NO: 101, after dosing at 700nmol/kg, had an 85.4% (±2.0) reduction in ACTH at 24 hours and an 80.8% (±4.4) reduction of ACTH at 192 hours.

### Experimental Method E: Peptide Pharmacokinetics

### Example E1

### Peptide Pharmacokinetics

Pharmacokinetics of the test peptides were evaluated *in vivo* as described herein. Male Sprague Dawley rats (300-450 g), male Beagle dogs (5-10 kg) and male cynomolgus monkeys (2-4 kg) were used in the pharmacokinetics (PK) study. All animal procedures were approved by the Institutional Animal Care and Use Committee at the Test Facility.

### Rat Study

For intravenous (IV) dosing, compounds were administered by intravenous bolus injection via the jugular vein cannula (JVC), at 0.1 mg/kg or 0.3 mg/kg in 0.01 M phosphate (pH 7.4, 1 mg/mL). For subcutaneous (SC) dosing, compounds were administered in the space over the dorsal region, at 3 mg/kg in 0.1M phosphate (pH 7.4, 0.6 mg/mL).

Serial Blood samples were collected via the JVC at 0.083- (5 minutes), 0.25 (15 minutes), 1, 4, 8, 24, and 48-hours post-dose for IV and 2, 6, 24, 48, 120, 144, and 168-hours post-dose for SC administration.

### Dog Study

For IV dosing, compounds were administered by intravenous bolus injection into one of the two peripheral tail veins, at either 0.1 mg/kg or 0.3 mg/kg in 0.01 M phosphate (pH 7.4, 1 mg/mL). Following dosing, the catheter was flushed with 3 mL of saline prior to removal. For SC dosing, compounds were administered at the intra-scapular skin, at 0.3 mg/kg or 0.9 mg/kg in 0.01M phosphate (pH 7.4, 5 mg/mL). The SC injection site was prepared by clipping away the hair and cleaning with alcohol. After administration the dose site was marked with indelible ink.

Serial blood samples were collected via cephalic venipuncture at 0.083 (5 minutes), 0.25 (15 minutes), 1, 2, 4, 8, 24, 48, 72, 96, 120, 144, and 168-hours post-dose for IV and 0.25 (15 minutes), 0.5 (30 minutes), 1, 2, 4, 8, 24, 48, 72, 96, 120, 144, and 168-hours post-dose for SC administration.

### Monkey Study

For IV dosing, compounds were administered by intravenous bolus injection into one of the two peripheral tail veins, at 0.1 mg/kg or 0.3 mg/kg in 0.01M phosphate (pH 7.4, 1 mg/mL). Following dosing, the catheter was flushed with 3 mL of saline prior to removal. For SC dosing, compounds were administered at the intra-scapular skin, at 0.3 mg/kg or 0.9 mg/kg in 0.01M phosphate (pH 7.4, 5 mg/mL). The SC injection site was prepared by clipping away the hair and cleaning with alcohol. After administration, the site was marked with a circle approximately 5cm in diameter.

Serial Blood samples were collected via femoral venipuncture at 0.083 (5 minutes), 0.25 (15 minutes), 1, 2, 4, 8, 24, 48, 72, 96, 120, 144, and 168-hours post-dose for IV and 0.25 (15 minutes), 0.5 (30 minutes), 1, 2, 4, 8, 24, 48, 72, 96, 120, 144, and 168-hours post-dose for SC administration.

### Sample Preparation and Analysis

Blood samples were collected into tubes with K₂EDTA. Tubes were stored on wet ice until processed to plasma by centrifugation (2,200 x g at nominal 4°C for 10 minutes) within 60 minutes of collection. Plasma samples were transferred into individual uniquely labeled matrix tubes and stored at nominal -80°C until analysis by liquid chromatography tandem-mass spectrometry (LC-MS/MS).

The plasma samples were prepared by solid phase extraction (SPE) using a Waters PRiME HLB Microelution plate. The SPE plate was conditioned using water:methanol (50:50, v/v) and 0.5% tween 80 in water. After pretreated samples were loaded, wells were washed with 5% methanol in water and 0.1% TFA in water:acetonitrile (80:20, v/v). Wells were eluted with 0.1% formic acid, 0.5% 2,2,2-trifluoroethanol in water:acetonitrile (20:80, v/v) followed by a dilution with 0.1% formic acid, 0.1% tween 80 in water for analysis by LC-MS/MS. The analyte and internal standard (IS) were separated by reversed-phase chromatography using a Shimadzu Nexera X2 UHPLC system and Phenomenex bioZen Peptide PS-C18 50x2.1-mm, 3µm column. The analyte and IS were detected using an Applied Biosystems Sciex API 6500 triple quadrupole mass spectrometer. The mass spectrometer was equipped with an electrospray ionization source operated in the positive-ion mode. The analyte and internal standard were monitored in the multiple-reaction-monitoring scan mode.

Noncompartmental pharmacokinetic parameters were calculated using Phoenix^{®} WinNonlin^{®} software (Version 8.3; Linear Up Log Down Interpolation Calculation Method; Certara L.P.).

Table 18, below, provides data for peptides of the disclosure. Peptides are dosed either intravenously (IV) or subcutaneously (SC) in rat, dog or cynomolgus monkey species. For IV dosing, clearance (IV CL) and volume of distribution (IV Vd) are given in mL/min/kg and L/kg, respectively. IV half-life (IV t_{1/2}) is given in hours. For SC dosing, concentration of the peptide in blood plasma (AUC) is given in ng*hr/mL, peak concentration (Cmax) is given in ng/mL and the time to reach peak plasma concentration (Tₘₐₓ) is given in hours. The time required for the concentration of the peptide in plasma to reduce to half of its initial value (t_{1/2}) is given in hours. The amount of an administered dose of a peptide that reaches the systemic circulation (F) is expressed as a percentage. For example, the peptide of SEQ ID NO: 95 was administered intravenously and subcutaneously in rat, dog and cynomolgus monkey species. In cynomolgus monkey, the peptide of SEQ ID NO: 95 was dosed intravenously at 0.3 mg/kg, had a clearance of 0.025 mL/min/kg, a volume of distribution of 0.066 L/kg with a half-life of 34.9 hours. In cynomolgus monkey, the peptide of SEQ ID NO: 95 was dosed subcutaneously at 0.9 mg/kg, the concentration of peptide in blood plasma was 626000 ng*hr/mL, with a peak concentration of 16700 mg/L at 5.3 hours and took 33.5 hours to reduce the concentration to half its initial value. 100% of the administered SC dose of a peptide of SEQ ID NO: 95 reached systemic circulation.

**Table 18**

| **SEQ ID NO:** | **Species** | **IV dose** mg/kg | **IV CL** mL/min /kg | **IV Vd** L/kg | **IV t1/2** hr | **SC Dose** mg/kg | **SC Tmax** hr | **SC AUC** ng*hr/mL | **SC Cmax** ng/mL | **SC t1/2** hr | **SC F** % |
|---|---|---|---|---|---|---|---|---|---|---|---|
| 4 | Rat | 0.3 | 0.074 | 0.078 | 12.3 | 3 | 24 | 66800 | 1350 | 27.6 | 7.6 |
| 6 | Rat | 0.3 | 0.139 | 0.114 | 10.5 | 3 | 6.0 | 280000 | 9790 | 10.8 | 83 |
| 9 | Rat | 0.3 | 0.083 | 0.085 | 11.7 | 3 | 4.7 | 366000 | 11100 | 14.7 | 54 |
| 20 | Rat | 0.3 | 0.094 | 0.127 | 17.1 | 3 | 3.3 | 425000 | 14900 | 15.9 | 91 |
| 50 | Rat | | | | | 3 | 4.7 | 703000 | 19000 | 20.3 | |
| 72 | Rat | | | | | 3 | 3.3 | 349000 | 14100 | 11.8 | |
| 78 | Rat | | | | | 3 | 6.0 | 279000 | 13400 | 12.7 | |
| 79 | Rat | | | | | 3 | 3.3 | 380000 | 15400 | 14.6 | |
| 95 | Rat | 0.3 | 0.096 | 0.129 | 16.8 | 3 | 4.7 | 499000 | 18600 | 22.8 | 100 |
| 101 | Rat | 0.1 | 0.045 | 0.078 | 21.5 | 3 | 24 | 708000 | 14700 | 20.0 | 79 |
| 95 | Dog | 0.3 | 0.0094 | 0.106 | 132 | 0.9 | 8 | 794000 | 8340 | 115 | 84 |
| 101 | Dog | 0.1 | 0.006 | 0.041 | 72.2 | 0.3 | 27 | 483000 | 3620 | 179 | 80 |
| 95 | Cyno | 0.3 | 0.025 | 0.066 | 34.9 | 0.9 | 5.3 | 626000 | 16700 | 33.5 | 100 |
| 101 | Cyno | 0.1 | 0.013 | 0.056 | 48.2 | 0.3 | 56 | 275000 | 2490 | 102 | 79 |

### Equivalents and Scope

Those skilled in the art will recognize or be able to ascertain using no more than routine experimentation, many equivalents to the specific embodiments in accordance with the Detailed Description provided herein. The scope of the present disclosure is not intended to be limited to the above Detailed Description, but rather is as set forth in the appended claims.

Where ranges are given, endpoints are included. Furthermore, it is to be understood that unless otherwise indicated or otherwise evident from the context and understanding of one of ordinary skill in the art, values that are expressed as ranges can assume any specific value or subrange within the stated ranges in different embodiments of the disclosure, to the tenth of the unit of the lower limit of the range, unless the context clearly dictates otherwise.

While the present disclosure has been described at some length and with some particularity with respect to the several described embodiments, it is not intended that it should be limited to any such particulars or embodiments or any particular embodiment, but it is to be construed with references to the appended claims so as to provide the broadest possible interpretation of such claims in view of the prior art and, therefore, to effectively encompass the intended scope of the disclosure.

In addition, section headings, the materials, methods, and examples are illustrative only and not intended to be limiting.
Various features of the present disclosure are emphasized in the embodiments which follow.
1. A peptide or a pharmaceutically acceptable salt thereof, comprising the following amino acid sequence (SEQ ID NO: 117): 
Y-Asp-R2-R3-R4-R5-R6-R7-Arg-R9-R10-R11-R12-R13-R14-R15-R16-R17-R18-
R19-R20-R21-R22-R23-R24-R25-R26-R27-R28-R29-R30-R31-R32-R33-CONH2
 wherein:
   **R2** is Leu or N-Me-Leu;
   **R3** is Thr, Ser, or D-Phe;
   **R4** is D-Phe, Phe, Leu, Trp, or His;
   **R5** is His, Glu, or CML (C^{α}-Me-Leu);
   **R6** is Leu, or CML (C^{α}-Me-Leu);
   **R7 is** Leu, CML (C^{α}-Me-Leu), or N-Me-Leu;
   **R9** is Glu, Thr, Nle, CML (C^{α}-Me-Leu), Ala, or **K*;**
   **R10** is Val, Met, Leu, Nle, Asp, or Asn;
   **R11** is Leu or Ile;
   **R12** is Glu, Aib, Ala, or **K*;**
   **R13** is Nle, Ile, Leu, Met, Asn, Asp, or **K*;**
   **R14** is **K*,** Glu, Ala, Thr, Aib, n-Me-Ala, or Gln;
   **R15** is Asn, Thr, Arg, **K*,** Gln, Glu, Ala, Aad, Gla, pcF, K&, K%, K^, E&, E%, or Lys;
   **R16** is Arg, Glu, Gln, Thr, Ala, Gln, Aib, or **K*;**
   **R17** is Glu, Gln, Nle, Aib, Ala, D-Ala, Asp, or **K*;**
   **R18** is Gln, Ser, Ala, or **K*;**
   **R19** is cVal, CML (C^{α}-Me-Leu) Glu, Gln, Leu, Aib, N-Me-Leu, D-Ala, CMV
      (C^{α}-Me-Val), CMP (C^{α}-Me-Phe) or **K*;**
   **R20** is Ala, Arg, Glu, Aib, or **K*;**
   **R21** is Gln, Glu, Aib, Ala, or **K*;**
   **R22** is E# or K#;
   **R23** is Ala, Leu, Glu, Gln, Aib, D-Ala, Aph(Hor), Nle, or Lys;
   **R24** is Glu, Deg, Ala, D-Ala, His, Gln, Lys, Aib, N-Me-Ala, CML (C^{α}-Me-Leu),
      N-Me-D-Ala, D-Aph(Cbm), D-CML (C^{α}-Me-D-Leu), D-Aph(Cbm), or **K*;**
   **R25** is K# or E#;
   **R26** is Asn, Glu, Lys, K(Ac), Ala, or Aib;
   **R27** is Arg, Glu, or **K*;**
   **R28** is Lys, Leu, Ile, Glu, K(Ac), Ala, Arg, or K(iPr);
   **R29** is Leu, Ile, Ala, Glu, K(Ac), CML (C^{α}-Me-Leu), N-Me-Leu or **K*;**
   **R30** is Nle, Leu, Phe, Met, K(Ac), Asp, Asn, Glu, 1Nal, 2Nal, or Cha;
   **R31** is Glu, Lys, CML (C^{α}-Me-Leu), Aib, Ala, or Asp;
   **R32** is cVal, Thr, Ser, Glu, Lys, Me-Lys, Aib, D-Ala, CML (C^{α}-Me-Leu), CMV (C^{α}- Me-
      Val), CMP (C^{α}-Me-Phe), βAla(2-thienyl), βAla(3-thienyl), or Ile;
   **R33** is Ala, Val, Lys, N-Me-Ala, or Ile; and
   Y is Ac or X*;
   wherein X* represents ((2-[2-(2-amino-ethoxy)-ethoxy]-acetyl))ₙ-(γGlu)ₘ-diacid, wherein: n is 1, 2 or 3; m is 0, 1, 2, or 3; and wherein the diacid is a C16, C18, C20, or C22 diacid, and wherein the linkage to the 2-[2-(2-amino-ethoxy)-ethoxy]-acetyl ((AEEA)) amine is via the nitrogen of the N-terminus;
   **K*** represents Lys-(Z)₀₋₁((2-[2-(2-amino-ethoxy)-ethoxy]-acetyl))ₙ-(γGlu)ₘ-diacid or
      Lys-(PEG12)-(γGlu)₁₋₂-diacid, wherein: Z is Glu, γGlu, Lys, Ala, Gln or His; n is 1, 2 or 3; m is 0, 1, 2, or 3; and wherein the diacid is a C16, C18, C20, or C22 diacid, and wherein the linkage to the 2-[2-(2-amino-ethoxy)-ethoxy]-acetyl ((AEEA)) amine is via the gamma carboxylate of the yGlu residue;
   with the proviso that only one amino acid in the Formula I sequence is **K*;** and with
      the proviso that if K* is present, Y is Ac, and if Y is X*, K* is not present; and
   with the further proviso that when **R22** is E#, **R25** is K#, and when **R22** is K#, **R25** is
      E#; wherein E# and K# represent a lactam bridge between the side chains of these two amino acids (E=Glu; K=Lys).
2. A peptide of Formula (I), or a pharmaceutically acceptable salt thereof, comprising the following amino acid sequence (SEQ ID. NO 107): 
Y-Asp-R2-R3-R4-R5-R6-R7-Arg-R9-R10-R11-R12-R13-R14-R15-R16-R17-R18-
R19-R20-R21-R22-R23-R24-R25-R26-R27-R28-R29-R30-R31-R32-R33-CONH2
(I)
 wherein:
   **R2** is Leu or N-Me-Leu;
   **R3** is Thr, Ser, or D-Phe;
   **R4** is D-Phe, Phe, Leu, Trp, or His;
   **R5** is His, Glu, or CML (C^{α}-Me-Leu);
   **R6** is Leu, or CML (C^{α}-Me-Leu);
   **R7 is** Leu, CML (C^{α}-Me-Leu), or N-Me-Leu;
   **R9** is Glu, Thr, Nle, CML (C^{α}-Me-Leu), Ala, or **K*;**
   **R10** is Val, Met, Leu, Nle, Asp, or Asn;
   **R11** is Leu or Ile;
   **R12** is Glu, Aib, Ala, or **K*;**
   **R13** is Nle, Ile, Leu, Met, Asn, Asp, or **K*;**
   **R14** is **K*,** Glu, Ala, Thr, Aib, N-Me-Ala, or Gln;
   **R15** is Arg, **K*,** Gln, Glu, Ala, Aad, Gla, pcF, K&, K%, K^, E&, E%, or Lys;
   **R16** is Glu, Gln, Thr, Ala, Gln, Aib, or **K*;**
   **R17** is Glu, Gln, Nle, Aib, Ala, D-Ala, Asp, or **K*;**
   **R18** is Gln, Ser, Ala, or **K*;**
   **R19** is cVal, CML (C^{α}-Me-Leu), Glu, Gln, Leu, Aib, N-Me-Leu, D-Ala, Me-Phe, CMV
      (C^{α}-Me-Val), CMP (C^{α}-Me-Phe), or **K*;**
   **R20** is Ala, Arg, Glu, Aib, or **K*;**
   **R21** is Gln, Glu, Aib, Ala, or **K*;**
   **R22** is E# or K#;
   **R23** is Ala, Leu, Glu, Gln, Aib, D-Ala, Aph(Hor), Nle, or Lys;
   **R24** is Glu, Deg, Ala, D-Ala, His, Gln, Lys, Aib, N-Me-Ala, CML (C^{α}-Me-Leu), N- Me-
      D-Ala, D-Aph(Cbm), D-CML (C^{α}-Me-D-Leu), D-Aph(Cbm), or **K*;**
   **R25** is K# or E#;
   **R26** is Asn, Glu, Lys, K(Ac), Ala, or Aib;
   **R27** is Arg, Glu, or **K*;**
   **R28** is Lys, Leu, Ile, Glu, K(Ac), Ala, Arg, or K(iPr);
   **R29** is Leu, Ile, Ala, Glu, K(Ac), CML (C^{α}-Me-Leu), N-Me-Leu or **K*;**
   **R30** is Nle, Leu, Phe, Met, K(Ac), Asp, Asn, Glu, 1Nal, 2Nal, or Cha;
   **R31** is Glu, Lys, CML (C^{α}-Me-Leu), Aib, Ala, or Asp;
   **R32** is cVal, Thr, Ser, Glu, Lys, Me-Lys, Aib, D-Ala, CML (C^{α}-Me-Leu), CMV (C^{α}- Me-
      Val), CMP (C^{α}-Me-Phe), βAla(2-thienyl), βAla(3-thienyl), or Ile;
   **R33** is Ala, Val, Lys, Na-Me-Ala, or Ile; and
   Y is Ac,
   **K*** is Lys-(2-[2-(2-amino-ethoxy)-ethoxy]-acetyl)ₙ-(γGlu)ₘ-diacid, wherein the diacid is a C16, C18, or C20 diacid, and wherein n = 1, 2 or 3 and m is 1, 2, or 3; and wherein the linkage to the 2-[2-(2-amino-ethoxy)-ethoxy]-acetyl amine is via the gamma carboxylate of the yGlu residue; with the proviso that only one amino acid in the sequence is **K*;** and with the further proviso that when **R22** is E#, then **R25** is K#, and when **R22** is K#, then **R25** is E#, wherein E# and K# represent a lactam bridge between the side chains of these two amino acids.
3. The peptide of embodiment 1 or 2, wherein the peptide is a peptide of Formula (Ia), or a pharmaceutically acceptable salt thereof, comprising the following amino acid sequence (SEQ ID NO: 108): 
Y-Asp-Leu-Thr-D-Phe-His-Leu-Leu-Arg-R9-Val-Leu-R12-R13-R14-R15-R16-R17-R18-
R19-R20-R21-R22-R23-R24-R25-Asn-R27-R28-R29-R30-R31-R32-R33-CONH2
(Ia)
 wherein:
   **R9** is Glu or **K*;**
   **R12** is Glu or **K*;**
   **R13** is Nle or **K*;**
   **R14** is Glu, Ala, **K*,** Thr, or Gln;
   **R15** is Asn, Thr, Arg, **K*,** Gln, Glu, Ala, Aad, Gla, pcF, K&, K%, K^, E&, E%, or Lys;
   **R16** is Arg, Glu, Gln, Thr, Ala, or **K*;**
   **R17** is Glu, Asp, or **K*;**
   **R18** is Gln or **K*;**
   **R19** is cVal, CML (C^{α}-Me-Leu), or **K*;**
   **R20** is Ala or **K*;**
   **R21** is Gln or **K*;**
   **R22** is E# or K#;
   **R23** is Ala or Lys;
   **R24** is Deg, Glu or **K*;**
   **R25** is E# or K#;
   **R27** is Arg or **K*;**
   **R28** is Lys or K(iPr);
   **R29** is Leu or **K*;**
   **R30** is Cha or Nle;
   **R31** is Glu or Asp;
   **R32** is cVal, CML (C^{α}-Me-Leu), or Ile;
   **R33** is Ile or Ala; and
   Y is Ac,
   **K*** represents Lys-(2-[2-(2-amino-ethoxy)-ethoxy]-acetyl)ₙ-(γGlu)ₘ-diacid, wherein the diacid is a C16, C18, or C20 diacid, and wherein n = 1, 2 or 3 and m is 1, 2, or 3; with the proviso that only one amino acid in the sequence is **K*;** and with the further proviso that when **R22** is E#, **R25** is K#, and when **R22** is K#, **R25** is E#.
4. The peptide of embodiment 3, wherein the peptide is a peptide of Formula (Ib), or a pharmaceutically acceptable salt thereof, comprising the following amino acid sequence (SEQ ID NO: 109): 
Ac-Asp-Leu-Thr-D-Phe-His-Leu-Leu-Arg-Glu-Val-Leu-Glu-Nle-R14-R15-R16-
R17-Gln-cVal-Ala-Gln-R22-Ala-Glu-R25-Asn-Arg-Lys-Leu-Nle-Glu-cVal-R33-
CONH2
(Ib)
 wherein:
   **R14** is **K*,** Glu, Ala, Thr, or Gln;
   **R15** is Asn, Thr, Arg, **K*,** Ala, Lys, Glu, or Gln;
   **R16** is Arg, Ala, Glu, Gln, or Thr;
   **R17** is Glu or Asp;
   **R22** is E# or K#;
   **R25** is K# or E#;
   **R33** is Ala or Ile; and
   **K*** represents Lys-(2-[2-(2-amino-ethoxy)-ethoxy]-acetyl)ₙ-(γGlu)ₘ-diacid, wherein the diacid is a C16, C18, or C20 diacid, n is 1, 2 or 3, and m is 1 or 2; with the proviso that one amino acid in the sequence is **K*;** and with the further proviso that when **R22** is E#, then **R25** is K#, and when **R22** is K#, then **R25** is E#.
5. The peptide of embodiment 4, wherein the peptide is a peptide of Formula (Ic), or a pharmaceutically acceptable salt thereof, comprising the following amino acid sequence (SEQ ID NO: 110): 
Ac-Asp-Leu-Thr-D-Phe-His-Leu-Leu-Arg-Glu-Val-Leu-Glu-Nle-K*-R15-R16-R17-
Gln-cVal-Ala-Gln-R22-Ala-Glu-R25-Asn-Arg-Lys-Leu-Nle-Glu-cVal-R33-CONH2
(Ic)
 wherein:
   **R15** is Asn, Thr, Ala, Glu, Lys, Gln or Arg;
   **R16** is Arg, Glu, Gln, Ala or Thr;
   **R17** is Asp or Glu;
   **R22** is E# or K#;
   **R25** is E# or K#;
   **R33** is Ile or Ala; and
   **K*** represents Lys-(2-[2-(2-amino-ethoxy)-ethoxy]-acetyl)ₙ-(γGlu)ₘ-diacid, wherein the diacid is a C16, C18, or C20 diacid, n is 1, 2 or 3, and m is 1 or 2; with the further proviso that when **R22** is E#, **R25** is K#, and when **R22** is K#, **R25** is E#.
6. The peptide of embodiment 5, wherein the diacid is a C18 or C20 diacid.
7. The peptide of embodiment 5, wherein **R15** is Gln, and the diacid in **K*** is a C18 diacid or a C20 diacid.
8. The peptide of embodiment 5, wherein **R15** is Arg, and the diacid in **K*** is a C18 diacid or a C20 diacid.
9. The peptide of embodiment 5, wherein **R33** is Ile, and the diacid in **K*** is a C18 diacid or
   a C20 diacid.
10. The peptide of embodiment 5, wherein **R33** is Ala, and the diacid in **K*** is a C18 diacid or a C20 diacid.
11. The peptide of embodiment 5, wherein **R15** is Gln, **R33** is Ile, and the diacid in **K*** is a C18 diacid or a C20 diacid.
12. The peptide of embodiment 5, wherein **R15** is Gln, **R33** is Ala, and the diacid in **K*** is a C18 diacid or a C20 diacid.
13. The peptide of embodiment 5, wherein **R15** is Arg, **R33** is Ile, and the diacid in **K*** is a C18 diacid or a C20 diacid.
14. The peptide of embodiment 5, wherein **R15** is Arg, **R16** is Glu, **R17** is Glu, **R22 is** E#, **R25** is K#, **R33** is Ala, n = 2, m = 1, and the diacid in **K*** is a C20 diacid.
15. The peptide of embodiment 5, wherein **R15** is Gln, **R16** is Glu, **R17** is Glu, **R22 is** E#, **R25** is K#, **R33** is Ala or Ile, n = 2, m = 1, and the diacid in **K*** is a C18 diacid.
16. The peptide of embodiment 5, wherein **R15** is Gln, **R16** is Glu, **R17** is Glu, **R22 is** E#, **R25** is K#, **R33** is Ala, n = 3, m = 1, and the diacid in **K*** is a C18 diacid.
17. The peptide of embodiment 5, wherein the peptide is a peptide of Formula (Id), or a pharmaceutically acceptable salt thereof, comprising the following amino acid sequence (SEQ ID NO: 111): 
Ac-Asp-Leu-Thr-D-Phe-His-Leu-Leu-Arg-Glu-Val-Leu-Glu-Nle-K*-R15-R16-Glu-
Gln-cVal-Ala-Gln-R22-Ala-Glu-R25-Asn-Arg-Lys-Leu-Nle-Glu-cVal-R33-CONH2
(Id)
 wherein:
   **R15** is Asn, Thr, Gln or Arg;
   **R16** is Arg, Ala or Glu;
   **R22** is E# or K#;
   **R25** is E# or K#;
   **R33** is Ile or Ala; and
   **K*** represents Lys-(2-[2-(2-amino-ethoxy)-ethoxy]-acetyl)ₙ-(γGlu)ₘ-diacid, wherein the diacid is a C18 or C20 diacid, and wherein n = 1, 2 or 3 and m is 1 or 2; with the proviso that when **R22** is E#, then **R25** is K#, and when **R22** is K#, then **R25** is E#.
18. The peptide of embodiment 5, wherein the peptide is a peptide of Formula (Ie), or a pharmaceutically acceptable salt thereof, comprising the following amino acid sequence (SEQ ID NO: 112): 
Ac-Asp-Leu-Thr-D-Phe-His-Leu-Leu-Arg-Glu-Val-Leu-Glu-Nle-K*-R15-R16-Glu-
Gln-cVal-Ala-Gln-R22-Ala-Glu-R25-Asn-Arg-Lys-Leu-Nle-Glu-cVal-R33-CONH2
(Ie)
 wherein:
   **R15** is Asn, Thr, Gln, Lys, or Arg;
   **R16** is Arg, Ala or Glu;
   **R22** is E# or K#;
   **R25** is E# or K#;
   **R33** is Ile or Ala; and
   **K*** represents Lys-(2-[2-(2-amino-ethoxy)-ethoxy]-acetyl)ₙ-(γGlu)ₘ-diacid, wherein the diacid is a C16, C18, or C20 diacid, n is 1, 2 or 3, and m is 1, 2 or 3, with the proviso that when **R22** is E#, **R25** is K#, and when **R22** is K#, **R25** is E#.
19. The peptide of embodiment 18 wherein **R15** is Arg, **R16** is Glu, **R22** is E#, **R25** is K#, **R33** is Ile, n=3, m=1, 2 or 3, and the diacid is a C18 diacid.
20. The peptide of embodiment 18 wherein **R15** is Arg, **R16** is Ala or Glu, **R22** is E#, **R25** is K#, **R33** is Ala, n=2, m=1, and the diacid is a C18 diacid.
21. The peptide of embodiment 18 wherein **R15** is Lys, **R16** is Ala, **R22** is E#, **R25** is K#, **R33** is Ala, n=2, m=1, and the diacid is a C18 diacid.
22. The peptide of embodiment 18 wherein **R15** is Arg, **R16** is Glu, **R22** is E#, **R25 is** K#, **R33** is Ala, n=3, m=1, and the diacid is a C20 diacid.
23. The peptide of embodiment 1 or 2, wherein the peptide is a peptide of Formula (If), or a pharmaceutically acceptable salt thereof, comprising the following amino acid sequence (SEQ ID NO: 113): 
Ac-Asp-Leu-Thr-D-Phe-His-Leu-Leu-Arg-Glu-Val-Leu-Glu-Nle-R14-K*-R16-Glu-
Gln-cVal-Ala-Gln-R22-Ala-Glu-R25-Asn-Arg-Lys-Leu-Nle-Glu-cVal-R33-CONH2
(If)
 wherein:
   **R14** is Glu, Thr, Ala, Gln;
   **R16** is Ala or Glu;
   **R22** is E# or K#;
   **R25** is E# or K#;
   **R33** is Ile or Ala; and
   **K*** represents Lys-X-(2-[2-(2-amino-ethoxy)-ethoxy]-acetyl)ₙ-(γGlu)ₘ-diacid, wherein X is absent or Gln, the diacid is a C16, C18, or C20 diacid, n is 1, 2 or 3, and m is 1, 2 or 3; with the proviso that when **R22** is E#, **R25** is K#, and when **R22** is K#, **R25** is E#.
24. The peptide of embodiment 23, wherein **R14** is Glu, **R16** is Glu, **R22** is E#, **R25** is K#, **R33** is Ile, X is Gln, n=2, m=1, and the diacid is a C18 diacid.
25. The peptide of embodiment 23, wherein **R14** is Thr, Gln or Ala, **R16** is Glu, **R22** is E#, **R25** is K#, **R33** is Ile, X is absent, n=2, m=1, and the diacid is a C18 diacid.
26. The peptide of embodiment 23, wherein **R14** is Glu, **R16** is Glu, **R22** is E#, **R25** is K#, **R33** is Ile, X is absent, n=2, m=2, and the diacid is a C18 diacid.
27. The peptide of embodiment 23, wherein **R14** is Gln, **R16** is Glu or Ala, **R22** is E#, **R25** is K#, **R33** is Ala, X is absent, n=2, m=1, and the diacid is a C18 diacid.
28. The peptide of embodiment 23, wherein **R14** is Thr, **R16** is Glu or Ala, **R22** is E#, **R25** is K#, **R33** is Ala, X is absent, n=2, m=1, and the diacid is a C18 diacid.
29. The peptide of embodiment 23, wherein **R14** is Thr or Gln, **R16** is Ala, **R22** is E#, **R25** is K#, **R33** is Ala, X is absent, n=3, m=1, and the diacid is a C18 diacid.
30. The peptide of embodiment 1 or 2, wherein the peptide is a peptide of Formula (Ig), or a pharmaceutically acceptable salt thereof, comprising the following amino acid sequence (SEQ ID NO: 114): 
Ac-Asp-Leu-Thr-D-Phe-His-Leu-Leu-Arg-Glu-Val-Leu-Glu-Nle-K*-Gln-R16-Glu-
Gln-cVal-Ala-Gln-R22-Ala-Glu-R25-Asn-Arg-Lys-Leu-Nle-Glu-cVal-R33-CONH2
(Ig)
 wherein:
   **R16** is Ala or Gln;
   **R22** is E# or K#;
   **R25** is E# or K#;
   **R33** is Ile or Ala; and
   **K*** represents Lys-(2-[2-(2-amino-ethoxy)-ethoxy]-acetyl)ₙ-(γGlu)ₘ-diacid, wherein the diacid is a C16, C18, or C20 diacid, n is 1, 2 or 3, and m is 1, 2 or 3, with the proviso that when **R22** is E#, **R25** is K#, and when **R22** is K#, **R25** is E#.
31. The peptide of embodiment 30, wherein **R16** is Glu, **R22** is E#, **R25** is K#, **R33** is Ala, n=2, m=1 or 2, and the diacid is a C18 diacid.
32. The peptide of embodiment 30, wherein **R16** is Glu, **R22** is E#, **R25** is K#, **R33** is Ala, n=3, m=1, and the diacid is a C18 diacid.
33. The peptide of embodiment 30, wherein **R16** is Ala, **R22** is E#, **R25** is K#, **R33** is Ala, n=2, m=1, and the diacid is a C18 diacid.
34. The peptide of embodiment 30, wherein **R16** is Glu, **R22** is E#, **R25** is K#, **R33** is Ile, n=2 or 3, m=1, and the diacid is a C18 diacid.
35. The peptide of embodiment 30, wherein **R16** is Glu, **R22** is E#, **R25** is K#, **R33** is Ala, n=2, m=1, and the diacid is a C20 diacid.
36. The peptide of embodiment 1 or 2, wherein the peptide is a peptide of Formula (Ih), or a pharmaceutically acceptable salt thereof, comprising the following amino acid sequence (SEQ ID NO: 115): 
Ac-Asp-Leu-Thr-D-Phe-His-Leu-Leu-Arg-K*-Val-Leu-Glu-Nle-R14-R15-Glu-Glu-
Gln-cVal-Ala-Gln-R22-Ala-Glu-R25-Asn-Arg-Lys-Leu-Nle-Glu-cVal-Ile-CONH2
(Ih)
 wherein:
   **R14** is Glu or Gln;
   **R15** is Arg, Gln, Glu, or Gla;
   **R22** is E# or K#;
   **R25** is E# or K#; and
   **K*** represents Lys-(2-[2-(2-amino-ethoxy)-ethoxy]-acetyl)ₙ-(γGlu)ₘ-diacid or PEG12-gGlu-diacid, wherein the diacid is a C16, C18, or C20 diacid, n is 1, 2 or 3, and m is 1, 2 or 3; with the proviso that when **R22** is E#, **R25** is K#, and when **R22** is K#, **R25** is E#.
37. The peptide of embodiment 36, wherein R14 is Gln, R15 is Gln or Gla, **R22** is E#, **R25** is K#, **K*** is Lys-(2-[2-(2-amino-ethoxy)-ethoxy]-acetyl)ₙ-(γGlu)ₘ-diacid, n=2, m=1, and the diacid is a C18 diacid.
38. The peptide of embodiment 36, wherein R14 is Glu, R15 is Gln, **R22** is E#, **R25** is K#, **K*** is Lys-(2-[2-(2-amino-ethoxy)-ethoxy]-acetyl)ₙ-(γGlu)ₘ-diacid, n=2, m=1, and the diacid is a C16 diacid or a C18 diacid.
39. The peptide of embodiment 36, wherein R14 is Glu, R15 is Arg, **R22** is E#, **R25** is K#, **K*** is PEG12-γGlu-diacid, and the diacid is a C18 diacid.
40. The peptide of embodiment 1 or 2, wherein the peptide is a peptide of Formula (Ik), or a pharmaceutically acceptable salt thereof, comprising the following amino acid sequence (SEQ ID NO: 116): 
X-Asp-Leu-Thr-D-Phe-His-Leu-Leu-Arg-R9-Val-Leu-R12-R13-R14-R15-R16-R17-
R18-cVal-R20-R21-R22-Ala-Glu-R25-Asn-R27-Lys-R29-Nle-Glu-cVal-R33-
CONH2
(Ik)
 wherein:
   X is Ac or (2-[2-(2-amino-ethoxy)-ethoxy]-acetyl)₂-γGlu-diacid, wherein the diacid is a C16, C18, or C20 diacid;
   **R9** is Glu or **K*;**
   **R12** is Glu or **K*;**
   **R13** is Nle or **K*;**
   **R14** is Thr, Glu, or **K*;**
   **R15** is Asn, Thr, Arg, Ala, Gln, or **K*;**
   **R16** is Arg, Ala or Glu;
   **R17** is Glu or **K*;**
   **R18** is Gln or **K*;**
   **R20** is Ala or **K*;**
   **R21** is Gln or **K*;**
   **R22** is E# or K#;
   **R25** is E# or K#;
   **R27** is Arg or **K***
   **R29** is Leu or **K*;**
   **R33** is Ile or Ala; and
   **K*** represents Lys-Z-(2-[2-(2-amino-ethoxy)-ethoxy]-acetyl)ₙ-(γGlu)ₘ-diacid,
   wherein Z is absent, Lys, Glu, γGlu, Ala, or Gln, the diacid is a C16, C18, or C20 diacid, n is 1, 2 or 3, and m is 1, 2 or 3; with the proviso that only one amino acid in the sequence is **K*;** and with the further proviso that when **R22** is E#, **R25** is K#, and when **R22** is K#, **R25** is E#.
41. The peptide of embodiment 40, wherein X is Ac, **R9** is **K*, R12** is Glu, **R13** is Nle, **R14** is Glu, **R15** is Arg, **R16** is Glu, **R17** is Glu, **R18** is Gln, **R20** is Ala, **R21** is Gln, **R22** is E#, **R25** is K#, **R27** is Arg, **R29** is Leu, **R33** is Ile, Z is absent, n=2, m=1, and the diacid is a C18 diacid.
42. The peptide of embodiment 40, wherein X is Ac, **R9** is Glu, **R12** is Glu, **R13** is **K*, R14** is Glu, **R15** is Arg, **R16** is Glu, **R17** is Glu, **R18** is Gln, **R20** is Ala, **R21** is Gln, **R22** is E#, **R25** is K#, R27 is Arg, **R29** is Leu, **R33** is Ile, Z is absent, n=2, m=1, and the diacid is a C18 diacid.
43. The peptide of embodiment 40, wherein X is Ac, **R9** is Glu, **R12** is Glu, **R13** is Nle, **R14** is **K*, R15** is Arg, Gln, Glu, or **K*,** R16 is Glu, **R17** is Glu, **R18** is Gln, **R20** is Ala, **R21** is Gln, **R22** is E#, **R25** is K#, R27 is Arg, R29 is Leu, **R33** is Ile, Z is absent, n=2, m=1, and the diacid is a C18 diacid.
44. The peptide of embodiment 40, wherein X is Ac, **R9** is Glu, **R12** is Glu, **R13** is Nle, **R14** is Glu, **R15** is **K*** or Arg, **R16** is Glu, **R17** is Glu, **R18** is Gln, **R20** is Ala, **R21** is Gln, **R22** is E#, **R25** is K#, **R27** is Arg, **R29** is Leu, **R33** is Ile, Z is absent, n=2, m=1, and the diacid is a C18 diacid.
45. The peptide of embodiment 40, wherein X is Ac, **R9** is Glu, **R12** is Glu, **R13** is Nle, **R14** is Glu, **R15** is Arg, **R16** is Glu, **R17** is Glu, **R18** is Gln, **R20** is Ala, **R21** is Gln, **R22** is E#, **R25** is K#, **R27** is Arg or **K*, R29** is Leu, **R33** is Ile, Z is absent, n=2, m=1, and the diacid is a C18 diacid.
46. The peptide of embodiment 40, wherein X is Ac, **R9** is Glu, **R12** is Glu, **R13** is Nle, **R14** is Glu, **R15** is Arg, **R16** is Glu, **R17** is Glu, **R18** is Gln, **R20** is Ala, **R21** is Gln, **R22** is E#, **R25** is K#, **R27** is Arg, **R29** is **K*, R33** is Ile, Z is absent, n=2, m=1, and the diacid is a C18 diacid.
47. The peptide of embodiment 40, wherein X is Ac, **R14** is Glu, **R9** is **K*, R12** is Glu, **R13** is Nle, **R15** is Arg, **R16** is Glu, **R17** is Glu, **R18** is Gln, **R20** is Ala, **R21** is Gln, **R22** is E#, **R25** is K#, **R27** is Arg, **R29** is Leu, **R33** is Ile, Z is absent, n=2, m=1, and the diacid is a C16 diacid.
48. The peptide of embodiment 40, wherein X is Ac, **R9** is Glu, **R12** is **K*, R13** is Nle, **R14** is Glu, **R15** is Arg, **R16** is Glu, **R17** is Glu, **R18** is Gln, **R20** is Ala, **R21** is Gln, **R22** is E#, **R25** is K#, **R27** is Arg, R29 is Leu, **R33** is Ile, Z is absent, n=2, m=1, and the diacid is a C16 diacid.
49. The peptide of embodiment 40, wherein X is Ac, **R9** is Glu, **R12** is Glu, **R13** is Nle, **R14** is Glu, **R15** is Arg, R16 is Glu, **R17** is **K*, R18** is Gln, **R20** is Ala, **R21** is Gln, **R22** is E#, **R25** is K#, R27 is Arg, R29 is Leu, **R33** is Ile, Z is absent, n=2, m=1, and the diacid is a C16 diacid.
50. The peptide of embodiment 40, wherein X is Ac, **R9** is Glu, **R12** is Glu, **R13** is Nle, **R14** is Glu, **R15** is Arg, R16 is Glu, **R17** is Glu, **R18** is **K*, R20** is Ala, **R21** is Gln, **R22** is E#, **R25** is K#, R27 is Arg, R29 is Leu, **R33** is Ile, Z is absent, n=2, m=1, and the diacid is a C16 diacid.
51. The peptide of embodiment 40, wherein X is Ac, **R9** is Glu, **R12** is Glu, **R13** is Nle, **R14** is Glu, **R15** is Arg, **R16** is Glu, **R17** is Glu, **R18** is Gln, **R20** is **K*, R21** is Gln, **R22** is E#, **R25** is K#, **R27** is Arg, **R29** is Leu, **R33** is Ile, Z is absent, n=2, m=1, and the diacid is a C16 diacid.
52. The peptide of embodiment 40, wherein X is Ac, **R9** is Glu, **R12** is Glu, **R13** is Nle, **R14** is Glu, **R15** is Arg, **R16** is Glu, **R17** is Glu, **R18** is Gln, **R20** is Ala, **R21** is **K*, R22** is E#, **R25** is K#, **R27** is Arg, **R29** is Leu, **R33** is Ile, Z is absent, n=2, m=1, and the diacid is a C16 diacid.
53. The peptide of embodiment 40, wherein X is Ac, **R9** is Glu, **R12** is Glu, **R13** is Nle, **R14** is Thr, **R15** is **K*, R16** is Glu or Ala, **R17** is Glu, **R18** is Gln, **R20** is Ala, **R21** is Gln, **R22** is E#, **R25** is K#, **R27** is Arg, **R29** is Leu, **R33** is Ile, Z is absent, n=2, m=1, and the diacid is a C16 diacid.
54. The peptide of embodiment 40, wherein X is Ac, **R9** is Glu, **R12** is Glu, **R13** is Nle, **R14** is Glu, **R15** is **K*, R16** is Glu, **R17** is Glu, **R18** is Gln, **R20** is Ala, **R21** is Gln, **R22** is E#, **R25** is K#, **R27** is Arg, **R29** is Leu, **R33** is Ile, Z is absent, n=2, m=2 or 3, and the diacid is a C16 diacid.
55. The peptide of embodiment 40, wherein X is Ac, **R9** is Glu, **R12** is Glu, **R13** is Nle, **R14** is Glu, **R15** is **K*, R16** is Glu, **R17** is Glu, **R18** is Gln, **R20** is Ala, **R21** is Gln, **R22** is E#, **R25** is K#, **R27** is Arg, **R29** is Leu, **R33** is Ile, Z is Glu, yGlu or Ala, n=2, m=1, and the diacid is a C16 diacid.
56. The peptide of embodiment 40, wherein X is Ac, **R9** is Glu, **R12** is Glu, **R13** is Nle, **R14** is Glu, **R15** is **K*,** R16 is Glu, **R17** is Glu, **R18** is Gln, **R20** is Ala, **R21** is Gln, **R22** is E#, **R25** is K#, **R27** is Arg, **R29** is Leu, **R33** is Ile, Z is Gln, n=2, m=1, and the diacid is a C16 diacid.
57. The peptide of any one of embodiments 1-56, wherein the solubility of the peptide is
   (i) at least about 100 mg/mL in about 10 mM phosphate at pH of about 7; or
   (ii) at least about 100 mg/mL in about 10 mM TRIS at pH of about 8, and
   wherein the solubility is measured by visual observation.
58. The peptide of any one of embodiments 1-57, wherein the peptide shows no aggregation as measured using size exclusion chromatography (SEC).
59. The peptide of any one of embodiments 1-58, wherein the peptide shows CRFR1 antagonist activity as measured using a cyclic AMP (cAMP) assay.
60. The peptide of any one of embodiments 1-60, wherein the peptide shows CRFR2 antagonist activity as measured using a cAMP assay.
61. The peptide of any one of embodiments 1-62, wherein the peptide is more selective for CRF1 over CRFR2α and/or CRFR2β as measured using a cAMP assay.
62. The peptide of any one of embodiments 1-56, wherein the peptide has:
   (i) CRFR1 antagonist IC₅₀ activity as measured using a cyclic AMP (cAMP) assay of less than about 5 nM;
   (ii) more than about 35% reduction in ACTH about 48 hours after peptide dosing in adrenalectomized rats;
   (iii) pharmacokinetic half-life of more than about 100 hours in cynomolgus monkeys;
   (iv) solubility more than or equal to about 100 mg/mL in about 10 mM phosphate buffer at pH of about 7; and
   (v) low or no aggregation as measured using size exclusion chromatography (SEC).
63. The peptide of any one of embodiments 1-56, wherein the peptide has:
   (i) CRFR1 antagonist IC₅₀ activity as measured using a cyclic AMP (cAMP) assay of less than about 5 nM;
   (ii) more than about 100% reduction in ACTH about 48 hours after peptide dosing in adrenalectomized rats;
   (iii) pharmacokinetic half-life of more than about 100 hours in cynomolgus monkeys;
   (iv) solubility more than or equal to about 100 mg/mL in about 10 mM phosphate buffer at pH of about 7; and
   (v) low or no aggregation as measured using size exclusion chromatography (SEC).
64. A peptide comprising or consisting of the following amino acid sequence (SEQ ID NO: 101): Ac-Asp-Leu-Thr-D-Phe-His-Leu-Leu-Arg-Glu-Val-Leu-Glu-Nle-**K***-Arg-Glu-Glu-Gln-cVal-Ala-Gln-E#-Ala-Glu-K#-Asn-Arg-Lys-Leu-Nle-Glu-cVal-Ala-CONH₂; wherein: **K*** represents Lys-(2-[2-(2-amino-ethoxy)-ethoxy]-acetyl)₂-γGlu-diacid; wherein the diacid is a C20 diacid; and wherein E# and K# represent a lactam bridge between the side chains of these two amino acids (E=Glu; K=Lys).
65. A peptide comprising or consisting of the following amino acid sequence (SEQ ID NO: 95):Ac-Asp-Leu-Thr-D-Phe-His-Leu-Leu-Arg-Glu-Val-Leu-Glu-Nle-**K***-Gln-Glu-Glu-Gln-cVal-Ala-Gln-E#-Ala-Glu-K#-Asn-Arg-Lys-Leu-Nle-Glu-cVal-Ala-CONH₂; wherein **K*** represents Lys-(2-[2-(2-amino-ethoxy)-ethoxy]-acetyl)₂-γGlu-diacid, wherein the diacid is a C18 diacid; and wherein E# and K# represent a lactam bridge between the side chains of these two amino acids (E=Glu; K=Lys).
66. A peptide comprising or consisting of the following amino acid sequence (SEQ ID NO: 50): Ac-Asp-Leu-Thr-D-Phe-His-Leu-Leu-Arg-Glu-Val-Leu-Glu-Nle-**K***-Gln-Glu-Glu-Gln-cVal-Ala-Gln-E#-Ala-Glu-K#-Asn-Arg-Lys-Leu-Nle-Glu-cVal-Ile-CONH₂; wherein **K*** represents Lys-(2-[2-(2-amino-ethoxy)-ethoxy]-acetyl)₂-γGlu-diacid, wherein the diacid is a C18 diacid; and wherein E# and K# represent a lactam bridge between the side chains of these two amino acids (E=Glu; K=Lys).
67. A peptide comprising or consisting of the following amino acid sequence (SEQ ID NO: 97): Ac-Asp-Leu-Thr-D-Phe-His-Leu-Leu-Arg-Glu-Val-Leu-Glu-Nle-**K***-Arg-Glu-Glu-Gln-cVal-Ala-Gln-E#-Ala-Glu-K#-Asn-Arg-Lys-Leu-Nle-Glu-cVal-Ala-CONH₂; wherein **K*** represents Lys-(2-[2-(2-amino-ethoxy)-ethoxy]-acetyl)₃-γGlu-diacid, wherein the diacid is a C18 diacid; and wherein E# and K# represent a lactam bridge between the side chains of these two amino acids (E=Glu; K=Lys).
68. A pharmaceutically acceptable salt of the peptide of any one of embodiments 64-67.
69. A method of treating congenital adrenal hyperplasia (CAH), in a subject in need thereof, comprising administering to the subject a therapeutically effective amount of the peptide of any one of embodiments 1-68.
70. A method for reducing the severity of one or more symptoms of congenital adrenal hyperplasia (CAH) selected from hirsutism, precocious puberty, fertility problems, acne, and growth impairment in a subject having classic congenital adrenal hyperplasia, comprising administering a therapeutically effective amount of the peptide of any one of embodiments 1-68.
71. The method of embodiment 70, wherein the growth impairment is selected from one or more of accelerated height velocity, accelerated weight velocity, and accelerated bone age.
72. A method of reducing the dosage of corticosteroid administered to a subject having congenital adrenal hyperplasia (CAH) for controlling CAH, comprising administering to the subject a therapeutically effective amount of the peptide of any one of embodiments 1-67.
73. A method of reducing the severity of one or more side effects of glucocorticoid treatment in a subject having CAH comprising administering to the subject a therapeutically effective amount of the peptide of any one of embodiments 1-67.
74. The method of embodiment 73, wherein the side effect is selected from osteoporosis, avascular necrosis of bone, myopathy, hyperglycemia, diabetes mellitus, dyslipidemia, weight gain, Cushing syndrome, Cushingoid features, growth suppression, adrenal suppression, gastritis, peptic ulcer, gastrointestinal bleeding, visceral perforation, hepatic steatosis, pancreatitis, hypertension, coronary heart disease, ischemic heart disease, heart failure, dermatoprosis, skin atrophy, ecchymosis, purpura, erosions, striae, delayed wound healing, easy bruising, acne, hirsutism, hair loss, mood changes, depression, euphoria, mood lability, irritability, akathisia, anxiety, cognitive impairment, psychosis, dementia, delirium, cataract, glaucoma, ptosis, mydriasis, opportunistic ocular infections, central serous chorioretinopathy, suppression of cell-mediated immunity, predisposition to infections, and reactivation of latent infections.
75. A method of treating or preventing testicular adrenal rest tumors (TART) in a subject in need thereof, comprising administering to the subject a therapeutically effective amount of the peptide of any one of embodiments 1-67, or a pharmaceutically acceptable salt thereof.
76. A method of treating or preventing ovarian adrenal rest tumors (OART) in a subject in need thereof, comprising administering to the subject a therapeutically effective amount of the peptide of any one of embodiments 1-67, or a pharmaceutically acceptable salt thereof.
77. The method of embodiment 75 or 76 wherein the subject has congenital adrenal hyperplasia (CAH), and the size and/or numbers of the tumors are decreased or reduced.
78. A method of treating congenital adrenal hyperplasia (CAH), in a subject in need thereof, comprising administering to the subject a therapeutically effective amount of the peptide of any one of embodiments 1-67, or pharmaceutically acceptable salt thereof, wherein the level of androstenedione (A4) in the subject is reduced from baseline by at least 5%, 10%, 15%, 20%, 25% and is maintained at the reduced level post 24 hours.
79. A method of treating congenital adrenal hyperplasia (CAH), in a subject in need thereof, comprising administering to the subject a therapeutically effective amount of the peptide of any one of embodiments 1-67, or pharmaceutically acceptable salt thereof, wherein the level of adrenocorticotropic hormone (ACTH) in the subject is reduced by at least 10% from baseline, and wherein the level of 17-hydroxyprogesterone (17-OHP) is reduced in the subject by at least about 10% from baseline.
80. The method of any one of embodiments 69-79, wherein the peptide comprises or consists of SEQ ID NO: 50, SEQ ID NO: 95, SEQ ID NO: 97, or SEQ ID NO: 101.
81. The method of any one of embodiments 69-79, comprising administering to the subject a pharmaceutically acceptable salt of the peptide of any one of embodiments 1-67.
82. Use of the peptide of any one of embodiments 1-67 or pharmaceutically acceptable salt thereof in the manufacture of a medicament for treating congenital adrenal hyperplasia (CAH) in a subject in need thereof.
83. Use of the peptide of any one of embodiments 1-67 or pharmaceutically acceptable salt thereof in the manufacture of a medicament for reducing the severity of one or more symptoms of congenital adrenal hyperplasia (CAH) selected from hirsutism, precocious puberty, fertility problems, acne, and growth impairment in a subject having classic congenital adrenal hyperplasia.
84. The use of embodiment 83, wherein the growth impairment is selected from one or more of accelerated height velocity, accelerated weight velocity, and accelerated bone age.
85. Use of the peptide of any one of embodiments 1-67 or pharmaceutically acceptable salt thereof in the manufacture of a medicament for reducing the dosage of corticosteroid administered to a subject having congenital adrenal hyperplasia (CAH) for controlling CAH.
86. Use of the peptide of any one of embodiments 1-67 or pharmaceutically acceptable salt thereof in the manufacture of a medicament for reducing the severity of one or more side effects of glucocorticoid treatment in a subject having CAH.
87. The use of embodiment 86, wherein the side effect is selected from osteoporosis, avascular necrosis of bone, myopathy, hyperglycemia, diabetes mellitus, dyslipidemia, weight gain, Cushing syndrome, Cushingoid features, growth suppression, adrenal suppression, gastritis, peptic ulcer, gastrointestinal bleeding, visceral perforation, hepatic steatosis, pancreatitis, hypertension, coronary heart disease, ischemic heart disease, heart failure, dermatoprosis, skin atrophy, ecchymosis, purpura, erosions, striae, delayed wound healing, easy bruising, acne, hirsutism, hair loss, mood changes, depression, euphoria, mood lability, irritability, akathisia, anxiety, cognitive impairment, psychosis, dementia, delirium, cataract, glaucoma, ptosis, mydriasis, opportunistic ocular infections, central serous chorioretinopathy, suppression of cell-mediated immunity, predisposition to infections, and reactivation of latent infections.
88. Use of the peptide of any one of embodiments 1-67 or pharmaceutically acceptable salt thereof in the manufacture of a medicament for treating testicular adrenal rest tumors (TART) in a subject in need thereof.
89. Use of the peptide of any one of embodiments 1-67 or pharmaceutically acceptable salt thereof in the manufacture of a medicament for treating ovarian adrenal rest tumors (OART) in a subject in need thereof.
90. Use of the peptide of any one of embodiments 1-67 or pharmaceutically acceptable salt thereof in the manufacture of a medicament for treating congenital adrenal hyperplasia (CAH), wherein the size and/or numbers of the tumors are decreased or reduced.
91. Use of the peptide of any one of embodiments 1-67 or pharmaceutically acceptable salt thereof in the manufacture of a medicament for treating congenital adrenal hyperplasia (CAH) in a subject in need thereof, wherein the level of androstenedione (A4) in the subject is reduced from baseline by at least 5%, 10%, 15%, 20%, 25% and is maintained at the reduced level post 24 hours.
92. Use of the peptide of any one of embodiments 1-67 or pharmaceutically acceptable salt thereof in the manufacture of a medicament for treating congenital adrenal hyperplasia (CAH) in a subject in need thereof, wherein the level of adrenocorticotropic hormone (ACTH) in the subject is reduced by at least 10% from baseline, and wherein the level of 17-hydroxyprogesterone (17-OHP) is reduced in the subject by at least about 10% from baseline.
93. The use of any one of embodiments 82-92, wherein the peptide comprises or consists of SEQ ID NO: 50, SEQ ID NO: 95, SEQ ID NO: 97, or SEQ ID NO: 101.
94. The use of any one of embodiments 82-92, wherein the peptide is a pharmaceutically acceptable salt of the peptide of any one of embodiments 1-67.
95. A peptide of any one of embodiments 1-67 or pharmaceutically acceptable salt thereof for use in treating congenital adrenal hyperplasia (CAH) in a subject in need thereof.
96. A peptide of any one of embodiments 1-67 or pharmaceutically acceptable salt thereof for use in reducing the severity of one or more symptoms of congenital adrenal hyperplasia (CAH) selected from hirsutism, precocious puberty, fertility problems, acne, and growth impairment in a subject having classic congenital adrenal hyperplasia.
97. The peptide of embodiment 96, wherein the growth impairment is selected from one or more of accelerated height velocity, accelerated weight velocity, and accelerated bone age.
98. A peptide of any one of embodiments 1-67 or pharmaceutically acceptable salt thereof for use in reducing the dosage of corticosteroid administered to a subject having congenital adrenal hyperplasia (CAH) for controlling CAH.
99. A peptide of any one of embodiments 1-67 or pharmaceutically acceptable salt thereof for use in reducing the severity of one or more side effects of glucocorticoid treatment in a subject having CAH.
100. The peptide of embodiment 99, wherein the side effect is selected from osteoporosis, avascular necrosis of bone, myopathy, hyperglycemia, diabetes mellitus, dyslipidemia, weight gain, Cushing syndrome, Cushingoid features, growth suppression, adrenal suppression, gastritis, peptic ulcer, gastrointestinal bleeding, visceral perforation, hepatic steatosis, pancreatitis, hypertension, coronary heart disease, ischemic heart disease, heart failure, dermatoprosis, skin atrophy, ecchymosis, purpura, erosions, striae, delayed wound healing, easy bruising, acne, hirsutism, hair loss, mood changes, depression, euphoria, mood lability, irritability, akathisia, anxiety, cognitive impairment, psychosis, dementia, delirium, cataract, glaucoma, ptosis, mydriasis, opportunistic ocular infections, central serous chorioretinopathy, suppression of cell-mediated immunity, predisposition to infections, and reactivation of latent infections.
101. A peptide of any one of embodiments 1-67 or pharmaceutically acceptable salt thereof for use in treating testicular adrenal rest tumors (TART) in a subject in need thereof.
102. A peptide of any one of embodiments 1-67 or pharmaceutically acceptable salt thereof for use in treating ovarian adrenal rest tumors (OART) in a subject in need thereof.
103. A peptide of any one of embodiments 1-67 or pharmaceutically acceptable salt thereof for use in treating congenital adrenal hyperplasia (CAH), wherein the size and/or numbers of the tumors are decreased or reduced.
104. A peptide of any one of embodiments 1-67 or pharmaceutically acceptable salt thereof for use in treating congenital adrenal hyperplasia (CAH) in a subject in need thereof, wherein the level of androstenedione (A4) in the subject is reduced from baseline by at least 5%, 10%, 15%, 20%, 25% and is maintained at the reduced level post 24 hours.
105. A peptide of any one of embodiments 1-67 or pharmaceutically acceptable salt thereof for use in treating congenital adrenal hyperplasia (CAH) in a subject in need thereof, wherein the level of adrenocorticotropic hormone (ACTH) in the subject is reduced by at least 10% from baseline, and wherein the level of 17-hydroxyprogesterone (17-OHP) is reduced in the subject by at least about 10% from baseline.
106. The peptide of any one of embodiments 95-105, wherein the peptide comprises or consists of SEQ ID NO: 50, SEQ ID NO: 95, SEQ ID NO: 97, or SEQ ID NO: 101.
107. The peptide of any one of embodiments 95-105, wherein the peptide is a pharmaceutically acceptable salt of the peptide of any one of embodiments 1-67.
108. A formulation comprising one or more peptides of any one of embodiments 1-67 in admixture with one or more suitable excipients.
109. A formulation comprising a pharmaceutically acceptable salt of the peptide of any one of embodiments 1-67, in admixture with one or more suitable excipients.
110. A subcutaneous injectable device comprising one or more peptides of any one of embodiments 1-67, or the formulation of embodiment 108.
111. A subcutaneous injectable device comprising a pharmaceutically acceptable salt of one or more peptides of any one of embodiments 1-67, or the formulation of embodiment 108.
113. The method of any one of embodiments 69-81 comprising administering to the subject with the subcutaneous injectable device of embodiment 110 or 111.
113. The method of embodiment 112, wherein the subject is a human.
114. The method of embodiment 113, wherein the human is administered a dose of 75 mg.
115. The method of embodiment 112 or 114, wherein the administration is at intervals of 7 days, 14 days or monthly.

## Claims

1. A corticotropin releasing factor (CRF) receptor antagonist fatty acid conjugated cyclic peptide comprising or consisting of the following amino acid sequence (SEQ ID NO: 110): 
Ac-Asp-Leu-Thr-D-Phe-His-Leu-Leu-Arg-Glu-Val-Leu-Glu-Nle-K*-R15-R16-R17-Gln-cVal-Ala-
Gln-R22-Ala-Glu-R25-Asn-Arg-Lys-Leu-Nle-Glu-cVal-R33-CONH2
(Ic)
 wherein:
**R15** is Asn, Thr, Ala, Glu, Lys, Gln or Arg;
**R16** is Arg, Glu, Gln, Ala or Thr;
**R17** is Asp or Glu;
**R22** is E# or K#;
**R25** is E# or K#;
**R33** is Ile or Ala; and
**K*** represents Lys-(2-[2-(2-amino-ethoxy)-ethoxy]-acetyl)ₙ-(γGlu)ₘ-diacid, wherein the diacid is a C16, C18, or C20 diacid, n is 1, 2 or 3, and m is 1 or 2; with the further proviso that when **R22** is E#, **R25** is K#, and when **R22** is K#, **R25** is E#, and
wherein E# and K# represent a lactam bridge between the side chains of these two amino acids (E=Glu; K=Lys).

2. The peptide of claim 1, wherein the peptide comprises or consists of the following amino acid sequence (SEQ ID NO: 111): 
Ac-Asp-Leu-Thr-D-Phe-His-Leu-Leu-Arg-Glu-Val-Leu-Glu-Nle-K*-R15-R16-Glu-Gln-cVal-Ala-
Gln-R22-Ala-Glu-R25-Asn-Arg-Lys-Leu-Nle-Glu-cVal-R33-CONH2
(Id)
 wherein:
**R15** is Asn, Thr, Gln or Arg;
**R16** is Arg, Ala or Glu;
**R22** is E# or K#;
**R25** is E# or K#;
**R33** is Ile or Ala; and
K* represents Lys-(2-[2-(2-amino-ethoxy)-ethoxy]-acetyl)n-(γGlu)m-diacid, wherein the diacid is a C18 or C20 diacid, and wherein n = 1, 2 or 3 and m is 1 or 2; with the proviso that when R22 is E#, then R25 is K#, and when R22 is K#, then R25 is E#.

3. The peptide of claim 1 or 2, wherein:
a) **R17** is Glu;
b) **R22** is E# and **R25** is K#;
c) n is 2 or 3;
d) m is 1; and/or
e) the diacid is a C18 or C20 diacid.

4. The peptide of any one of claims 1-3, wherein:
a) **R15** is Gln, and the diacid in **K*** is a C18 diacid or a C20 diacid;
b) **R15** is Arg, and the diacid in **K*** is a C18 diacid or a C20 diacid;
c) **R33** is Ile, and the diacid in **K*** is a C18 diacid or a C20 diacid;
d) **R33** is Ala, and the diacid in **K*** is a C18 diacid or a C20 diacid;
e) **R15** is Gln, **R33** is Ile, and the diacid in **K*** is a C18 diacid or a C20 diacid;
t) **R15 is** Gln, **R33** is Ala, and the diacid in **K*** is a C18 diacid or a C20 diacid;
g) **R15** is Arg, **R33** is Ile, and the diacid in **K*** is a C18 diacid or a C20 diacid;
h) **R15** is Gln, **R16** is Glu, **R17** is Glu, **R22** is E#, **R25** is K#, **R33** is Ala or Ile, n = 2, m = 1, and the diacid in **K*** is a C18 diacid, or
i) **R15 is** Gln, **R16** is Glu, **R17** is Glu, **R22** is E#, **R25** is K#, **R33** is Ala, n = 3, m = 1, and the diacid in **K*** is a C18 diacid.

5. The peptide of any one of claims 1-4, wherein the peptide comprises or consists of:
a) any one of SEQ ID NOs 9, 50, 72, or 79;
b) any one of SEQ ID NOs 9, 50, 52, 79, 97, 98, or 102, or
c) any one of SEQ ID NOs 79, 96, 97, 98, 99, 100, 102, 103, 104, 105, or 106.

6. A pharmaceutically acceptable salt of the peptide of any one of claims 1-5.

7. The peptide of any one of claims 1-5 or the pharmaceutically acceptable salt of claim 6, for use in therapy.

8. The peptide of any one of claims 1-5 or the pharmaceutically acceptable salt of claim 6, for use in treating congenital adrenal hyperplasia (CAH) in a subject in need thereof.

9. The peptide or pharmaceutically acceptable salt for use according to claim 8, wherein the CAH is classic CAH.

10. The peptide of any one of claims 1-5 or the pharmaceutically acceptable salt of claim 6, for use in treating a subject having congenital adrenal hyperplasia (CAH), wherein the treatment allows the dosage of corticosteroid administered to the subject for controlling CAH to be reduced.

11. The peptide or pharmaceutically acceptable salt for use according to any one of claims 7-10, wherein the peptide or pharmaceutically acceptable salt thereof is administered to the subject by subcutaneous injection.

12. The peptide or pharmaceutically acceptable salt for use according to any one of claims 7-11, wherein the peptide or pharmaceutically acceptable salt thereof is administered to the subject once weekly, twice monthly, or monthly.

13. The peptide or pharmaceutically acceptable salt for use according to any one of claims 7-12, wherein the peptide or pharmaceutically acceptable salt thereof is administered to the subject weekly in a dose of about 50 mg to about 250 mg, about 75 mg to about 230 mg and is delivered in less than 1 mL volume from a subcutaneous injection.

14. A pre-filled subcutaneous injection device comprising:
a) the peptide of any one of claims 1-5;
b) the pharmaceutically accept salt of claim 6, or
b) a pharmaceutical composition comprising the peptide of any one of claims 1-5 or the pharmaceutically acceptable salt of claim 6, in admixture with one or more suitable excipients.
